# EUROPEAN PATENT APPLICATION

(11) **EP 2 078 727 A1**
(43) Date of publication of application: **15.07.2009**
(21) Application number: 09154797.6
(22) Date of filing: 13.03.2003
(51) Int. Cl.: C07K 14/38, A61K 38/00

(54) **Mutagenesis of aspergillus fungi and identification of genes essential for growth**

(30) Priority: 13.03.2002 US 363543 P; 19.12.2002 US 434407 P
(62) Divisional of application: 03710137.5
(71) Applicant: Bayer CropScience SA, 69009 Lyon (FR); Institut Pasteur, 75015 Paris (FR)
(72) Inventor: Grosjean-Cournoyer, Marie-Claire, 69250, CURIS AU MONT D'OR (FR); D'enfert, Christophe Didier, 75011, PARIS (FR); Firon, Arnaud, 75015, PARIS (FR); Villalba, François, 69130, ECULLY (FR); Lebrun, Marc-Henri, 69002, LYON (FR); Beffa, Roland, 69230, SAINT GENIS LAVAL (FR)
(74) Representative: Warcoin, Jacques

(57) **Abstract**

The present invention is directed to polynucleotides encoding proteins Essential For the Growth (EFG) of filamentous fungi. The invention also deals with namely polypeptides encoded by said polynucleotides, screening assays for identifying compounds capable of inhibiting said EFG proteins activities, pharmaceutical or phytosanitary compositions comprising such compounds.

SEQUENCE LISTING

<110> Institut Pasteur
<110> Bayer CropScience SA

<120> MUTAGENESIS OF ASPERGILLUS FUNGI AND IDENTIFICATION OF GENES ESSENTIAL FOR
GROWTH

<130> D20128

<150> US 60/363 543
<151> 2002-03-13

<150> US 60/434 407
<151> 2002-12-19

<160> 199

<170> PatentIn Ver. 3.2

<210> 1
<211> 2802
<212> DNA
<213> Aspergillus fumigatus

<400> 1
atgcctcacc gcgcggcatc cccagcggtg tcggaaaatg agttcgacat cacaggcgct 60
ctcttccaga acgacagcga ctccgacaac gaacagccct cggccaagtc caaacgacaa 120
cctccgaaga aggttccctc gcaggcgctc gacttcctcg gcgatgtgaa tgaagacgac 180
aatgacgacg aggcttttat tgccgagcag caaacttctg ccaaccgcaa ggcctcgaat 240
ctcaaaggtc gcactgttaa gaagggtggt ggtttccaag ccatgggctt gagtgccaat 300
ctgttaaagg caatcgctcg gaaaggcttc tcggtaccga ctcccattca gcgcaagacc 360
attcccgtta ttatggacga ccaggatgta gttggtatgg cacggactgg ttcaggaaag 420
acggccgctt tcgttatccc aatgatcgag aaattgaaga gccatagcac caaggttgga 480
gcccgcggtc tggtcttgtc cccatcgaga gagctggcac tgcagacatt gaaagtcgtc 540
aaggaactgg gtagaggcac tgacctgaag tcggttcttc ttgttggtgg agacagcctg 600
gaggagcaat tcgcgatgat cgccggcaat ccagatatta ttattgcaac acctggtcga 660
ttcctgcatt tgaaggtgga aatgaacctg gacttgtcca gtatccgcta tgtcgttttc 720
gacgaggctg atcgactgtt tgagatgggt ttcgccgcgc aactaacaga gattttgcac 780
ggtcttcctg cgaatcggca gactctcctg ttctctgcca ctcttccgaa gtcccttgtc 840
gagtttgccc gcgccggctt gcaggaacct acactagtcc gtctggatac cgagagcaag 900
atctcgcccg atcttcagaa tgctttcttc tctgtgaaat cctcagagaa ggaaggggcc 960
ttgctctaca tccttcatga ggtcatcaag atgccgactg ggccaaccga ggtttcgcaa 1020
caaaggaaag aagaagatgc aagcgccaag aacttgaaga acaagaagag gaagagagcg 1080
gaaatggaga aggctgtcaa tacgagagag tctccaacca agcattcgac aatcgttttt 1140
gccgcgacga agcaccatgt cgattatctt tactcactac tctgcgaggc gggattcgcc 1200
gtctcctacg tttacggctc tttagatcaa accgcccgaa agatccaggt tcaaaacttt 1260
agaacgggca tgaccaatat cctcgttgtc accgacgttg cggctagagg tattgatatt 1320
cctatcctgg caaatgtcat taattacgat ttcccctccc agccaaagat ttttgttcac 1380
cgtgtcggcc gaactgctcg tgccgggcgc aagggctgga gttacagtct ggtccgcgat 1440
gcagacgctc cttatttact tgatttgcaa ctattcctag gaagaaggct ggttgttggc 1500
cgcgaattcg gggatcaagt gaacttcgcc gaagacgttg taacgggcag tctacctagg 1560
gatgggctct ctcaaagctg tgagtgggta accaaggtcc tggacgataa tgcagacctt 1620
gcagcccaac gcacggtcgc tgctaagggt gagaagctct atatgcgaac ccggaacgcg 1680
gcatctcttg agagtgcgaa gcgatcgaaa caggtggttt cttccgacaa ttggacaagc 1740
gtacacccgc tcttccaaga tgaaactagt aatctggagg ccgagcggga gaagatgctc 1800
gcccgtattg gtggttaccg gccgccagag acaatctttg aggtcaacaa ccggcggatg 1860
ggcaaacatg agaacgtcga cgctctcgat acgattaaga gagttcgtag cactctagag 1920
tccaagaaga agcgtgcaca agcgaatgaa aagtccgagt tcctcgaaga cggtcccgac 1980
gacggaaagg cagtcaacga agccaaagaa accgagagcg agggagcatt ttctgatgag 2040
gacgacgacg ttcccaccgg cgtggcagat aacatgtcga tggcatccga ttcagagctg 2100

gaagtcacct tctcgtcata ttcaaaatca aaggacaaca aggcgaagaa agcgagcgcg 2160
gcatctttcc agaaccctga atacttcatg tcctataccc cgaataacac ctccctggcg 2220
gaggaccgag cttatggtgt gcattccggt accaactcca acttcgccca ggcctcacgc 2280
agcgcgacca tggatctggc aggcgacgat ggaggccgcg ggttcggcga ggctcgtacg 2340
ctgatgcgct gggacaagcg acacaagaag tacgtggctc gacagaatga cgaggacggc 2400
tccaagggca cacgcctcgt ccgcggtgag agcggggcga agatcgcagc cagcttccgg 2460
agcggacggt tcgacgcttg gaagagagaa aatcgtctcg gccgcttgcc tcgggtgggt 2520
gaggccgaag ctgctaatct cgctgctggt ctcaacgcag ccatctcagg caagcggttc 2580
aggcatcgca aggagcaggc gcccaagaag gctgatcctc tccgtggtga ttacgagaag 2640
atgaagaaga aggctgaact cgccaaggag cgagcaatgt ctaaggctgg cggcgctgca 2700
ccacgtggca agagcgagct gaagagtacg gacgatatcc ggattgcgcg caaattgaag 2760
cagaagagac gagagaagaa tgctcgtccc tcgaggaaga ag 2802

<210> 2
<211> 2802
<212> DNA
<213> Aspergillus fumigatus

<400> 2
   atgcctcacc gcgcggcatc cccagcggtg tcggaaaatg agttcgacat cacaggcgct 60
   ctcttccaga acgacagcga ctccgacaac gaacagccct cggccaagtc caaacgacaa 120
   cctccgaaga aggttccctc gcaggcgctc gacttcctcg gcgatgtgaa tgaagacgac 180
   aatgacgacg aggcttttat tgccgagcag caaacttctg ccaaccgcaa ggcctcgaat 240
   ctcaaaggtc gcactgttaa gaagggtggt ggtttccaag ccatgggctt gagtgccaat 300
   ctgttaaagg caatcgctcg gaaaggcttc tcggtaccga ctcccattca gcgcaagacc 360
   attcccgtta ttatggacga ccaggatgta gttggtatgg cacggactgg ttcaggaaag 420
   acggccgctt tcgttatccc aatgatcgag aaattgaaga gccatagcac caaggttgga 480
   gcccgcggtc tggtcttgtc cccatcgaga gagctggcac tgcagacatt gaaagtcgtc 540
   aaggaactgg gtagaggcac tgacctgaag tcggttcttc ttgttggtgg agacagcctg 600
   gaggagcaat tcgcgatgat cgccggcaat ccagatatta ttattgcaac acctggtcga 660
   ttcctgcatt tgaaggtgga aatgaacctg gacttgtcca gtatccgcta tgtcgttttc 720
   gacgaggctg atcgactgtt tgagatgggt ttcgccgcgc aactaacaga gattttgcac 780
   ggtcttcctg cgaatcggca gactctcctg ttctctgcca ctcttccgaa gtcccttgtc 840
   gagtttgccc gcgccggctt gcaggaacct acactagtcc gtctggatac cgagagcaag 900
   atctcgcccg atcttcagaa tgctttcttc tctgtgaaat cctcagagaa ggaaggggcc 960
   ttgctctaca tccttcatga ggtcatcaag atgccgactg ggccaaccga ggtttcgcaa 1020
   caaaggaaag aagaagatgc aagcgccaag aacttgaaga acaagaagag gaagagagcg 1080
   gaaatggaga aggctgtcaa tacgagagag tctccaacca agcattcgac aatcgttttt 1140
   gccgcgacga agcaccatgt cgattatctt tactcactac tctgcgaggc gggattcgcc 1200
   gtctcctacg tttacggctc tttagatcaa accgcccgaa agatccaggt tcaaaacttt 1260
   agaacggcca tgaccaatat cctcgttgtc accgacgttg cggctagagg tattgatatt 1320
   cctatcctgg caaatgtcat taattacgat ttcccctccc agccaaagat ttttgttcac 1380
   cgtgtcggcc gaactgctcg tgccgggcgc aagggctgga gttacagtct ggtccgcgat 1440
   gcagacgctc cttatttact tgatttgcaa ctattcctag gaagaaggct ggttgttggc 1500
   cgcgaattcg gggatcaagt gaacttcgcc gaagacgttg taacgggcag tctacctagg 1560
   gatgggctct ctcaaagctg tgagtgggta accaaggtcc tggacgataa tgcagacctt 1620
   gcagcccaac gcacggtcgc tgctaagggt gagaagctct atatgcgaac ccggaacgcg 1680
   gcatctcttg agagtgcgaa gcgatcgaaa caggtggttt cttccgacaa ttggacaagc 1740
   gtacacccgc tcttccaaga tgaaactagt aatctggagg ccgagcggga gaagatgctc 1800
   gcccgtattg gtggttaccg gccgccagag acaatctttg aggtcaacaa ccggcggatg 1860
   ggcaaacatg agaacgtcga cgctctcgat acgattaaga gagttcgtag cactctagag 1920
   tccaagaaga agcgtgcaca agcgaatgaa aagtccgagt tcctcgaaga cggtcccgac 1980
   gacggaaagg cagtcaacga agccaaagaa accgagagcg agggagcatt ttctgatgag 2040
   gacgacgacg ttcccaccgg cgtggcagat aacatgtcga tggcatccga ttcagagctg 2100
   gaagtcacct tctcgtcata ttcaaaatca aaggacaaca aggcgaagaa agcgagcgcg 2160
   gcatctttcc agaaccctga atacttcatg tcctataccc cgaataacac ctccctggcg 2220
   gaggaccgag cttatggtgt gcattccggt accaactcca acttcgccca ggcctcacgc 2280
   agcgcgacca tggatctggc aggcgacgat ggaggccgcg ggttcggcga ggctcgtacg 2340
   ctgatgcgct gggacaagcg acacaagaag tacgtggctc gacagaatga cgaggacggc 2400
   tccaagggca cacgcctcgt ccgcggtgag agcggggcga agatcgcagc cagcttccgg 2460
   agcggacggt tcgacgcttg gaagagagaa aatcgtctcg gccgcttgcc tcgggtgggt 2520
   gaggccgaag ctgctaatct cgctgctggt ctcaacgcag ccatctcagg caagcggttc 2580
   aggcatcgca aggagcaggc gcccaagaag gctgatcctc tccgtggtga ttacgagaag 2640
   atgaagaaga aggctgaact cgccaaggag cgagcaatgt ctaaggctgg cggcgctgca 2700
   ccacgtggca agagcgagct gaagagtacg gacgatatcc ggattgcgcg caaattgaag 2760
   cagaagagac gagagaagaa tgctcgtccc tcgaggaaga ag 2802

<210> 3
<211> 934
<212> PRT
<213> Aspergillus fumigatus

<400> 3
Met Pro His Arg Ala Ala Ser Pro Ala Val Ser Glu Asn Glu Phe Asp
1               5                   10                  15

Ile Thr Gly Ala Leu Phe Gln Asn Asp Ser Asp Ser Asp Asn Glu Gln
            20                  25                  30

Pro Ser Ala Lys Ser Lys Arg Gln Pro Pro Lys Lys Val Pro Ser Gln
        35                  40                  45

Ala Leu Asp Phe Leu Gly Asp Val Asn Glu Asp Asp Asn Asp Asp Glu
    50                  55                  60

Ala Phe Ile Ala Glu Gln Gln Thr Ser Ala Asn Arg Lys Ala Ser Asn
65                  70                  75                  80

Leu Lys Gly Arg Thr Val Lys Lys Gly Gly Gly Phe Gln Ala Met Gly
                85                  90                  95

Leu Ser Ala Asn Leu Leu Lys Ala Ile Ala Arg Lys Gly Phe Ser Val
            100                 105                 110

Pro Thr Pro Ile Gln Arg Lys Thr Ile Pro Val Ile Met Asp Asp Gln
        115                 120                 125

Asp Val Val Gly Met Ala Arg Thr Gly Ser Gly Lys Thr Ala Ala Phe
    130                 135                 140

Val Ile Pro Met Ile Glu Lys Leu Lys Ser His Ser Thr Lys Val Gly
145                 150                 155                 160

Ala Arg Gly Leu Val Leu Ser Pro Ser Arg Glu Leu Ala Leu Gln Thr
                165                 170                 175

Leu Lys Val Val Lys Glu Leu Gly Arg Gly Thr Asp Leu Lys Ser Val
            180                 185                 190

Leu Leu Val Gly Gly Asp Ser Leu Glu Glu Gln Phe Ala Met Ile Ala
        195                 200                 205

Gly Asn Pro Asp Ile Ile Ile Ala Thr Pro Gly Arg Phe Leu His Leu
    210                 215                 220

Lys Val Glu Met Asn Leu Asp Leu Ser Ser Ile Arg Tyr Val Val Phe
225                 230                 235                 240

Asp Glu Ala Asp Arg Leu Phe Glu Met Gly Phe Ala Ala Gln Leu Thr
                245                 250                 255

Glu Ile Leu His Gly Leu Pro Ala Asn Arg Gln Thr Leu Leu Phe Ser
            260                 265                 270

Ala Thr Leu Pro Lys Ser Leu Val Glu Phe Ala Arg Ala Gly Leu Gln
        275                 280                 285

Glu Pro Thr Leu Val Arg Leu Asp Thr Glu Ser Lys Ile Ser Pro Asp
    290                 295                 300

Leu Gln Asn Ala Phe Phe Ser Val Lys Ser Ser Glu Lys Glu Gly Ala
305                 310                 315                 320

Leu Leu Tyr Ile Leu His Glu Val Ile Lys Met Pro Thr Gly Pro Thr
                325                 330                 335

Glu Val Ser Gln Gln Arg Lys Glu Glu Asp Ala Ser Ala Lys Asn Leu
            340                 345                 350

Lys Asn Lys Lys Arg Lys Arg Ala Glu Met Glu Lys Ala Val Asn Thr
        355                 360                 365

Arg Glu Ser Pro Thr Lys His Ser Thr Ile Val Phe Ala Ala Thr Lys
    370                 375                 380

His His Val Asp Tyr Leu Tyr Ser Leu Leu Cys Glu Ala Gly Phe Ala
385                 390                 395                 400

Val Ser Tyr Val Tyr Gly Ser Leu Asp Gln Thr Ala Arg Lys Ile Gln
                405                 410                 415

Val Gln Asn Phe Arg Thr Gly Met Thr Asn Ile Leu Val Val Thr Asp

            420                 425                 430

Val Ala Ala Arg Gly Ile Asp Ile Pro Ile Leu Ala Asn Val Ile Asn
        435                 440                 445

Tyr Asp Phe Pro Ser Gln Pro Lys Ile Phe Val His Arg Val Gly Arg
    450                 455                 460

Thr Ala Arg Ala Gly Arg Lys Gly Trp Ser Tyr Ser Leu Val Arg Asp
465                 470                 475                 480

Ala Asp Ala Pro Tyr Leu Leu Asp Leu Gln Leu Phe Leu Gly Arg Arg
                485                 490                 495

Leu Val Val Gly Arg Glu Phe Gly Asp Gln Val Asn Phe Ala Glu Asp
            500                 505                 510

Val Val Thr Gly Ser Leu Pro Arg Asp Gly Leu Ser Gln Ser Cys Glu
        515                 520                 525

Trp Val Thr Lys Val Leu Asp Asp Asn Ala Asp Leu Ala Ala Gln Arg
    530                 535                 540

Thr Val Ala Ala Lys Gly Glu Lys Leu Tyr Met Arg Thr Arg Asn Ala
545                 550                 555                 560

Ala Ser Leu Glu Ser Ala Lys Arg Ser Lys Gln Val Val Ser Ser Asp
                565                 570                 575

Asn Trp Thr Ser Val His Pro Leu Phe Gln Asp Glu Thr Ser Asn Leu
            580                 585                 590

Glu Ala Glu Arg Glu Lys Met Leu Ala Arg Ile Gly Gly Tyr Arg Pro
        595                 600                 605

Pro Glu Thr Ile Phe Glu Val Asn Asn Arg Arg Met Gly Lys His Glu
    610                 615                 620

Asn Val Asp Ala Leu Asp Thr Ile Lys Arg Val Arg Ser Thr Leu Glu
625                 630                 635                 640

Ser Lys Lys Lys Arg Ala Gln Ala Asn Glu Lys Ser Glu Phe Leu Glu
                645                 650                 655

Asp Gly Pro Asp Asp Gly Lys Ala Val Asn Glu Ala Lys Glu Thr Glu
            660                 665                 670

Ser Glu Gly Ala Phe Ser Asp Glu Asp Asp Asp Val Pro Thr Gly Val
        675                 680                 685

Ala Asp Asn Met Ser Met Ala Ser Asp Ser Glu Leu Glu Val Thr Phe
    690                 695                 700

Ser Ser Tyr Ser Lys Ser Lys Asp Asn Lys Ala Lys Lys Ala Ser Ala
705                 710                 715                 720

Ala Ser Phe Gln Asn Pro Glu Tyr Phe Met Ser Tyr Thr Pro Asn Asn
                725                 730                 735

Thr Ser Leu Ala Glu Asp Arg Ala Tyr Gly Val His Ser Gly Thr Asn
            740                 745                 750

Ser Asn Phe Ala Gln Ala Ser Arg Ser Ala Thr Met Asp Leu Ala Gly
        755                 760                 765

Asp Asp Gly Gly Arg Gly Phe Gly Glu Ala Arg Thr Leu Met Arg Trp
    770                 775                 780

Asp Lys Arg His Lys Lys Tyr Val Ala Arg Gln Asn Asp Glu Asp Gly
785                 790                 795                 800

Ser Lys Gly Thr Arg Leu Val Arg Gly Glu Ser Gly Ala Lys Ile Ala
                805                 810                 815

Ala Ser Phe Arg Ser Gly Arg Phe Asp Ala Trp Lys Arg Glu Asn Arg
            820                 825                 830

Leu Gly Arg Leu Pro Arg Val Gly Glu Ala Glu Ala Ala Asn Leu Ala
        835                 840                 845

Ala Gly Leu Asn Ala Ala Ile Ser Gly Lys Arg Phe Arg His Arg Lys
    850                 855                 860

Glu Gln Ala Pro Lys Lys Ala Asp Pro Leu Arg Gly Asp Tyr Glu Lys
865                 870                 875                 880

Met Lys Lys Lys Ala Glu Leu Ala Lys Glu Arg Ala Met Ser Lys Ala
                885                 890                 895

Gly Gly Ala Ala Pro Arg Gly Lys Ser Glu Leu Lys Ser Thr Asp Asp
            900                 905                 910

Ile Arg Ile Ala Arg Lys Leu Lys Gln Lys Arg Arg Glu Lys Asn Ala
        915                 920                 925

Arg Pro Ser Arg Lys Lys
    930

<210> 4
<211> 1735
<212> DNA
<213> Aspergillus fumigatus

<400> 4
atgagtgcaa tcctttctgc agacgatttg aacgatttca tttctcccgg ggttgcttgc 60
atcaagcccg ttgagagtct accacaaaaa gaatcccagt cggaggtatc tttcctgtct 120
taccagtcat ctgttgatat cagccaatag gctaacgctc atttccaatt caatagaatc 180
cctatgaggt gacaaaggaa gacaaagttc aaccggaaaa ccttcccccg gctcagattt 240
cattgactga ttgccttgca tgctccggat gtgtcacgtc tgcggaagca gtgttgatat 300
ccttgcaatc acatacggag gttctcaata ctcttgattc gtaccccgaa ttgccgcttg 360
gttctacaag ctaccaaaga ggcacacaaa aagttggatc agcagacagc gatggtcgca 420
tctttgttgc tagcgtcagc cctcaagtca gggcgagctt ggcagccaca tacggaatca 480
ccgagcggga ggcgaaatat atgattgacc aatttcttat gggccctcac ggtctcagag 540
ctggtggaaa acatggcaat gggtttacat gggttgtgga cacgaacgtt atgcgtgaag 600
cagtgttggc tctgacagcg gacgaggtca cgagctcttt attatcaact ggatcgggca 660
gccttcccaa gagtccaatt ctttcgtccg cttgccccgg ctggatatgt tatgctgaaa 720
aaacacaccc ttttatcctt ccgcatttat ctcgcctcaa gtctcctcag gcgttgagcg 780
gcacatttct gaagtcagtg ctaagcaagg cacttggggt cccgccttct cagatatggc 840
atttagctat catgccatgc ttcgacaaga agctggaagc tagccgggaa gagctgacag 900
acattgcatg ggcttcaacc ttcacccagt cacagacaac acccgtccgc gacgttgact 960
gtgtcataac cacccgtgag ctactaactt tagccactgc tagggggctt tctctaccca 1020
atttgccgct caaaccattg cccgcgtcat gtttaactcc atttccagat caagccctag 1080
aatcattttt gttctctaag agctcgtcgg gccaaacagt cgaatcaggg acatctggag 1140
gctatcttca tcacgtcctc caaatcttcc aagccagaaa ccccggcagc aagattgtca 1200
cccagcgtgg gcgcaacgcc gatgttgtgg aatatgtgct catgtcgtct ggggatgagc 1260
ctctttttag ggcggctcgg tattatggct tcaggaatat acaaaatctc gtcagaaaac 1320
ttaaacccgc acgcgtgtca agactgccag gcgccaagcc gcaagcggtc tcttcaagtg 1380
caaatcgacg acagcccatg tcaaggaacg cagctccggc tggaacaggc gctgattatg 1440
catatgttga agtcatggct tgtcctggcg gctgtaccaa tggtggtggg caaataagga 1500
ttgaagatgc ccgggaggct gttccgaacg cactaaaaga gacatcgact gaaactcctg 1560
tggctgcacc gaaacccacg ccgcatgagc agcgtgcctg gctagcccgg gtagatgaag 1620
cgtactactc tgcggactcg gatagcgagg gatctgtcac gacggagccg gtttctgtcc 1680
tgtcaaggga taaccagatt catgagtttt tgaactattg gtcagagaag gttga      1735

<210> 5
<211> 1592
<212> DNA
<213> Aspergillus fumigatus

<400> 5
atgagtgcaa tcctttctgc agacgatttg aacgatttca tttctcccgg ggttgcttgc 60
atcaagcccg ttgagagtct accacaaaaa gaatcccagt cggagaatcc ctatgaggtg 120
acaaaggaag acaaagttca accggaaaac cttcccccgg ctcagatttc attgactgat 180
tgccttgcat gctccggatg tgtcacgtct gcggaagcag tgttgatatc cttgcaatca 240
catacggagg ttctcaatac tcttgattcc gatggtcgca tctttgttgc tagcgtcagc 300
cctcaagtca gggcgagctt ggcagccaca tacggaatca ccgagcggga ggcgaaatat 360
atgattgacc aatttcttat gggccctcac ggtctcagag ctggtggaaa acatggcaat 420

gggtttacat gggttgtgga cacgaacgtt atgcgtgaag cagtgttggc tctgacagcg 480
gacgaggtca cgagctcttt attatcaact ggatcgggca gccttcccaa gagtccaatt 540
ctttcgtccg cttgccccgg ctggatatgt tatgctgaaa aaacacaccc ttttatcctt 600
ccgcatttat ctcgcctcaa gtctcctcag gcgttgagcg gcacatttct gaagtcagtg 660
ctaagcaagg cacttggggt cccgccttct cagatatggc atttagctat catgccatgc 720
ttcgacaaga agctggaagc tagccgggaa gagctgacag acattgcatg ggcttcaacc 780
ttcacccagt cacagacaac acccgtccgc gacgttgact gtgtcataac cacccgtgag 840
ctactaactt tagccactgc tagggggctt tctctaccca atttgccgct caaaccattg 900
cccgcgtcat gtttaactcc atttccagat caagccctag aatcattttt gttctctaag 960
agctcgtcgg gccaaacagt cgaatcaggg acatctggag gctatcttca tcacgtcctc 1020
caaatcttcc aagccagaaa ccccggcagc aagattgtca cccagcgtgg gcgcaacgcc 1080
gatgttgtgg aatatgtgct catgtcgtct ggggatgagc ctctttttag ggcggctcgg 1140
tattatggct tcaggaatat acaaaatctc gtcagaaaac ttaaacccgc acgcgtgtca 1200
agactgccag gcgccaagcc gcaagcggtc tcttcaagtg caaatcgacg acagcccatg 1260
tcaaggaacg cagctccggc tggaacaggc gctgattatg catatgttga agtcatggct 1320
tgtcctggcg gctgtaccaa tggtggtggg caaataagga ttgaagatgc ccgggaggct 1380
gttccgaacg cactaaaaga gacatcgact gaaactcctg tggctgcacc gaaacccacg 1440
ccgcatgagc agcgtgcctg gctagcccgg gtagatgaag cgtactactc tgcggactcg 1500
gatagcgagg gatctgtcac gacggagccg gtttctgtcc tgtcaaggga taaccagatt 1560
catgagtttt tgaactattg gtcagagaag gt 1592

<210> 6
<211> 530
<212> PRT
<213> Aspergillus fumigatus

<400> 6
Met Ser Ala Ile Leu Ser Ala Asp Asp Leu Asn Asp Phe Ile Ser Pro
1               5                   10                  15

Gly Val Ala Cys Ile Lys Pro Val Glu Ser Leu Pro Gln Lys Glu Ser
            20                  25                  30

Gln Ser Glu Asn Pro Tyr Glu Val Thr Lys Glu Asp Lys Val Gln Pro
        35                  40                  45

Glu Asn Leu Pro Pro Ala Gln Ile Ser Leu Thr Asp Cys Leu Ala Cys
    50                  55                  60

Ser Gly Cys Val Thr Ser Ala Glu Ala Val Leu Ile Ser Leu Gln Ser
65                  70                  75                  80

His Thr Glu Val Leu Asn Thr Leu Asp Ser Asp Gly Arg Ile Phe Val
                85                  90                  95

Ala Ser Val Ser Pro Gln Val Arg Ala Ser Leu Ala Ala Thr Tyr Gly
            100                 105                 110

Ile Thr Glu Arg Glu Ala Lys Tyr Met Ile Asp Gln Phe Leu Met Gly
        115                 120                 125

Pro His Gly Leu Arg Ala Gly Gly Lys His Gly Asn Gly Phe Thr Trp
    130                 135                 140

Val Val Asp Thr Asn Val Met Arg Glu Ala Val Leu Ala Leu Thr Ala
145                 150                 155                 160

Asp Glu Val Thr Ser Ser Leu Leu Ser Thr Gly Ser Gly Ser Leu Pro
                165                 170                 175

Lys Ser Pro Ile Leu Ser Ser Ala Cys Pro Gly Trp Ile Cys Tyr Ala
            180                 185                 190

Glu Lys Thr His Pro Phe Ile Leu Pro His Leu Ser Arg Leu Lys Ser
        195                 200                 205

Pro Gln Ala Leu Ser Gly Thr Phe Leu Lys Ser Val Leu Ser Lys Ala
    210                 215                 220

Leu Gly Val Pro Pro Ser Gln Ile Trp His Leu Ala Ile Met Pro Cys
225                 230                 235                 240

Phe Asp Lys Lys Leu Glu Ala Ser Arg Glu Glu Leu Thr Asp Ile Ala
                245                 250                 255

Trp Ala Ser Thr Phe Thr Gln Ser Gln Thr Thr Pro Val Arg Asp Val
            260                 265                 270

Asp Cys Val Ile Thr Thr Arg Glu Leu Leu Thr Leu Ala Thr Ala Arg
        275                 280                 285

Gly Leu Ser Leu Pro Asn Leu Pro Leu Lys Pro Leu Pro Ala Ser Cys
    290                 295                 300

Leu Thr Pro Phe Pro Asp Gln Ala Leu Glu Ser Phe Leu Phe Ser Lys
305                 310                 315                 320

Ser Ser Ser Gly Gln Thr Val Glu Ser Gly Thr Ser Gly Gly Tyr Leu
                325                 330                 335

His His Val Leu Gln Ile Phe Gln Ala Arg Asn Pro Gly Ser Lys Ile
            340                 345                 350

Val Thr Gln Arg Gly Arg Asn Ala Asp Val Val Glu Tyr Val Leu Met
        355                 360                 365

Ser Ser Gly Asp Glu Pro Leu Phe Arg Ala Ala Arg Tyr Tyr Gly Phe
    370                 375                 380

Arg Asn Ile Gln Asn Leu Val Arg Lys Leu Lys Pro Ala Arg Val Ser
385                 390                 395                 400

Arg Leu Pro Gly Ala Lys Pro Gln Ala Val Ser Ser Ser Ala Asn Arg
                405                 410                 415

Arg Gln Pro Met Ser Arg Asn Ala Ala Pro Ala Gly Thr Gly Ala Asp
            420                 425                 430

Tyr Ala Tyr Val Glu Val Met Ala Cys Pro Gly Gly Cys Thr Asn Gly
        435                 440                 445

Gly Gly Gln Ile Arg Ile Glu Asp Ala Arg Glu Ala Val Pro Asn Ala
    450                 455                 460

Leu Lys Glu Thr Ser Thr Glu Thr Pro Val Ala Ala Pro Lys Pro Thr
465                 470                 475                 480

Pro His Glu Gln Arg Ala Trp Leu Ala Arg Val Asp Glu Ala Tyr Tyr
                485                 490                 495

Ser Ala Asp Ser Asp Ser Glu Gly Ser Val Thr Thr Glu Pro Val Ser

500                 505                 510

Val Leu Ser Arg Asp Asn Gln Ile His Glu Phe Leu Asn Tyr Trp Ser
        515                 520                 525

Glu Lys
    530

<210> 7
<211> 942
<212> DNA
<213> Aspergillus fumigatus

<400> 7
atgactaccg gggctggtac gatctctcat tccaacacct atcatcgtat tcctcgccgt 60
taactgacca atccaccagt gcaaaggttc cgtccagtgg tggtatcggg tccctctggg 120
actgggaagt cgaccttgct caagagactc ttcgctgaat accccgatac tttcgattta 180
tccgtgtctc gtacgtctaa ccccttgcca accctcattg actatgcctg cgaattgttt 240
cttttggtgg aattgcgctg aacggtgttt gttatattta gataccactc gagctccccg 300
tcccggggaa gaaaatggac gtgagtatta cttcacaact aaagaagatt tcctggatct 360
tgtgagcaag aatgccttta tcgagcatgc gcagtttggt ggcaattact acggtactac 420
tgtgcaggca gtgaaggatg ttgcgcagaa gggcaagatc tgcgttctcg acattgagat 480
gaggtaataa tagtcctgca acgtgaactg atatgaccgg agaagcagag gaaatccatc 540
atcaaatgga ttgtagttca acccaaacaa cagctgacga ctgaattgca atagggcgtg 600
aaacaagtca agcgcaccga tcttgatgct cgattcttat ttttagcacc cccgtccctt 660
gaagaactag agaaaagact gcgtgggaga gcaaccgaga ctgaggagag cttgacggta 720
tggctgtcct ccacattcct tcacttcccc aactcgccag actgtcccgc tggaattcta 780
actttgcgtc agaaacgcct tgcccaagct aaaaatgaat tggaatatgc ggcgcagcct 840
ggctctcatg ataagattgt cgtgaacgat gacctggaga aggcttataa ggaactgcgg 900
gattggattg tcgacggtgg taactttgga gcgcgtcaat ga                   942

<210> 8
<211> 600
<212> DNA
<213> Aspergillus fumigatus

<400> 8
atgactaccg gggctgtgca aaggttccgt ccagtggtgg tatcgggtcc ctctgggact 60
gggaagtcga ccttgctcaa gagactcttc gctgaatacc ccgatacttt cgatttatcc 120
gtgtctcata ccactcgagc tccccgtccc ggggaagaaa atggacgtga gtattacttc 180
acaactaaag aagatttcct ggatcttgtg agcaagaatg cctttatcga gcatgcgcag 240
tttggtggca attactacgg tactactgtg caggcagtga aggatgttgc gcagaagggc 300
aagatctgcg ttctcgacat tgagatgagg ggcgtgaaac aagtcaagcg caccgatctt 360
gatgctcgat tcttattttt agcacccccg tcccttgaag aactagagaa aagactgcgt 420
gggagagcaa ccgagactga ggagagcttg acgaaacgcc ttgcccaagc taaaaatgaa 480
ttggaatatg cggcgcagcc tggctctcat gataagattg tcgtgaacga tgacctggag 540
aaggcttata aggaactgcg ggattggatt gtcgacggtg gtaactttgg agcgcgtcaa 600

<210> 9
<211> 200
<212> PRT
<213> Aspergillus fumigatus

<400> 9
Met Thr Thr Gly Ala Val Gln Arg Phe Arg Pro Val Val Val Ser Gly
1               5                   10                  15

Pro Ser Gly Thr Gly Lys Ser Thr Leu Leu Lys Arg Leu Phe Ala Glu
            20                  25                  30

Tyr Pro Asp Thr Phe Asp Leu Ser Val Ser His Thr Thr Arg Ala Pro
        35                  40                  45

Arg Pro Gly Glu Glu Asn Gly Arg Glu Tyr Tyr Phe Thr Thr Lys Glu
    50                  55                  60

Asp Phe Leu Asp Leu Val Ser Lys Asn Ala Phe Ile Glu His Ala Gln
65                  70                  75                  80

Phe Gly Gly Asn Tyr Tyr Gly Thr Thr Val Gln Ala Val Lys Asp Val
                85                  90                  95

Ala Gln Lys Gly Lys Ile Cys Val Leu Asp Ile Glu Met Arg Gly Val
            100                 105                 110

Lys Gln Val Lys Arg Thr Asp Leu Asp Ala Arg Phe Leu Phe Leu Ala
        115                 120                 125

Pro Pro Ser Leu Glu Glu Leu Glu Lys Arg Leu Arg Gly Arg Ala Thr
    130                 135                 140

Glu Thr Glu Glu Ser Leu Thr Lys Arg Leu Ala Gln Ala Lys Asn Glu
145                 150                 155                 160

Leu Glu Tyr Ala Ala Gln Pro Gly Ser His Asp Lys Ile Val Val Asn
                165                 170                 175

Asp Asp Leu Glu Lys Ala Tyr Lys Glu Leu Arg Asp Trp Ile Val Asp
            180                 185                 190

Gly Gly Asn Phe Gly Ala Arg Gln
        195                 200

<210> 10
<211> 2059
<212> DNA
<213> Aspergillus fumigatus

<400> 10
atgttagaag ccttcgaagt cttgacaaca tctggggtgg tgctgtggtc gaagtcgtat 60
gcgccggtcg gagcgcatgt tgtcaacagc ctaatcaacg atgtcttcat tgaggagaag 120
gttcgagcgc agaatcaggc agcgagcagt gcagctccta tctacaagaa ggaaaagtat 180
actctgaaat ggaagcaagt aaaggatttc aatctgatat ttgtggtatg ttcacgccgc 240
tcgttgattc aatggcgcca ctgaccgatt ccataggctg tatatcaatc tctgctacat 300
cttggttgga tcgacaaact cttggataat gtttcgacca tattcatcga cttatataag 360
gatgagctaa ggagcacacg ggctaggatt attgagtacc cattcgataa gtacttcgac 420
cagcaggtgc gagagcttga ggacaatgct ggggctccta catcagaatc tctcgtagta 480
gagatcaacg agagaaagga ccctcttgtc tcatcagata acggcgggcc acctccgcca 540
cccgtgcctg gtctgctgaa aggtatctga cgtcgataat ttttctctgc tagtgatcat 600
attgctaact acctccgaag cgcaacgtcc agttgcgcag ggcgtggcga cctcggacga 660
gggttcgcca ccccaaaccc cagatctttc tcgatcgtca acgcccattt caggtcatct 720
attgaccgcg aaaggagggc ctgctggccg cgcctctcgt cgcgcacgca aagcggccaa 780
cgcgagcgct accgcttctt ctggagatga aagcattcgg aaggggaaaa cattgaaaag 840
tggaaaaaag atgcgcaagt gggatgctga tggctttgcg gatgaggacg acggcaaggt 900
cctcgattac tccgcccccg cagatggtga ggacgcaccg gctcctgtag tcgaggctgt 960
tgcgcaggaa tcctggggac gccgaacagg caagggccaa tttgtgctga aagatctagg 1020

ggatgaagtc cattccattc ttgagaatgc tgatcatgaa aagacaaagt cttcctcgtc 1080
cacgggcttt gttgggtctg gagtcaacgc acttggtgga ttcttccgta atattgtcgg 1140
cggcaaggtc cttactgagg ctgacttgga gaaacccttg aaagccatgg aagaccattt 1200
gctgaagaag aacgttgcgc gcgaagcggc cgtccgtcta tgtcaaggcg tccagcgcga 1260
attagttggc aagaagacag gcaactttca aagtgttgat gcagcactgc gctccgcaat 1320
ggagtcctcg ttgcgcaaaa tattgacgcc aacgtcatct ctcgatctac tgcgtgagat 1380
cgatgctgtt agatctccga cgagcaaagg acaggctcct cgcccatatg tcatttccat 1440
cgtgggcgtg aacggtgttg ggaagtcgac aaatctgggc aaaatttgtt acttccttct 1500
ccagaataac tatcgtgttc tgattgcagc ctgtgacacc ttccgctctg gagccgtgga 1560
gcagttacga gtccatgctc gcaatttgaa ggaacttagt acccgggaga atgctggaga 1620
ggttgaactc tacgagaagg gatatggaaa ggatgcagcg aatgtagcga aggatgcagt 1680
ggagtacggt gcggcgaatc atttcgacgt tgtgttgatt gatactgccg gtcgccgtca 1740
taacgaccaa cgccttatgt cttcgctcga gaagttcgcc aagttcgcca aaccagataa 1800
gatcttcatg gtcggtgaag ctctggtcgg tacggacagc gtgatgcagg ctcgcaactt 1860
caaccaagct ttcggcactg ggagaaacct cgatgggttc atcatcagta aatgtgatac 1920
cgttggtgac atggtaggta cgcttgtcag catggtgcat gctacaggca ttcctattgt 1980
ttttctgggt gtaggccagc actatggtga tttgaggggc ctaagtgttc cttgggctgt 2040
caatctgctg atgaagtga 2059

<210> 11
<211> 1923
<212> DNA
<213> Aspergillus fumigatus

<400> 11
atgttagaag ccttcgaagt cttgacaaca tctggggtgg tgctgtggtc gaagtcgtat 60
gcgccggtcg gagcgcatgt tgtcaacagc ctaatcaacg atgtcttcat tgaggagaag 120
gttcgagcgc agaatcaggc agcgagcagt gcagctccta tctacaagaa ggaaaagtat 180
actctgaaat ggaagcaagt aaaggatttc aatctgatat ttgtggctgt atatcaatct 240
ctgctacatc ttggttggat cgacaaactc ttggataatg tttcgaccat attcatcgac 300
ttatataagg atgagctaag gagcacacgg gctaggatta ttgagtaccc attcgataag 360
tacttcgacc agcaggtgcg agagcttgag gacaatgctg gggctcctac atcagaatct 420
ctcgtagtag agatcaacga gagaaaggac cctcttgtct catcagataa cggcgggcca 480
cctccgccac ccgtgcctgt tgcgcagggc gtggcgacct cggacgaggg ttcgccaccc 540
caaaccccag atctttctcg atcgtcaacg cccatttcag gtcatctatt gaccgcgaaa 600
ggagggcctg ctggccgcgc ctctcgtcgc gcacgcaaag cggccaacgc gagcgctacc 660
gcttcttctg gagatgaaag cattcggaag gggaaaacat tgaaaagtgg aaaaaagatg 720
cgcaagtggg atgctgatgg ctttgcggat gaggacgacg gcaaggtcct cgattactcc 780
gcccccgcag atggtgagga cgcaccggct cctgtagtcg aggctgttgc gcaggaatcc 840
tggggacgcc gaacaggcaa gggccaattt gtgctgaaag atctagggga tgaagtccat 900
tccattcttg agaatgctga tcatgaaaag acaaagtctt cctcgtccac gggctttgtt 960
gggtctggag tcaacgcact tggtggattc ttccgtaata ttgtcggcgg caaggtcctt 1020
actgaggctg acttggagaa acccttgaaa gccatggaag accatttgct gaagaagaac 1080
gttgcgcgcg aagcggccgt ccgtctatgt caaggcgtcc agcgcgaatt agttggcaag 1140
aagacaggca actttcaaag tgttgatgca gcactgcgct ccgcaatgga gtcctcgttg 1200
cgcaaaatat tgacgccaac gtcatctctc gatctactgc gtgagatcga tgctgttaga 1260
tctccgacga gcaaaggaca ggctcctcgc ccatatgtca tttccatcgt gggcgtgaac 1320
ggtgttggga agtcgacaaa tctgggcaaa atttgttact tccttctcca gaataactat 1380
cgtgttctga ttgcagcctg tgacaccttc cgctctggag ccgtggagca gttacgagtc 1440
catgctcgca atttgaagga acttagtacc cgggagaatg ctggagaggt tgaactctac 1500
gagaagggat atggaaagga tgcagcgaat gtagcgaagg atgcagtgga gtacggtgcg 1560
gcgaatcatt tcgacgttgt gttgattgat actgccggtc gccgtcataa cgaccaacgc 1620
cttatgtctt cgctcgagaa gttcgccaag ttcgccaaac cagataagat cttcatggtc 1680
ggtgaagctc tggtcggtac ggacagcgtg atgcaggctc gcaacttcaa ccaagctttc 1740
ggcactggga gaaacctcga tgggttcatc atcagtaaat gtgataccgt tggtgacatg 1800
gtaggtacgc ttgtcagcat ggtgcatgct acaggcattc ctattgtttt tctgggtgta 1860
ggccagcact atggtgattt gaggggccta agtgttcctt gggctgtcaa tctgctgatg 1920
aag 1923

<210> 12
<211> 641
<212> PRT
<213> Aspergillus fumigatus

<400> 12
Met Leu Glu Ala Phe Glu Val Leu Thr Thr Ser Gly Val Val Leu Trp
1               5                   10                  15

Ser Lys Ser Tyr Ala Pro Val Gly Ala His Val Val Asn Ser Leu Ile
            20                  25                  30

Asn Asp Val Phe Ile Glu Glu Lys Val Arg Ala Gln Asn Gln Ala Ala
        35                  40                  45

Ser Ser Ala Ala Pro Ile Tyr Lys Lys Glu Lys Tyr Thr Leu Lys Trp
    50                  55                  60

Lys Gln Val Lys Asp Phe Asn Leu Ile Phe Val Ala Val Tyr Gln Ser
65                  70                  75                  80

Leu Leu His Leu Gly Trp Ile Asp Lys Leu Leu Asp Asn Val Ser Thr
                85                  90                  95

Ile Phe Ile Asp Leu Tyr Lys Asp Glu Leu Arg Ser Thr Arg Ala Arg
            100                 105                 110

Ile Ile Glu Tyr Pro Phe Asp Lys Tyr Phe Asp Gln Gln Val Arg Glu
        115                 120                 125

Leu Glu Asp Asn Ala Gly Ala Pro Thr Ser Glu Ser Leu Val Val Glu
    130                 135                 140

Ile Asn Glu Arg Lys Asp Pro Leu Val Ser Ser Asp Asn Gly Gly Pro
145                 150                 155                 160

Pro Pro Pro Pro Val Pro Val Ala Gln Gly Val Ala Thr Ser Asp Glu
                165                 170                 175

Gly Ser Pro Pro Gln Thr Pro Asp Leu Ser Arg Ser Ser Thr Pro Ile
            180                 185                 190

Ser Gly His Leu Leu Thr Ala Lys Gly Gly Pro Ala Gly Arg Ala Ser
        195                 200                 205

Arg Arg Ala Arg Lys Ala Ala Asn Ala Ser Ala Thr Ala Ser Ser Gly
    210                 215                 220

Asp Glu Ser Ile Arg Lys Gly Lys Thr Leu Lys Ser Gly Lys Lys Met
225                 230                 235                 240

Arg Lys Trp Asp Ala Asp Gly Phe Ala Asp Glu Asp Asp Gly Lys Val
                245                 250                 255

Leu Asp Tyr Ser Ala Pro Ala Asp Gly Glu Asp Ala Pro Ala Pro Val
            260                 265                 270

Val Glu Ala Val Ala Gln Glu Ser Trp Gly Arg Arg Thr Gly Lys Gly
        275                 280                 285

Gln Phe Val Leu Lys Asp Leu Gly Asp Glu Val His Ser Ile Leu Glu

290                 295                 300

Asn Ala Asp His Glu Lys Thr Lys Ser Ser Ser Ser Thr Gly Phe Val
305                 310                 315                 320

Gly Ser Gly Val Asn Ala Leu Gly Gly Phe Phe Arg Asn Ile Val Gly
                325                 330                 335

Gly Lys Val Leu Thr Glu Ala Asp Leu Glu Lys Pro Leu Lys Ala Met
            340                 345                 350

Glu Asp His Leu Leu Lys Lys Asn Val Ala Arg Glu Ala Ala Val Arg
        355                 360                 365

Leu Cys Gln Gly Val Gln Arg Glu Leu Val Gly Lys Lys Thr Gly Asn
    370                 375                 380

Phe Gln Ser Val Asp Ala Ala Leu Arg Ser Ala Met Glu Ser Ser Leu
385                 390                 395                 400

Arg Lys Ile Leu Thr Pro Thr Ser Ser Leu Asp Leu Leu Arg Glu Ile
                405                 410                 415

Asp Ala Val Arg Ser Pro Thr Ser Lys Gly Gln Ala Pro Arg Pro Tyr
            420                 425                 430

Val Ile Ser Ile Val Gly Val Asn Gly Val Gly Lys Ser Thr Asn Leu
        435                 440                 445

Gly Lys Ile Cys Tyr Phe Leu Leu Gln Asn Asn Tyr Arg Val Leu Ile
    450                 455                 460

Ala Ala Cys Asp Thr Phe Arg Ser Gly Ala Val Glu Gln Leu Arg Val
465                 470                 475                 480

His Ala Arg Asn Leu Lys Glu Leu Ser Thr Arg Glu Asn Ala Gly Glu
                485                 490                 495

Val Glu Leu Tyr Glu Lys Gly Tyr Gly Lys Asp Ala Ala Asn Val Ala
            500                 505                 510

Lys Asp Ala Val Glu Tyr Gly Ala Ala Asn His Phe Asp Val Val Leu
        515                 520                 525

Ile Asp Thr Ala Gly Arg Arg His Asn Asp Gln Arg Leu Met Ser Ser
    530                 535                 540

Leu Glu Lys Phe Ala Lys Phe Ala Lys Pro Asp Lys Ile Phe Met Val
545                 550                 555                 560

Gly Glu Ala Leu Val Gly Thr Asp Ser Val Met Gln Ala Arg Asn Phe
                565                 570                 575

Asn Gln Ala Phe Gly Thr Gly Arg Asn Leu Asp Gly Phe Ile Ile Ser
            580                 585                 590

Lys Cys Asp Thr Val Gly Asp Met Val Gly Thr Leu Val Ser Met Val
        595                 600                 605

His Ala Thr Gly Ile Pro Ile Val Phe Leu Gly Val Gly Gln His Tyr
    610                 615                 620

Gly Asp Leu Arg Gly Leu Ser Val Pro Trp Ala Val Asn Leu Leu Met
625                 630                 635                 640

Lys

<210> 13
<211> 1564
<212> DNA
<213> Aspergillus fumigatus

<400> 13
atgcggtggt gcctcactct tctggcattc tgcttcttgg cagttgtacg tgcattaagt 60
agctccggca gtcgtctgtt ggttgttttg gaagatgcca cagaaaagga attatactcg 120
aaattatggg ctgacctaga aggtgctcta acctactgaa cttctacgtt aatatgctaa 180
tattaattgg tagctcgagg atataacctc gacttcgaat cccccaagaa tgacaagctc 240
agcctgttcg aactcggaga ccgagtctac gaccacatgc ttctcctgcc tcccaagtca 300
aagggttagc gttaccctta gacatgtcca tatgctctgc tttgtacatc tcaattgacc 360
tcttggccag gctatggacc ctcccttacc cccaagaata tcattgattt catgaacaag 420
gacggtaacg tcctcctcgc cttgtcgggc aagtccacaa ccgccagcgc tatcagctcg 480
ctgctattgg agctcgatct ccatctccct gtcgatcgtt cctctgtcac cgtcgatcac 540
ttcaactacg atacactttc tgcctccgat aagcatgatg ttctgctact ccaccgacca 600
ggcaagttga ggtccgatac caaggctttc tttgatggcg agggcgttgt agcatttccc 660
agagccgtcc cccacaccct gggcgatgca aaccctctca ttgcgcctat tctgcgagcg 720
cccgccactg cgtatagtta caaccccaag gaggacgcgt cgtcagttga ggatgttgca 780
gctacgggtt cgcagttggc tctggtctcg gccatgcagg ctagaaactc cgctcggttc 840
actctactgg gatccgtgga gagtctgcag gatcagtggt ttctgcgact tgtcaaggct 900
cctggtgatg ggaagcagat gaagacggtc aaccaggaat tcgccaagca gcttactgcg 960
tggacattca aggaaaccgg agtcctcaag gtcggaaaga tcgagcatca tctggctgaa 1020
gatggtgaaa tcactcccga gaagctgaac cctaagatct atcgaataaa gaatgaaact 1080
gtaagtgaca gccatctgag gttccattgc ctatttgcat gctcaccctt ctcaacaggt 1140
ctttagcatt gaactttccg aatacaacta tgatcgttac gcgcccttcg aggttccaac 1200
tggcgatgcc gtccagctcg agtttaccat gctgtctccc ttccatcgcc tgaacttgga 1260
acccgtccgt cgaacagata acagtacagt ttacagcaca cgattcacca cccccgatca 1320
gcatggaatc ttctccttcc gagtgaacta caagcgcccg ttcctcacga acatcgaaga 1380
aaaacttgag gtgaccgttc gtcatttcgc tcataacgag tacccccgaa gctggaaaat 1440
cagcggtgga tgggtctgga ttgcgggtct gtggtccgtc atcgctggct tcttagtatt 1500
cgttgttgca tggctttact cagcgccttc tgccgccgca ctgaacacaa agaagacaca 1560
ataa                                                              1564

<210> 14
<211> 1380
<212> DNA
<213> Aspergillus fumigatus

<400> 14
atgcggtggt gcctcactct tctggcattc tgcttcttgg cagttgtacg tgcattaagt 60
agctccggca gtcgtctgtt ggttgttttg gaagatgcca cagaaaagga attatactcg 120
aaattatggg ctgacctaga aggatataac ctcgacttcg aatcccccaa gaatgacaag 180
ctcagcctgt tcgaactcgg agaccgagtc tacgaccaca tgcttctcct gcctcccaag 240
tcaaagggct atggaccctc ccttaccccc aagaatatca ttgatttcat gaacaaggac 300
ggtaacgtcc tcctcgcctt gtcgggcaag tccacaaccg ccagcgctat cagctcgctg 360
ctattggagc tcgatctcca tctccctgtc gatcgttcct ctgtcaccgt cgatcacttc 420
aactacgata cactttctgc ctccgataag catgatgttc tgctactcca ccgaccaggc 480
aagttgaggt ccgataccaa ggctttcttt gatggcgagg gcgttgtagc atttcccaga 540
gccgtccccc acaccctggg cgatgcaaac cctctcattg cgcctattct gcgagcgccc 600
gccactgcgt atagttacaa ccccaaggag gacgcgtcgt cagttgagga tgttgcagct 660

acgggttcgc agttggctct ggtctcggcc atgcaggcta gaaactccgc tcggttcact 720
ctactgggat ccgtggagag tctgcaggat cagtggtttt ctgcgactgt caaggctcct 780
ggtgatggga agcagatgaa gacggtcaac caggaattcg ccaagcagct tactgcgtgg 840
acattcaagg aaaccggagt cctcaaggtc ggaaagatcg agcatcatct ggctgaagat 900
ggtgaaatca ctcccgagaa gctgaaccct aagatctatc gaataaagaa tgaaactgtc 960
tttagcattg aactttccga atacaactat gatcgttacg cgcccttcga ggttccaact 1020
ggcgatgccg tccagctcga gtttaccatg ctgtctccct tccatcgcct gaacttggaa 1080
cccgtccgtc gaacagataa cagtacagtt tacagcacac gattcaccac ccccgatcag 1140
catggaatct tctccttccg agtgaactac aagcgcccgt tcctcacgaa catcgaagaa 1200
aaacttgagg tgaccgttcg tcatttcgct cataacgagt acccccgaag ctggaaaatc 1260
agcggtggat gggtctggat tgcgggtctg tggtccgtca tcgctggctt cttagtattc 1320
gttgttgcat ggctttactc agcgccttct gccgccgcac tgaacacaaa gaagacacaa 1380

<210> 15
<211> 460
<212> PRT
<213> Aspergillus fumigatus

<400> 15
Met Arg Trp Cys Leu Thr Leu Leu Ala Phe Cys Phe Leu Ala Val Val
1               5                   10                  15

Arg Ala Leu Ser Ser Ser Gly Ser Arg Leu Leu Val Val Leu Glu Asp
            20                  25                  30

Ala Thr Glu Lys Glu Leu Tyr Ser Lys Leu Trp Ala Asp Leu Glu Gly
        35                  40                  45

Tyr Asn Leu Asp Phe Glu Ser Pro Lys Asn Asp Lys Leu Ser Leu Phe
    50                  55                  60

Glu Leu Gly Asp Arg Val Tyr Asp His Met Leu Leu Leu Pro Pro Lys
65                  70                  75                  80

Ser Lys Gly Tyr Gly Pro Ser Leu Thr Pro Lys Asn Ile Ile Asp Phe
                85                  90                  95

Met Asn Lys Asp Gly Asn Val Leu Leu Ala Leu Ser Gly Lys Ser Thr
            100                 105                 110

Thr Ala Ser Ala Ile Ser Ser Leu Leu Leu Glu Leu Asp Leu His Leu
        115                 120                 125

Pro Val Asp Arg Ser Ser Val Thr Val Asp His Phe Asn Tyr Asp Thr
    130                 135                 140

Leu Ser Ala Ser Asp Lys His Asp Val Leu Leu Leu His Arg Pro Gly
145                 150                 155                 160

Lys Leu Arg Ser Asp Thr Lys Ala Phe Phe Asp Gly Glu Gly Val Val
                165                 170                 175

Ala Phe Pro Arg Ala Val Pro His Thr Leu Gly Asp Ala Asn Pro Leu
            180                 185                 190

Ile Ala Pro Ile Leu Arg Ala Pro Ala Thr Ala Tyr Ser Tyr Asn Pro
        195                 200                 205

Lys Glu Asp Ala Ser Ser Val Glu Asp Val Ala Ala Thr Gly Ser Gln
    210                 215                 220

Leu Ala Leu Val Ser Ala Met Gln Ala Arg Asn Ser Ala Arg Phe Thr
225                 230                 235                 240

Leu Leu Gly Ser Val Glu Ser Leu Gln Asp Gln Trp Phe Ser Ala Thr
                245                 250                 255

Val Lys Ala Pro Gly Asp Gly Lys Gln Met Lys Thr Val Asn Gln Glu
            260                 265                 270

Phe Ala Lys Gln Leu Thr Ala Trp Thr Phe Lys Glu Thr Gly Val Leu
        275                 280                 285

Lys Val Gly Lys Ile Glu His His Leu Ala Glu Asp Gly Glu Ile Thr
    290                 295                 300

Pro Glu Lys Leu Asn Pro Lys Ile Tyr Arg Ile Lys Asn Glu Thr Val
305                 310                 315                 320

Phe Ser Ile Glu Leu Ser Glu Tyr Asn Tyr Asp Arg Tyr Ala Pro Phe
                325                 330                 335

Glu Val Pro Thr Gly Asp Ala Val Gln Leu Glu Phe Thr Met Leu Ser
            340                 345                 350

Pro Phe His Arg Leu Asn Leu Glu Pro Val Arg Arg Thr Asp Asn Ser
        355                 360                 365

Thr Val Tyr Ser Thr Arg Phe Thr Thr Pro Asp Gln His Gly Ile Phe
    370                 375                 380

Ser Phe Arg Val Asn Tyr Lys Arg Pro Phe Leu Thr Asn Ile Glu Glu
385                 390                 395                 400

Lys Leu Glu Val Thr Val Arg His Phe Ala His Asn Glu Tyr Pro Arg
                405                 410                 415

Ser Trp Lys Ile Ser Gly Gly Trp Val Trp Ile Ala Gly Leu Trp Ser
            420                 425                 430

Val Ile Ala Gly Phe Leu Val Phe Val Val Ala Trp Leu Tyr Ser Ala
        435                 440                 445

Pro Ser Ala Ala Ala Leu Asn Thr Lys Lys Thr Gln
    450                 455                 460

<210> 16
<211> 2376
<212> DNA
<213> Aspergillus fumigatus

<400> 16
atgtctcagt atcagcttac tgtggccacc agggccaatc agccctatgt acttcctgtc 60
ctactggtcg caacttccat caacgaggca cgaccaagcc cagtgatatc gatcacctat 120
gaggatactg cggttcttcg tgaaggagac aaggccgtcg tgcaatacac tggagctagc 180
ggtaatccta tctttggcct tatcaatgct gttcaggaac tccgcaaaga cttccccttc 240
cttaacagca aggatgagaa gctggtaaga ggcgccatgg agccttactg ctgatgagca 300
ctgataagtg atactaaccc tccttttata ggagaatgaa tggctgtctc agttggaagc 360
atttgctcct ctagatttca aggcccttga ccctgaattg cagcgcctcg atacccacct 420

cctgctgaga tctttcgtcg tcggttacgc tctctcgacg gccgacattg ccctttgggg 480
tgccatccga ggcaaccgtg tcgcagttgc cgcgatcaag aagggctcac ttgtcaatgt 540
gactcgttgg ttctatttct tggaggatct gtgcccgtgg gccacatcta cactggaggt 600
cttgaaccag gctgtgcgag agaagaaggc cgccaaggcg aaggagggag ctagctacga 660
catcgctctt ctcaacactg aaaaaggcgt ggtgacaagg tttcctcccg agccttcagg 720
ttatcttcac atcggtcacg caaaagctgc gctgctcaac gactactttg cccacgagaa 780
gtataatggc acccttcttg tccgctttga cgacacaaat ccttcgaacg agaagctcga 840
gttccaggac gcgatcattg aagatcttgc tctcatgggc atcaagcccg acaagatgag 900
ctacaccagt gactactttg acgagcttta ccagtacgcc cttcaaatca tcaaggacgg 960
taacgcctac gccgacgata ccgagaagga ggtcatggct gagcagagaa tgaatggaaa 1020
acccagcaag cgtcgtgatg catccgtcga ggagaacctt gcccgcttcg aggagatgaa 1080
gaagggtacc cctgagggtc tccgttggtg tatccgagcc aagatgtctg tcgataaccc 1140
caacaaggcc atgcgtgatc ctgtcattta ccgctgcaac cctgcccctc accaccgcac 1200
tgggacgaag tggaagatct atcctaccta tgacttcgcc tgccctatcg tcgattcaat 1260
tgagggtgtg actcatgccc tcagaaccat tgaataccgc gatcgcaacc ctcagtacca 1320
gtggttcttg gacacgctca agcttcgcca tgtccaaatc tgggattttg ctcgcatgaa 1380
cttcattcgc accttgctgt ctaagagaaa acttaccaag ctcgttaacc aaggtgtcgt 1440
ctggggatgg gatgagtaag tttacctttg cttgcaaacg gattcttgtc ttactaacga 1500
tgtcagtcct cgtttcccca ccatccgagt aagtaacatg cctagtcatg cctgcgaatc 1560
cccttattca tccggcattt tttaccatct cacctacttc ccatgtactg ttaacttccc 1620
acgctaatcc tttcataggg catccgacga aggggaatga ctatccctgc tctgagagaa 1680
ttcattctta agcagggacc cagcaagaac atcaccaacc ttgactggac cctgatctgg 1740
gcgaccaaca agaagtacat tgatcctgtc gcacctcgtc acactgccat tctcaagaag 1800
gatatggtca aggcgatcgt caagggaggc ccggctacac cttacacgga agagaaacct 1860
aagcacggca agaaccctgc agttggtatg aagaaggtgg tttttggtaa cacggtcatt 1920
ttcgaccaga aagatgccaa gagcttcaag caagatgaag agatcacctt gatgagctgg 1980
ggtaatgcca ttgtccgtaa gatcgagacc gatcctacct caggcatcgt caaggagctg 2040
gagctggagc tccacctgga aggtgacttc aaaaagaccg agaagaaggt cacgtggctc 2100
tctactgagg gacaggacct aattcccgtt gaattggtcg atttcgacta tctcctcaac 2160
aaggacaccc tgcaggagga cgacgtcctt gaggatgtcc tgaacaagaa caccgagttc 2220
agagaggacg ctgttgctga ctgcaacgtc gctgaactga aagaaggtga catcatccag 2280
tttgagcgca agggctatta ccgtgttgac cgggcctatg taccgggcaa gccggctgtt 2340
ttgttcaaca ttcccacggg caagacgggc aaatag 2376

<210> 17
<211> 2145
<212> DNA
<213> Aspergillus fumigatus

<400> 17
atgtctcagt atcagcttac tgtggccacc agggccaatc agccctatgt acttcctgtc 60
ctactggtcg caacttccat caacgaggca cgaccaagcc cagtgatatc gatcacctat 120
gaggatactg cggttcttcg tgaaggagac aaggccgtcg tgcaatacac tggagctagc 180
ggtaatccta tctttggcct tatcaatgct gttcaggaac tccgcaaaga cttccccttc 240
cttaacagca aggatgagaa gctggagaat gaatggctgt ctcagttgga agcatttgct 300
cctctagatt tcaaggccct tgaccctgaa ttgcagcgcc tcgataccca cctcctgctg 360
agatctttcg tcgtcggtta cgctctctcg acggccgaca ttgccctttg gggtgccatc 420
cgaggcaacc gtgtcgcagt tgccgcgatc aagaagggct cacttgtcaa tgtgactcgt 480
tggttctatt tcttggagga tctgtgcccg tgggccacat ctacactgga ggtcttgaac 540
caggctgtgc gagagaagaa ggccgccaag gcgaaggagg gagctagcta cgacatcgct 600
cttctcaaca ctgaaaaagg cgtggtgaca aggtttcctc ccgagccttc aggttatctt 660
cacatcggtc acgcaaaagc tgcgctgctc aacgactact ttgcccacga gaagtataat 720
ggcacccttc ttgtccgctt tgacgacaca aatccttcga acgagaagct cgagttccag 780
gacgcgatca ttgaagatct tgctctcatg ggcatcaagc ccgacaagat gagctacacc 840
agtgactact ttgacgagct ttaccagtac gcccttcaaa tcatcaagga cggtaacgcc 900
tacgccgacg ataccgagaa ggaggtcatg gctgagcaga gaatgaatgg aaaacccagc 960
aagcgtcgtg atgcatccgt cgaggagaac cttgcccgct tcgaggagat gaagaagggt 1020
acccctgagg gtctccgttg gtgtatccga gccaagatgt ctgtcgataa ccccaacaag 1080
gccatgcgtg atcctgtcat ttaccgctgc aaccctgccc ctcaccaccg cactgggacg 1140
aagtggaaga tctatcctac ctatgacttc gcctgcccta tcgtcgattc aattgagggt 1200

gtgactcatg ccctcagaac cattgaatac cgcgatcgca accctcagta ccagtggttc 1260
ttggacacgc tcaagcttcg ccatgtccaa atctgggatt ttgctcgcat gaacttcatt 1320
cgcaccttgc tgtctaagag aaaacttacc aagctcgtta accaaggtgt cgtctgggga 1380
tgggatgatc ctcgtttccc caccatccga ggcatccgac gaaggggaat gactatccct 1440
gctctgagag aattcattct taagcaggga cccagcaaga acatcaccaa ccttgactgg 1500
accctgatct gggcgaccaa caagaagtac attgatcctg tcgcacctcg tcacactgcc 1560
attctcaaga aggatatggt caaggcgatc gtcaagggag gcccggctac accttacacg 1620
gaagagaaac ctaagcacgg caagaaccct gcagttggta tgaagaaggt ggtttttggt 1680
aacacggtca ttttcgacca gaaagatgcc aagagcttca agcaagatga agagatcacc 1740
ttgatgagct ggggtaatgc cattgtccgt aagatcgaga ccgatcctac ctcaggcatc 1800
gtcaaggagc tggagctgga gctccacctg gaaggtgact tcaaaaagac cgagaagaag 1860
gtcacgtggc tctctactga gggacaggac ctaattcccg ttgaattggt cgatttcgac 1920
tatctcctca acaaggacac cctgcaggag gacgacgtcc ttgaggatgt cctgaacaag 1980
aacaccgagt tcagagagga cgctgttgct gactgcaacg tcgctgaact gaaagaaggt 2040
gacatcatcc agtttgagcg caaggctatt taccgtgttg accgggccta tgtaccgggc 2100
aagccggctg ttttgttcaa cattcccacg ggcaagacgg gcaaa 2145

<210> 18
<211> 715
<212> PRT
<213> Aspergillus fumigatus

<400> 18
Met Ser Gln Tyr Gln Leu Thr Val Ala Thr Arg Ala Asn Gln Pro Tyr
1               5                   10                  15

Val Leu Pro Val Leu Leu Val Ala Thr Ser Ile Asn Glu Ala Arg Pro
            20                  25                  30

Ser Pro Val Ile Ser Ile Thr Tyr Glu Asp Thr Ala Val Leu Arg Glu
        35                  40                  45

Gly Asp Lys Ala Val Val Gln Tyr Thr Gly Ala Ser Gly Asn Pro Ile
    50                  55                  60

Phe Gly Leu Ile Asn Ala Val Gln Glu Leu Arg Lys Asp Phe Pro Phe
65                  70                  75                  80

Leu Asn Ser Lys Asp Glu Lys Leu Glu Asn Glu Trp Leu Ser Gln Leu
                85                  90                  95

Glu Ala Phe Ala Pro Leu Asp Phe Lys Ala Leu Asp Pro Glu Leu Gln
            100                 105                 110

Arg Leu Asp Thr His Leu Leu Leu Arg Ser Phe Val Val Gly Tyr Ala
        115                 120                 125

Leu Ser Thr Ala Asp Ile Ala Leu Trp Gly Ala Ile Arg Gly Asn Arg
    130                 135                 140

Val Ala Val Ala Ala Ile Lys Lys Gly Ser Leu Val Asn Val Thr Arg
145                 150                 155                 160

Trp Phe Tyr Phe Leu Glu Asp Leu Cys Pro Trp Ala Thr Ser Thr Leu
                165                 170                 175

Glu Val Leu Asn Gln Ala Val Arg Glu Lys Lys Ala Ala Lys Ala Lys
            180                 185                 190

Glu Gly Ala Ser Tyr Asp Ile Ala Leu Leu Asn Thr Glu Lys Gly Val

195                 200                 205

Val Thr Arg Phe Pro Pro Glu Pro Ser Gly Tyr Leu His Ile Gly His
    210                 215                 220

Ala Lys Ala Ala Leu Leu Asn Asp Tyr Phe Ala His Glu Lys Tyr Asn
225                 230                 235                 240

Gly Thr Leu Leu Val Arg Phe Asp Asp Thr Asn Pro Ser Asn Glu Lys
                245                 250                 255

Leu Glu Phe Gln Asp Ala Ile Ile Glu Asp Leu Ala Leu Met Gly Ile
            260                 265                 270

Lys Pro Asp Lys Met Ser Tyr Thr Ser Asp Tyr Phe Asp Glu Leu Tyr
        275                 280                 285

Gln Tyr Ala Leu Gln Ile Ile Lys Asp Gly Asn Ala Tyr Ala Asp Asp
    290                 295                 300

Thr Glu Lys Glu Val Met Ala Glu Gln Arg Met Asn Gly Lys Pro Ser
305                 310                 315                 320

Lys Arg Arg Asp Ala Ser Val Glu Glu Asn Leu Ala Arg Phe Glu Glu
                325                 330                 335

Met Lys Lys Gly Thr Pro Glu Gly Leu Arg Trp Cys Ile Arg Ala Lys
            340                 345                 350

Met Ser Val Asp Asn Pro Asn Lys Ala Met Arg Asp Pro Val Ile Tyr
        355                 360                 365

Arg Cys Asn Pro Ala Pro His His Arg Thr Gly Thr Lys Trp Lys Ile
    370                 375                 380

Tyr Pro Thr Tyr Asp Phe Ala Cys Pro Ile Val Asp Ser Ile Glu Gly
385                 390                 395                 400

Val Thr His Ala Leu Arg Thr Ile Glu Tyr Arg Asp Arg Asn Pro Gln
                405                 410                 415

Tyr Gln Trp Phe Leu Asp Thr Leu Lys Leu Arg His Val Gln Ile Trp
            420                 425                 430

Asp Phe Ala Arg Met Asn Phe Ile Arg Thr Leu Leu Ser Lys Arg Lys
        435                 440                 445

Leu Thr Lys Leu Val Asn Gln Gly Val Val Trp Gly Trp Asp Asp Pro
    450                 455                 460

Arg Phe Pro Thr Ile Arg Gly Ile Arg Arg Arg Gly Met Thr Ile Pro
465                 470                 475                 480

Ala Leu Arg Glu Phe Ile Leu Lys Gln Gly Pro Ser Lys Asn Ile Thr
                485                 490                 495

Asn Leu Asp Trp Thr Leu Ile Trp Ala Thr Asn Lys Lys Tyr Ile Asp
            500                 505                 510

Pro Val Ala Pro Arg His Thr Ala Ile Leu Lys Lys Asp Met Val Lys
        515                 520                 525

Ala Ile Val Lys Gly Gly Pro Ala Thr Pro Tyr Thr Glu Glu Lys Pro
    530                 535                 540

Lys His Gly Lys Asn Pro Ala Val Gly Met Lys Lys Val Val Phe Gly
545                 550                 555                 560

Asn Thr Val Ile Phe Asp Gln Lys Asp Ala Lys Ser Phe Lys Gln Asp
                565                 570                 575

Glu Glu Ile Thr Leu Met Ser Trp Gly Asn Ala Ile Val Arg Lys Ile
            580                 585                 590

Glu Thr Asp Pro Thr Ser Gly Ile Val Lys Glu Leu Glu Leu Glu Leu
        595                 600                 605

His Leu Glu Gly Asp Phe Lys Lys Thr Glu Lys Lys Val Thr Trp Leu
    610                 615                 620

Ser Thr Glu Gly Gln Asp Leu Ile Pro Val Glu Leu Val Asp Phe Asp
625                 630                 635                 640

Tyr Leu Leu Asn Lys Asp Thr Leu Gln Glu Asp Asp Val Leu Glu Asp
                645                 650                 655

Val Leu Asn Lys Asn Thr Glu Phe Arg Glu Asp Ala Val Ala Asp Cys
            660                 665                 670

Asn Val Ala Glu Leu Lys Glu Gly Asp Ile Ile Gln Phe Glu Arg Lys
        675                 680                 685

Gly Tyr Tyr Arg Val Asp Arg Ala Tyr Val Pro Gly Lys Pro Ala Val
    690                 695                 700

Leu Phe Asn Ile Pro Thr Gly Lys Thr Gly Lys
705                 710                 715

<210> 19
<211> 2639
<212> DNA
<213> Aspergillus fumigatus

<400> 19
atgtcgccat caatatccta catttcaggc cagcttaggc agctaatata ctatcatctc 60
gataacaatt tgtgccgtaa tgcgctgttc ctcgccggtc gtttacatgc ttacgagccc 120
cgaacggcgg aagcgtcgta tttactcgct ctctgccatc ttcagaacgg gcaagtcaaa 180
gccgcatacg attacagcag gaattttgga tcgagaggca cccacctcgg ctgctcctat 240
gtcttcgcgc aagcgtgctt ggacctagga aagtatctgg aaggtatcac agcgttagag 300
cggagtaaag gcctttgggc ttcgaagaac cactggagta agtattccat gcgctgatac 360
ttgcagtcga cctgcattta tttcgttgct ttctgttact gacacatttc caccttgtgc 420
tctagataag cacagtgaga cgcgaagaca acatctgccg gatgcagccg cagtattctg 480
cctgttaggt aaattatggc atgcgcataa ggacatcaac aaagctgtgg aatgctatgt 540
tgaatctctg aagctgaatc ccttcatgtg ggatgcgttc caagggttgt gcgacaccgg 600
taagctttgg agaataaacc taatacatct agccatggct aattgtgttc taccgccaag 660
gagtcaatgt ccgcgtgtca aacatctaca agttgaattc tgaattgctg gccgtattgt 720
cttcatcgcc acaggcggat gctgagccaa tatccgataa gtctgcacac acgaatgggc 780
cactgcaagc gcaggcgaat gttaatccaa gttccgatcc ttttgcctcg actacttctc 840
gcagtgattc aggtaccagc catgggagct ctgccttgtg ggaaaaacta aatggaagca 900
cagtaagcgt ggcgtcatcg ggagtgccag catcaatcgt gcatgaagga gccgaaacgc 960

cgagtggtca aagcagcgga tctgatgagt tccggttagc taacggaatg aacggcgcgg 1020
atgcttcttg ggaccctcct ttagctcctg caaggaaaaa cagaacgatc caggcaataa 1080
gcggcgagta tccaatggac cctcctccca agatgaaacc cactgggatc cgaccaagga 1140
caaggaccag gactgagccg gaggaccaaa tttcagccca gatagaccgg gaggcaacaa 1200
atgcgcccag ggtcggggac cgcaaacgaa ctgtttctgg tcaggtagcg catccaccga 1260
cgtcacaacc cacagaacca ggagcacccc agcggcggag tgtgcgactc ttcaaccaga 1320
ttaaacccac gaccagcaaa ttgtcggcgt ccgcgctggg agtcaaggat gctagagaag 1380
tcaagaaagc gaaagccaca ggtacgaagg ggcgtacgac aaccaccacc atgggacgag 1440
tagtgagtgg cagccgaaaa catgccagcg aacatcatga tgcagatggt aaagacggac 1500
ggtcggtacc gtccgcccac actcacgcca tctccaaagg cgctgctcaa gaaagatcga 1560
aagaaatcga ggcgttgacc tggctgctgg agctattctc gaaacttgct tctggattct 1620
ttgccttgtg tcgctaccga tgcccagagt caatccagat cttcaattcg ctctctcaag 1680
gccaacggga aacaccgtgg gttctcgctc agattggacg agcgtactat gagcaggcta 1740
tgtattccga ggcagaaaag tacttctacc gtgtgaagac catggcaccc tcgcgcttgg 1800
aagacatgga gatctactcg actgtccttt ggcatctgaa gaacgatgtt gagttagcct 1860
atttggcgca tgagttgatg gaaacagacc gcctgtcgcc acaggcgtgg tgcgccatcg 1920
gtaattcgtt ttcccaccag cgagatcatg accaggcctt gaagtgcttt aagcgggcaa 1980
cccagctgga tcctcagttt gcctacgggt ttactcttca agggcacgag tatgttgcca 2040
acgaagaata cgacaaggcg cttgatgcat accgtcacgg tatcagcgcg gatagtcggc 2100
attacaatgc ttggtacgga ctgggcacgg tttatgacaa aatgggcaaa ctggactttg 2160
ccgaacaaca cttccggaat gcggcaagca ttaacccgac caacgcagtt ttgatctgct 2220
gcattggatt ggtactggaa aaaatgaaca accctaaagc ggctctcgtg caatatggtc 2280
gcgcttgttc cttggcacct cattccgtac tcgcgcgatt ccgcaaggcc cgcgcattga 2340
tgaagctcca ggagctcaaa ttagctctgt ctgagttgaa gattctcaaa gacatggctc 2400
cagacgaagc taacgtgcat tatctgttgg gtaagctcta caaaatgctt cacgacaaag 2460
ccaatgccat taagcacttc acaactgctt tgaacttgga tccaaaggta tgcctatcac 2520
ttccattccg tcaaattacc agacaatact aacggattcg gttacaggca gcacaataca 2580
tcaaggatgc catggaatct cttgacgatg acgaggagga tgatgaggac atgagctga 2639

<210> 20
<211> 2427
<212> DNA
<213> Aspergillus fumigatus

<400> 20
atgtcgccat caatatccta catttcaggc cagcttaggc agctaatata ctatcatctc 60
gataacaatt tgtgccgtaa tgcgctgttc ctcgccggtc gtttacatgc ttacgagccc 120
cgaacggcgg aagcgtcgta tttactcgct ctctgccatc ttcagaacgg gcaagtcaaa 180
gccgcatacg attacagcag gaattttgga tcgagaggca cccacctcgg ctgctcctat 240
gtcttcgcgc aagcgtgctt ggacctagga aagtatctgg aaggtatcac agcgttagag 300
cggagtaaag gcctttgggc ttcgaagaac cactggaata agcacagtga gacgcgaaga 360
caacatctgc cggatgcagc cgcagtattc tgcctgttag gtaaattatg gcatgcgcat 420
aaggacatca acaaagctgt ggaatgctat gttgaatctc tgaagctgaa tcccttcatg 480
tgggatgcgt tccaagggtt gtgcgacacc ggagtcaatg tccgcgtgtc aaacatctac 540
aagttgaatt ctgaattgct ggccgtattg tcttcatcgc cacaggcgga tgctgagcca 600
atatccgata agtctgcaca cacgaatggg ccactgcaag cgcaggcgaa tgttaatcca 660
agttccgatc cttttgcctc gactacttct cgcagtgatt caggtaccag ccatgggagc 720
tctgccttgt gggaaaaact aaatggaagc acagtaagcg tggcgtcatc gggagtgcca 780
gcatcaatcg tgcatgaagg agccgaaacg ccgagtggtc aaagcagcgg atctgatgag 840
ttccggttag ctaacggaat gaacggcgcg gatgcttctt gggaccctcc tttagctcct 900
gcaaggaaaa acagaacgat ccaggcaata agcggcgagt atccaatgga ccctcctccc 960
aagatgaaac ccactgggat ccgaccaagg acaaggacca ggactgagcc ggaggaccaa 1020
atttcagccc agatagaccg ggaggcaaca aatgcgccca gggtcgggga ccgcaaacga 1080
actgtttctg gtcaggtagc gcatccaccg acgtcacaac ccacagaacc aggagcaccc 1140
cagcggcgga gtgtgcgact cttcaaccag attaaaccca cgaccagcaa attgtcggcg 1200
tccgcgctgg gagtcaagga tgctagagaa gtcaagaaag cgaaagccac aggtacgaag 1260
gggcgtacga caaccaccac catgggacga gtagtgagtg gcagccgaaa acatgccagc 1320
gaacatcatg atgcagatgg taaagacgga cggtcggtac cgtccgccca cactcacgcc 1380
atctccaaag gcgctgctca agaaagatcg aaagaaatcg aggcgttgac ctggctgctg 1440
gagctattct cgaaacttgc ttctggattc tttgccttgt gtcgctaccg atgcccagag 1500

tcaatccaga tcttcaattc gctctctcaa ggccaacggg aaacaccgtg ggttctcgct 1560
cagattggac gagcgtacta tgagcaggct atgtattccg aggcagaaaa gtacttctac 1620
cgtgtgaaga ccatggcacc ctcgcgcttg gaagacatgg agatctactc gactgtcctt 1680
tggcatctga agaacgatgt tgagttagcc tatttggcgc atgagttgat ggaaacagac 1740
cgcctgtcgc cacaggcgtg gtgcgccatc ggtaattcgt tttcccacca gcgagatcat 1800
gaccaggcct tgaagtgctt taagcgggca acccagctgg atcctcagtt tgcctacggg 1860
tttactcttc aagggcacga gtatgttgcc aacgaagaat acgacaaggc gcttgatgca 1920
taccgtcacg gtatcagcgc ggatagtcgg cattacaatg cttggtacgg actgggcacg 1980
gtttatgaca aaatgggcaa actggacttt gccgaacaac acttccggaa tgcggcaagc 2040
attaacccga ccaacgcagt tttgatctgc tgcattggat tggtactgga aaaaatgaac 2100
aaccctaaag cggctctcgt gcaatatggt cgcgcttgtt ccttggcacc tcattccgta 2160
ctcgcgcgat tccgcaaggc ccgcgcattg atgaagctcc aggagctcaa attagctctg 2220
tctgagttga agattctcaa agacatggct ccagacgaag ctaacgtgca ttatctgttg 2280
ggtaagctct acaaaatgct tcacgacaaa gccaatgcca ttaagcactt cacaactgct 2340
ttgaacttgg atccaaaggc agcacaatac atcaaggatg ccatggaatc tcttgacgat 2400
gacgaggagg atgatgagga catgagc 2427

<210> 21
<211> 809
<212> PRT
<213> Aspergillus fumigatus

<400> 21
Met Ser Pro Ser Ile Ser Tyr Ile Ser Gly Gln Leu Arg Gln Leu Ile
1               5                   10                  15

Tyr Tyr His Leu Asp Asn Asn Leu Cys Arg Asn Ala Leu Phe Leu Ala
            20                  25                  30

Gly Arg Leu His Ala Tyr Glu Pro Arg Thr Ala Glu Ala Ser Tyr Leu
        35                  40                  45

Leu Ala Leu Cys His Leu Gln Asn Gly Gln Val Lys Ala Ala Tyr Asp
    50                  55                  60

Tyr Ser Arg Asn Phe Gly Ser Arg Gly Thr His Leu Gly Cys Ser Tyr
65                  70                  75                  80

Val Phe Ala Gln Ala Cys Leu Asp Leu Gly Lys Tyr Leu Glu Gly Ile
                85                  90                  95

Thr Ala Leu Glu Arg Ser Lys Gly Leu Trp Ala Ser Lys Asn His Trp
            100                 105                 110

Asn Lys His Ser Glu Thr Arg Arg Gln His Leu Pro Asp Ala Ala Ala
        115                 120                 125

Val Phe Cys Leu Leu Gly Lys Leu Trp His Ala His Lys Asp Ile Asn
    130                 135                 140

Lys Ala Val Glu Cys Tyr Val Glu Ser Leu Lys Leu Asn Pro Phe Met
145                 150                 155                 160

Trp Asp Ala Phe Gln Gly Leu Cys Asp Thr Gly Val Asn Val Arg Val
                165                 170                 175

Ser Asn Ile Tyr Lys Leu Asn Ser Glu Leu Leu Ala Val Leu Ser Ser
            180                 185                 190

Ser Pro Gln Ala Asp Ala Glu Pro Ile Ser Asp Lys Ser Ala His Thr

        195                 200                 205

Asn Gly Pro Leu Gln Ala Gln Ala Asn Val Asn Pro Ser Ser Asp Pro
    210                 215                 220

Phe Ala Ser Thr Thr Ser Arg Ser Asp Ser Gly Thr Ser His Gly Ser
225                 230                 235                 240

Ser Ala Leu Trp Glu Lys Leu Asn Gly Ser Thr Val Ser Val Ala Ser
                245                 250                 255

Ser Gly Val Pro Ala Ser Ile Val His Glu Gly Ala Glu Thr Pro Ser
            260                 265                 270

Gly Gln Ser Ser Gly Ser Asp Glu Phe Arg Leu Ala Asn Gly Met Asn
        275                 280                 285

Gly Ala Asp Ala Ser Trp Asp Pro Pro Leu Ala Pro Ala Arg Lys Asn
    290                 295                 300

Arg Thr Ile Gln Ala Ile Ser Gly Glu Tyr Pro Met Asp Pro Pro Pro
305                 310                 315                 320

Lys Met Lys Pro Thr Gly Ile Arg Pro Arg Thr Arg Thr Arg Thr Glu
                325                 330                 335

Pro Glu Asp Gln Ile Ser Ala Gln Ile Asp Arg Glu Ala Thr Asn Ala
            340                 345                 350

Pro Arg Val Gly Asp Arg Lys Arg Thr Val Ser Gly Gln Val Ala His
        355                 360                 365

Pro Pro Thr Ser Gln Pro Thr Glu Pro Gly Ala Pro Gln Arg Arg Ser
    370                 375                 380

Val Arg Leu Phe Asn Gln Ile Lys Pro Thr Thr Ser Lys Leu Ser Ala
385                 390                 395                 400

Ser Ala Leu Gly Val Lys Asp Ala Arg Glu Val Lys Lys Ala Lys Ala
                405                 410                 415

Thr Gly Thr Lys Gly Arg Thr Thr Thr Thr Thr Met Gly Arg Val Val
            420                 425                 430

Ser Gly Ser Arg Lys His Ala Ser Glu His His Asp Ala Asp Gly Lys
        435                 440                 445

Asp Gly Arg Ser Val Pro Ser Ala His Thr His Ala Ile Ser Lys Gly
    450                 455                 460

Ala Ala Gln Glu Arg Ser Lys Glu Ile Glu Ala Leu Thr Trp Leu Leu
465                 470                 475                 480

Glu Leu Phe Ser Lys Leu Ala Ser Gly Phe Phe Ala Leu Cys Arg Tyr
                485                 490                 495

Arg Cys Pro Glu Ser Ile Gln Ile Phe Asn Ser Leu Ser Gln Gly Gln
            500                 505                 510

Arg Glu Thr Pro Trp Val Leu Ala Gln Ile Gly Arg Ala Tyr Tyr Glu
        515                 520                 525

Gln Ala Met Tyr Ser Glu Ala Glu Lys Tyr Phe Tyr Arg Val Lys Thr
    530                 535                 540

Met Ala Pro Ser Arg Leu Glu Asp Met Glu Ile Tyr Ser Thr Val Leu
545                 550                 555                 560

Trp His Leu Lys Asn Asp Val Glu Leu Ala Tyr Leu Ala His Glu Leu
                565                 570                 575

Met Glu Thr Asp Arg Leu Ser Pro Gln Ala Trp Cys Ala Ile Gly Asn
            580                 585                 590

Ser Phe Ser His Gln Arg Asp His Asp Gln Ala Leu Lys Cys Phe Lys
        595                 600                 605

Arg Ala Thr Gln Leu Asp Pro Gln Phe Ala Tyr Gly Phe Thr Leu Gln
    610                 615                 620

Gly His Glu Tyr Val Ala Asn Glu Glu Tyr Asp Lys Ala Leu Asp Ala
625                 630                 635                 640

Tyr Arg His Gly Ile Ser Ala Asp Ser Arg His Tyr Asn Ala Trp Tyr
                645                 650                 655

Gly Leu Gly Thr Val Tyr Asp Lys Met Gly Lys Leu Asp Phe Ala Glu
            660                 665                 670

Gln His Phe Arg Asn Ala Ala Ser Ile Asn Pro Thr Asn Ala Val Leu
        675                 680                 685

Ile Cys Cys Ile Gly Leu Val Leu Glu Lys Met Asn Asn Pro Lys Ala
    690                 695                 700

Ala Leu Val Gln Tyr Gly Arg Ala Cys Ser Leu Ala Pro His Ser Val
705                 710                 715                 720

Leu Ala Arg Phe Arg Lys Ala Arg Ala Leu Met Lys Leu Gln Glu Leu
                725                 730                 735

Lys Leu Ala Leu Ser Glu Leu Lys Ile Leu Lys Asp Met Ala Pro Asp
            740                 745                 750

Glu Ala Asn Val His Tyr Leu Leu Gly Lys Leu Tyr Lys Met Leu His
        755                 760                 765

Asp Lys Ala Asn Ala Ile Lys His Phe Thr Thr Ala Leu Asn Leu Asp
    770                 775                 780

Pro Lys Ala Ala Gln Tyr Ile Lys Asp Ala Met Glu Ser Leu Asp Asp
785                 790                 795                 800

Asp Glu Glu Asp Asp Glu Asp Met Ser
                805

<210> 22
<211> 1836
<212> DNA
<213> Aspergillus fumigatus

<400> 22
gcccactacg gtttacaatc gggaattccc gatgaggtgg actttgcatt gtatcacctt 60
gttcagattt cgaatcaacg atgggataaa ttcaagtttg agggtttccc cttgcttgcg 120
gagaacctca tggcaaaggc cctggatatc tcccttgtca caaccggggt gaagtgggag 180
cttcagtatg atgttcttca actcagtgat cgcgtcaatg agctgaactc gctacatggc 240
acacgagatc tgttggagaa gatcaaacaa atgccagtta cattgccgga agacaccctc 300
gagacgtacg aattcaacca ccttctgcgc aacgttaaag aagcgaccct ggtactacgc 360
aatatggtcc ttctgaaaga gaatgcctac tatgtgtcac ggtacgcgaa aggcctgctc 420
cgagacttcc tcgtcattat gatcaacttg cccaatcagc ctcgtctcaa cgagatcaag 480
aacgacgctt tggacattgc agaggaggtc accaagttta tgaagaccga tccggaagat 540
ccactttgga tctcacttct caattgtctc gggtcgtcag atcgtgctca cgtggtccgc 600
gcactctggg ctctcaccca tttctccact gaattagacg agccagaggc gaaccgggca 660
atggaacgga taccaaaaga gactttgcag cagctctact ttcacactct tctcgacttg 720
gacaaagata ttctcagtgg tgcattggac ttctggtacc agtatacact ttcgtccgag 780
aacattgaga ctttgattga ggtcttcaac ttgcctaccg tcttcgtccc ccggatggtc 840
gcactgttaa cgcacgaagg ccgaccgaac aagaaggaaa ctgtgttgca agaagaaaag 900
gtggcccccc caccgtcgga tatccctcgt gtaccgcccg agctcatgaa agagctgatg 960
gagctttcgg agcctgaacg gagctcgcgt tggctccggt gctgctttgt ggaggacctc 1020
gagtgcgaga tcacccaaat tgccttgtgg caggcgtatc agagcagatt tgcagaccct 1080
cgccttcctg gtggcggtgt tctccctgcg gctgaattta tcaaaaatgt cagtacgact 1140
ttcacgaacg cgcaagcaca ggtgatcaat ggccctggtg cagccacgaa attcatcatc 1200
aaaggcattc ggcccctgga gaccgcctat accttcgagg gctttcccta catttactgc 1260
aagtgggcgg acaactcgaa gccaagcaag acgtgtcagc gtgctttcaa gtcgccggca 1320
gaacttcgcc atcacgtctt cacggaacac atgaacctca agcctactga aacgccggga 1380
cactataacc tggagaaggc ggagtcgccc gttcatacct gcctttggga caactgcacg 1440
aaattccggt cgtctggtcc gagtgccaat actgcaatgg tcgctgggca cgtctccgca 1500
catctgcccg aggaacgtgc gccagatgcg gagccgccga catccaaacg tgcggttctc 1560
caagagcgca tcgtccgcaa atggtactac ctggacactc cagtcaatga gcgaggcgag 1620
ccgtttggag tggcctacaa ggcggcgtta gtattgcgta accttgcccg aaacctgcct 1680
acgggtattg caccgcagta caacggcttt tcatggaaga aagccgtctt ccttagtcat 1740
cgtccaaaga tcatcgaagc atgggaccgc aaccgctcat tgcgcaagga acttaccgag 1800
ttgatcatgg taatagaaaa agaggattat tactga 1836

<210> 23
<211> 1836
<212> DNA
<213> Aspergillus fumigatus

<400> 23
gcccactacg gtttacaatc gggaattccc gatgaggtgg actttgcatt gtatcacctt 60
gttcagattt cgaatcaacg atgggataaa ttcaagtttg agggtttccc cttgcttgcg 120
gagaacctca tggcaaaggc cctggatatc tcccttgtca caaccggggt gaagtgggag 180
cttcagtatg atgttcttca actcagtgat cgcgtcaatg agctgaactc gctacatggc 240
acacgagatc tgttggagaa gatcaaacaa atgccagtta cattgccgga agacaccctc 300
gagacgtacg aattcaacca ccttctgcgc aacgttaaag aagcgaccct ggtactacgc 360
aatatggtcc ttctgaaaga gaatgcctac tatgtgtcac ggtacgcgaa aggcctgctc 420
cgagacttcc tcgtcattat gatcaacttg cccaatcagc ctcgtctcaa cgagatcaag 480
aacgacgctt tggacattgc agaggaggtc accaagttta tgaagaccga tccggaagat 540
ccactttgga tctcacttct caattgtctc gggtcgtcag atcgtgctca cgtggtccgc 600
gcactctggg ctctcaccca tttctccact gaattagacg agccagaggc gaaccgggca 660
atggaacgga taccaaaaga gactttgcag cagctctact ttcacactct tctcgacttg 720
gacaaagata ttctcagtgg tgcattggac ttctggtacc agtatacact ttcgtccgag 780
aacattgaga ctttgattga ggtcttcaac ttgcctaccg tcttcgtccc ccggatggtc 840
gcactgttaa cgcacgaagg ccgaccgaac aagaaggaaa ctgtgttgca agaagaaaag 900
gtggcccccc caccgtcgga tatccctcgt gtaccgcccg agctcatgaa agagctgatg 960
gagctttcgg agcctgaacg gagctcgcgt tggctccggt gctgctttgt ggaggacctc 1020
gagtgcgaga tcacccaaat tgccttgtgg caggcgtatc agagcagatt tgcagaccct 1080
cgccttcctg gtggcggtgt tctccctgcg gctgaattta tcaaaaatgt cagtacgact 1140
ttcacgaacg cgcaagcaca ggtgatcaat ggccctggtg cagccacgaa attcatcatc 1200

aaaggcattc ggcccctgga gaccgcctat accttcgagg gctttcccta catttactgc 1260
aagtgggcgg acaactcgaa gccaagcaag acgtgtcagc gtgctttcaa gtcgccggca 1320
gaacttcgcc atcacgtctt cacggaacac atgaacctca agcctactga aacgccggga 1380
cactataacc tggagaaggc ggagtcgccc gttcatacct gcctttggga caactgcacg 1440
aaattccggt cgtctggtcc gagtgccaat actgcaatgg tcgctgggca cgtctccgca 1500
catctgcccg aggaacgtgc gccagatgcg gagccgccga catccaaacg tgcggttctc 1560
caagagcgca tcgtccgcaa atggtactac ctggacactc cagtcaatga gcgaggcgag 1620
ccgtttggag tggcctacaa ggcggcgtta gtattgcgta accttgcccg aaacctgcct 1680
acgggtattg caccgcagta caacgggctt tcatggaaga aagccgtctt ccttagtcat 1740
cgtccaaaga tcatcgaagc atgggaccgc aaccgctcat tgcgcaagga acttaccgag 1800
ttgatcatgg taatagaaaa agaggattat tactga 1836

<210> 24
<211> 611
<212> PRT
<213> Aspergillus fumigatus

<400> 24
Ala His Tyr Gly Leu Gln Ser Gly Ile Pro Asp Glu Val Asp Phe Ala
1               5                   10                  15

Leu Tyr His Leu Val Gln Ile Ser Asn Gln Arg Trp Asp Lys Phe Lys
            20                  25                  30

Phe Glu Gly Phe Pro Leu Leu Ala Glu Asn Leu Met Ala Lys Ala Leu
        35                  40                  45

Asp Ile Ser Leu Val Thr Thr Gly Val Lys Trp Glu Leu Gln Tyr Asp
    50                  55                  60

Val Leu Gln Leu Ser Asp Arg Val Asn Glu Leu Asn Ser Leu His Gly
65                  70                  75                  80

Thr Arg Asp Leu Leu Glu Lys Ile Lys Gln Met Pro Val Thr Leu Pro
                85                  90                  95

Glu Asp Thr Leu Glu Thr Tyr Glu Phe Asn His Leu Leu Arg Asn Val
            100                 105                 110

Lys Glu Ala Thr Leu Val Leu Arg Asn Met Val Leu Leu Lys Glu Asn
        115                 120                 125

Ala Tyr Tyr Val Ser Arg Tyr Ala Lys Gly Leu Leu Arg Asp Phe Leu
    130                 135                 140

Val Ile Met Ile Asn Leu Pro Asn Gln Pro Arg Leu Asn Glu Ile Lys
145                 150                 155                 160

Asn Asp Ala Leu Asp Ile Ala Glu Glu Val Thr Lys Phe Met Lys Thr
                165                 170                 175

Asp Pro Glu Asp Pro Leu Trp Ile Ser Leu Leu Asn Cys Leu Gly Ser
            180                 185                 190

Ser Asp Arg Ala His Val Val Arg Ala Leu Trp Ala Leu Thr His Phe
        195                 200                 205

Ser Thr Glu Leu Asp Glu Pro Glu Ala Asn Arg Ala Met Glu Arg Ile
    210                 215                 220

Pro Lys Glu Thr Leu Gln Gln Leu Tyr Phe His Thr Leu Leu Asp Leu
225                 230                 235                 240

Asp Lys Asp Ile Leu Ser Gly Ala Leu Asp Phe Trp Tyr Gln Tyr Thr
                245                 250                 255

Leu Ser Ser Glu Asn Ile Glu Thr Leu Ile Glu Val Phe Asn Leu Pro
            260                 265                 270

Thr Val Phe Val Pro Arg Met Val Ala Leu Leu Thr His Glu Gly Arg
        275                 280                 285

Pro Asn Lys Lys Glu Thr Val Leu Gln Glu Glu Lys Val Ala Pro Pro
    290                 295                 300

Pro Ser Asp Ile Pro Arg Val Pro Pro Glu Leu Met Lys Glu Leu Met
305                 310                 315                 320

Glu Leu Ser Glu Pro Glu Arg Ser Ser Arg Trp Leu Arg Cys Cys Phe
                325                 330                 335

Val Glu Asp Leu Glu Cys Glu Ile Thr Gln Ile Ala Leu Trp Gln Ala
            340                 345                 350

Tyr Gln Ser Arg Phe Ala Asp Pro Arg Leu Pro Gly Gly Gly Val Leu
        355                 360                 365

Pro Ala Ala Glu Phe Ile Lys Asn Val Ser Thr Thr Phe Thr Asn Ala
    370                 375                 380

Gln Ala Gln Val Ile Asn Gly Pro Gly Ala Ala Thr Lys Phe Ile Ile
385                 390                 395                 400

Lys Gly Ile Arg Pro Leu Glu Thr Ala Tyr Thr Phe Glu Gly Phe Pro
                405                 410                 415

Tyr Ile Tyr Cys Lys Trp Ala Asp Asn Ser Lys Pro Ser Lys Thr Cys
            420                 425                 430

Gln Arg Ala Phe Lys Ser Pro Ala Glu Leu Arg His His Val Phe Thr
        435                 440                 445

Glu His Met Asn Leu Lys Pro Thr Glu Thr Pro Gly His Tyr Asn Leu
    450                 455                 460

Glu Lys Ala Glu Ser Pro Val His Thr Cys Leu Trp Asp Asn Cys Thr
465                 470                 475                 480

Lys Phe Arg Ser Ser Gly Pro Ser Ala Asn Thr Ala Met Val Ala Gly
                485                 490                 495

His Val Ser Ala His Leu Pro Glu Glu Arg Ala Pro Asp Ala Glu Pro
            500                 505                 510

Pro Thr Ser Lys Arg Ala Val Leu Gln Glu Arg Ile Val Arg Lys Trp
        515                 520                 525

Tyr Tyr Leu Asp Thr Pro Val Asn Glu Arg Gly Glu Pro Phe Gly Val
    530                 535                 540

Ala Tyr Lys Ala Ala Leu Val Leu Arg Asn Leu Ala Arg Asn Leu Pro

545                 550                 555                 560

Thr Gly Ile Ala Pro Gln Tyr Asn Gly Leu Ser Trp Lys Lys Ala Val
                565                 570                 575

Phe Leu Ser His Arg Pro Lys Ile Ile Glu Ala Trp Asp Arg Asn Arg
            580                 585                 590

Ser Leu Arg Lys Glu Leu Thr Glu Leu Ile Met Val Ile Glu Lys Glu
        595                 600                 605

Asp Tyr Tyr
    610

<210> 25
<211> 1542
<212> DNA
<213> Aspergillus fumigatus

<400> 25
atggtctaca tcggcatccc caagaactac acggcttcgc cgtcttcctt tgccggaact 60
ccgtccttga cgatcaatta cgaggcaacg caggatcttg attctaccaa tgcttttgaa 120
ggtttgttga cgccggtgac acgtgtgaga gcagcgctgg acagaggctg acagtctaca 180
ggtccagaga aactcttgga ggtgtggttc gcgccttccg ctcaggaatt aggtccagcg 240
cagcccgccg gtctgaaggc tgttccggag gagatctgga aggacatgtt ggatctcgtc 300
aattgccagg tcctctcgat tgtttcgtca gaggatgtgg acgcctacct gctctccgag 360
tctagcatgt tcgtttggcc tcacaaactc atcttgaaga cttgtggtac caccactctt 420
ctgtctggtc tcccacgcat tctcgagatt gccgctttgt tcggtggctt ccccaagtct 480
accgcccctt ctcgcggaat ctccgtcgcc gctgcgccct accgcgtctt ctacagccgc 540
aagaacttcc tgttccccga ccgccagcgg ggccctcacc gcagctggag agatgaagtg 600
cggactatgg ataagctctt cctcaacggc agcgcctaca tgattggcaa gatgaatggc 660
gagcactggt acttgtacct gactgaacct cataccatgc tcaccccgcc aacgagcccg 720
ggagccaaga ccgagtttac ggaaacggag accaaggtcc tcagtgtacc ccagggcgct 780
gctctgcaga ctgattcgga ggatgagact ttggaagtct tgatgaccga cttggatgag 840
gagaacgcca agcagttcta cctcgagaat gccactgccg ttgcggagaa ccgttatcgc 900
aactcaaatt cggagaagag tggccatgtt gatgttttca gcaacacttc ctccgatatc 960
agcgattttg actccgacgg aagccaggtt ctgcctccag agttgactac cgagggtcac 1020
gcgctcggaa ccgtggtctc tgaagcctgt ggactttcct ctgtgtatcc taaggagaag 1080
tatcccgatt cgcgcatcga tgcctacctg tttacaccat gcggcttctc cgccaacggc 1140
gtgattccgc ctcctgaggg aaaagctgga acccactact tcacagtaca cgtcactcca 1200
gagccgcact gttcatatgc gtcctttgag accaacgtac cgcactcgca gaacggccag 1260
actaccgctg gaatcatcaa gcaagtggtc gacatcttca agcctggtcg cttcagcgtg 1320
actctcttcg aggccaagcc agcgctgagc caggtcgaag acgagtggaa ggaagccaag 1380
tacctggccg ctcgtcggac cgccaaaatg gaacatgtgg agggatatcg ccgagtggac 1440
cggattgtcc acgacctcga cggctatgag cttgtcttcc gctattatga acgcctggac 1500
tggaaagggg gggcccctcg gctgggagag gagagatctt ga                   1542

<210> 26
<211> 1479
<212> DNA
<213> Aspergillus fumigatus

<400> 26
   atggtctaca tcggcatccc caagaactac acggcttcgc cgtcttcctt tgccggaact 60
   ccgtccttga cgatcaatta cgaggcaacg caggatcttg attctaccaa tgcttttgaa 120
   ggtccagaga aactcttgga ggtgtggttc gcgccttccg ctcaggaatt aggtccagcg 180
   cagcccgccg gtctgaaggc tgttccggag gagatctgga aggacatgtt ggatctcgtc 240
   aattgccagg tcctctcgat tgtttcgtca gaggatgtgg acgcctacct gctctccgag 300
   tctagcatgt tcgtttggcc tcacaaactc atcttgaaga cttgtggtac caccactctt 360
   ctgtctggtc tcccacgcat tctcgagatt gccgctttgt tcggtggctt ccccaagtct 420
   accgcccctt ctcgcggaat ctccgtcgcc gctgcgccct accgcgtctt ctacagccgc 480
   aagaacttcc tgttccccga ccgccagcgg ggccctcacc gcagctggag agatgaagtg 540
   cggactatgg ataagctctt cctcaacggc agcgcctaca tgattggcaa gatgaatggc 600
   gagcactggt acttgtacct gactgaacct cataccatgc tcaccccgcc aacgagcccg 660
   ggagccaaga ccgagtttac ggaaacggag accaaggtcc tcagtgtacc ccagggcgct 720
   gctctgcaga ctgattcgga ggatgagact ttggaagtct tgatgaccga cttggatgag 780
   gagaacgcca agcagttcta cctcgagaat gccactgccg ttgcggagaa ccgttatcgc 840
   aactcaaatt cggagaagag tggccatgtt gatgttttca gcaacacttc ctccgatatc 900
   agcgattttg actccgacgg aagccaggtt ctgcctccag agttgactac cgagggtcac 960
   gcgctcggaa ccgtggtctc tgaagcctgt ggactttcct ctgtgtatcc taaggagaag 1020
   tatcccgatt cgcgcatcga tgcctacctg tttacaccat gcggcttctc cgccaacggc 1080
   gtgattccgc ctcctgaggg aaaagctgga acccactact tcacagtaca cgtcactcca 1140
   gagccgcact gttcatatgc gtcctttgag accaacgtac cgcactcgca gaacggccag 1200
   actaccgctg gaatcatcaa gcaagtggtc gacatcttca agcctggtcg cttcagcgtg 1260
   actctcttcg aggccaagcc agcgctgagc caggtcgaag acgagtggaa ggaagccaag 1320
   tacctggccg ctcgtcggac cgccaaaatg gaacatgtgg agggatatcg ccgagtggac 1380
   cggattgtcc acgacctcga cggctatgag cttgtcttcc gctattatga acgcctggac 1440
   tggaaagggg gggcccctcg gctgggagag gagagatct 1479

<210> 27
<211> 493
<212> PRT
<213> Aspergillus fumigatus

<400> 27
Met Val Tyr Ile Gly Ile Pro Lys Asn Tyr Thr Ala Ser Pro Ser Ser
1               5                   10                  15

Phe Ala Gly Thr Pro Ser Leu Thr Ile Asn Tyr Glu Ala Thr Gln Asp
            20                  25                  30

Leu Asp Ser Thr Asn Ala Phe Glu Gly Pro Glu Lys Leu Leu Glu Val
        35                  40                  45

Trp Phe Ala Pro Ser Ala Gln Glu Leu Gly Pro Ala Gln Pro Ala Gly
    50                  55                  60

Leu Lys Ala Val Pro Glu Glu Ile Trp Lys Asp Met Leu Asp Leu Val
65                  70                  75                  80

Asn Cys Gln Val Leu Ser Ile Val Ser Ser Glu Asp Val Asp Ala Tyr
                85                  90                  95

Leu Leu Ser Glu Ser Ser Met Phe Val Trp Pro His Lys Leu Ile Leu
            100                 105                 110

Lys Thr Cys Gly Thr Thr Thr Leu Leu Ser Gly Leu Pro Arg Ile Leu
        115                 120                 125

Glu Ile Ala Ala Leu Phe Gly Gly Phe Pro Lys Ser Thr Ala Pro Ser
    130                 135                 140

Arg Gly Ile Ser Val Ala Ala Ala Pro Tyr Arg Val Phe Tyr Ser Arg
145                 150                 155                 160

Lys Asn Phe Leu Phe Pro Asp Arg Gln Arg Gly Pro His Arg Ser Trp
                165                 170                 175

Arg Asp Glu Val Arg Thr Met Asp Lys Leu Phe Leu Asn Gly Ser Ala
                180                 185                 190

Tyr Met Ile Gly Lys Met Asn Gly Glu His Trp Tyr Leu Tyr Leu Thr
            195                 200                 205

Glu Pro His Thr Met Leu Thr Pro Pro Thr Ser Pro Gly Ala Lys Thr
    210                 215                 220

Glu Phe Thr Glu Thr Glu Thr Lys Val Leu Ser Val Pro Gln Gly Ala
225                 230                 235                 240

Ala Leu Gln Thr Asp Ser Glu Asp Glu Thr Leu Glu Val Leu Met Thr
                245                 250                 255

Asp Leu Asp Glu Glu Asn Ala Lys Gln Phe Tyr Leu Glu Asn Ala Thr
            260                 265                 270

Ala Val Ala Glu Asn Arg Tyr Arg Asn Ser Asn Ser Glu Lys Ser Gly
        275                 280                 285

His Val Asp Val Phe Ser Asn Thr Ser Ser Asp Ile Ser Asp Phe Asp
    290                 295                 300

Ser Asp Gly Ser Gln Val Leu Pro Pro Glu Leu Thr Thr Glu Gly His
305                 310                 315                 320

Ala Leu Gly Thr Val Val Ser Glu Ala Cys Gly Leu Ser Ser Val Tyr
                325                 330                 335

Pro Lys Glu Lys Tyr Pro Asp Ser Arg Ile Asp Ala Tyr Leu Phe Thr
            340                 345                 350

Pro Cys Gly Phe Ser Ala Asn Gly Val Ile Pro Pro Pro Glu Gly Lys
        355                 360                 365

Ala Gly Thr His Tyr Phe Thr Val His Val Thr Pro Glu Pro His Cys
    370                 375                 380

Ser Tyr Ala Ser Phe Glu Thr Asn Val Pro His Ser Gln Asn Gly Gln
385                 390                 395                 400

Thr Thr Ala Gly Ile Ile Lys Gln Val Val Asp Ile Phe Lys Pro Gly
                405                 410                 415

Arg Phe Ser Val Thr Leu Phe Glu Ala Lys Pro Ala Leu Ser Gln Val
            420                 425                 430

Glu Asp Glu Trp Lys Glu Ala Lys Tyr Leu Ala Ala Arg Arg Thr Ala
        435                 440                 445

Lys Met Glu His Val Glu Gly Tyr Arg Arg Val Asp Arg Ile Val His
    450                 455                 460

Asp Leu Asp Gly Tyr Glu Leu Val Phe Arg Tyr Tyr Glu Arg Leu Asp
465                 470                 475                 480

Trp Lys Gly Gly Ala Pro Arg Leu Gly Glu Glu Arg Ser
                485                 490

<210> 28
<211> 637
<212> DNA
<213> Aspergillus fumigatus

<400> 28
atgggtcgcg ttagaaccaa ggtaagttac agatgaagca tcatgagtta tcttcaaaaa 60
agccccaaaa gagtatcatt tctgacgaaa tgggtttttc ttcaatagac agtcaagagg 120
tccgccaagg tcatcatcga gcgctactac cccaagttga cgctcgactt tgagaccaac 180
aagcgtcttt gcgatgagat cgctatcatt gcctccaagc gccttcgcaa caaggtgggc 240
aatccatcac tgagccgtac aacagtcgga atttgacttg ctgacgaaaa ctagattgct 300
ggttacacca cccaccttat gaagcgtatc cagcgtggcc ctgtccgcgg tatctctttc 360
aagctgcagg aggaggagcg tgagcgcaag gatcagtacg ttcctgaggt ttccgctctg 420
gatgtttccc agaccgagtc cggccagctc gatgtcgatg ccgacaccaa ggaccttctc 480
aagtccatgg gcgtaagttc tgttctcaac gcggttgttc gtggttttaa agcagtccgt 540
taacttatat tgcccactac agttcgacaa tctcaaggtc aacgttgtca acgtctccca 600
acatcaggtt caggagcgcc cccgccgctt ccggtag 637

<210> 29
<211> 417
<212> DNA
<213> Aspergillus fumigatus

<400> 29
atgggtcgcg ttagaaccaa gacagtcaag aggtccgcca aggtcatcat cgagcgctac 60
taccccaagt tgacgctcga ctttgagacc aacaagcgtc tttgcgatga gatcgctatc 120
attgcctcca agcgccttcg caacaagatt gctggttaca ccacccacct tatgaagcgt 180
atccagcgtg gccctgtccg cggtatctct ttcaagctgc aggaggagga gcgtgagcgc 240
aaggatcagt acgttcctga ggtttccgct ctggatgttt cccagaccga gtccggccag 300
ctcgatgtcg atgccgacac caaggacctt ctcaagtcca tgggcttcga caatctcaag 360
gtcaacgttg tcaacgtctc ccaacatcag gttcaggagc gcccccgccg cttccgg 417

<210> 30
<211> 139
<212> PRT
<213> Aspergillus fumigatus

<400> 30
Met Gly Arg Val Arg Thr Lys Thr Val Lys Arg Ser Ala Lys Val Ile
1               5                   10                  15

Ile Glu Arg Tyr Tyr Pro Lys Leu Thr Leu Asp Phe Glu Thr Asn Lys
            20                  25                  30

Arg Leu Cys Asp Glu Ile Ala Ile Ile Ala Ser Lys Arg Leu Arg Asn
        35                  40                  45

Lys Ile Ala Gly Tyr Thr Thr His Leu Met Lys Arg Ile Gln Arg Gly
    50                  55                  60

Pro Val Arg Gly Ile Ser Phe Lys Leu Gln Glu Glu Glu Arg Glu Arg
65                  70                  75                  80

Lys Asp Gln Tyr Val Pro Glu Val Ser Ala Leu Asp Val Ser Gln Thr
                85                  90                  95

Glu Ser Gly Gln Leu Asp Val Asp Ala Asp Thr Lys Asp Leu Leu Lys
            100                 105                 110

Ser Met Gly Phe Asp Asn Leu Lys Val Asn Val Val Asn Val Ser Gln
        115                 120                 125

His Gln Val Gln Glu Arg Pro Arg Arg Phe Arg
    130                 135

<210> 31
<211> 1035
<212> DNA
<213> Aspergillus fumigatus

<400> 31
atggcggttg gaaagtatgc caattcactt ctattattgt tctgaacgct tttagcatgt 60
gtctggatac ggtggtttac aggtactgat ccgggaacag gaacaagcgc ttgtcgaagg 120
gcaagaaggg tgttaagaag aggaccgttg atcctttctc caggaaggac gaatactctg 180
ttaaggtatg tcgacgtgga ctgtgtaagt cgaccgcagc taatctatat caggcgcctt 240
ccactttcca gatcagagag tatgttgcac gcatatgatg tcgaatgcag gataaaggcg 300
attcacaatg gtagtggaga ttatgctgac tgaattatag tgtcgggaag actctggtca 360
accgcaccag tggtctcaag aacgccaatg actccctgaa gggtcgaatt ttcgaggtct 420
cgctggctga cctgcagaat gatgaagacc atgctttccg caaggttaag cttcgtgtgg 480
acgaggttca gggcaagaac tgtttgacca acttccacgg tcttgatttc acaaccgaca 540
aattgcgatc cctcgtgcgc aagtggcagt cgctgatcga agccatgtca ctgtgaagac 600
gaccgatgat tatctccttc ggctttttgc tatcgccttc accaagagac gcccgaacca 660
gattaagaag accacatatg ctcgttcttc tcaaatccgt gccatccgca agaagatgat 720
tgaaatcatg cagagggagg cagccagctg ctctctcgct cagctcactc acaagctcat 780
tcctgaggtc attggtcgtg agatcgagaa ggctacccag ggaatctatc ctttgcagaa 840
tgtgtgtgac cctgttattc ttactcggga tgaagactaa ctgcaatcta ggtccatatt 900
cgcaaggtca agcttcttaa ggctcccaag ttcgacctgg gtgcactgct gaatctgcac 960
ggtgaatcta caaccgatga taagggccac aaggtcgaga gagagttcaa ggagcaggtt 1020
ctcgaaagcg tttaa                                                  1035

<210> 32
<211> 768
<212> DNA
<213> Aspergillus fumigatus

<400> 32
atggcggttg gaaagaacaa gcgcttgtcg aagggcaaga agggtgttaa gaagaggacc 60
gttgatcctt tctccaggaa ggacgaatac tctgttaagg cgccttccac tttccagatc 120
agagatgtcg ggaagactct ggtcaaccgc accagtggtc tcaagaacgc caatgactcc 180
ctgaagggtc gaattttcga ggtctcgctg gctgacctgc agaatgatga agaccatgct 240
ttccgcaagg ttaagcttcg tgtggacgag gttcagggca agaactgttt gaccaacttc 300
cacggtcttg atttcacaac cgacaaattg cgatccctcg tgcgcaagtg gcagtcgctg 360
atcgaagcca atgtcactgt gaagacgacc gatgattatc tccttcggct ttttgctatc 420
gccttcacca agagacgccc gaaccagatt aagaagacca catatgctcg ttcttctcaa 480
atccgtgcca tccgcaagaa gatgattgaa atcatgcaga gggaggcagc cagctgctct 540
ctcgctcagc tcactcacaa gctcattcct gaggtcattg gtcgtgagat cgagaaggct 600
acccagggaa tctatccttt gcagaatgtc catattcgca aggtcaagct tcttaaggct 660
cccaagttcg acctgggtgc actgctgaat ctgcacggtg aatctacaac cgatgataag 720
ggccacaagg tcgagagaga gttcaaggag caggttctcg aaagcgtt              768

<210> 33
<211> 256
<212> PRT
<213> Aspergillus fumigatus

<400> 33
Met Ala Val Gly Lys Asn Lys Arg Leu Ser Lys Gly Lys Lys Gly Val
1               5                   10                  15

Lys Lys Arg Thr Val Asp Pro Phe Ser Arg Lys Asp Glu Tyr Ser Val
            20                  25                  30

Lys Ala Pro Ser Thr Phe Gln Ile Arg Asp Val Gly Lys Thr Leu Val
        35                  40                  45

Asn Arg Thr Ser Gly Leu Lys Asn Ala Asn Asp Ser Leu Lys Gly Arg
    50                  55                  60

Ile Phe Glu Val Ser Leu Ala Asp Leu Gln Asn Asp Glu Asp His Ala
65                  70                  75                  80

Phe Arg Lys Val Lys Leu Arg Val Asp Glu Val Gln Gly Lys Asn Cys
                85                  90                  95

Leu Thr Asn Phe His Gly Leu Asp Phe Thr Thr Asp Lys Leu Arg Ser
            100                 105                 110

Leu Val Arg Lys Trp Gln Ser Leu Ile Glu Ala Asn Val Thr Val Lys
        115                 120                 125

Thr Thr Asp Asp Tyr Leu Leu Arg Leu Phe Ala Ile Ala Phe Thr Lys
    130                 135                 140

Arg Arg Pro Asn Gln Ile Lys Lys Thr Thr Tyr Ala Arg Ser Ser Gln
145                 150                 155                 160

Ile Arg Ala Ile Arg Lys Lys Met Ile Glu Ile Met Gln Arg Glu Ala
                165                 170                 175

Ala Ser Cys Ser Leu Ala Gln Leu Thr His Lys Leu Ile Pro Glu Val
            180                 185                 190

Ile Gly Arg Glu Ile Glu Lys Ala Thr Gln Gly Ile Tyr Pro Leu Gln
        195                 200                 205

Asn Val His Ile Arg Lys Val Lys Leu Leu Lys Ala Pro Lys Phe Asp
    210                 215                 220

Leu Gly Ala Leu Leu Asn Leu His Gly Glu Ser Thr Thr Asp Asp Lys
225                 230                 235                 240

Gly His Lys Val Glu Arg Glu Phe Lys Glu Gln Val Leu Glu Ser Val
                245                 250                 255

<210> 34
<211> 614
<212> DNA
<213> Aspergillus fumigatus

<400> 34
cctgtcggag atgatcaaag gcagcacctc gaattttcga ggaacactgc gaatagtttc 60

aatcatgtat atggacccat tttcccgtca ccagaagcaa ttatatgtaa gtggtttttg 120
cttctggcgg aacggtcctg tgttgggaaa ttgacggcta tcaatagcgc ctgctaaacg 180
ggttatgtcc ctcaaagaac cgacgttgaa aatgtccaag tcccatgccg acagacgctc 240
aaggatcatt cttacggatt cgcccgcaga aatctccaaa aagatcaatg ctgcgctcac 300
agactcggaa ttaaccatta catatgaccc agtccgtcga cctggagtgg cgaatttaat 360
agagatcttg agtcacttcg atggacgaac ttgcgatgag attgccatgg aataccgttc 420
agccagtctt cgcgctctaa aggaacatct ggccagaacg ttgtccaatc atcttgagcc 480
aataagagag aagtatctct cacttgtagg agatcagact gactaccttg attctatagc 540
agaacagggt tctgaagccg cgcgggccaa cgctgaattg acaatggagc aagtcaaagt 600
cgctatgggc ttaa 614

<210> 35
<211> 552
<212> DNA
<213> Aspergillus fumigatus

<400> 35
cctgtcggag atgatcaaag gcagcacctc gaattttcga ggaacactgc gaatagtttc 60
aatcatgtat atggacccat tttcccgtca ccagaagcaa ttatatcgcc tgctaaacgg 120
gttatgtccc tcaaagaacc gacgttgaaa atgtccaagt cccatgccga cagacgctca 180
aggatcattc ttacggattc gcccgcagaa atctccaaaa agatcaatgc tgcgctcaca 240
gactcggaat taaccattac atatgaccca gtccgtcgac ctggagtggc gaatttaata 300
gagatcttga gtcacttcga tggacgaact tgcgatgaga ttgccatgga ataccgttca 360
gccagtcttc gcgctctaaa ggaacatctg gccagaacgt tgtccaatca tcttgagcca 420
ataagagaga agtatctctc acttgtagga gatcagactg actaccttga ttctatagca 480
gaacagggtt ctgaagccgc gcgggccaac gctgaattga caatggagca agtcaaagtc 540
gctatgggct ta 552

<210> 36
<211> 184
<212> PRT
<213> Aspergillus fumigatus

<400> 36
Pro Val Gly Asp Asp Gln Arg Gln His Leu Glu Phe Ser Arg Asn Thr
1               5                   10                  15

Ala Asn Ser Phe Asn His Val Tyr Gly Pro Ile Phe Pro Ser Pro Glu
            20                  25                  30

Ala Ile Ile Ser Pro Ala Lys Arg Val Met Ser Leu Lys Glu Pro Thr
        35                  40                  45

Leu Lys Met Ser Lys Ser His Ala Asp Arg Arg Ser Arg Ile Ile Leu
    50                  55                  60

Thr Asp Ser Pro Ala Glu Ile Ser Lys Lys Ile Asn Ala Ala Leu Thr
65                  70                  75                  80

Asp Ser Glu Leu Thr Ile Thr Tyr Asp Pro Val Arg Arg Pro Gly Val
                85                  90                  95

Ala Asn Leu Ile Glu Ile Leu Ser His Phe Asp Gly Arg Thr Cys Asp
            100                 105                 110

Glu Ile Ala Met Glu Tyr Arg Ser Ala Ser Leu Arg Ala Leu Lys Glu
        115                 120                 125

His Leu Ala Arg Thr Leu Ser Asn His Leu Glu Pro Ile Arg Glu Lys

130                 135                 140

Tyr Leu Ser Leu Val Gly Asp Gln Thr Asp Tyr Leu Asp Ser Ile Ala
145                 150                 155                 160

Glu Gln Gly Ser Glu Ala Ala Arg Ala Asn Ala Glu Leu Thr Met Glu
                165                 170                 175

Gln Val Lys Val Ala Met Gly Leu
            180

<210> 37
<211> 819
<212> DNA
<213> Aspergillus fumigatus

<400> 37
atggcaactt cgactgggac cggatgggct cagctccggc agcaagcccg ttcgcttgag 60
actcaggtac ggaactcgaa actacgctat aatgaggctt tactcgtgat ttggatgttg 120
acaataatgt tcctagaccg agagtctgtt tcacacctat gcgcagtatg catcgatgac 180
gaagctgcct ccgaaaccct cagaagaaga acaacggatt gaatcgcaac tgaaggatct 240
tcttgaaaag gtgtgcactt tgaggccctc tagtccagcc caacagacga tcatgctgac 300
acgatccgat catagcgtga agccctcatc tcccagctct cccgtctcct tgactccgaa 360
gccactctta ccgcatctgc cctgaaacag agcaatcttg cccgcaatcg cgaagtcctc 420
caggatcatc gccgcgaatt gcagcgcctg aacgccgcaa tcgccgagtc ccgcgaccga 480
gccaatcttc tgtctaacgt ccgctccgac attgatgcct accgcaattc aaaccccgcc 540
gcggctgagg cagactacat gctcgaggag cggggtcgta tagatgaaag ccataacatg 600
atagatggtg tcctaagcca ggcgtatgca atcaacgaga gttttgggct acaacgtgaa 660
accctggcca gcatcaatcg ccgtatcgtc ggtgctgcca ataaggtacc aggaatgaat 720
gcattgattg gtaagattgg gacgaagagg agacgtgacg caatcatctt gggggctttc 780
atcggctttt gtttcttgat ggtgttcttc ttccgatga                         819

<210> 38
<211> 681
<212> DNA
<213> Aspergillus fumigatus

<400> 38
atggcaactt cgactgggac cggatgggct cagctccggc agcaagcccg ttcgcttgag 60
actcagaccg agagtctgtt tcacacctat gcgcagtatg catcgatgac gaagctgcct 120
ccgaaaccct cagaagaaga acaacggatt gaatcgcaac tgaaggatct tcttgaaaag 180
cgtgaagccc tcatctccca gctctcccgt ctccttgact ccgaagccac tcttaccgca 240
tctgccctga aacagagcaa tcttgcccgc aatcgcgaag tcctccagga tcatcgccgc 300
gaattgcagc gcctgaacgc cgcaatcgcc gagtcccgcg accgagccaa tcttctgtct 360
aacgtccgct ccgacattga tgcctaccgc aattcaaacc ccgccgcggc tgaggcagac 420
tacatgctcg aggagcgggg tcgtatagat gaaagccata acatgataga tggtgtccta 480
agccaggcgt atgcaatcaa cgagagtttt gggctacaac gtgaaaccct ggccagcatc 540
aatcgccgta tcgtcggtgc tgccaataag gtaccaggaa tgaatgcatt gattggtaag 600
attgggacga agaggagacg tgacgcaatc atcttggggg ctttcatcgg cttttgtttc 660
ttgatggtgt tcttcttccg a                                            681

<210> 39
<211> 227
<212> PRT
<213> Aspergillus fumigatus

<400> 39

Met Ala Thr Ser Thr Gly Thr Gly Trp Ala Gln Leu Arg Gln Gln Ala
1               5                   10                  15

Arg Ser Leu Glu Thr Gln Thr Glu Ser Leu Phe His Thr Tyr Ala Gln
            20                  25                  30

Tyr Ala Ser Met Thr Lys Leu Pro Pro Lys Pro Ser Glu Glu Glu Gln
        35                  40                  45

Arg Ile Glu Ser Gln Leu Lys Asp Leu Leu Glu Lys Arg Glu Ala Leu
    50                  55                  60

Ile Ser Gln Leu Ser Arg Leu Leu Asp Ser Glu Ala Thr Leu Thr Ala
65                  70                  75                  80

Ser Ala Leu Lys Gln Ser Asn Leu Ala Arg Asn Arg Glu Val Leu Gln
                85                  90                  95

Asp His Arg Arg Glu Leu Gln Arg Leu Asn Ala Ala Ile Ala Glu Ser
            100                 105                 110

Arg Asp Arg Ala Asn Leu Leu Ser Asn Val Arg Ser Asp Ile Asp Ala
        115                 120                 125

Tyr Arg Asn Ser Asn Pro Ala Ala Ala Glu Ala Asp Tyr Met Leu Glu
    130                 135                 140

Glu Arg Gly Arg Ile Asp Glu Ser His Asn Met Ile Asp Gly Val Leu
145                 150                 155                 160

Ser Gln Ala Tyr Ala Ile Asn Glu Ser Phe Gly Leu Gln Arg Glu Thr
                165                 170                 175

Leu Ala Ser Ile Asn Arg Arg Ile Val Gly Ala Ala Asn Lys Val Pro
            180                 185                 190

Gly Met Asn Ala Leu Ile Gly Lys Ile Gly Thr Lys Arg Arg Arg Asp
        195                 200                 205

Ala Ile Ile Leu Gly Ala Phe Ile Gly Phe Cys Phe Leu Met Val Phe
    210                 215                 220

Phe Phe Arg
225

<210> 40
<211> 1601
<212> DNA
<213> Aspergillus fumigatus

<400> 40
atgtcacaaa atcgacctgg ggtgttctcg aatctgcgca tgggtggtaa ggaacatcca 60
aatgctgagt ccaattgttc agaaaacatt acccaggagc ctgtggaact aactgctttg 120
ctttccgacc atacagaagt cgtccgcgag aaggtccagg atggactgac aggggaaact 180
aaggagattt cgtactcaca atgtaaaatc gtcggcaatg gatcgtttgg tgtcgtcttt 240
cagacgaaaa tgatgccaag cggcgaggat gctgccatta agagggtcct tcaagacaag 300
cgcttcaaag tatgtgtaca ttataagggc aattgccctc gctgcccaac ccaaagatac 360
tgtcgctgac gagataccag aatcgagaac tgcagattat gcggattgtt cgccatccta 420
acatcgtaga attgaaagcc ttctattact cgaacggcga gagggtatgc gactctcctt 480

tgtctcccca ttcgttctag tttgccgttt gctgactacc ctaccattgt ctttcacaga 540
aggatgaagt gtacctaaac ctcgttctcg aatacgtacc agaaaccgtg tatcgggcgt 600
cgcggtactt taataaactc aaaacgacta tgccaatgtt ggaagtcaag ctgtatatct 660
atcaattgtt ccgttccctg gcatacatcc attcacaagg catctgccac cgtgacatca 720
agccccagaa tctcttactt gatccatcca ccggcatcct caaactctgc gactttggtt 780
cggccaagat tctggtagag aatgagccca acgtttccta tatctgttcc cgctactatc 840
gtgcgccgga attgatcttt ggcgccacta attacacaac aaagatcggt aagtcttgac 900
tgattcctcc ttcaagtttg gtactgtcat gctgacgatc gtcaagacgt gtggtccacg 960
ggttgtgtga tggctgaact catgcttggt cagccattgt tccctggaga gtcgggaatt 1020
gaccaactgg tggaaatcat caaggttctt ggaaccccta ctcgggagca gatccgcacc 1080
atgaacccaa actatatgga gcacaaattc cctcaaatca agccacaccc attcaacaag 1140
gtgaccacgc tcttaaagaa cttcttgcga atatgcactg acttgatgac cccaggtttt 1200
ccggagagct cctcacgagg ccattgatct gatctcagct ttgctagaat acacgccgac 1260
acaacgtctc tccgctatcg aggcgatgtg ccacccgttc ttcgacgaac tcagagatcc 1320
caatacgcga ctgcccgact ctcggcaccc tggtggcgct gctagagacc tccccaatct 1380
ctttgatttc tccagacatg gtttgttgtc acttgaggcc caaattcatt cttccagatg 1440
gcttattcgc tgatcactct tttgtagaac tttctattgc acctgcattg aacagccggc 1500
tggttccccc tcatgcacgc gccgctctcg aggcccgggg gctagacatt gacaacttca 1560
ctcctctcac gaaggaggag atgatggcac gtctcgactg a 1601

<210> 41
<211> 1182
<212> DNA
<213> Aspergillus fumigatus

<400> 41
atgtcacaaa atcgacctgg ggtgttctcg aatctgcgca tgggtgaagt cgtccgcgag 60
aaggtccagg atggactgac aggggaaact aaggagattt cgtactcaca atgtaaaatc 120
gtcggcaatg gatcgtttgg tgtcgtcttt cagacgaaaa tgatgccaag cggcgaggat 180
gctgccatta agagggtcct tcaagacaag cgcttcaaaa atcgagaact gcagattatg 240
cggattgttc gccatcctaa catcgtagaa ttgaaagcct tctattactc gaacggcgag 300
aggaaggatg aagtgtacct aaacctcgtt ctcgaatacg taccagaaac cgtgtatcgg 360
gcgtcgcggt actttaataa actcaaaacg actatgccaa tgttggaagt caagctgtat 420
atctatcaat tgttccgttc cctggcatac atccattcac aaggcatctg ccaccgtgac 480
atcaagcccc agaatctctt acttgatcca tccaccggca tcctcaaact ctgcgacttt 540
ggttcggcca agattctggt agagaatgag cccaacgttt cctatatctg ttcccgctac 600
tatcgtgcgc cggaattgat ctttggcgcc actaattaca caacaaagat cgacgtgtgg 660
tccacgggtt gtgtgatggc tgaactcatg cttggtcagc cattgttccc tggagagtcg 720
ggaattgacc aactggtgga aatcatcaag gttcttggaa cccctactcg ggagcagatc 780
cgcaccatga acccaaacta tatggagcac aaattccctc aaatcaagcc acacccattc 840
aacaaggttt tccggagagc tcctcacgag gccattgatc tgatctcagc tttgctagaa 900
tacacgccga cacaacgtct ctccgctatc gaggcgatgt gccacccgtt cttcgacgaa 960
ctcagagatc ccaatacgcg actgcccgac tctcggcacc ctggtggcgc tgctagagac 1020
ctccccaatc tctttgattt ctccagacat gaactttcta ttgcacctgc attgaacagc 1080
cggctggttc cccctcatgc acgcgccgct ctcgaggccc gggggctaga cattgacaac 1140
ttcactcctc tcacgaagga ggagatgatg gcacgtctcg ac 1182

<210> 42
<211> 394
<212> PRT
<213> Aspergillus fumigatus

<400> 42
Met Ser Gln Asn Arg Pro Gly Val Phe Ser Asn Leu Arg Met Gly Glu
1               5                   10                  15

Val Val Arg Glu Lys Val Gln Asp Gly Leu Thr Gly Glu Thr Lys Glu
            20                  25                  30

Ile Ser Tyr Ser Gln Cys Lys Ile Val Gly Asn Gly Ser Phe Gly Val
        35                  40                  45

Val Phe Gln Thr Lys Met Met Pro Ser Gly Glu Asp Ala Ala Ile Lys
    50                  55                  60

Arg Val Leu Gln Asp Lys Arg Phe Lys Asn Arg Glu Leu Gln Ile Met
65                  70                  75                  80

Arg Ile Val Arg His Pro Asn Ile Val Glu Leu Lys Ala Phe Tyr Tyr
                85                  90                  95

Ser Asn Gly Glu Arg Lys Asp Glu Val Tyr Leu Asn Leu Val Leu Glu
            100                 105                 110

Tyr Val Pro Glu Thr Val Tyr Arg Ala Ser Arg Tyr Phe Asn Lys Leu
        115                 120                 125

Lys Thr Thr Met Pro Met Leu Glu Val Lys Leu Tyr Ile Tyr Gln Leu
    130                 135                 140

Phe Arg Ser Leu Ala Tyr Ile His Ser Gln Gly Ile Cys His Arg Asp
145                 150                 155                 160

Ile Lys Pro Gln Asn Leu Leu Leu Asp Pro Ser Thr Gly Ile Leu Lys
                165                 170                 175

Leu Cys Asp Phe Gly Ser Ala Lys Ile Leu Val Glu Asn Glu Pro Asn
            180                 185                 190

Val Ser Tyr Ile Cys Ser Arg Tyr Tyr Arg Ala Pro Glu Leu Ile Phe
        195                 200                 205

Gly Ala Thr Asn Tyr Thr Thr Lys Ile Asp Val Trp Ser Thr Gly Cys
    210                 215                 220

Val Met Ala Glu Leu Met Leu Gly Gln Pro Leu Phe Pro Gly Glu Ser
225                 230                 235                 240

Gly Ile Asp Gln Leu Val Glu Ile Ile Lys Val Leu Gly Thr Pro Thr
                245                 250                 255

Arg Glu Gln Ile Arg Thr Met Asn Pro Asn Tyr Met Glu His Lys Phe
            260                 265                 270

Pro Gln Ile Lys Pro His Pro Phe Asn Lys Val Phe Arg Arg Ala Pro
        275                 280                 285

His Glu Ala Ile Asp Leu Ile Ser Ala Leu Leu Glu Tyr Thr Pro Thr
    290                 295                 300

Gln Arg Leu Ser Ala Ile Glu Ala Met Cys His Pro Phe Phe Asp Glu
305                 310                 315                 320

Leu Arg Asp Pro Asn Thr Arg Leu Pro Asp Ser Arg His Pro Gly Gly
                325                 330                 335

Ala Ala Arg Asp Leu Pro Asn Leu Phe Asp Phe Ser Arg His Glu Leu
            340                 345                 350

Ser Ile Ala Pro Ala Leu Asn Ser Arg Leu Val Pro Pro His Ala Arg

355                 360                 365

Ala Ala Leu Glu Ala Arg Gly Leu Asp Ile Asp Asn Phe Thr Pro Leu
    370                 375                 380

Thr Lys Glu Glu Met Met Ala Arg Leu Asp
385                 390

<210> 43
<211> 2209
<212> DNA
<213> Aspergillus fumigatus

<400> 43
accgcgcgat cctgctgtgc tgagtcagca ttggattact tcattagacc tcgacagtca 60
acagcatttc tccacttaac cacctacaac ctaagacagc acgggaactg gttatatttg 120
ttcgagtcag ttacttcagg gtgttttatt aaatcaatgt ggaagtcttt taaagaaaag 180
catgccagca agtttggggg aggctcggct gaagcggcag cctcagacgg tggccaagat 240
ctgaccacga tattggatag atcccaacgc ggggaattga cagtgcttgt tgcgctaatc 300
gcacagcgga tgcgggatgg aatagaacaa aacttctcca atgctcctcc ttcgtcaggc 360
cagtctgtca actacgaaga aaaacggaca ggctccctgc cccaatctac agatgcacaa 420
gaagaccagt catcctccgg cagcgcagcg aacggttcaa gaaccgaccc tcaattcaaa 480
gacccggaga ctgcgacatg tgcactatct aaatatgatg actggaggga ctccgtcctt 540
cttcgaattg gtgaagtcgt caacagggat ccagaacatg gtgaagttca ggcgaatgaa 600
aacccaccct caggacagca atcccagcag atccgctccg aggaggatga ccgttccatt 660
cgtaagctac gtgaggtctt tccgccggtg gagaccagcc tctctcaatt gccagaagcg 720
aagaaactct taattctcca ctcattgcta ctcctagttt tgagtcttga acactataac 780
gcctggtctc gggtcctgat gctgttcgtg acgtccagct tagggctgga cgtgaaatta 840
ctaaacgaag atgaagtcaa agtggcgagg gggttgcttg acactgctct ggcactgtcg 900
tctaacgccc caaggcagga tgagagtcga agtcgcgatt catcccgaaa atggaaggtt 960
gggatcgcgt cagttgcggg tgctgccctg atcgggatca ctggtggact ggctgcgccc 1020
cttgttgcag ctgggcttgg tactgtcatg ggcggccttg ggcttggcgc caccgccgca 1080
gcagggtatc tcggagctct cgctggaagt ggtgttgtcg tcggcggact tttcggtgct 1140
tatggtgggc ggatgaccgg tcgtatggtt gacaagtacg cacgggaggt ggatgatttt 1200
gcctttctgc cgattcgtgg ttctcggcat cgatccgaag acgaaagaga agctgcccat 1260
caggatcacc ggctgcgggt taccatcggc gtgaccggat ggctgacaga ggaggacaat 1320
ttcgtgatcc cgtggcgagt gatcggagcg gaatcggagg tgtttggtct ccgctgggaa 1380
accgagcccc tgatgaatct gggaaatgcg cttgaccttt tggtaaccag cgccgcatgg 1440
actgccggtg aacaagtcct gaagaagaca ttcctctccc aactcttgac cgctgtcgcg 1500
ctgccgcttg gccttctcaa ggtcgcacgt gtggtggaca atccgtttag cgtagcgaag 1560
gctcgggcgg acaaggctgg ggaggttctc gcggatgctc ttatcagtaa agtgcagggc 1620
gagcgaccag tcaccctcat tggctactcc ttagggtctc gggtgatttt cgcttgcctt 1680
caaagcttgg cgaaacggcg cgcgttcggc ttggtggaat ccgcaattct gatgggagct 1740
cccaccccgt cgaattcaga acaatggtgt cgcatccgca gtgttgtgag tggacgcctt 1800
gtcaacgtgt actcggaaaa cgactccgtg ctggctctct tatatcgaac aagcagcctc 1860
cagcttgggg ttgcaggctt gcagcctgtt gaaggtgtct caggcgttga gaatctggac 1920
gttagcgacc tgatcagcgg ccatctccgt tatcagtttc tcgttggcag gatcttgagc 1980
gttgttggac ttgagagcat tgatgctcgc gaggtcgcac tggaggaggc cgcattagaa 2040
gccaaagatc ggaggcagga gcaggaaagg gctcataacg aacgacaggc tggatttatg 2100
ggcgagggtc ggtcaccaag ccagcggctg gaaagccagg aggatctgca gggcgaagag 2160
gacagattac agaaagagat gggaaaagca cgagtgcggc actcttaga 2209

<210> 44
<211> 2209
<212> DNA
<213> Aspergillus fumigatus

<400> 44

accgcgcgat cctgctgtgc tgagtcagca ttggattact tcattagacc tcgacagtca 60
acagcatttc tccacttaac cacctacaac ctaagacagc acgggaactg gttatatttg 120
ttcgagtcag ttacttcagg gtgttttatt aaatcaatgt ggaagtcttt taaagaaaag 180
catgccagca agtttggggg aggctcggct gaagcggcag cctcagacgg tggccaagat 240
ctgaccacga tattggatag atcccaacgc ggggaattga cagtgcttgt tgcgctaatc 300
gcacagcgga tgcgggatgg aatagaacaa aacttctcca atgctcctcc ttcgtcaggc 360
cagtctgtca actacgaaga aaaacggaca ggctccctgc cccaatctac agatgcacaa 420
gaagaccagt catcctccgg cagcgcagcg aacggttcaa gaaccgaccc tcaattcaaa 480
gacccggaga ctgcgacatg tgcactatct aaatatgatg actggaggga ctccgtcctt 540
cttcgaattg gtgaagtcgt caacagggat ccagaacatg gtgaagttca ggcgaatgaa 600
aacccaccct caggacagca atcccagcag atccgctccg aggaggatga ccgttccatt 660
cgtaagctac gtgaggtctt tccgccggtg gagaccagcc tctctcaatt gccagaagcg 720
aagaaactct taattctcca ctcattgcta ctcctagttt tgagtcttga acactataac 780
gcctggtctc gggtcctgat gctgttcgtg acgtccagct tagggctgga cgtgaaatta 840
ctaaacgaag atgaagtcaa agtggcgagg gggttgcttg acactgctct ggcactgtcg 900
tctaacgccc caaggcagga tgagagtcga agtcgcgatt catcccgaaa atggaaggtt 960
gggatcgcgt cagttgcggg tgctgccctg atcgggatca ctggtggact ggctgcgccc 1020
cttgttgcag ctgggcttgg tactgtcatg ggcggccttg ggcttggcgc caccgccgca 1080
gcagggtatc tcggagctct cgctggaagt ggtgttgtcg tcggcggact tttcggtgct 1140
tatggtgggc ggatgaccgg tcgtatggtt gacaagtacg cacgggaggt ggatgatttt 1200
gcctttctgc cgattcgtgg ttctcggcat cgatccgaag acgaaagaga agctgcccat 1260
caggatcacc ggctgcgggt taccatcggc gtgaccggat ggctgacaga ggaggacaat 1320
ttcgtgatcc cgtggcgagt gatcggagcg gaatcggagg tgtttggtct ccgctgggaa 1380
accgagcccc tgatgaatct gggaaatgcg cttgaccttt tggtaaccag cgccgcatgg 1440
actgccggtg aacaagtcct gaagaagaca ttcctctccc aactcttgac cgctgtcgcg 1500
ctgccgcttg gccttctcaa ggtcgcacgt gtggtggaca atccgtttag cgtagcgaag 1560
gctcgggcgg acaaggctgg ggaggttctc gcggatgctc ttatcagtaa agtgcagggc 1620
gagcgaccag tcaccctcat tggctactcc ttagggtctc gggtgatttt cgcttgcctt 1680
caaagcttgg cgaaacggcg cgcgttcggc ttggtggaat ccgcaattct gatgggagct 1740
cccaccccgt cgaattcaga acaatggtgt cgcatccgca gtgttgtgag tggacgcctt 1800
gtcaacgtgt actcggaaaa cgactccgtg ctggctctct tatatcgaac aagcagcctc 1860
cagcttgggg ttgcaggctt gcagcctgtt gaaggtgtct caggcgttga gaatctggac 1920
gttagcgacc tgatcagcgg ccatctccgt tatcagtttc tcgttggcag gatcttgagc 1980
gttgttggac ttgagagcat tgatgctcgc gaggtcgcac tggaggaggc cgcattagaa 2040
gccaaagatc ggaggcagga gcaggaaagg gctcataacg aacgacaggc tggatttatg 2100
ggcgagggtc ggtcaccaag ccagcggctg gaaagccagg aggatctgca gggcgaagag 2160
gacagattac agaaagagat gggaaaagca cgagtgcggc actcttaga 2209

<210> 45
<211> 735
<212> PRT
<213> Aspergillus fumigatus

<400> 45
Thr Ala Arg Ser Cys Cys Ala Glu Ser Ala Leu Asp Tyr Phe Ile Arg
1               5                   10                  15

Pro Arg Gln Ser Thr Ala Phe Leu His Leu Thr Thr Tyr Asn Leu Arg
            20                  25                  30

Gln His Gly Asn Trp Leu Tyr Leu Phe Glu Ser Val Thr Ser Gly Cys
        35                  40                  45

Phe Ile Lys Ser Met Trp Lys Ser Phe Lys Glu Lys His Ala Ser Lys
    50                  55                  60

Phe Gly Gly Gly Ser Ala Glu Ala Ala Ala Ser Asp Gly Gly Gln Asp
65                  70                  75                  80

Leu Thr Thr Ile Leu Asp Arg Ser Gln Arg Gly Glu Leu Thr Val Leu

                85                  90                  95

Val Ala Leu Ile Ala Gln Arg Met Arg Asp Gly Ile Glu Gln Asn Phe
            100                 105                 110

Ser Asn Ala Pro Pro Ser Ser Gly Gln Ser Val Asn Tyr Glu Glu Lys
        115                 120                 125

Arg Thr Gly Ser Leu Pro Gln Ser Thr Asp Ala Gln Glu Asp Gln Ser
    130                 135                 140

Ser Ser Gly Ser Ala Ala Asn Gly Ser Arg Thr Asp Pro Gln Phe Lys
145                 150                 155                 160

Asp Pro Glu Thr Ala Thr Cys Ala Leu Ser Lys Tyr Asp Asp Trp Arg
                165                 170                 175

Asp Ser Val Leu Leu Arg Ile Gly Glu Val Val Asn Arg Asp Pro Glu
            180                 185                 190

His Gly Glu Val Gln Ala Asn Glu Asn Pro Pro Ser Gly Gln Gln Ser
        195                 200                 205

Gln Gln Ile Arg Ser Glu Glu Asp Asp Arg Ser Ile Arg Lys Leu Arg
    210                 215                 220

Glu Val Phe Pro Pro Val Glu Thr Ser Leu Ser Gln Leu Pro Glu Ala
225                 230                 235                 240

Lys Lys Leu Leu Ile Leu His Ser Leu Leu Leu Leu Val Leu Ser Leu
                245                 250                 255

Glu His Tyr Asn Ala Trp Ser Arg Val Leu Met Leu Phe Val Thr Ser
            260                 265                 270

Ser Leu Gly Leu Asp Val Lys Leu Leu Asn Glu Asp Glu Val Lys Val
        275                 280                 285

Ala Arg Gly Leu Leu Asp Thr Ala Leu Ala Leu Ser Ser Asn Ala Pro
    290                 295                 300

Arg Gln Asp Glu Ser Arg Ser Arg Asp Ser Ser Arg Lys Trp Lys Val
305                 310                 315                 320

Gly Ile Ala Ser Val Ala Gly Ala Ala Leu Ile Gly Ile Thr Gly Gly
                325                 330                 335

Leu Ala Ala Pro Leu Val Ala Ala Gly Leu Gly Thr Val Met Gly Gly
            340                 345                 350

Leu Gly Leu Gly Ala Thr Ala Ala Ala Gly Tyr Leu Gly Ala Leu Ala
        355                 360                 365

Gly Ser Gly Val Val Val Gly Gly Leu Phe Gly Ala Tyr Gly Gly Arg
    370                 375                 380

Met Thr Gly Arg Met Val Asp Lys Tyr Ala Arg Glu Val Asp Asp Phe
385                 390                 395                 400

Ala Phe Leu Pro Ile Arg Gly Ser Arg His Arg Ser Glu Asp Glu Arg
                405                 410                 415

Glu Ala Ala His Gln Asp His Arg Leu Arg Val Thr Ile Gly Val Thr
            420                 425                 430

Gly Trp Leu Thr Glu Glu Asp Asn Phe Val Ile Pro Trp Arg Val Ile
        435                 440                 445

Gly Ala Glu Ser Glu Val Phe Gly Leu Arg Trp Glu Thr Glu Pro Leu
    450                 455                 460

Met Asn Leu Gly Asn Ala Leu Asp Leu Leu Val Thr Ser Ala Ala Trp
465                 470                 475                 480

Thr Ala Gly Glu Gln Val Leu Lys Lys Thr Phe Leu Ser Gln Leu Leu
                485                 490                 495

Thr Ala Val Ala Leu Pro Leu Gly Leu Leu Lys Val Ala Arg Val Val
            500                 505                 510

Asp Asn Pro Phe Ser Val Ala Lys Ala Arg Ala Asp Lys Ala Gly Glu
        515                 520                 525

Val Leu Ala Asp Ala Leu Ile Ser Lys Val Gln Gly Glu Arg Pro Val
    530                 535                 540

Thr Leu Ile Gly Tyr Ser Leu Gly Ser Arg Val Ile Phe Ala Cys Leu
545                 550                 555                 560

Gln Ser Leu Ala Lys Arg Arg Ala Phe Gly Leu Val Glu Ser Ala Ile
                565                 570                 575

Leu Met Gly Ala Pro Thr Pro Ser Asn Ser Glu Gln Trp Cys Arg Ile
            580                 585                 590

Arg Ser Val Val Ser Gly Arg Leu Val Asn Val Tyr Ser Glu Asn Asp
        595                 600                 605

Ser Val Leu Ala Leu Leu Tyr Arg Thr Ser Ser Leu Gln Leu Gly Val
    610                 615                 620

Ala Gly Leu Gln Pro Val Glu Gly Val Ser Gly Val Glu Asn Leu Asp
625                 630                 635                 640

Val Ser Asp Leu Ile Ser Gly His Leu Arg Tyr Gln Phe Leu Val Gly
                645                 650                 655

Arg Ile Leu Ser Val Val Gly Leu Glu Ser Ile Asp Ala Arg Glu Val
            660                 665                 670

Ala Leu Glu Glu Ala Ala Leu Glu Ala Lys Asp Arg Arg Gln Glu Gln
        675                 680                 685

Glu Arg Ala His Asn Glu Arg Gln Ala Gly Phe Met Gly Glu Gly Arg
    690                 695                 700

Ser Pro Ser Gln Arg Leu Glu Ser Gln Glu Asp Leu Gln Gly Glu Glu
705                 710                 715                 720

Asp Arg Leu Gln Lys Glu Met Gly Lys Ala Arg Val Arg His Ser
                725                 730                 735

<210> 46
<211> 510
<212> DNA
<213> Aspergillus fumigatus

<400> 46
atggccgata tcgatgtcaa ggttgctcaa tggaagcttg ttgaggttgg ccgtgttgtg 60
ctgatccgca gcggtcctta caccggcaag cttgctgcca ttgtcgagat catcgaccac 120
aagcgtgtac gtttttcaac ggagaaattc tgagcgcagg acggaaagat catggtcgga 180
tgtgatattg acaaagaggc gcgatcatag gtcctggttg acggtccttc caccgaggag 240
aacaagatcg ttccccgtca cgctcttcct ctcgctcacg ccactctcac ccccttcgtc 300
attcccaaac tcccccgcgc tgccggcact ggccccgtca agaagctctg ggagaagaac 360
gagatcgatg gaaagtgggc taagagcacc attgctcaga agactgagcg cgctgagcgg 420
aggaagaacc ttaccgactt cgagcgcttc aaggtcctca gactcaagaa gcaggtacgt 480
tcagtttgcg aaactatggg agaattgtga 510

<210> 47
<211> 423
<212> DNA
<213> Aspergillus fumigatus

<400> 47
atggccgata tcgatgtcaa ggttgctcaa tggaagcttg ttgaggttgg ccgtgttgtg 60
ctgatccgca gcggtcctta caccggcaag cttgctgcca ttgtcgagat catcgaccac 120
aagcgtgtcc tggttgacgg tccttccacc gaggagaaca agatcgttcc ccgtcacgct 180
cttcctctcg ctcacgccac tctcaccccc ttcgtcattc ccaaactccc ccgcgctgcc 240
ggcactggcc ccgtcaagaa gctctgggag aagaacgaga tcgatggaaa gtgggctaag 300
agcaccattg ctcagaagac tgagcgcgct gagcggagga agaaccttac cgacttcgag 360
cgcttcaagg tcctcagact caagaagcag gtacgttcag tttgcgaaac tatgggagaa 420
ttg 423

<210> 48
<211> 141
<212> PRT
<213> Aspergillus fumigatus

<400> 48
Met Ala Asp Ile Asp Val Lys Val Ala Gln Trp Lys Leu Val Glu Val
1               5                   10                  15

Gly Arg Val Val Leu Ile Arg Ser Gly Pro Tyr Thr Gly Lys Leu Ala
            20                  25                  30

Ala Ile Val Glu Ile Ile Asp His Lys Arg Val Leu Val Asp Gly Pro
        35                  40                  45

Ser Thr Glu Glu Asn Lys Ile Val Pro Arg His Ala Leu Pro Leu Ala
    50                  55                  60

His Ala Thr Leu Thr Pro Phe Val Ile Pro Lys Leu Pro Arg Ala Ala
65                  70                  75                  80

Gly Thr Gly Pro Val Lys Lys Leu Trp Glu Lys Asn Glu Ile Asp Gly
                85                  90                  95

Lys Trp Ala Lys Ser Thr Ile Ala Gln Lys Thr Glu Arg Ala Glu Arg
            100                 105                 110

Arg Lys Asn Leu Thr Asp Phe Glu Arg Phe Lys Val Leu Arg Leu Lys
        115                 120                 125

Lys Gln Val Arg Ser Val Cys Glu Thr Met Gly Glu Leu
    130                 135                 140

<210> 49
<211> 1413
<212> DNA
<213> Aspergillus fumigatus

<400> 49
atggctctcc gccggccatt aacacttccg aggcacattc tcaatggagc ttgtttaggc 60
ttgcgaccag ctgtgtctcg cgccgctctg gcttatgggc aggagcagag gaaagggctt 120
gcaacagcag ttcccccggt cactcaaaat gcggctgggt ccaaaggccc cacggcaatg 180
gtcttcctca acatgggtgg gccatcgaag attgacgaag tggaagattt tctgagcaga 240
ttatttgtat gcattcctca atatgcccga tgctaccacc atgtatgagt ctgacaaact 300
ctctcttcct ataccaggcc gatggcgatc tgattcctct cggacgactt caatcatacc 360
tcggccctct catcgctaag cgcagaaccc caaagatcca acggcaatac tcggatattg 420
gtggagggtc accgatcagg aaatggtccg agtatcagtg cgaggaaatg tgcagattgc 480
tagacaaaat caatcccgaa acggctcctc acaagcctta cgtcgcgttc cggtacgccg 540
accctctgac ggaagaaatg tacacaaagt tgctggaaga tggattcggc aacggaaagg 600
gcgggcgcgc tgtcgcgttc acacagtacc cccaatattc gtgctccacc acgggtagct 660
cgctgaacga gttgtggaaa tggagaacca ggcttgaggg taagcgtgca aatggcaaca 720
tggaccccgc tggtgccatc cagtggagtg tcattgatcg atggccaacg caccctggcc 780
tcgtggaggc tttcgcccgg aacattgagg agcagctgaa gacataccca gaggagaagc 840
gaaacggtgt cgttctcttg ttctcagccc acagtctgcc catgagtgtt gtcaacagag 900
gtgagactca tcttcttacc gaacaacaag atttgctcgc taacacattt cctaggcgac 960
ccatatcctg ctgaagttgc tgcaactgtg catgctgtca tgcaaagatt gaatttcagc 1020
aatccttacc gactgtgctg gcagtcccaa gtgggaccgt cagcttggct tggagcccaa 1080
actagcgata cggtcgaaaa ctatgtcaaa cgtggacaga ccgatattat tctagttccc 1140
attgccttca ccagcgacca tattgagact ctgtacgagt tggatctgga agtgataaag 1200
gaagcaaact ccccgggagt caagagagcc gagagtttga atggtaaccc cattttcatt 1260
caggcattag cagacattgc ccaagagcac ctccgtaagg gagagaagtg ctcactacag 1320
atgactctgc gctgtcaagg ctgtaagagc gaacggtgcc tggaacagaa gaaattcttt 1380
gctggcgacc gattttcttc tcttgtagtt tag 1413

<210> 50
<211> 1284
<212> DNA
<213> Aspergillus fumigatus

<400> 50
   atggctctcc gccggccatt aacacttccg aggcacattc tcaatggagc ttgtttaggc 60
   ttgcgaccag ctgtgtctcg cgccgctctg gcttatgggc aggagcagag gaaagggctt 120
   gcaacagcag ttcccccggt cactcaaaat gcggctgggt ccaaaggccc cacggcaatg 180
   gtcttcctca acatgggtgg gccatcgaag attgacgaag tggaagattt tctgagcaga 240
   ttatttgccg atggcgatct gattcctctc ggacgacttc aatcatacct cggccctctc 300
   atcgctaagc gcagaacccc aaagatccaa cggcaatact cggatattgg tggagggtca 360
   ccgatcagga aatggtccga gtatcagtgc gaggaaatgt gcagattgct agacaaaatc 420
   aatcccgaaa cggctcctca caagccttac gtcgcgttcc ggtacgccga ccctctgacg 480
   gaagaaatgt acacaaagtt gctggaagat ggattcggca acggaaaggc cgggcgcgct 540
   gtcgcgttca cacagtaccc ccaatattcg tgctccacca cgggtagctc gctgaacgag 600
   ttgtggaaat ggagaaccag gcttgagggt aagcgtgcaa atggcaacat ggaccccgct 660
   ggtgccatcc agtggagtgt cattgatcga tggccaacgc accctggcct cgtggaggct 720
   ttcgcccgga acattgagga gcagctgaag acatacccag aggagaagcg aaacggtgtc 780
   gttctcttgt tctcagccca cagtctgccc atgagtgttg tcaacagagg cgacccatat 840
   cctgctgaag ttgctgcaac tgtgcatgct gtcatgcaaa gattgaattt cagcaatcct 900
   taccgactgt gctggcagtc ccaagtggga ccgtcagctt ggcttggagc ccaaactagc 960
   gatacggtcg aaaactatgt caaacgtgga cagaccgata ttattctagt tcccattgcc 1020
   ttcaccagcg accatattga gactctgtac gagttggatc tggaagtgat aaaggaagca 1080
   aactccccgg gagtcaagag agccgagagt ttgaatggta accccatttt cattcaggca 1140
   ttagcagaca ttgcccaaga gcacctccgt aagggagaga agtgctcact acagatgact 1200
   ctgcgctgtc aaggctgtaa gagcgaacgg tgcctggaac agaagaaatt ctttgctggc 1260
   gaccgatttt cttctcttgt agtt 1284

<210> 51
<211> 428
<212> PRT
<213> Aspergillus fumigatus

<400> 51
Met Ala Leu Arg Arg Pro Leu Thr Leu Pro Arg His Ile Leu Asn Gly
1               5                   10                  15

Ala Cys Leu Gly Leu Arg Pro Ala Val Ser Arg Ala Ala Leu Ala Tyr
            20                  25                  30

Gly Gln Glu Gln Arg Lys Gly Leu Ala Thr Ala Val Pro Pro Val Thr
        35                  40                  45

Gln Asn Ala Ala Gly Ser Lys Gly Pro Thr Ala Met Val Phe Leu Asn
    50                  55                  60

Met Gly Gly Pro Ser Lys Ile Asp Glu Val Glu Asp Phe Leu Ser Arg
65                  70                  75                  80

Leu Phe Ala Asp Gly Asp Leu Ile Pro Leu Gly Arg Leu Gln Ser Tyr
                85                  90                  95

Leu Gly Pro Leu Ile Ala Lys Arg Arg Thr Pro Lys Ile Gln Arg Gln
            100                 105                 110

Tyr Ser Asp Ile Gly Gly Gly Ser Pro Ile Arg Lys Trp Ser Glu Tyr
        115                 120                 125

Gln Cys Glu Glu Met Cys Arg Leu Leu Asp Lys Ile Asn Pro Glu Thr
    130                 135                 140

Ala Pro His Lys Pro Tyr Val Ala Phe Arg Tyr Ala Asp Pro Leu Thr
145                 150                 155                 160

Glu Glu Met Tyr Thr Lys Leu Leu Glu Asp Gly Phe Gly Asn Gly Lys
                165                 170                 175

Gly Gly Arg Ala Val Ala Phe Thr Gln Tyr Pro Gln Tyr Ser Cys Ser
            180                 185                 190

Thr Thr Gly Ser Ser Leu Asn Glu Leu Trp Lys Trp Arg Thr Arg Leu
        195                 200                 205

Glu Gly Lys Arg Ala Asn Gly Asn Met Asp Pro Ala Gly Ala Ile Gln
    210                 215                 220

Trp Ser Val Ile Asp Arg Trp Pro Thr His Pro Gly Leu Val Glu Ala
225                 230                 235                 240

Phe Ala Arg Asn Ile Glu Glu Gln Leu Lys Thr Tyr Pro Glu Glu Lys
                245                 250                 255

Arg Asn Gly Val Val Leu Leu Phe Ser Ala His Ser Leu Pro Met Ser
            260                 265                 270

Val Val Asn Arg Gly Asp Pro Tyr Pro Ala Glu Val Ala Ala Thr Val
        275                 280                 285

His Ala Val Met Gln Arg Leu Asn Phe Ser Asn Pro Tyr Arg Leu Cys
    290                 295                 300

Trp Gln Ser Gln Val Gly Pro Ser Ala Trp Leu Gly Ala Gln Thr Ser
305                 310                 315                 320

Asp Thr Val Glu Asn Tyr Val Lys Arg Gly Gln Thr Asp Ile Ile Leu
                325                 330                 335

Val Pro Ile Ala Phe Thr Ser Asp His Ile Glu Thr Leu Tyr Glu Leu
            340                 345                 350

Asp Leu Glu Val Ile Lys Glu Ala Asn Ser Pro Gly Val Lys Arg Ala
        355                 360                 365

Glu Ser Leu Asn Gly Asn Pro Ile Phe Ile Gln Ala Leu Ala Asp Ile
    370                 375                 380

Ala Gln Glu His Leu Arg Lys Gly Glu Lys Cys Ser Leu Gln Met Thr
385                 390                 395                 400

Leu Arg Cys Gln Gly Cys Lys Ser Glu Arg Cys Leu Glu Gln Lys Lys
                405                 410                 415

Phe Phe Ala Gly Asp Arg Phe Ser Ser Leu Val Val
            420                 425

<210> 52
<211> 1536
<212> DNA
<213> Aspergillus fumigatus

<400> 52
atgatttatc tccggtcctc gttgctgagg tctggattgg ctcgagatcc tgctcgcctg 60
tgttcacaat gcttctcacg actctcacca tcacgacgac ctgtcgcagt tcgcagcttc 120
ttctcctcat ctcggctgcg ggctggcatt gccgatcatg aatcaactcc ctcgactgtc 180
caaaagacct atttttctgc caatcggacc gcagatggct tacttgcatc cttatccgcc 240
gtcaatagct cccctcgaag tattgccgac aatgcgttat cacagggtgc agccagttcg 300
gagtcgatta cttcacagtc tacttcacaa gagttacctc atcgccggag gaagcggtta 360
aaggaagagg cggccaagaa taatgctgca gaaaccgaac tccctcctga tgcctcgtct 420
caattgtcca ccctctcatc agccctccct gcgacttccc tgcgccgcaa gctggctgcg 480
tttctcgccc tcacaaagcc tcgtctctcg ttcctgatcg tgttgacgac tacctccgct 540
tatgggatgt acccgatctc ctctcttctc acacttgacc cttcaatgac tcccctaccg 600
accctctcga cctcaacctt gacctttctc tacctgacca caggaacctt cttgtcttca 660
tgcagcgcca ataccttgaa tatgctcctt gaacctaaat acgatgccct catgtcacgg 720
acacggaacc ggccgttagt gcgggggcta ctctcacgcc gtgctgcggt attgtttgcg 780
attgcgactg ctgctgcagg tctcggtttg ttatacattg gaacgaaccc tacgactact 840
gcgctctccg ccagtaatat ctgtctctat gcctttgtgt atacgccgct gaagcgtata 900
tcagtgatca acacctgggt aggcgccgtg gtaggaggca ttcctccgtt gatgggttgg 960
accgctgcag caggccagac agcgaccact ggccacgaca gctggcggga catgttgttc 1020

agcaaggata gcatcggtgg ttggctcctg ggtggcattc tctttgcatg gcagtttcct 1080
catttcaatg ctttgtccta catgatccgt gaagagtaca aggcagccgg gtacaggatg 1140
ctcgcatgga ctaatcccgc cgcaaatgcc cgtgtcgcac tacgatattc tcttctcatg 1200
tttcctttct ccgtcggtct ctggtgggta ggagttgtcg gtaatggttt cctggttgga 1260
agcacggcgg ccaatggctg gctagtcaaa gaggcctaca aattctggcg gcaccaaggc 1320
gccaacggca gtgctcgacg cctcttctgg gccagtattt ggcagctgcc aatcctcctt 1380
gtcggtggtc tggtcacgaa gaaaggtctc tgggatggtg tctggaacaa tgttttcggt 1440
cagcctgtgg aagacgagga tgactatctc tgggaggatg aggatgaagt ggcagaggcg 1500
gagcgcaaga tgatacctgc gaagacgagt agctcg 1536

<210> 53
<211> 1536
<212> DNA
<213> Aspergillus fumigatus

<400> 53
atgatttatc tccggtcctc gttgctgagg tctggattgg ctcgagatcc tgctcgcctg 60
tgttcacaat gcttctcacg actctcacca tcacgacgac ctgtcgcagt tcgcagcttc 120
ttctcctcat ctcggctgcg ggctggcatt gccgatcatg aatcaactcc ctcgactgtc 180
caaaagacct atttttctgc caatcggacc gcagatggct tacttgcatc cttatccgcc 240
gtcaatagct cccctcgaag tattgccgac aatgcgttat cacagggtgc agccagttcg 300
gagtcgatta cttcacagtc tacttcacaa gagttacctc atcgccggag gaagcggtta 360
aaggaagagg cggccaagaa taatgctgca gaaaccgaac tccctcctga tgcctcgtct 420
caattgtcca ccctctcatc agccctccct gcgacttccc tgcgccgcaa gctggctgcg 480
tttctcgccc tcacaaagcc tcgtctctcg ttcctgatcg tgttgacgac tacctccgct 540
tatgggatgt acccgatctc ctctcttctc acacttgacc cttcaatgac tcccctaccg 600
accctctcga cctcaacctt gacctttctc tacctgacca caggaacctt cttgtcttca 660
tgcagcgcca ataccttgaa tatgctcctt gaacctaaat acgatgccct catgtcacgg 720
acacggaacc ggccgttagt gcgggggcta ctctcacgcc gtgctgcggt attgtttgcg 780
attgcgactg ctgctgcagg tctcggtttg ttatacattg gaacgaaccc tacgactact 840
gcgctctccg ccagtaatat ctgtctctat gcctttgtgt atacgccgct gaagcgtata 900
tcagtgatca acacctgggt aggcgccgtg gtaggaggca ttcctccgtt gatgggttgg 960
accgctgcag caggccagac agcgaccact ggccacgaca gctggcggga catgttgttc 1020
agcaaggata gcatcggtgg ttggctcctg ggtggcattc tctttgcatg gcagtttcct 1080
catttcaatg ctttgtccta catgatccgt gaagagtaca aggcagccgg gtacaggatg 1140
ctcgcatgga ctaatcccgc cgcaaatgcc cgtgtcgcac tacgatattc tcttctcatg 1200
tttcctttct ccgtcggtct ctggtgggta ggagttgtcg gtaatggttt cctggttgga 1260
agcacggcgg ccaatggctg gctagtcaaa gaggcctaca aattctggcg gcaccaaggc 1320
gccaacggca gtgctcgacg cctcttctgg gccagtattt ggcagctgcc aatcctcctt 1380
gtcggtggtc tggtcacgaa gaaaggtctc tgggatggtg tctggaacaa tgttttcggt 1440
cagcctgtgg aagacgagga tgactatctc tgggaggatg aggatgaagt ggcagaggcg 1500
gagcgcaaga tgatacctgc gaagacgagt agctcg 1536

<210> 54
<211> 512
<212> PRT
<213> Aspergillus fumigatus

<400> 54
Met Ile Tyr Leu Arg Ser Ser Leu Leu Arg Ser Gly Leu Ala Arg Asp
1               5                   10                  15

Pro Ala Arg Leu Cys Ser Gln Cys Phe Ser Arg Leu Ser Pro Ser Arg
            20                  25                  30

Arg Pro Val Ala Val Arg Ser Phe Phe Ser Ser Ser Arg Leu Arg Ala
        35                  40                  45

Gly Ile Ala Asp His Glu Ser Thr Pro Ser Thr Val Gln Lys Thr Tyr

50                  55                  60

Phe Ser Ala Asn Arg Thr Ala Asp Gly Leu Leu Ala Ser Leu Ser Ala
65                  70                  75                  80

Val Asn Ser Ser Pro Arg Ser Ile Ala Asp Asn Ala Leu Ser Gln Gly
                85                  90                  95

Ala Ala Ser Ser Glu Ser Ile Thr Ser Gln Ser Thr Ser Gln Glu Leu
            100                 105                 110

Pro His Arg Arg Arg Lys Arg Leu Lys Glu Glu Ala Ala Lys Asn Asn
        115                 120                 125

Ala Ala Glu Thr Glu Leu Pro Pro Asp Ala Ser Ser Gln Leu Ser Thr
    130                 135                 140

Leu Ser Ser Ala Leu Pro Ala Thr Ser Leu Arg Arg Lys Leu Ala Ala
145                 150                 155                 160

Phe Leu Ala Leu Thr Lys Pro Arg Leu Ser Phe Leu Ile Val Leu Thr
                165                 170                 175

Thr Thr Ser Ala Tyr Gly Met Tyr Pro Ile Ser Ser Leu Leu Thr Leu
            180                 185                 190

Asp Pro Ser Met Thr Pro Leu Pro Thr Leu Ser Thr Ser Thr Leu Thr
        195                 200                 205

Phe Leu Tyr Leu Thr Thr Gly Thr Phe Leu Ser Ser Cys Ser Ala Asn
    210                 215                 220

Thr Leu Asn Met Leu Leu Glu Pro Lys Tyr Asp Ala Leu Met Ser Arg
225                 230                 235                 240

Thr Arg Asn Arg Pro Leu Val Arg Gly Leu Leu Ser Arg Arg Ala Ala
                245                 250                 255

Val Leu Phe Ala Ile Ala Thr Ala Ala Ala Gly Leu Gly Leu Leu Tyr
            260                 265                 270

Ile Gly Thr Asn Pro Thr Thr Thr Ala Leu Ser Ala Ser Asn Ile Cys
        275                 280                 285

Leu Tyr Ala Phe Val Tyr Thr Pro Leu Lys Arg Ile Ser Val Ile Asn
    290                 295                 300

Thr Trp Val Gly Ala Val Val Gly Gly Ile Pro Pro Leu Met Gly Trp
305                 310                 315                 320

Thr Ala Ala Ala Gly Gln Thr Ala Thr Thr Gly His Asp Ser Trp Arg
                325                 330                 335

Asp Met Leu Phe Ser Lys Asp Ser Ile Gly Gly Trp Leu Leu Gly Gly
            340                 345                 350

Ile Leu Phe Ala Trp Gln Phe Pro His Phe Asn Ala Leu Ser Tyr Met
        355                 360                 365

Ile Arg Glu Glu Tyr Lys Ala Ala Gly Tyr Arg Met Leu Ala Trp Thr
    370                 375                 380

Asn Pro Ala Ala Asn Ala Arg Val Ala Leu Arg Tyr Ser Leu Leu Met
385                 390                 395                 400

Phe Pro Phe Ser Val Gly Leu Trp Trp Val Gly Val Val Gly Asn Gly
                405                 410                 415

Phe Leu Val Gly Ser Thr Ala Ala Asn Gly Trp Leu Val Lys Glu Ala
            420                 425                 430

Tyr Lys Phe Trp Arg His Gln Gly Ala Asn Gly Ser Ala Arg Arg Leu
        435                 440                 445

Phe Trp Ala Ser Ile Trp Gln Leu Pro Ile Leu Leu Val Gly Gly Leu
    450                 455                 460

Val Thr Lys Lys Gly Leu Trp Asp Gly Val Trp Asn Asn Val Phe Gly
465                 470                 475                 480

Gln Pro Val Glu Asp Glu Asp Asp Tyr Leu Trp Glu Asp Glu Asp Glu
                485                 490                 495

Val Ala Glu Ala Glu Arg Lys Met Ile Pro Ala Lys Thr Ser Ser Ser
            500                 505                 510

<210> 55
<211> 1626
<212> DNA
<213> Aspergillus fumigatus

<400> 55
atgctcaacg ccgcggttgc tgccccgcga tgttttgtat atcccactga tcgcgcagca 60
atgcgcttgg gctttgctct tcgtctctcc tctcctgcac ctctcttctc aacagcacct 120
ttccgtcgac agttgcatgc ttccggcgtc cgatcaattg aacctgttat ctttcgaaat 180
agccttgaaa agactcttga ggctcatcga tcctccaatc gagccagtct gatccgcaag 240
gtgattaacc acgattgtcc tgctgaaacg ccccctccaa ttttaccact tgagaatcgt 300
gctggtcatg atcaatcatc tcaaaaggcc tcctccgtgt caaatgcaga gtcagagtcc 360
ccccggtctt ctgcgcctgc gagacgagcg cagaggaagg cccgttcgcc cagccaagta 420
gccaccccgc agccccagac aacagaatat ccacaactgc aatggcatgc agatgaaacc 480
aagggccgac cggcacaaag tccttggctg aagtacttga ctaccgattg gaaaacgccc 540
gatgccgttt cgcgtctcga cgcggagatc cgcgctcttg agctctacat gacaccgacc 600
ccgtcggagc ggactgagat agatcggctg gttgcagata tgggtaggtt gctagcggga 660
atcgtcccca gcccgcccca ggtaaccggt tcatggcgga cgcgatttgc cttgagccac 720
tcgggtctcg attttgtctt acctgtcccg gattcagacc gatccacccg tgacgttcgc 780
aagccgagtg ccacacggcc caaggtgctc cagacttaca aaaagctctt acatgaagtg 840
ggacatgcgc ttcagcagtc cccctcgttc gcggagcgag tccgcatcat aggcagccgt 900
ttccccgtcc tctcagccat ccatcgcccc acgggccgcc tgctgcagtt ccactgcggt 960
gaagggctac cggcctctgt cgaatacatc atggattacc aggccgagta tccctcgatc 1020
cggccgctct acgtgaccgc tcgcctgatc ctggaggcgc ggggtaggta tggccgtact 1080
cagatgtcta ttgaatccga tgccctcgta atgcttctcg tggccttcct caaaatgaac 1140
cacgggcgtt ttcagcggcc cgactgtctc ggcgagcagc tgatcgcgtt tctgcgcgcc 1200
tacggcagcg atattgacct gaccaccacc ggtgtgtccg tcgatccccc cagttggttc 1260
aatgctagta cggtcaaacg cgccagcgcc ctgtacgcgc ccgatgatct acccgcgcat 1320
ctgcgcggcc agcgctccct catcagcctc aagagaacag cagccgccag acgcaatctg 1380
cctgccgcca gccggctgtg cgtgcaggac cccaccaatt acatgaatga tctgggccgc 1440
agctgcgtgc gtacgttgga actccagcac acgttctcgc ttgctcatga ccgtctcggc 1500

gcaagtctca agcgctggga tgacagtgaa ccggccgcga acgttagtat cctgacacgg 1560
gccctgcaag caaacttttc tgattttgaa aatctacgcg ccaaatcgct taagctcaac 1620
gcgacc 1626

<210> 56
<211> 1626
<212> DNA
<213> Aspergillus fumigatus

<400> 56
atgctcaacg ccgcggttgc tgccccgcga tgttttgtat atcccactga tcgcgcagca 60
atgcgcttgg gctttgctct tcgtctctcc tctcctgcac ctctcttctc aacagcacct 120
ttccgtcgac agttgcatgc ttccggcgtc cgatcaattg aacctgttat ctttcgaaat 180
agccttgaaa agactcttga ggctcatcga tcctccaatc gagccagtct gatccgcaag 240
gtgattaacc acgattgtcc tgctgaaacg ccccctccaa ttttaccact tgagaatcgt 300
gctggtcatg atcaatcatc tcaaaaggcc tcctccgtgt caaatgcaga gtcagagtcc 360
ccccggtctt ctgcgcctgc gagacgagcg cagaggaagg cccgttcgcc cagccaagta 420
gccaccccgc agccccagac aacagaatat ccacaactgc aatggcatgc agatgaaacc 480
aagggccgac cggcacaaag tccttggctg aagtacttga ctaccgattg gaaaacgccc 540
gatgccgttt cgcgtctcga cgcggagatc cgcgctcttg agctctacat gacaccgacc 600
ccgtcggagc ggactgagat agatcggctg gttgcagata tgggtaggtt gctagcggga 660
atcgtcccca gcccgcccca ggtaaccggt tcatggcgga cgcgatttgc cttgagccac 720
tcgggtctcg attttgtctt acctgtcccg gattcagacc gatccacccg tgacgttcgc 780
aagccgagtg ccacacggcc caaggtgctc cagacttaca aaaagctctt acatgaagtg 840
ggacatgcgc ttcagcagtc cccctcgttc gcggagcgag tccgcatcat aggcagccgt 900
ttccccgtcc tctcagccat ccatcgcccc acgggccgcc tgctgcagtt ccactgcggt 960
gaagggctac cggcctctgt cgaatacatc atggattacc aggccgagta tccctcgatc 1020
cggccgctct acgtgaccgc tcgcctgatc ctggaggcgc ggggtaggta tggccgtact 1080
cagatgtcta ttgaatccga tgccctcgta atgcttctcg tggccttcct caaaatgaac 1140
cacgggcgtt ttcagcggcc cgactgtctc ggcgagcagc tgatcgcgtt tctgcgcgcc 1200
tacggcagcg atattgacct gaccaccacc ggtgtgtccg tcgatccccc cagttggttc 1260
aatgctagta cggtcaaacg cgccagcgcc ctgtacgcgc ccgatgatct acccgcgcat 1320
ctgcgcggcc agcgctccct catcagcctc aagagaacag cagccgccag acgcaatctg 1380
cctgccgcca gccggctgtg cgtgcaggac cccaccaatt acatgaatga tctgggccgc 1440
agctgcgtgc gtacgttgga actccagcac acgttctcgc ttgctcatga ccgtctcggc 1500
gcaagtctca agcgctggga tgacagtgaa ccggccgcga acgttagtat cctgacacgg 1560
gccctgcaag caaacttttc tgattttgaa aatctacgcg ccaaatcgct taagctcaac 1620
gcgacc 1626

<210> 57
<211> 542
<212> PRT
<213> Aspergillus fumigatus

<400> 57
Met Leu Asn Ala Ala Val Ala Ala Pro Arg Cys Phe Val Tyr Pro Thr
1               5                   10                  15

Asp Arg Ala Ala Met Arg Leu Gly Phe Ala Leu Arg Leu Ser Ser Pro
            20                  25                  30

Ala Pro Leu Phe Ser Thr Ala Pro Phe Arg Arg Gln Leu His Ala Ser
        35                  40                  45

Gly Val Arg Ser Ile Glu Pro Val Ile Phe Arg Asn Ser Leu Glu Lys
    50                  55                  60

Thr Leu Glu Ala His Arg Ser Ser Asn Arg Ala Ser Leu Ile Arg Lys
65                  70                  75                  80

Val Ile Asn His Asp Cys Pro Ala Glu Thr Pro Pro Pro Ile Leu Pro
                85                  90                  95

Leu Glu Asn Arg Ala Gly His Asp Gln Ser Ser Gln Lys Ala Ser Ser
            100                 105                 110

Val Ser Asn Ala Glu Ser Glu Ser Pro Arg Ser Ser Ala Pro Ala Arg
        115                 120                 125

Arg Ala Gln Arg Lys Ala Arg Ser Pro Ser Gln Val Ala Thr Pro Gln
    130                 135                 140

Pro Gln Thr Thr Glu Tyr Pro Gln Leu Gln Trp His Ala Asp Glu Thr
145                 150                 155                 160

Lys Gly Arg Pro Ala Gln Ser Pro Trp Leu Lys Tyr Leu Thr Thr Asp
                165                 170                 175

Trp Lys Thr Pro Asp Ala Val Ser Arg Leu Asp Ala Glu Ile Arg Ala
            180                 185                 190

Leu Glu Leu Tyr Met Thr Pro Thr Pro Ser Glu Arg Thr Glu Ile Asp
        195                 200                 205

Arg Leu Val Ala Asp Met Gly Arg Leu Leu Ala Gly Ile Val Pro Ser
    210                 215                 220

Pro Pro Gln Val Thr Gly Ser Trp Arg Thr Arg Phe Ala Leu Ser His
225                 230                 235                 240

Ser Gly Leu Asp Phe Val Leu Pro Val Pro Asp Ser Asp Arg Ser Thr
                245                 250                 255

Arg Asp Val Arg Lys Pro Ser Ala Thr Arg Pro Lys Val Leu Gln Thr
            260                 265                 270

Tyr Lys Lys Leu Leu His Glu Val Gly His Ala Leu Gln Gln Ser Pro
        275                 280                 285

Ser Phe Ala Glu Arg Val Arg Ile Ile Gly Ser Arg Phe Pro Val Leu
    290                 295                 300

Ser Ala Ile His Arg Pro Thr Gly Arg Leu Leu Gln Phe His Cys Gly
305                 310                 315                 320

Glu Gly Leu Pro Ala Ser Val Glu Tyr Ile Met Asp Tyr Gln Ala Glu
                325                 330                 335

Tyr Pro Ser Ile Arg Pro Leu Tyr Val Thr Ala Arg Leu Ile Leu Glu
            340                 345                 350

Ala Arg Gly Arg Tyr Gly Arg Thr Gln Met Ser Ile Glu Ser Asp Ala
        355                 360                 365

Leu Val Met Leu Leu Val Ala Phe Leu Lys Met Asn His Gly Arg Phe
    370                 375                 380

Gln Arg Pro Asp Cys Leu Gly Glu Gln Leu Ile Ala Phe Leu Arg Ala
385                 390                 395                 400

Tyr Gly Ser Asp Ile Asp Leu Thr Thr Thr Gly Val Ser Val Asp Pro
                405                 410                 415

Pro Ser Trp Phe Asn Ala Ser Thr Val Lys Arg Ala Ser Ala Leu Tyr
            420                 425                 430

Ala Pro Asp Asp Leu Pro Ala His Leu Arg Gly Gln Arg Ser Leu Ile
        435                 440                 445

Ser Leu Lys Arg Thr Ala Ala Ala Arg Arg Asn Leu Pro Ala Ala Ser
    450                 455                 460

Arg Leu Cys Val Gln Asp Pro Thr Asn Tyr Met Asn Asp Leu Gly Arg
465                 470                 475                 480

Ser Cys Val Arg Thr Leu Glu Leu Gln His Thr Phe Ser Leu Ala His
                485                 490                 495

Asp Arg Leu Gly Ala Ser Leu Lys Arg Trp Asp Asp Ser Glu Pro Ala
            500                 505                 510

Ala Asn Val Ser Ile Leu Thr Arg Ala Leu Gln Ala Asn Phe Ser Asp
        515                 520                 525

Phe Glu Asn Leu Arg Ala Lys Ser Leu Lys Leu Asn Ala Thr
    530                 535                 540

<210> 58
<211> 2356
<212> DNA
<213> Aspergillus fumigatus

<400> 58
atggggttgg aggatccgcg gcgagcagtc caggcagagt cgtatctgga gactacccgg 60
tcgcagctcg tcgatgcgct tatatcacgg ccgaatcttg agagtggctt gggtcgaagg 120
aattttgttc ctcctaggca ggtcccggtt gtcgacgcca tggagcggac gaaaaatgct 180
atacaatcaa ataattccag ctcgcgggcg caactttcgg atgcattacc cgagagcgag 240
aaatcacaga gtgcagggca ggttattgtc ccgacacgga tgcaggaact tctcgaccgc 300
ggtcgtccca ttgaggcggc ccaatttttt ctggagacac atgccgcttc attgaaaggc 360
atatcctcag acaggaagga aatggctacg aaggtattct tcgtcaactg caaagaggac 420
aatgtgttta ttgccagaag tgtgtttgag cggttggaag aggtggatag aatcacgcct 480
gagatgtgga agacgttgat gctggctttg gcgaagaaag ggtgcattga atcagttgcg 540
agtgtttata cgcgatacat gcgcaagttc ccttgccctc cggagatggt cgacgttgtg 600
ctgcggagtt tgcttgaatc ccaccgactc accactgcga aatggttcct cttgcgcaat 660
ctacaacatg accgtgattg cggtttgtgc ggagcctacc tctccggcct gtggcggaag 720
acaagaagca tcgagttatt gaacgggcag ttgaaaaaga tattgaccat tcttccaaag 780
ttcgaaaaac aacctagcga caaactattt aacccggtga tcaaggcata tgttgaattt 840
gggcgggttg ccgatgccga agctctggtg catgatatga ccactttata tgggatccct 900
cttcgttgcc gaactcaggg cctattggta tacgccaagg ctctgaactg tgactgggag 960
ggagttgacg caggactgca agagatgcac aaactcaagc tgacgagacg ccggcgagac 1020
ttccttccta tttttgatcg aatatttctg gagtactggg tctcacattc gggaattgag 1080
attcgcaatt ttgtgttccg gtaccttgat aaattcgaca ttgtccccga tcgcgtgctc 1140
tacaagcaca tcttggaggc tttcgtggag aaaggagaca aggaaatgat tgctgaattt 1200
acaagtatgg cgaagcagcg atcgtggaat atccccataa acgagcagca attcttggag 1260
atactacggt ctcgtcgcct cgcattggaa ggagcaccag tggggttctg gcaaatgctg 1320
caggcagcgc gtgtcaagta cgggcacagc tccacgtctc agcgtatcat gggctacgac 1380
caacagtcat tccctttgcc ggaagtcaac agcatgccat atacacagaa tccgctatcg 1440
tggtaccaga gaacgatgca agagaccacg ccgtcgaagc ctgtcgacca atatcagaaa 1500
ctccataagc agatgaccca ttttctgcac gctggaaagc tgaaggaagc attgaagtgc 1560

tttcaaaatg ctaaaaatgc ccggttccag atgaggcagc ttcacgttga attggcggtc 1620
atagcgactt tgcttgagga cggccttagt gcagcgcgca gtctcatcga gtctgaatgg 1680
cggactatcc gtcaccttgt ccgcttctct cctatcttct ttcgtcaagt catggcggtc 1740
gacgaggatg cgggtggcca cattgtccag atggcagtcc tacgcttcta ccagctttgt 1800
tggtctacga aacacatgaa ggtcaagcat caccttactg ttgcgacaag ccgtcgcttg 1860
atctcccaac ataagccgga aatggctctg gagcttctga cggccgtgta caagtcccgt 1920
tataggtttg cagcgacctt tgacggggtt tgcatgaaga tgtttgcgcg cgccttcgcc 1980
gcgacagaca acattctagg cttacgatgg tgtattctta ctgctttatc acgcgatagt 2040
gcactcaatc atgattttgt ggtggaagtt cgccgaatct tgggcactct aagtccacct 2100
tccgcggtgg atgccaccgc cggccccgtc actcatgagc agctggaata cctttattac 2160
atcgccgatc tgctcgagga gaagaatgag ggtgcgcccc cgatatggga gctcaagcat 2220
gacgccacac tgaagcagtc ttcgaggcga cagttgaagc aacctcttga cgcaagccgt 2280
cttttcaacc agtccgatgt ccgcgagacc gtcaagcgat gggacgaaga atacgagctg 2340
gaggcagtgc tgggta 2356

<210> 59
<211> 2356
<212> DNA
<213> Aspergillus fumigatus

<400> 59
atggggttgg aggatccgcg gcgagcagtc caggcagagt cgtatctgga gactacccgg 60
tcgcagctcg tcgatgcgct tatatcacgg ccgaatcttg agagtggctt gggtcgaagg 120
aattttgttc ctcctaggca ggtcccggtt gtcgacgcca tggagcggac gaaaaatgct 180
atacaatcaa ataattccag ctcgcgggcg caactttcgg atgcattacc cgagagcgag 240
aaatcacaga gtgcagggca ggttattgtc ccgacacgga tgcaggaact tctcgaccgc 300
ggtcgtccca ttgaggcggc ccaatttttt ctggagacac atgccgcttc attgaaaggc 360
atatcctcag acaggaagga aatggctacg aaggtattct tcgtcaactg caaagaggac 420
aatgtgttta ttgccagaag tgtgtttgag cggttggaag aggtggatag aatcacgcct 480
gagatgtgga agacgttgat gctggctttg gcgaagaaag ggtgcattga atcagttgcg 540
agtgtttata cgcgatacat gcgcaagttc ccttgccctc cggagatggt cgacgttgtg 600
ctgcggagtt tgcttgaatc ccaccgactc accactgcga aatggttcct cttgcgcaat 660
ctacaacatg accgtgattg cggtttgtgc ggagcctacc tctccggcct gtggcggaag 720
acaagaagca tcgagttatt gaacgggcag ttgaaaaaga tattgaccat tcttccaaag 780
ttcgaaaaac aacctagcga caaactattt aacccggtga tcaaggcata tgttgaattt 840
gggcgggttg ccgatgccga agctctggtg catgatatga ccactttata tgggatccct 900
cttcgttgcc gaactcaggg cctattggta tacgccaagg ctctgaactg tgactgggag 960
ggagttgacg caggactgca agagatgcac aaactcaagc tgacgagacg ccggcgagac 1020
ttccttccta tttttgatcg aatatttctg gagtactggg tctcacattc gggaattgag 1080
attcgcaatt ttgtgttccg gtaccttgat aaattcgaca ttgtccccga tcgcgtgctc 1140
tacaagcaca tcttggaggc tttcgtggag aaaggagaca aggaaatgat tgctgaattt 1200
acaagtatgg cgaagcagcg atcgtggaat atccccataa acgagcagca attcttggag 1260
atactacggt ctcgtcgcct cgcattggaa ggagcaccag tggggttctg gcaaatgctg 1320
caggcagcgc gtgtcaagta cgggcacagc tccacgtctc agcgtatcat gggctacgac 1380
caacagtcat tccctttgcc ggaagtcaac agcatgccat atacacagaa tccgctatcg 1440
tggtaccaga gaacgatgca agagaccacg ccgtcgaagc ctgtcgacca atatcagaaa 1500
ctccataagc agatgaccca ttttctgcac gctggaaagc tgaaggaagc attgaagtgc 1560
tttcaaaatg ctaaaaatgc ccggttccag atgaggcagc ttcacgttga attggcggtc 1620
atagcgactt tgcttgagga cggccttagt gcagcgcgca gtctcatcga gtctgaatgg 1680
cggactatcc gtcaccttgt ccgcttctct cctatcttct ttcgtcaagt catggcggtc 1740
gacgaggatg cgggtggcca cattgtccag atggcagtcc tacgcttcta ccagctttgt 1800
tggtctacga aacacatgaa ggtcaagcat caccttactg ttgcgacaag ccgtcgcttg 1860
atctcccaac ataagccgga aatggctctg gagcttctga cggccgtgta caagtcccgt 1920
tataggtttg cagcgacctt tgacggggtt tgcatgaaga tgtttgcgcg cgccttcgcc 1980
gcgacagaca acattctagg cttacgatgg tgtattctta ctgctttatc acgcgatagt 2040
gcactcaatc atgattttgt ggtggaagtt cgccgaatct tgggcactct aagtccacct 2100
tccgcggtgg atgccaccgc cggccccgtc actcatgagc agctggaata cctttattac 2160
atcgccgatc tgctcgagga gaagaatgag ggtgcgcccc cgatatggga gctcaagcat 2220
gacgccacac tgaagcagtc ttcgaggcga cagttgaagc aacctcttga cgcaagccgt 2280
cttttcaacc agtccgatgt ccgcgagacc gtcaagcgat gggacgaaga atacgagctg 2340

gaggcagtgc tgggta                                                    2356

<210> 60
<211> 785
<212> PRT
<213> Aspergillus fumigatus

<400> 60
Met Gly Leu Glu Asp Pro Arg Arg Ala Val Gln Ala Glu Ser Tyr Leu
1               5                   10                  15

Glu Thr Thr Arg Ser Gln Leu Val Asp Ala Leu Ile Ser Arg Pro Asn
            20                  25                  30

Leu Glu Ser Gly Leu Gly Arg Arg Asn Phe Val Pro Pro Arg Gln Val
        35                  40                  45

Pro Val Val Asp Ala Met Glu Arg Thr Lys Asn Ala Ile Gln Ser Asn
    50                  55                  60

Asn Ser Ser Ser Arg Ala Gln Leu Ser Asp Ala Leu Pro Glu Ser Glu
65                  70                  75                  80

Lys Ser Gln Ser Ala Gly Gln Val Ile Val Pro Thr Arg Met Gln Glu
                85                  90                  95

Leu Leu Asp Arg Gly Arg Pro Ile Glu Ala Ala Gln Phe Phe Leu Glu
            100                 105                 110

Thr His Ala Ala Ser Leu Lys Gly Ile Ser Ser Asp Arg Lys Glu Met
        115                 120                 125

Ala Thr Lys Val Phe Phe Val Asn Cys Lys Glu Asp Asn Val Phe Ile
    130                 135                 140

Ala Arg Ser Val Phe Glu Arg Leu Glu Glu Val Asp Arg Ile Thr Pro
145                 150                 155                 160

Glu Met Trp Lys Thr Leu Met Leu Ala Leu Ala Lys Lys Gly Cys Ile
                165                 170                 175

Glu Ser Val Ala Ser Val Tyr Thr Arg Tyr Met Arg Lys Phe Pro Cys
            180                 185                 190

Pro Pro Glu Met Val Asp Val Val Leu Arg Ser Leu Leu Glu Ser His
        195                 200                 205

Arg Leu Thr Thr Ala Lys Trp Phe Leu Leu Arg Asn Leu Gln His Asp
    210                 215                 220

Arg Asp Cys Gly Leu Cys Gly Ala Tyr Leu Ser Gly Leu Trp Arg Lys
225                 230                 235                 240

Thr Arg Ser Ile Glu Leu Leu Asn Gly Gln Leu Lys Lys Ile Leu Thr
                245                 250                 255

Ile Leu Pro Lys Phe Glu Lys Gln Pro Ser Asp Lys Leu Phe Asn Pro
            260                 265                 270

Val Ile Lys Ala Tyr Val Glu Phe Gly Arg Val Ala Asp Ala Glu Ala

        275                 280                 285

Leu Val His Asp Met Thr Thr Leu Tyr Gly Ile Pro Leu Arg Cys Arg
    290                 295                 300

Thr Gln Gly Leu Leu Val Tyr Ala Lys Ala Leu Asn Cys Asp Trp Glu
305                 310                 315                 320

Gly Val Asp Ala Gly Leu Gln Glu Met His Lys Leu Lys Leu Thr Arg
                325                 330                 335

Arg Arg Arg Asp Phe Leu Pro Ile Phe Asp Arg Ile Phe Leu Glu Tyr
            340                 345                 350

Trp Val Ser His Ser Gly Ile Glu Ile Arg Asn Phe Val Phe Arg Tyr
        355                 360                 365

Leu Asp Lys Phe Asp Ile Val Pro Asp Arg Val Leu Tyr Lys His Ile
    370                 375                 380

Leu Glu Ala Phe Val Glu Lys Gly Asp Lys Glu Met Ile Ala Glu Phe
385                 390                 395                 400

Thr Ser Met Ala Lys Gln Arg Ser Trp Asn Ile Pro Ile Asn Glu Gln
                405                 410                 415

Gln Phe Leu Glu Ile Leu Arg Ser Arg Arg Leu Ala Leu Glu Gly Ala
            420                 425                 430

Pro Val Gly Phe Trp Gln Met Leu Gln Ala Ala Arg Val Lys Tyr Gly
        435                 440                 445

His Ser Ser Thr Ser Gln Arg Ile Met Gly Tyr Asp Gln Gln Ser Phe
    450                 455                 460

Pro Leu Pro Glu Val Asn Ser Met Pro Tyr Thr Gln Asn Pro Leu Ser
465                 470                 475                 480

Trp Tyr Gln Arg Thr Met Gln Glu Thr Thr Pro Ser Lys Pro Val Asp
                485                 490                 495

Gln Tyr Gln Lys Leu His Lys Gln Met Thr His Phe Leu His Ala Gly
            500                 505                 510

Lys Leu Lys Glu Ala Leu Lys Cys Phe Gln Asn Ala Lys Asn Ala Arg
        515                 520                 525

Phe Gln Met Arg Gln Leu His Val Glu Leu Ala Val Ile Ala Thr Leu
    530                 535                 540

Leu Glu Asp Gly Leu Ser Ala Ala Arg Ser Leu Ile Glu Ser Glu Trp
545                 550                 555                 560

Arg Thr Ile Arg His Leu Val Arg Phe Ser Pro Ile Phe Phe Arg Gln
                565                 570                 575

Val Met Ala Val Asp Glu Asp Ala Gly Gly His Ile Val Gln Met Ala
            580                 585                 590

Val Leu Arg Phe Tyr Gln Leu Cys Trp Ser Thr Lys His Met Lys Val
        595                 600                 605

Lys His His Leu Thr Val Ala Thr Ser Arg Arg Leu Ile Ser Gln His
    610                 615                 620

Lys Pro Glu Met Ala Leu Glu Leu Leu Thr Ala Val Tyr Lys Ser Arg
625                 630                 635                 640

Tyr Arg Phe Ala Ala Thr Phe Asp Gly Val Cys Met Lys Met Phe Ala
                645                 650                 655

Arg Ala Phe Ala Ala Thr Asp Asn Ile Leu Gly Leu Arg Trp Cys Ile
            660                 665                 670

Leu Thr Ala Leu Ser Arg Asp Ser Ala Leu Asn His Asp Phe Val Val
        675                 680                 685

Glu Val Arg Arg Ile Leu Gly Thr Leu Ser Pro Pro Ser Ala Val Asp
    690                 695                 700

Ala Thr Ala Gly Pro Val Thr His Glu Gln Leu Glu Tyr Leu Tyr Tyr
705                 710                 715                 720

Ile Ala Asp Leu Leu Glu Glu Lys Asn Glu Gly Cys Ala Pro Ile Trp
                725                 730                 735

Glu Leu Lys His Asp Ala Thr Leu Lys Gln Ser Ser Arg Arg Gln Leu
            740                 745                 750

Lys Gln Pro Leu Asp Ala Ser Arg Leu Phe Asn Gln Ser Asp Val Arg
        755                 760                 765

Glu Thr Val Lys Arg Trp Asp Glu Glu Tyr Glu Leu Glu Ala Val Leu
    770                 775                 780

Gly
785


<210> 61
<211> 21
<212> DNA
<213> Artificial sequence

<220>
<223> PCR primer Imp1

<400> 61
atgaaggcgt aagttccttg c                                                21


<210> 62
<211> 19
<212> DNA
<213> Artificial sequence

<220>
<223> PCR primer Imp2

<400> 62
gtgtggagga agaaagagc                                                   19

<210> 63
<211> 20
<212> DNA
<213> Artificial sequence

<220>
<223> PCR primer ppyr1

<400> 63
ggaagacggg cagttagtcc                                                    20


<210> 64
<211> 21
<212> DNA
<213> Artificial sequence

<220>
<223> PCR primer ppyr3

<400> 64
cccaggcttt acactttatg c                                                  21


<210> 65
<211> 35
<212> DNA
<213> Artificial sequence

<220>
<223> PCR primer PCRa1 1


<220>
<223> misc_feature
<223> (21)..(30)
<223> n is a, c, g, or t

<400> 65
ggccacgcgt cgactagtag nnnnnnnnnn gatat                                   35


<210> 66
<211> 35
<212> DNA
<213> Artificial sequence

<220>
<223> PCR primer PCRa1 3


<220>
<223> misc_feature
<223> (21)..(30)
<223> n is a, c, g, or t

<400> 66
ggccacgcgt cgactagtac nnnnnnnnnn acgtc                                   35

<210> 67
<211> 35
<212> DNA
<213> Artificial sequence

<220>
<223> PCR primer PCRa1 4


<220>
<223> misc_feature
<223> (21)..(30)
<223> n is a, c, g, or t

<400> 67
ggccacgcgt cgactagtac nnnnnnnnnn tggac                                35


<210> 68
<211> 35
<212> DNA
<213> Artificial sequence

<220>
<223> PCR primer PCRa1 5


<220>
<223> misc_feature
<223> (21)..(30)
<223> n is a, c, g, or t

<400> 68
ggccacgcgt cgactagtac nnnnnnnnnn acgtg                                35


<210> 69
<211> 21
<212> DNA
<213> Artificial sequence

<220>
<223> PCR primer ppyr2N

<400> 69
cgaagttgac gttcagtatg c                                              21


<210> 70
<211> 20
<212> DNA
<213> Artificial sequence


<220>
<223> PCR primer ppyr4N

<400> 70
tgaccatgat tacgccaagc                                                20

<210> 71
<211> 20
<212> DNA
<213> Artificial sequence

<220>
<223> PCR primer PCRa1 2N

<400> 71
ggccacgcgt cgactagtac                                                    20


<210> 72
<211> 19
<212> DNA
<213> Artificial sequence

<220>
<223> PCR primer 10.175.2

<400> 72
gttggatctt tgggttctg                                                     19


<210> 73
<211> 20
<212> DNA
<213> Artificial sequence

<220>
<223> PCR primer 10.304.4

<400> 73
cgcgaatctg atgacatagc                                                    20


<210> 74
<211> 20
<212> DNA
<213> Artificial sequence

<220>
<223> PCR primer 10.4.20.2

<400> 74
ctcttcgctt catcgtaccc                                                    20


<210> 75
<211> 20
<212> DNA
<213> Artificial sequence

<220>
<223> PCR primer 11.4.9.4

<400> 75
attagtccat gcgagcatcc                                                    20


<210> 76

<211> 20
<212> DNA
<213> Artificial sequence

<220>
<223> PCR primer 11.6.20.2

<400> 76
gcctgagcct agtccatcac                                                    20


<210> 77
<211> 20
<212> DNA
<213> Artificial sequence

<220>
<223> PCR primer 2.1.1.1

<400> 77
ctcgcaggtc gatttcactc                                                    20


<210> 78
<211> 20
<212> DNA
<213> Artificial sequence

<220>
<223> PCR primer 2.1.1.2

<400> 78
ggaggaaacc ttgtcaccac                                                    20


<210> 79
<211> 20
<212> DNA
<213> Artificial sequence

<220>
<223> PCR primer 2.1.1.5

<400> 79
taccgagaag gaggtcatgg                                                    20


<210> 80
<211> 20
<212> DNA
<213> Artificial sequence

<220>
<223> PCR primer 2.1.1.6

<400> 80
tccagtcaag gttggtgatg                                                    20


<210> 81
<211> 19

<212> DNA
<213> Artificial sequence

<220>
<223> PCR primer 2.1.1.9

<400> 81
cgagaccatc ctacctcag                                                    19


<210> 82
<211> 20
<212> DNA
<213> Artificial sequence

<220>
<223> PCR primer 4.3.4.2

<400> 82
acactcaccg ccttaaccac                                                   20


<210> 83
<211> 20
<212> DNA
<213> Artificial sequence

<220>
<223> PCR primer 5.3.11.2

<400> 83
agtgcccttc attcagttcc                                                   20


<210> 84
<211> 21
<212> DNA
<213> Artificial sequence

<220>
<223> PCR primer 6.8.13.2

<400> 84
gcgactttga gggaactatc c                                                 21


<210> 85
<211> 20
<212> DNA
<213> Artificial sequence

<220>
<223> PCR primer 7.5.9.3

<400> 85
caccacccac cttatgaagc                                                   20


<210> 86
<211> 20
<212> DNA

<213> Artificial sequence

<220>
<223> PCR primer 7.5.9.4

<400> 86
accaggagaa tcagcgacac                                                    20


<210> 87
<211> 20
<212> DNA
<213> Artificial sequence

<220>
<223> PCR primer 8.62.2

<400> 87
gggacgaaga atacgagctg                                                    20


<210> 88
<211> 19
<212> DNA
<213> Artificial sequence

<220>
<223> PCR primer Gt11f1

<400> 88
ctgaatatcg acggtttcc                                                     19


<210> 89
<211> 19
<212> DNA
<213> Artificial sequence

<220>
<223> PCR primer Gt11f3

<400> 89
gcacattggc tgaatatcg                                                     19


<210> 90
<211> 24
<212> DNA
<213> Artificial sequence

<220>
<223> PCR primer Gt11Rev

<400> 90
ttgacaccag accaactggt aatg                                               24


<210> 91
<211> 3052
<212> DNA
<213> Aspergillus fumigatus

<220>
<223> Phylum CEA228; clone 8-47; contig 4842 region 234347-231296
      Genomic sequence containing 3′ and 5′-ends and the coding region

<400> 91
cgagccgctc caagctattc gggatgagac tccggacgcg atccaggaga acacgattgg      60
attagaacaa ttgaaggagg ccatgtccaa agaacgagtc gtaggacgga gcagaaggat     120
acatcgcgct gagagcgcca gaaatgaccg cccggacgga gatgcttggg atgggaatgc     180
gcttggctat gcgtccagga ctaaggggaa gttctttgtc gttgagacag gcaatgaacc     240
tggcaattga ttcggcatgc ctctttccca aaagccattt caatattact gcacaatagc     300
ctgcttcatc ttgcgtgcga cgggctgcac ccctgctatg agagaccgcg cgatcctgct     360
gtgctgagtc agcattggat tacttcatta gacctcgaca gtcaacagca tttctccact     420
taaccaccta caacctaaga cagcacggga actggttata tttgttcgag tcagttactt     480
cagggtgttt tattaaatca atgtggaagt cttttaaaga aaagcatgcc agcaagtttg     540
ggggaggctc ggctgaagcg gcagcctcag acggtggcca agatctgacc acgatattgg     600
atagatccca acgcggggaa ttgacagtgc ttgttgcgct aatcgcacag cggatgcggg     660
atggaataga acaaaacttc tccaatgctc ctccttcgtc aggccagtct gtcaactacg     720
aagaaaaacg gacaggctcc ctgccccaat ctacagatgc acaagaagac cagtcatcct     780
ccggcagcgc agcgaacggt tcaagaaccg accctcaatt caaagacccg gagactgcga     840
catgtgcact atctaaatat gatgactgga gggactccgt ccttcttcga attggtgaag     900
tcgtcaacag ggatccagaa catggtgaag ttcaggcgaa tgaaaaccca ccctcaggac     960
agcaatccca gcagatccgc tccgaggagg atgaccgttc cattcgtaag ctacgtgagg    1020
tctttccgcc ggtggagacc agcctctctc aattgccaga agcgaagaaa ctcttaattc    1080
tccactcatt gctactccta gttttgagtc ttgaacacta taacgcctgg tctcgggtcc    1140
tgatgctgtt cgtgacgtcc agcttagggc tggacgtgaa attactaaac gaagatgaag    1200
tcaaagtggc gagggggttg cttgacactg ctctggcact gtcgtctaac gccccaaggc    1260
aggatgagag tcgaagtcgc gattcatccc gaaaatggaa ggttgggatc gcgtcagttg    1320
cgggtgctgc cctgatcggg atcactggtg gactggctgc gccccttgtt gcagctgggc    1380
ttggtactgt catgggcggc cttggcttgg gcgccaccgc cgcagcaggg tatctcggag    1440
ctctcgctgg aagtggtgtt gtcgtcggcg gacttttcgg tgcttatggt gggcggatga    1500
ccggtcgtat ggttgacaag tacgcacggg aggtggatga ttttgccttt ctgccgattc    1560
gtggttctcg gcatcgatcc gaagacgaaa gagaagctgc ccatcaggat caccggctgc    1620
gggttaccat cggcgtgacc ggatggctga cagaggagga caatttcgtg atcccgtggc    1680
gagtgatcgg agcggaatcg gaggtgtttg gtctccgctg ggaaaccgag cccctgatga    1740
atctgggaaa tgcgcttgac cttttggtaa ccagcgccgc atggactgcc ggtgaacaag    1800
tcctgaagaa gacattcctc tcccaactct tgaccgctgt cgcgctgccg cttggccttc    1860
tcaaggtcgc acgtgtggtg gacaatccgt ttagcgtagc gaaggctcgg gcggacaagg    1920
ctggggaggt tctcgcggat gctcttatca gtaaagtgca gggcgagcga ccagtcaccc    1980
tcattggcta ctccttaggg tctcgggtga ttttcgcttg ccttcaaagc ttggcgaaac    2040
ggcgcgcgtt cggcttggtg gaatccgcaa ttctgatggg agctcccacc ccgtcgaatt    2100
cagaacaatg gtgtcgcatc cgcagtgttg tgagtggacg ccttgtcaac gtgtactcgg    2160
aaaacgactc cgtgctggct ctcttatatc gaacaagcag cctccagctt ggggttgcag    2220
gcttgcagcc tgttgaaggt gtctcaggcg ttgagaatct ggacgttagc gacctgatca    2280
gcggccatct ccgttatcag tttctcgttg gcaggatctt gagcgttgtt ggacttgaga    2340
gcattgatgc tcgcgaggtc gcactggagg aggccgcatt agaagccaaa gatcggaggc    2400
aggagcagga aagggctcat aacgaacgac aggctggatt tatgggcgag ggtcggtcac    2460
caagccagcg gctggaaagc caggaggatc tgcagggcga agaggacaga ttacagaaag    2520
agatgggaaa agcacgagtg cggcactctt agatagtaca tttcaaagcg aatggaacaa    2580
tagctatgat gatttttcct tttctttctg gttatattag agatggacgt ctaaaatcat    2640
tatcccacaa tgatcgaagc agtggaaatg atacatgagg gacacgcaat caggtcagtc    2700
agcagtcgca gtctacccaa ggtaaactca cggacagcag ctaatatccg acagatccgg    2760
gacaattcat ctgaatttga ggctaaaata tgaccaacca agcctccaac caaccccgcc    2820
agtcactggg gctgcaaagc ttggtctagt gaaagtagcc gatcactagt ccaggcaacc    2880
gtgcatcaaa tggcctgtat gcgaacatgg tccctggtta acggaagcca gtctgtttcc    2940
aagcgaggtc aatttcaacg gtgtcctctc tagtggttgt cttcctgcaa ccaggcatgg    3000
tatgatttgt cggcctttag tgccatttcc tgagtcaagc aggacaggtc aa            3052

<210> 92
<211> 2052

<212> DNA
<213> Aspergillus fumigatus

<220>
<223> Phylum CEA228; clone 8-47; contig 4842 region 234347-231296
      Genomic sequence containing the coding region

<400> 92
atgtggaagt cttttaaaga aaagcatgcc agcaagtttg ggggaggctc ggctgaagcg      60
gcagcctcag acggtggcca agatctgacc acgatattgg atagatccca acgcggggaa     120
ttgacagtgc ttgttgcgct aatcgcacag cggatgcggg atggaataga acaaaacttc     180
tccaatgctc ctccttcgtc aggccagtct gtcaactacg aagaaaaacg gacaggctcc     240
ctgccccaat ctacagatgc acaagaagac cagtcatcct ccggcagcgc agcgaacggt     300
tcaagaaccg accctcaatt caaagacccg gagactgcga catgtgcact atctaaatat     360
gatgactgga gggactccgt ccttcttcga attggtgaag tcgtcaacag ggatccagaa     420
catggtgaag ttcaggcgaa tgaaaaccca ccctcaggac agcaatccca gcagatccgc     480
tccgaggagg atgaccgttc cattcgtaag ctacgtgagg tctttccgcc ggtggagacc     540
agcctctctc aattgccaga agcgaagaaa ctcttaattc tccactcatt gctactccta     600
gttttgagtc ttgaacacta taacgcctgg tctcgggtcc tgatgctgtt cgtgacgtcc     660
agcttagggc tggacgtgaa attactaaac gaagatgaag tcaaagtggc gagggggttg     720
cttgacactg ctctggcact gtcgtctaac gccccaaggc aggatgagag tcgaagtcgc     780
gattcatccc gaaaatggaa ggttgggatc gcgtcagttg cgggtgctgc cctgatcggg     840
atcactggtg gactggctgc gccccttgtt gcagctgggc ttggtactgt catgggcggc     900
cttgggcttg gcgccaccgc cgcagcaggg tatctcggag ctctcgctgg aagtggtgtt     960
gtcgtcggcg gacttttcgg tgcttatggt gggcggatga ccggtcgtat ggttgacaag    1020
tacgcacggg aggtggatga ttttgccttt ctgccgattc gtggttctcg gcatcgatcc    1080
gaagacgaaa gagaagctgc ccatcaggat caccggctgc gggttaccat cggcgtgacc    1140
ggatggctga cagaggagga caatttcgtg atcccgtggc gagtgatcgg agcggaatcg    1200
gaggtgtttg gtctccgctg ggaaaccgag cccctgatga atctgggaaa tgcgcttgac    1260
cttttggtaa ccagcgccgc atggactgcc ggtgaacaag tcctgaagaa gacattcctc    1320
tcccaactct tgaccgctgt cgcgctgccg cttggccttc tcaaggtcgc acgtgtggtg    1380
gacaatccgt ttagcgtagc gaaggctcgg gcggacaagg ctggggaggt tctcgcggat    1440
gctcttatca gtaaagtgca gggcgagcga ccagtcaccc tcattggcta ctccttaggg    1500
tctcgggtga ttttcgcttg ccttcaaagc ttggcgaaac ggcgcgcgtt cggcttggtg    1560
gaatccgcaa ttctgatggg agctcccacc ccgtcgaatt cagaacaatg gtgtcgcatc    1620
cgcagtgttg tgagtggacg ccttgtcaac gtgtactcgg aaaacgactc cgtgctggct    1680
ctcttatatc gaacaagcag cctccagctt ggggttgcag gcttgcagcc tgttgaaggt    1740
gtctcaggcg ttgagaatct ggacgttagc gacctgatca gcggccatct ccgttatcag    1800
tttctcgttg gcaggatctt gagcgttgtt ggacttgaga gcattgatgc tcgcgaggtc    1860
gcactggagg aggccgcatt agaagccaaa gatcggaggc aggagcagga aagggctcat    1920
aacgaacgac aggctggatt tatgggcgag ggtcggtcac caagccagcg gctggaaagc    1980
caggaggatc tgcagggcga agaggacaga ttacagaaag agatgggaaa agcacgagtg    2040
cggcactctt ag                                                        2052


<210> 93
<211> 2052
<212> DNA
<213> Aspergillus fumigatus

<220>
<223> Phylum CEA228; clone 8-47; contig 4842 region 234347-231296
      Coding region without introns

<400> 93
atgtggaagt cttttaaaga aaagcatgcc agcaagtttg ggggaggctc ggctgaagcg      60
gcagcctcag acggtggcca agatctgacc acgatattgg atagatccca acgcggggaa     120
ttgacagtgc ttgttgcgct aatcgcacag cggatgcggg atggaataga acaaaacttc     180
tccaatgctc ctccttcgtc aggccagtct gtcaactacg aagaaaaacg gacaggctcc     240
ctgccccaat ctacagatgc acaagaagac cagtcatcct ccggcagcgc agcgaacggt     300
tcaagaaccg accctcaatt caaagacccg gagactgcga catgtgcact atctaaatat     360

gatgactgga gggactccgt ccttcttcga attggtgaag tcgtcaacag ggatccagaa 420
catggtgaag ttcaggcgaa tgaaaaccca ccctcaggac agcaatccca gcagatccgc 480
tccgaggagg atgaccgttc cattcgtaag ctacgtgagg tctttccgcc ggtggagacc 540
agcctctctc aattgccaga agcgaagaaa ctcttaattc tccactcatt gctactccta 600
gttttgagtc ttgaacacta taacgcctgg tctcgggtcc tgatgctgtt cgtgacgtcc 660
agcttagggc tggacgtgaa attactaaac gaagatgaag tcaaagtggc gagggggttg 720
cttgacactg ctctggcact gtcgtctaac gccccaaggc aggatgagag tcgaagtcgc 780
gattcatccc gaaaatggaa ggttgggatc gcgtcagttg cgggtgctgc cctgatcggg 840
atcactggtg gactggctgc gccccttgtt gcagctgggc ttggtactgt catgggcggc 900
cttgggcttg gcgccaccgc cgcagcaggg tatctcggag ctctcgctgg aagtggtgtt 960
gtcgtcggcg gacttttcgg tgcttatggt gggcggatga ccggtcgtat ggttgacaag 1020
tacgcacggg aggtggatga ttttgccttt ctgccgattc gtggttctcg gcatcgatcc 1080
gaagacgaaa gagaagctgc ccatcaggat caccggctgc gggttaccat cggcgtgacc 1140
ggatggctga cagaggagga caatttcgtg atcccgtggc gagtgatcgg agcggaatcg 1200
gaggtgtttg gtctccgctg ggaaaccgag cccctgatga atctgggaaa tgcgcttgac 1260
cttttggtaa ccagcgccgc atggactgcc ggtgaacaag tcctgaagaa gacattcctc 1320
tcccaactct tgaccgctgt cgcgctgccg cttggccttc tcaaggtcgc acgtgtggtg 1380
gacaatccgt ttagcgtagc gaaggctcgg gcggacaagg ctggggaggt tctcgcggat 1440
gctcttatca gtaaagtgca gggcgagcga ccagtcaccc tcattggcta ctccttaggg 1500
tctcgggtga ttttcgcttg ccttcaaagc ttggcgaaac ggcgcgcgtt cggcttggtg 1560
gaatccgcaa ttctgatggg agctcccacc ccgtcgaatt cagaacaatg gtgtcgcatc 1620
cgcagtgttg tgagtggacg ccttgtcaac gtgtactcgg aaaacgactc cgtgctggct 1680
ctcttatatc gaacaagcag cctccagctt ggggttgcag gcttgcagcc tgttgaaggt 1740
gtctcaggcg ttgagaatct ggacgttagc gacctgatca gcggccatct ccgttatcag 1800
tttctcgttg gcaggatctt gagcgttgtt ggacttgaga gcattgatgc tcgcgaggtc 1860
gcactggagg aggccgcatt agaagccaaa gatcggaggc aggagcagga aagggctcat 1920
aacgaacgac aggctggatt tatgggcgag ggtcggtcac caagccagcg gctggaaagc 1980
caggaggatc tgcagggcga agaggacaga ttacagaaag agatgggaaa agcacgagtg 2040
cggcactctt ag 2052

<210> 94
<211> 683
<212> PRT
<213> Aspergillus fumigatus

<220>
<223> Phylum CEA228; clone 8-47; contig 4842 region 234347-231296
      Protein sequence

<400> 94

Met Trp Lys Ser Phe Lys Glu Lys His Ala Ser Lys Phe Gly Gly Gly
1               5                   10                  15

Ser Ala Glu Ala Ala Ala Ser Asp Gly Gly Gln Asp Leu Thr Thr Ile
            20                  25                  30

Leu Asp Arg Ser Gln Arg Gly Glu Leu Thr Val Leu Val Ala Leu Ile
        35                  40                  45

Ala Gln Arg Met Arg Asp Gly Ile Glu Gln Asn Phe Ser Asn Ala Pro
    50                  55                  60

Pro Ser Ser Gly Gln Ser Val Asn Tyr Glu Glu Lys Arg Thr Gly Ser
65                  70                  75                  80

Leu Pro Gln Ser Thr Asp Ala Gln Glu Asp Gln Ser Ser Ser Gly Ser
                85                  90                  95

Ala Ala Asn Gly Ser Arg Thr Asp Pro Gln Phe Lys Asp Pro Glu Thr

            100                 105                 110

Ala Thr Cys Ala Leu Ser Lys Tyr Asp Asp Trp Arg Asp Ser Val Leu
        115                 120                 125

Leu Arg Ile Gly Glu Val Val Asn Arg Asp Pro Glu His Gly Glu Val
    130                 135                 140

Gln Ala Asn Glu Asn Pro Pro Ser Gly Gln Gln Ser Gln Gln Ile Arg
145                 150                 155                 160

Ser Glu Glu Asp Asp Arg Ser Ile Arg Lys Leu Arg Glu Val Phe Pro
                165                 170                 175

Pro Val Glu Thr Ser Leu Ser Gln Leu Pro Glu Ala Lys Lys Leu Leu
            180                 185                 190

Ile Leu His Ser Leu Leu Leu Leu Val Leu Ser Leu Glu His Tyr Asn
        195                 200                 205

Ala Trp Ser Arg Val Leu Met Leu Phe Val Thr Ser Ser Leu Gly Leu
    210                 215                 220

Asp Val Lys Leu Leu Asn Glu Asp Glu Val Lys Val Ala Arg Gly Leu
225                 230                 235                 240

Leu Asp Thr Ala Leu Ala Leu Ser Ser Asn Ala Pro Arg Gln Asp Glu
                245                 250                 255

Ser Arg Ser Arg Asp Ser Ser Arg Lys Trp Lys Val Gly Ile Ala Ser
            260                 265                 270

Val Ala Gly Ala Ala Leu Ile Gly Ile Thr Gly Gly Leu Ala Ala Pro
        275                 280                 285

Leu Val Ala Ala Gly Leu Gly Thr Val Met Gly Gly Leu Gly Leu Gly
    290                 295                 300

Ala Thr Ala Ala Ala Gly Tyr Leu Gly Ala Leu Ala Gly Ser Gly Val
305                 310                 315                 320

Val Val Gly Gly Leu Phe Gly Ala Tyr Gly Gly Arg Met Thr Gly Arg
                325                 330                 335

Met Val Asp Lys Tyr Ala Arg Glu Val Asp Asp Phe Ala Phe Leu Pro
            340                 345                 350

Ile Arg Gly Ser Arg His Arg Ser Glu Asp Glu Arg Glu Ala Ala His
        355                 360                 365

Gln Asp His Arg Leu Arg Val Thr Ile Gly Val Thr Gly Trp Leu Thr
    370                 375                 380

Glu Glu Asp Asn Phe Val Ile Pro Trp Arg Val Ile Gly Ala Glu Ser
385                 390                 395                 400

Glu Val Phe Gly Leu Arg Trp Glu Thr Glu Pro Leu Met Asn Leu Gly
                405                 410                 415

Asn Ala Leu Asp Leu Leu Val Thr Ser Ala Ala Trp Thr Ala Gly Glu
            420                 425                 430

Gln Val Leu Lys Lys Thr Phe Leu Ser Gln Leu Leu Thr Ala Val Ala
        435                 440                 445

Leu Pro Leu Gly Leu Leu Lys Val Ala Arg Val Val Asp Asn Pro Phe
    450                 455                 460

Ser Val Ala Lys Ala Arg Ala Asp Lys Ala Gly Glu Val Leu Ala Asp
465                 470                 475                 480

Ala Leu Ile Ser Lys Val Gln Gly Glu Arg Pro Val Thr Leu Ile Gly
                485                 490                 495

Tyr Ser Leu Gly Ser Arg Val Ile Phe Ala Cys Leu Gln Ser Leu Ala
            500                 505                 510

Lys Arg Arg Ala Phe Gly Leu Val Glu Ser Ala Ile Leu Met Gly Ala
        515                 520                 525

Pro Thr Pro Ser Asn Ser Glu Gln Trp Cys Arg Ile Arg Ser Val Val
    530                 535                 540

Ser Gly Arg Leu Val Asn Val Tyr Ser Glu Asn Asp Ser Val Leu Ala
545                 550                 555                 560

Leu Leu Tyr Arg Thr Ser Ser Leu Gln Leu Gly Val Ala Gly Leu Gln
                565                 570                 575

Pro Val Glu Gly Val Ser Gly Val Glu Asn Leu Asp Val Ser Asp Leu
            580                 585                 590

Ile Ser Gly His Leu Arg Tyr Gln Phe Leu Val Gly Arg Ile Leu Ser
        595                 600                 605

Val Val Gly Leu Glu Ser Ile Asp Ala Arg Glu Val Ala Leu Glu Glu
    610                 615                 620

Ala Ala Leu Glu Ala Lys Asp Arg Arg Gln Glu Gln Glu Arg Ala His
625                 630                 635                 640

Asn Glu Arg Gln Ala Gly Phe Met Gly Glu Gly Arg Ser Pro Ser Gln
                645                 650                 655

Arg Leu Glu Ser Gln Glu Asp Leu Gln Gly Glu Glu Asp Arg Leu Gln
            660                 665                 670

Lys Glu Met Gly Lys Ala Arg Val Arg His Ser
        675                 680


<210> 95
<211> 3814
<212> DNA
<213> Aspergillus fumigatus

<220>
<223> Phylum CEA229; clone 8-62; contig 4938 region 215653-219466
      Genomic sequence containing 3' and 5'-ends and the coding region

<400> 95
ctttagtagc agttgtttct gaatattgca gtcatttcaa agagtccacg atgaagctca      60

gaggagctgc ttacgattat atcgctaaaa gtacattaag ggacgctctt tgaaactttg 120
tgttggacgt cacttataca gatcgaaggg tttgcaggaa aataggagac tactccatgt 180
aggtaggtag cattttacta tcgacgctga tagttgtcgt atgatctgag ctggatccgg 240
agagcgatcg gaggactacg gcacgtgaca aagcctgcga gtgcaccgcc atgcaaactt 300
tccgctggag ctcagcaaaa agtccaccga aattgccctg ttgaatattc caacccaatt 360
gtgcccattg tcgtccccca acttttcttt cgggcattgt ccccattagt tgagctttat 420
ctcagtttac tgtttatatc aatgcttcgc aggtgacgcc catagaaccc gccgcctgcg 480
gtgcaagagg ttctaccacc atgttctcga aaccaaccaa cagccccccg gctgtcccct 540
ctcgaaatgc tctccgagtt ctgcgtagat tagcgcttgc cggctcaact gtgggcagtt 600
tctgcacggt cgcggccatc acctacgatg tccatcgccg agtccgagtc gccgagcgca 660
ttgtcgagaa caaacgggcc ttacagacct ccgcacccaa ctacgatgcc acgtctgccg 720
cgaaacgact cgcccgtatg atggaagcag cggaagcagg cgaatttatg ggattggcgt 780
cgttgaagga ggcggacaga aagatccgag aagggcaagc tacgcaggat gacgatgtgg 840
tagctttgca ccaggagggg ggctttcgac gtccgggcat ggggttggag gatccgcggc 900
gagcagtcca ggcagagtcg tatctggaga ctacccggtc gcagctcgtc gatgcgctta 960
tatcacggcc gaatcttgag agtggcttgg gtcgaaggaa ttttgttcct cctaggcagg 1020
tcccggttgt cgacgccatg gagcggacga aaaatgctat acaatcaaat aattccagct 1080
cgcgggcgca actttcggat gcattacccg agagcgagaa atcacagagt gcagggcagg 1140
ttattgtccc gacacggatg caggaacttc tcgaccgcgg tcgtcccatt gaggcggccc 1200
aattttttct ggagacacat gccgcttcat tgaaaggcat atcctcagac aggaaggaaa 1260
tggctacgaa ggtattcttc gtcaactgca aagaggacaa tgtgtttatt gccagaagtg 1320
tgtttgagcg gttggaagag gtggatagaa tcacgcctga gatgtggaag acgttgatgc 1380
tggctttggc gaagaaaggg tgcattgaat cagttgcgag tgtttatacg cgatacatgc 1440
gcaagttccc ttgccctccg gagatggtcg acgttgtgct gcggagtttg cttgaatccc 1500
accgactcac cactgcgaaa tggttcctct tgcgcaatct acaacatgac cgtgattgcg 1560
gtttgtgcgg agcctacctc tccggcctgt ggcggaagac aagaagcatc gagttattga 1620
acgggcagtt gaaaaagata ttgaccattc ttccaaagtt cgaaaaacaa cctagcgaca 1680
aactatttaa cccggtgatc aaggcatatg ttgaatttgg gcgggttgcc gatgccgaag 1740
ctctggtgca tgatatgacc actttatatg ggatccctct tcgttgccga actcagggcc 1800
tattggtata cgccaaggct ctgaactgtg actgggaggg agttgacgca ggactgcaag 1860
agatgcacaa actcaagctg acgagacgcc ggcgagactt ccttcctatt tttgatcgaa 1920
tatttctgga gtactgggtc tcacattcgg gaattgagat tcgcaatttt gtgttccggt 1980
accttgataa attcgacatt gtccccgatc gcgtgctcta caagcacatc ttggaggctt 2040
tcgtggagaa aggagacaag gaaatgattg ctgaatttac aagtatggcg aagcagcgat 2100
cgtggaatat ccccataaac gagcagcaat tcttggagat actacggtct cgtcgcctcg 2160
cattggaagg agcaccagtg gggttctggc aaatgctgca ggcagcgcgt gtcaagtacg 2220
ggcacagctc cacgtctcag cgtatcatgg gctacgacca acagtcattc cctttgccgg 2280
aagtcaacag catgccatat acacagaatc cgctatcgtg gtaccagaga acgatgcaag 2340
agaccacgcc gtcgaagcct gtcgaccaat atcagaaact ccataagcag atgacccatt 2400
ttctgcacgc tggaaagctg aaggaagcat tgaagtgctt tcaaaatgct aaaaatgccc 2460
ggttccagat gaggcagctt cacgttgaat tggcggtcat agcgactttg cttgaggacg 2520
gccttagtgc agcgcgcagt ctcatcgagt ctgaatggcg gactatccgt caccttgtcc 2580
gcttctctcc tatcttcttt cgtcaagtca tggcggtcga cgaggatgcg ggtggccaca 2640
ttgtccagat ggcagtccta cgcttctacc agctttgttg gtctacgaaa cacatgaagg 2700
tcaagcatca ccttactgtt gcgacaagcc gtcgcttgat ctcccaacat aagccggaaa 2760
tggctctgga gcttctgacg gccgtgtaca agtcccgtta taggtttgca gcgacctttg 2820
acggggtttg catgaagatg tttgcgcgcg ccttcgccgc gacagacaac attctaggct 2880
tacgatggtg tattcttact gctttatcac gcgatagtgc actcaatcat gattttgtgg 2940
tggaagttcg ccgaatcttg ggcactctaa gtccaccttc cgcggtggat gccaccgccg 3000
gccccgtcac tcatgagcag ctggaatacc tttattacat cgccgatctg ctcgaggaga 3060
agaatgaggg gtgcgccccg atatgggagc tcaagcatga cgccacactg aagcagtctt 3120
cgaggcgaca gttgaagcaa cctcttgacg caagccgtct tttcaaccag tccgatgtcc 3180
gcgagaccgt caagcgatgg gacgaagaat acgagctgga ggcagtgctg ggtaggattg 3240
acaatgaccc gaactcgatt actgctcgat ggaacgagag tcgtcgttca catcagaaag 3300
aggcgggttt atgacgatcg gtctgatacc atctaggaag tggttgcaac tgcaacgcct 3360
ggccgagcct ttattgaatc atgtgatagg ctccttagcc actgtatata tactattgac 3420
actctgtact ttactctcct gtgcggtttg tgactgccat tatttctcat cggaaataga 3480
atagagatgg tgagattgga tgcaaatatc acggatatgg ccgaaacccc ttggctctcc 3540
ttttgcaggc aacaacgccg aacggtatcc aataataaac actgtaattg atcggatggg 3600
atcagaccag aagatgctct tttccggtcc tttcagtgat catacggtgt tcctgaattt 3660
tctctcacca ataagaatct agcactgcgg aaggaggaaa gccagtcttt cacatgtcac 3720

gccactgtcc actcttggcc cctttccagt aaaaacactt aaagttctcc gatctttttc 3780
tatctccgcc atcccatcct tttcatcaat tgct 3814

<210> 96
<211> 2814
<212> DNA
<213> Aspergillus fumigatus

<220>
<223> Phylum CEA229; clone 8-62; contig 4938 region 215653-219466
      Genomic sequence containing the coding region

<400> 96
atgttctcga aaccaaccaa cagccccccg gctgtcccct ctcgaaatgc tctccgagtt 60
ctgcgtagat tagcgcttgc cggctcaact gtgggcagtt tctgcacggt cgcggccatc 120
acctacgatg tccatcgccg agtccgagtc gccgagcgca ttgtcgagaa caaacgggcc 180
ttacagacct ccgcacccaa ctacgatgcc acgtctgccg cgaaacgact cgcccgtatg 240
atggaagcag cggaagcagg cgaatttatg ggattggcgt cgttgaagga ggcggacaga 300
aagatccgag aagggcaagc tacgcaggat gacgatgtgg tagctttgca ccaggagggg 360
ggctttcgac gtccgggcat ggggttggag gatccgcggc gagcagtcca ggcagagtcg 420
tatctggaga ctacccggtc gcagctcgtc gatgcgctta tatcacggcc gaatcttgag 480
agtggcttgg gtcgaaggaa ttttgttcct cctaggcagg tcccggttgt cgacgccatg 540
gagcggacga aaaatgctat acaatcaaat aattccagct cgcgggcgca actttcggat 600
gcattacccg agagcgagaa atcacagagt gcagggcagg ttattgtccc gacacggatg 660
caggaacttc tcgaccgcgg tcgtcccatt gaggcggccc aattttttct ggagacacat 720
gccgcttcat tgaaaggcat atcctcagac aggaaggaaa tggctacgaa ggtattcttc 780
gtcaactgca aagaggacaa tgtgtttatt gccagaagtg tgtttgagcg gttggaagag 840
gtggatagaa tcacgcctga gatgtggaag acgttgatgc tggctttggc gaagaaaggg 900
tgcattgaat cagttgcgag tgtttatacg cgatacatgc gcaagttccc ttgccctccg 960
gagatggtcg acgttgtgct gcggagtttg cttgaatccc accgactcac cactgcgaaa 1020
tggttcctct tgcgcaatct acaacatgac cgtgattgcg gtttgtgcgg agcctacctc 1080
tccggcctgt ggcggaagac aagaagcatc gagttattga acgggcagtt gaaaaagata 1140
ttgaccattc ttccaaagtt cgaaaaacaa cctagcgaca aactatttaa cccggtgatc 1200
aaggcatatg ttgaatttgg gcgggttgcc gatgccgaag ctctggtgca tgatatgacc 1260
actttatatg ggatccctct tcgttgccga actcagggcc tattggtata cgccaaggct 1320
ctgaactgtg actgggaggg agttgacgca ggactgcaag agatgcacaa actcaagctg 1380
acgagacgcc ggcgagactt ccttcctatt tttgatcgaa tatttctgga gtactgggtc 1440
tcacattcgg gaattgagat tcgcaatttt gtgttccggt accttgataa attcgacatt 1500
gtccccgatc gcgtgctcta caagcacatc ttggaggctt tcgtggagaa aggagacaag 1560
gaaatgattg ctgaatttac aagtatggcg aagcagcgat cgtggaatat ccccataaac 1620
gagcagcaat tcttggagat actacggtct cgtcgcctcg cattggaagg agcaccagtg 1680
gggttctggc aaatgctgca ggcagcgcgt gtcaagtacg ggcacagctc cacgtctcag 1740
cgtatcatgg gctacgacca acagtcattc cctttgccgg aagtcaacag catgccatat 1800
acacagaatc cgctatcgtg gtaccagaga acgatgcaag agaccacgcc gtcgaagcct 1860
gtcgaccaat atcagaaact ccataagcag atgacccatt ttctgcacgc tggaaagctg 1920
aaggaagcat tgaagtgctt tcaaaatgct aaaaatgccc ggttccagat gaggcagctt 1980
cacgttgaat tggcggtcat agcgactttg cttgaggacg gccttagtgc agcgcgcagt 2040
ctcatcgagt ctgaatggcg gactatccgt caccttgtcc gcttctctcc tatcttcttt 2100
cgtcaagtca tggcggtcga cgaggatgcg ggtggccaca ttgtccagat ggcagtccta 2160
cgcttctacc agctttgttg gtctacgaaa cacatgaagg tcaagcatca ccttactgtt 2220
gcgacaagcc gtcgcttgat ctcccaacat aagccggaaa tggctctgga gcttctgacg 2280
gccgtgtaca agtcccgtta taggtttgca gcgacctttg acggggtttg catgaagatg 2340
tttgcgcgcg ccttcgccgc gacagacaac attctaggct tacgatggtg tattcttact 2400
gctttatcac gcgatagtgc actcaatcat gattttgtgg tggaagttcg ccgaatcttg 2460
ggcactctaa gtccaccttc cgcggtggat gccaccgccg gccccgtcac tcatgagcag 2520
ctggaatacc tttattacat cgccgatctg ctcgaggaga agaatgaggg gtgcgccccg 2580
atatgggagc tcaagcatga cgccacactg aagcagtctt cgaggcgaca gttgaagcaa 2640
cctcttgacg caagccgtct tttcaaccag tccgatgtcc gcgagaccgt caagcgatgg 2700
gacgaagaat acgagctgga ggcagtgctg ggtaggattg acaatgaccc gaactcgatt 2760
actgctcgat ggaacgagag tcgtcgttca catcagaaag aggcgggttt atga 2814

<210> 97
<211> 2814
<212> DNA
<213> Aspergillus fumigatus

<220>
<223> Phylum CEA229; clone 8-62; contig 4938 region 215653-219466
      Coding region without introns

<400> 97
atgttctcga aaccaaccaa cagccccccg gctgtcccct ctcgaaatgc tctccgagtt      60
ctgcgtagat tagcgcttgc cggctcaact gtgggcagtt tctgcacggt cgcggccatc     120
acctacgatg tccatcgccg agtccgagtc gccgagcgca ttgtcgagaa caaacgggcc     180
ttacagacct ccgcacccaa ctacgatgcc acgtctgccg cgaaacgact cgcccgtatg     240
atggaagcag cggaagcagg cgaatttatg ggattggcgt cgttgaagga ggcggacaga     300
aagatccgag aagggcaagc tacgcaggat gacgatgtgg tagctttgca ccaggagggg     360
ggctttcgac gtccgggcat ggggttggag gatccgcggc gagcagtcca ggcagagtcg     420
tatctggaga ctacccggtc gcagctcgtc gatgcgctta tatcacggcc gaatcttgag     480
agtggcttgg gtcgaaggaa ttttgttcct cctaggcagg tcccggttgt cgacgccatg     540
gagcggacga aaaatgctat acaatcaaat aattccagct cgcgggcgca actttcggat     600
gcattacccg agagcgagaa atcacagagt gcagggcagg ttattgtccc gacacggatg     660
caggaacttc tcgaccgcgg tcgtcccatt gaggcggccc aattttttct ggagacacat     720
gccgcttcat tgaaaggcat atcctcagac aggaaggaaa tggctacgaa ggtattcttc     780
gtcaactgca aagaggacaa tgtgtttatt gccagaagtg tgtttgagcg gttggaagag     840
gtggatagaa tcacgcctga gatgtggaag acgttgatgc tggctttggc gaagaaaggg     900
tgcattgaat cagttgcgag tgtttatacg cgatacatgc gcaagttccc ttgccctccg     960
gagatggtcg acgttgtgct gcggagtttg cttgaatccc accgactcac cactgcgaaa    1020
tggttcctct tgcgcaatct acaacatgac cgtgattgcg gtttgtgcgg agcctacctc    1080
tccggcctgt ggcggaagac aagaagcatc gagttattga acgggcagtt gaaaaagata    1140
ttgaccattc ttccaaagtt cgaaaaacaa cctagcgaca aactatttaa cccggtgatc    1200
aaggcatatg ttgaatttgg gcgggttgcc gatgccgaag ctctggtgca tgatatgacc    1260
actttatatg ggatccctct tcgttgccga actcagggcc tattggtata cgccaaggct    1320
ctgaactgtg actgggaggg agttgacgca ggactgcaag agatgcacaa actcaagctg    1380
acgagacgcc ggcgagactt ccttcctatt tttgatcgaa tatttctgga gtactgggtc    1440
tcacattcgg gaattgagat tcgcaatttt gtgttccggt accttgataa attcgacatt    1500
gtccccgatc gcgtgctcta caagcacatc ttggaggctt tcgtggagaa aggagacaag    1560
gaaatgattg ctgaatttac aagtatggcg aagcagcgat cgtggaatat ccccataaac    1620
gagcagcaat tcttggagat actacggtct cgtcgcctcg cattggaagg agcaccagtg    1680
gggttctggc aaatgctgca ggcagcgcgt gtcaagtacg ggcacagctc cacgtctcag    1740
cgtatcatgg gctacgacca acagtcattc cctttgccgg aagtcaacag catgccatat    1800
acacagaatc cgctatcgtg gtaccagaga acgatgcaag agaccacgcc gtcgaagcct    1860
gtcgaccaat atcagaaact ccataagcag atgacccatt ttctgcacgc tggaaagctg    1920
aaggaagcat tgaagtgctt tcaaaatgct aaaaatgccc ggttccagat gaggcagctt    1980
cacgttgaat tggcggtcat agcgactttg cttgaggacg gccttagtgc agcgcgcagt    2040
ctcatcgagt ctgaatggcg gactatccgt caccttgtcc gcttctctcc tatcttcttt    2100
cgtcaagtca tggcggtcga cgaggatgcg ggtggccaca ttgtccagat ggcagtccta    2160
cgcttctacc agctttgttg gtctacgaaa cacatgaagg tcaagcatca ccttactgtt    2220
gcgacaagcc gtcgcttgat ctcccaacat aagccggaaa tggctctgga gcttctgacg    2280
gccgtgtaca agtcccgtta taggtttgca gcgacctttg acggggtttg catgaagatg    2340
tttgcgcgcg ccttcgccgc gacagacaac attctaggct tacgatggtg tattcttact    2400
gctttatcac gcgatagtgc actcaatcat gattttgtgg tggaagttcg ccgaatcttg    2460
ggcactctaa gtccaccttc cgcggtggat gccaccgccg gccccgtcac tcatgagcag    2520
ctggaatacc tttattacat cgccgatctg ctcgaggaga agaatgaggg gtgcgccccg    2580
atatgggagc tcaagcatga cgccacactg aagcagtctt cgaggcgaca gttgaagcaa    2640
cctcttgacg caagccgtct tttcaaccag tccgatgtcc gcgagaccgt caagcgatgg    2700
gacgaagaat acgagctgga ggcagtgctg ggtaggattg acaatgaccc gaactcgatt    2760
actgctcgat ggaacgagag tcgtcgttca catcagaaag aggcgggttt atga          2814

<210> 98
<211> 937
<212> PRT
<213> Aspergillus fumigatus

<220>
<223> Phylum CEA229; clone 8-62; contig 4938 region 215653-219466
      Protein sequence

<400> 98

Met Phe Ser Lys Pro Thr Asn Ser Pro Pro Ala Val Pro Ser Arg Asn
1               5                   10                  15

Ala Leu Arg Val Leu Arg Arg Leu Ala Leu Ala Gly Ser Thr Val Gly
            20                  25                  30

Ser Phe Cys Thr Val Ala Ala Ile Thr Tyr Asp Val His Arg Arg Val
        35                  40                  45

Arg Val Ala Glu Arg Ile Val Glu Asn Lys Arg Ala Leu Gln Thr Ser
    50                  55                  60

Ala Pro Asn Tyr Asp Ala Thr Ser Ala Ala Lys Arg Leu Ala Arg Met
65                  70                  75                  80

Met Glu Ala Ala Glu Ala Gly Glu Phe Met Gly Leu Ala Ser Leu Lys
                85                  90                  95

Glu Ala Asp Arg Lys Ile Arg Glu Gly Gln Ala Thr Gln Asp Asp Asp
            100                 105                 110

Val Val Ala Leu His Gln Glu Gly Gly Phe Arg Arg Pro Gly Met Gly
        115                 120                 125

Leu Glu Asp Pro Arg Arg Ala Val Gln Ala Glu Ser Tyr Leu Glu Thr
    130                 135                 140

Thr Arg Ser Gln Leu Val Asp Ala Leu Ile Ser Arg Pro Asn Leu Glu
145                 150                 155                 160

Ser Gly Leu Gly Arg Arg Asn Phe Val Pro Pro Arg Gln Val Pro Val
                165                 170                 175

Val Asp Ala Met Glu Arg Thr Lys Asn Ala Ile Gln Ser Asn Asn Ser
            180                 185                 190

Ser Ser Arg Ala Gln Leu Ser Asp Ala Leu Pro Glu Ser Glu Lys Ser
        195                 200                 205

Gln Ser Ala Gly Gln Val Ile Val Pro Thr Arg Met Gln Glu Leu Leu
    210                 215                 220

Asp Arg Gly Arg Pro Ile Glu Ala Ala Gln Phe Phe Leu Glu Thr His
225                 230                 235                 240

Ala Ala Ser Leu Lys Gly Ile Ser Ser Asp Arg Lys Glu Met Ala Thr
                245                 250                 255

Lys Val Phe Phe Val Asn Cys Lys Glu Asp Asn Val Phe Ile Ala Arg
            260                 265                 270

Ser Val Phe Glu Arg Leu Glu Glu Val Asp Arg Ile Thr Pro Glu Met
        275                 280                 285

Trp Lys Thr Leu Met Leu Ala Leu Ala Lys Lys Gly Cys Ile Glu Ser
    290                 295                 300

Val Ala Ser Val Tyr Thr Arg Tyr Met Arg Lys Phe Pro Cys Pro Pro
305                 310                 315                 320

Glu Met Val Asp Val Val Leu Arg Ser Leu Leu Glu Ser His Arg Leu
                325                 330                 335

Thr Thr Ala Lys Trp Phe Leu Leu Arg Asn Leu Gln His Asp Arg Asp
            340                 345                 350

Cys Gly Leu Cys Gly Ala Tyr Leu Ser Gly Leu Trp Arg Lys Thr Arg
        355                 360                 365

Ser Ile Glu Leu Leu Asn Gly Gln Leu Lys Lys Ile Leu Thr Ile Leu
    370                 375                 380

Pro Lys Phe Glu Lys Gln Pro Ser Asp Lys Leu Phe Asn Pro Val Ile
385                 390                 395                 400

Lys Ala Tyr Val Glu Phe Gly Arg Val Ala Asp Ala Glu Ala Leu Val
                405                 410                 415

His Asp Met Thr Thr Leu Tyr Gly Ile Pro Leu Arg Cys Arg Thr Gln
            420                 425                 430

Gly Leu Leu Val Tyr Ala Lys Ala Leu Asn Cys Asp Trp Glu Gly Val
        435                 440                 445

Asp Ala Gly Leu Gln Glu Met His Lys Leu Lys Leu Thr Arg Arg Arg
    450                 455                 460

Arg Asp Phe Leu Pro Ile Phe Asp Arg Ile Phe Leu Glu Tyr Trp Val
465                 470                 475                 480

Ser His Ser Gly Ile Glu Ile Arg Asn Phe Val Phe Arg Tyr Leu Asp
                485                 490                 495

Lys Phe Asp Ile Val Pro Asp Arg Val Leu Tyr Lys His Ile Leu Glu
            500                 505                 510

Ala Phe Val Glu Lys Gly Asp Lys Glu Met Ile Ala Glu Phe Thr Ser
        515                 520                 525

Met Ala Lys Gln Arg Ser Trp Asn Ile Pro Ile Asn Glu Gln Gln Phe
    530                 535                 540

Leu Glu Ile Leu Arg Ser Arg Arg Leu Ala Leu Glu Gly Ala Pro Val
545                 550                 555                 560

Gly Phe Trp Gln Met Leu Gln Ala Ala Arg Val Lys Tyr Gly His Ser
                565                 570                 575

Ser Thr Ser Gln Arg Ile Met Gly Tyr Asp Gln Gln Ser Phe Pro Leu
            580                 585                 590

Pro Glu Val Asn Ser Met Pro Tyr Thr Gln Asn Pro Leu Ser Trp Tyr
        595                 600                 605

Gln Arg Thr Met Gln Glu Thr Thr Pro Ser Lys Pro Val Asp Gln Tyr
    610                 615                 620

Gln Lys Leu His Lys Gln Met Thr His Phe Leu His Ala Gly Lys Leu
625                 630                 635                 640

Lys Glu Ala Leu Lys Cys Phe Gln Asn Ala Lys Asn Ala Arg Phe Gln
                645                 650                 655

Met Arg Gln Leu His Val Glu Leu Ala Val Ile Ala Thr Leu Leu Glu
            660                 665                 670

Asp Gly Leu Ser Ala Ala Arg Ser Leu Ile Glu Ser Glu Trp Arg Thr
        675                 680                 685

Ile Arg His Leu Val Arg Phe Ser Pro Ile Phe Phe Arg Gln Val Met
    690                 695                 700

Ala Val Asp Glu Asp Ala Gly Gly His Ile Val Gln Met Ala Val Leu
705                 710                 715                 720

Arg Phe Tyr Gln Leu Cys Trp Ser Thr Lys His Met Lys Val Lys His
                725                 730                 735

His Leu Thr Val Ala Thr Ser Arg Arg Leu Ile Ser Gln His Lys Pro
            740                 745                 750

Glu Met Ala Leu Glu Leu Leu Thr Ala Val Tyr Lys Ser Arg Tyr Arg
        755                 760                 765

Phe Ala Ala Thr Phe Asp Gly Val Cys Met Lys Met Phe Ala Arg Ala
    770                 775                 780

Phe Ala Ala Thr Asp Asn Ile Leu Gly Leu Arg Trp Cys Ile Leu Thr
785                 790                 795                 800

Ala Leu Ser Arg Asp Ser Ala Leu Asn His Asp Phe Val Val Glu Val
                805                 810                 815

Arg Arg Ile Leu Gly Thr Leu Ser Pro Pro Ser Ala Val Asp Ala Thr
            820                 825                 830

Ala Gly Pro Val Thr His Glu Gln Leu Glu Tyr Leu Tyr Tyr Ile Ala
        835                 840                 845

Asp Leu Leu Glu Glu Lys Asn Glu Gly Cys Ala Pro Ile Trp Glu Leu
    850                 855                 860

Lys His Asp Ala Thr Leu Lys Gln Ser Ser Arg Arg Gln Leu Lys Gln
865                 870                 875                 880

Pro Leu Asp Ala Ser Arg Leu Phe Asn Gln Ser Asp Val Arg Glu Thr
                885                 890                 895

Val Lys Arg Trp Asp Glu Glu Tyr Glu Leu Glu Ala Val Leu Gly Arg
            900                 905                 910

Ile Asp Asn Asp Pro Asn Ser Ile Thr Ala Arg Trp Asn Glu Ser Arg

915                 920                 925

Arg Ser His Gln Lys Glu Ala Gly Leu
    930                 935

<210> 99
<211> 2401
<212> DNA
<213> Aspergillus fumigatus

<220>
<223> Phylum CEA230; clone 9-11; contig 4899 region 9642-7242
      Genomic sequence containing 3' and 5'-ends and the coding region

<400> 99
acatggatga atcactgccc gatatcgaga agtcgcttga accgtacctc tcacattttg      60
gttatcggag tgacggatcg gatcagagcg cagtatttga tcttttagaa agtgagcgtt     120
tcactcataa caagagcccc ttgacagaca ttcgcaaggg ttaacctcat ggttgtggtt     180
tggtttggtt tgctctgctt ataattctct cggcatgtcg gtaatcaatc cacttttctc     240
tggggccctt ctacacatgc agcaatttca ttggtcatct gagatataca gatttgtaag     300
gcagtatcag tatcatcagg atacgcttct atgtagtatg agctcaaaga acagatagtt     360
gagaagtagt actgatatca cgtggtttaa gtattttgtc ggagtgctaa gcattacaag     420
tggcatatcc tttcagaata gaaaaccatc aggcaaacgc gattatcacc agaatttcca     480
tctcagtcta cagattggat atgcagagat ccaattgcca cagcagtcaa gtcgtcagaa     540
ttctgcgtgg agcgaggaga cctcgcattc gtgcaggaaa acctgtgacg tcgagagaag     600
ttttccgcag atggagtagc tcagagacgg cgaaatcatc ttcagctgca aatcagacca     660
tattctctgg gatccagcca accggcgtac cgcaccttgg aaattatctg ggagctctac     720
gtgaatgggt acggctacaa aatgctgcga aggagggaac aagattgttt ttttctatcg     780
tcgacttgca tgcgttgaca gtgccacaag acgcctccca gctacgaaat tggagaaaag     840
agacatttgc aacactcatt gccgttggtt tagacccgaa tcgctcaacg attttctacc     900
agtccgccgt atgcagtatg aatatgggtt gtaaagttta ctgaccatgt cattaggtcc     960
atgcacacgc cgaactattt tggattttgt gcacaatagc ctctatggga tatctctccc    1020
gaatgacaca gtggaaggta atacccagat caagtgccaa cctggtccaa tggtcagaag    1080
gaacagctag ctgacgtcgg ccagagcaaa ctccagttgc ctgataacgc aaatctggaa    1140
gactcgacgg cgaggtcaag gttgcgattg gggttgttct cataccctgt tctgcaggcg    1200
gcggatattc tagttcatag gtatgctgcc caatcgttgg tacattttca cattactgat    1260
gtagatcaga gctactcatg ttcctgtcgg agatgatcaa aggcagcacc tcgaattttc    1320
gaggaacact gcgaatagtt tcaatcatgt atatggaccc attttcccgt caccagaagc    1380
aattatatgt aagtggtttt tgcttctggc ggaacggtcc tgtgttggga aattgacggc    1440
tatcaatagc gcctgctaaa cgggttatgt ccctcaaaga accgacgttg aaaatgtcca    1500
agtcccatgc cgacagacgc tcaaggatca ttcttacgga ttcgcccgca gaaatctcca    1560
aaaagatcaa tgctgcgctc acagactcgg aattaaccat tacatatgac ccagtccgtc    1620
gacctggagt ggcgaattta atagagatct tgagtcactt cgatggacga acttgcgatg    1680
agattgccat ggaataccgt tcagccagtc ttcgcgctct aaaggaacat ctggccagaa    1740
cgttgtccaa tcatcttgag ccaataagag agaagtatct ctcacttgta ggagatcaga    1800
ctgactacct tgattctata gcagaacagg gttctgaagc cgcgcgggcc aacgctgaat    1860
tgacaatgga gcaagtcaaa gtcgctatgg gcttaattta gcccttcata tacgttgtca    1920
tcatgctccg ccgggtacac atgtaggctc agttggtcat actctgatga tcagttcatg    1980
atggtggcca ctattgacaa ttacccacca ctattgcctg ttcaggtact actcaaacgc    2040
tgattataca atataatgca tgcatatata tctacgtgtg tgtgtgtgtg tatacatatg    2100
tatgaatatg tgcatagcta atcaaatctc gcttgggttg gtgaaagaaa tgcgacatat    2160
gctttagaga acattctcgc ctgggattga attcatttca caaccgtata gaggtacatc    2220
atctagttat tggcagggat acgtcatcca tactggataa tggctgctgc ctacagaccc    2280
agactggtag gtatgtccac tgcgtggaga gcactaaaga ggcccagctt ggtgtgcggc    2340
aagtgggagc attactagga gcatgcagtg tccttcgtac atgtaccttt tacatatgct    2400
c                                                                    2401

<210> 100
<211> 1401

<212> DNA
<213> Aspergillus fumigatus

<220>
<223> Phylum CEA230; clone 9-11; contig 4899 region 9642-7242
      Genomic sequence containing the coding region

<400> 100
atgcagagat ccaattgcca cagcagtcaa gtcgtcagaa ttctgcgtgg agcgaggaga      60
cctcgcattc gtgcaggaaa acctgtgacg tcgagagaag ttttccgcag atggagtagc     120
tcagagacgg cgaaatcatc ttcagctgca aatcagacca tattctctgg gatccagcca     180
accggcgtac cgcaccttgg aaattatctg ggagctctac gtgaatgggt acggctacaa     240
aatgctgcga aggagggaac aagattgttt ttttctatcg tcgacttgca tgcgttgaca     300
gtgccacaag acgcctccca gctacgaaat tggagaaaag agacatttgc aacactcatt     360
gccgttggtt tagacccgaa tcgctcaacg attttctacc agtccgccgt atgcagtatg     420
aatatgggtt gtaaagttta ctgaccatgt cattaggtcc atgcacacgc cgaactattt     480
tggattttgt gcacaatagc ctctatggga tatctctccc gaatgacaca gtggaaggta     540
atacccagat caagtgccaa cctggtccaa tggtcagaag gaacagctag ctgacgtcgg     600
ccagagcaaa ctccagttgc ctgataacgc aaatctggaa gactcgacgg cgaggtcaag     660
gttgcgattg gggttgttct cataccctgt tctgcaggcg gcggatattc tagttcatag     720
gtatgctgcc caatcgttgg tacattttca cattactgat gtagatcaga gctactcatg     780
ttcctgtcgg agatgatcaa aggcagcacc tcgaattttc gaggaacact gcgaatagtt     840
tcaatcatgt atatggaccc attttcccgt caccagaagc aattatatgt aagtggtttt     900
tgcttctggc ggaacggtcc tgtgttggga aattgacggc tatcaatagc gcctgctaaa     960
cgggttatgt ccctcaaaga accgacgttg aaaatgtcca agtcccatgc cgacagacgc    1020
tcaaggatca ttcttacgga ttcgcccgca gaaatctcca aaaagatcaa tgctgcgctc    1080
acagactcgg aattaaccat tacatatgac ccagtccgtc gacctggagt ggcgaattta    1140
atagagatct tgagtcactt cgatggacga acttgcgatg agattgccat ggaataccgt    1200
tcagccagtc ttcgcgctct aaaggaacat ctggccagaa cgttgtccaa tcatcttgag    1260
ccaataagag agaagtatct ctcacttgta ggagatcaga ctgactacct tgattctata    1320
gcagaacagg gttctgaagc cgcgcgggcc aacgctgaat tgacaatgga gcaagtcaaa    1380
gtcgctatgg gcttaattta g                                              1401


<210> 101
<211> 1200
<212> DNA
<213> Aspergillus fumigatus

<220>
<223> Phylum CEA230; clone 9-11; contig 4899 region 9642-7242
      Coding region without introns

<400> 101
atgcagagat ccaattgcca cagcagtcaa gtcgtcagaa ttctgcgtgg agcgaggaga      60
cctcgcattc gtgcaggaaa acctgtgacg tcgagagaag ttttccgcag atggagtagc     120
tcagagacgg cgaaatcatc ttcagctgca aatcagacca tattctctgg gatccagcca     180
accggcgtac cgcaccttgg aaattatctg ggagctctac gtgaatgggt acggctacaa     240
aatgctgcga aggagggaac aagattgttt ttttctatcg tcgacttgca tgcgttgaca     300
gtgccacaag acgcctccca gctacgaaat tggagaaaag agacatttgc aacactcatt     360
gccgttggtt tagacccgaa tcgctcaacg attttctacc agtccgccgt ccatgcacac     420
gccgaactat tttggatttt gtgcacaata gcctctatgg gatatctctc ccgaatgaca     480
cagtggaaga aggaacagct agctgacgtc ggccagagca aactccagtt gcctgataac     540
gcaaatctgg aagactcgac ggcgaggtca aggttgcgat tggggttgtt ctcataccct     600
gttctgcagg cggcggatat tctaatcaga gctactcatg ttcctgtcgg agatgatcaa     660
aggcagcacc tcgaattttc gaggaacact gcgaatagtt tcaatcatgt atatggaccc     720
attttcccgt caccagaagc aattatatcg cctgctaaac gggttatgtc cctcaaagaa     780
ccgacgttga aaatgtccaa gtcccatgcc gacagacgct caaggatcat tcttacggat     840
tcgcccgcag aaatctccaa aaagatcaat gctgcgctca cagactcgga attaaccatt     900
acatatgacc cagtccgtcg acctggagtg gcgaatttaa tagagatctt gagtcacttc     960
gatggacgaa cttgcgatga gattgccatg gaataccgtt cagccagtct tcgcgctcta    1020

aaggaacatc tggccagaac gttgtccaat catcttgagc caataagaga gaagtatctc 1080
tcacttgtag gagatcagac tgactacctt gattctatag cagaacaggg ttctgaagcc 1140
gcgcgggcca acgctgaatt gacaatggag caagtcaaag tcgctatggg cttaatttag 1200

<210> 102
<211> 399
<212> PRT
<213> Aspergillus fumigatus

<220>
<223> Phylum CEA230; clone 9-11; contig 4899 region 9642-7242
      Protein sequence

<400> 102

Met Gln Arg Ser Asn Cys His Ser Ser Gln Val Val Arg Ile Leu Arg
1               5                   10                  15

Gly Ala Arg Arg Pro Arg Ile Arg Ala Gly Lys Pro Val Thr Ser Arg
            20                  25                  30

Glu Val Phe Arg Arg Trp Ser Ser Ser Glu Thr Ala Lys Ser Ser Ser
        35                  40                  45

Ala Ala Asn Gln Thr Ile Phe Ser Gly Ile Gln Pro Thr Gly Val Pro
    50                  55                  60

His Leu Gly Asn Tyr Leu Gly Ala Leu Arg Glu Trp Val Arg Leu Gln
65                  70                  75                  80

Asn Ala Ala Lys Glu Gly Thr Arg Leu Phe Phe Ser Ile Val Asp Leu
                85                  90                  95

His Ala Leu Thr Val Pro Gln Asp Ala Ser Gln Leu Arg Asn Trp Arg
            100                 105                 110

Lys Glu Thr Phe Ala Thr Leu Ile Ala Val Gly Leu Asp Pro Asn Arg
        115                 120                 125

Ser Thr Ile Phe Tyr Gln Ser Ala Val His Ala His Ala Glu Leu Phe
    130                 135                 140

Trp Ile Leu Cys Thr Ile Ala Ser Met Gly Tyr Leu Ser Arg Met Thr
145                 150                 155                 160

Gln Trp Lys Lys Glu Gln Leu Ala Asp Val Gly Gln Ser Lys Leu Gln
                165                 170                 175

Leu Pro Asp Asn Ala Asn Leu Glu Asp Ser Thr Ala Arg Ser Arg Leu
            180                 185                 190

Arg Leu Gly Leu Phe Ser Tyr Pro Val Leu Gln Ala Ala Asp Ile Leu
        195                 200                 205

Ile Arg Ala Thr His Val Pro Val Gly Asp Asp Gln Arg Gln His Leu
    210                 215                 220

Glu Phe Ser Arg Asn Thr Ala Asn Ser Phe Asn His Val Tyr Gly Pro
225                 230                 235                 240

Ile Phe Pro Ser Pro Glu Ala Ile Ile Ser Pro Ala Lys Arg Val Met
                245                 250                 255

Ser Leu Lys Glu Pro Thr Leu Lys Met Ser Lys Ser His Ala Asp Arg
            260                 265                 270

Arg Ser Arg Ile Ile Leu Thr Asp Ser Pro Ala Glu Ile Ser Lys Lys
        275                 280                 285

Ile Asn Ala Ala Leu Thr Asp Ser Glu Leu Thr Ile Thr Tyr Asp Pro
    290                 295                 300

Val Arg Arg Pro Gly Val Ala Asn Leu Ile Glu Ile Leu Ser His Phe
305                 310                 315                 320

Asp Gly Arg Thr Cys Asp Glu Ile Ala Met Glu Tyr Arg Ser Ala Ser
                325                 330                 335

Leu Arg Ala Leu Lys Glu His Leu Ala Arg Thr Leu Ser Asn His Leu
            340                 345                 350

Glu Pro Ile Arg Glu Lys Tyr Leu Ser Leu Val Gly Asp Gln Thr Asp
        355                 360                 365

Tyr Leu Asp Ser Ile Ala Glu Gln Gly Ser Glu Ala Ala Arg Ala Asn
    370                 375                 380

Ala Glu Leu Thr Met Glu Gln Val Lys Val Ala Met Gly Leu Ile
385                 390                 395


<210> 103
<211> 3805
<212> DNA
<213> Aspergillus fumigatus

<220>
<223> Phylum CEA231; clone 10-80; contig 4940 region 54154-50350
      Genomic sequence containing 3' and 5'-ends and the coding region

<400> 103
cacccatttt gtagtaattc tcgagaaatc cgctctgaat acccaccagg ggaaagatgt      60
caattatcac tatgagaaac aaaaggctgc tattttacct cttcgggacg tgttggacgc     120
gtcgcggggc atgctatgtt tgtcctgttt gcaccaaaca ggaattgccg taaaatggga     180
acagagtttg ttagctcacc gcctttggat tttcactgct aaaacaaaaa aaaagtgaga     240
tttaccatca cgacaggcct ggatctgttc tggtcggaag gagacggcag gcgatttgga     300
tttctatttc gcccttgcaa agtaattgga tgcaaagggt tctgtgttct attttgcgtc     360
ttgcaacttg atggaattcg aagcctgagc tgtcctgagc ttcaaaatat cttctctcgc     420
cctcgggcga cctcaaacca cttttctttc ccatattttg tttggagcgg cctgcgccag     480
ctgcgcaaca gagttccaag atgcctcacc gcgcggcatc cccagcggtg tcggaaaatg     540
agttcgacat cacaggcgct ctcttccaga acgacagcga ctccgacaac gaacagccct     600
cggccaagtc caaacgacaa cctccgaaga aggttccctc gcaggcgctc gacttcctcg     660
gcgatgtgaa tgaagacgac aatgacgacg aggcttttat tgccgagcag caaacttctg     720
ccaaccgcaa ggcctcgaat ctcaaaggtc gcactgttaa gaagggtggt ggtttccaag     780
ccatgggctt gagtgccaat ctgttaaagg caatcgctcg gaaaggcttc tcggtaccga     840
ctcccattca gcgcaagacc attcccgtta ttatggacga ccaggatgta gttggtatgg     900
cacggactgg ttcaggaaag acggccgctt tcgttatccc aatgatcgag aaattgaaga     960
gccatagcac caaggttgga gcccgcggtc tggtcttgtc cccatcgaga gagctggcac    1020
tgcagacatt gaaagtcgtc aaggaactgg gtagaggcac tgacctgaag tcggttcttc    1080
ttgttggtgg agacagcctg gaggagcaat tcgcgatgat cgccggcaat ccagatatta    1140
ttattgcaac acctggtcga ttcctgcatt tgaaggtgga aatgaacctg gacttgtcca    1200

gtatccgcta tgtcgttttc gacgaggctg atcgactgtt tgagatgggt ttcgccgcgc 1260
aactaacaga gattttgcac ggtcttcctg cgaatcggca gactctcctg ttctctgcca 1320
ctcttccgaa gtcccttgtc gagtttgccc gcgccggctt gcaggaacct acactagtcc 1380
gtctggatac cgagagcaag atctcgcccg atcttcagaa tgctttcttc tctgtgaaat 1440
cctcagagaa ggaaggggcc ttgctctaca tccttcatga ggtcatcaag atgccgactg 1500
ggccaaccga ggtttcgcaa caaaggaaag aagaagatgc aagcgccaag aacttgaaga 1560
acaagaagag gaagagagcg gaaatggaga aggctgtcaa tacgagagag tctccaacca 1620
agcattcgac aatcgttttt gccgcgacga agcaccatgt cgattatctt tactcactac 1680
tctgcgaggc gggattcgcc gtctcctacg tttacggctc tttagatcaa accgcccgaa 1740
agatccaggt tcaaaacttt agaacggcca tgaccaatat cctcgttgtc accgacgttg 1800
cggctagagg tattgatatt cctatcctgg caaatgtcat taattacgat ttcccctccc 1860
agccaaagat ttttgttcac cgtgtcggcc gaactgctcg tgccgggcgc aagggctgga 1920
gttacagtct ggtccgcgat gcagacgctc cttatttact tgatttgcaa ctattcctag 1980
gaagaaggct ggttgttggc cgcgaattcg gggatcaagt gaacttcgcc gaagacgttg 2040
taacgggcag tctacctagg gatgggctct ctcaaagctg tgagtgggta accaaggtcc 2100
tggacgataa tgcagacctt gcagcccaac gcacggtcgc tgctaagggt gagaagctct 2160
atatgcgaac ccggaacgcg gcatctcttg agagtgcgaa gcgatcgaaa caggtggttt 2220
cttccgacaa ttggacaagc gtacacccgc tcttccaaga tgaaactagt aatctggagg 2280
ccgagcggga gaagatgctc gcccgtattg gtggttaccg gccgccagag acaatctttg 2340
aggtcaacaa ccggcggatg ggcaaacatg agaacgtcga cgctctcgat acgattaaga 2400
gagttcgtag cactctagag tccaagaaga agcgtgcaca agcgaatgaa aagtccgagt 2460
tcctcgaaga cggtcccgac gacggaaagg cagtcaacga agccaaagaa accgagagcg 2520
agggagcatt ttctgatgag gacgacgacg ttcccaccgg cgtggcagat aacatgtcga 2580
tggcatccga ttcagagctg gaagtcacct tctcgtcata ttcaaaatca aaggacaaca 2640
aggcgaagaa agcgagcgcg gcatctttcc agaaccctga atacttcatg tcctataccc 2700
cgaataacac ctccctggcg gaggaccgag cttatggtgt gcattccggt accaactcca 2760
acttcgccca ggcctcacgc agcgcgacca tggatctggc aggcgacgat ggaggccgcg 2820
ggttcggcga ggctcgtacg ctgatgcgct gggacaagcg acacaagaag tacgtggctc 2880
gacagaatga cgaggacggc tccaaggcca cacgcctcgt ccgcggtgag agcggggcga 2940
agatcgcagc cagcttccgg agcggacggt tcgacgcttg gaagagagaa aatcgtctcg 3000
gccgcttgcc tcgggtgggt gaggccgaag ctgctaatct cgctgctggt ctcaacgcag 3060
ccatctcagg caagcggttc aggcatcgca aggagcaggc gcccaagaag gctgatcctc 3120
tccgtggtga ttacgagaag atgaagaaga aggctgaact cgccaaggag cgagcaatgt 3180
ctaaggctgg cggcgctgca ccacgtggca agagcgagct gaagagtacg gacgatatcc 3240
ggattgcgcg caaattgaag cagaagagac gagagaagaa tgctcgtccc tcgaggaaga 3300
agtaaccggg aggttaattt tcagagtcat gtatatacca ggaattgcct atgatacctg 3360
ctactgtgta attttttttt atactaaagc ccaatccaaa acactcttgg gaacgaccga 3420
ggccaaacat tcctggtgac agagattacc tttcagcact tatgccaaac cacaaagctc 3480
tctcagtaaa accgccaata cgttaacacc cctccaatac tccgtacagc agctctcatc 3540
gcatcactga gaaaatgtcg cttgatattc tacccaacag tgaagaagac ccacagaccc 3600
aagccgaaaa tttcaaggtg accggaattt tctcggccgc aaaggagaaa caagatagaa 3660
tgcaaatgga cgatctccca atctccatca gctggactat tgattccgac ggtgaatagt 3720
ggagcatctg tgctgcgcta ttgggttctc cgtgacgccg ctcctttggt gactcgtttt 3780
ctttgctttt tccttcagca cttgg 3805

<210> 104
<211> 2805
<212> DNA
<213> Aspergillus fumigatus

<220>
<223> Phylum CEA231; clone 10-80; contig 4940 region 54154-50350
      Genomic sequence containing the coding region

<400> 104
atgcctcacc gcgcggcatc cccagcggtg tcggaaaatg agttcgacat cacaggcgct 60
ctcttccaga acgacagcga ctccgacaac gaacagccct cggccaagtc caaacgacaa 120
cctccgaaga aggttccctc gcaggcgctc gacttcctcg gcgatgtgaa tgaagacgac 180
aatgacgacg aggcttttat tgccgagcag caaacttctg ccaaccgcaa ggcctcgaat 240
ctcaaaggtc gcactgttaa gaagggtggt ggtttccaag ccatgggctt gagtgccaat 300

ctgttaaagg caatcgctcg gaaaggcttc tcggtaccga ctcccattca gcgcaagacc 360
attcccgtta ttatggacga ccaggatgta gttggtatgg cacggactgg ttcaggaaag 420
acggccgctt tcgttatccc aatgatcgag aaattgaaga gccatagcac caaggttgga 480
gcccgcggtc tggtcttgtc cccatcgaga gagctggcac tgcagacatt gaaagtcgtc 540
aaggaactgg gtagaggcac tgacctgaag tcggttcttc ttgttggtgg agacagcctg 600
gaggagcaat tcgcgatgat cgccggcaat ccagatatta ttattgcaac acctggtcga 660
ttcctgcatt tgaaggtgga aatgaacctg gacttgtcca gtatccgcta tgtcgttttc 720
gacgaggctg atcgactgtt tgagatgggt ttcgccgcgc aactaacaga gattttgcac 780
ggtcttcctg cgaatcggca gactctcctg ttctctgcca ctcttccgaa gtcccttgtc 840
gagtttgccc gcgccggctt gcaggaacct acactagtcc gtctggatac cgagagcaag 900
atctcgcccg atcttcagaa tgctttcttc tctgtgaaat cctcagagaa ggaaggggcc 960
ttgctctaca tccttcatga ggtcatcaag atgccgactg ggccaaccga ggtttcgcaa 1020
caaaggaaag aagaagatgc aagcgccaag aacttgaaga acaagaagag gaagagagcg 1080
gaaatggaga aggctgtcaa tacgagagag tctccaacca agcattcgac aatcgttttt 1140
gccgcgacga agcaccatgt cgattatctt tactcactac tctgcgaggc gggattcgcc 1200
gtctcctacg tttacggctc tttagatcaa accgcccgaa agatccaggt tcaaaacttt 1260
agaacgggca tgaccaatat cctcgttgtc accgacgttg cggctagagg tattgatatt 1320
cctatcctgg caaatgtcat taattacgat ttcccctccc agccaaagat ttttgttcac 1380
cgtgtcggcc gaactgctcg tgccgggcgc aagggctgga gttacagtct ggtccgcgat 1440
gcagacgctc cttatttact tgatttgcaa ctattcctag gaagaaggct ggttgttggc 1500
cgcgaattcg gggatcaagt gaacttcgcc gaagacgttg taacgggcag tctacctagg 1560
gatgggctct ctcaaagctg tgagtgggta accaaggtcc tggacgataa tgcagacctt 1620
gcagcccaac gcacggtcgc tgctaagggt gagaagctct atatgcgaac ccggaacgcg 1680
gcatctcttg agagtgcgaa gcgatcgaaa caggtggttt cttccgacaa ttggacaagc 1740
gtacacccgc tcttccaaga tgaaactagt aatctggagg ccgagcggga gaagatgctc 1800
gcccgtattg gtggttaccg gccgccagag acaatctttg aggtcaacaa ccggcggatg 1860
ggcaaacatg agaacgtcga cgctctcgat acgattaaga gagttcgtag cactctagag 1920
tccaagaaga agcgtgcaca agcgaatgaa aagtccgagt tcctcgaaga cggtcccgac 1980
gacggaaagg cagtcaacga agccaaagaa accgagagcg agggagcatt ttctgatgag 2040
gacgacgacg ttcccaccgg cgtggcagat aacatgtcga tggcatccga ttcagagctg 2100
gaagtcacct tctcgtcata ttcaaaatca aaggacaaca aggcgaagaa agcgagcgcg 2160
gcatctttcc agaaccctga atacttcatg tcctataccc cgaataacac ctccctggcg 2220
gaggaccgag cttatggtgt gcattccggt accaactcca acttcgccca ggcctcacgc 2280
agcgcgacca tggatctggc aggcgacgat ggaggccgcg ggttcggcga ggctcgtacg 2340
ctgatgcgct gggacaagcg acacaagaag tacgtggctc gacagaatga cgaggacggc 2400
tccaagggca cacgcctcgt ccgcggtgag agcggggcga agatcgcagc cagcttccgg 2460
agcggacggt tcgacgcttg gaagagagaa aatcgtctcg gccgcttgcc tcgggtgggt 2520
gaggccgaag ctgctaatct cgctgctggt ctcaacgcag ccatctcagg caagcggttc 2580
aggcatcgca aggagcaggc gcccaagaag gctgatcctc tccgtggtga ttacgagaag 2640
atgaagaaga aggctgaact cgccaaggag cgagcaatgt ctaaggctgg cggcgctgca 2700
ccacgtggca agagcgagct gaagagtacg gacgatatcc ggattgcgcg caaattgaag 2760
cagaagagac gagagaagaa tgctcgtccc tcgaggaaga agtaa 2805

<210> 105
<211> 2805
<212> DNA
<213> Aspergillus fumigatus

<220>
<223> Phylum CEA231; clone 10-80; contig 4940 region 54154-50350
      Coding region without introns

<400> 105
atgcctcacc gcgcggcatc cccagcggtg tcggaaaatg agttcgacat cacaggcgct 60
ctcttccaga acgacagcga ctccgacaac gaacagccct cggccaagtc caaacgacaa 120
cctccgaaga aggttccctc gcaggcgctc gacttcctcg gcgatgtgaa tgaagacgac 180
aatgacgacg aggcttttat tgccgagcag caaacttctg ccaaccgcaa ggcctcgaat 240
ctcaaaggtc gcactgttaa gaagggtggt ggtttccaag ccatgggctt gagtgccaat 300
ctgttaaagg caatcgctcg gaaaggcttc tcggtaccga ctcccattca gcgcaagacc 360
attcccgtta ttatggacga ccaggatgta gttggtatgg cacggactgg ttcaggaaag 420

acggccgctt tcgttatccc aatgatcgag aaattgaaga gccatagcac caaggttgga 480
gcccgcggtc tggtcttgtc cccatcgaga gagctggcac tgcagacatt gaaagtcgtc 540
aaggaactgg gtagaggcac tgacctgaag tcggttcttc ttgttggtgg agacagcctg 600
gaggagcaat tcgcgatgat cgccggcaat ccagatatta ttattgcaac acctggtcga 660
ttcctgcatt tgaaggtgga aatgaacctg gacttgtcca gtatccgcta tgtcgttttc 720
gacgaggctg atcgactgtt tgagatgggt ttcgccgcgc aactaacaga gattttgcac 780
ggtcttcctg cgaatcggca gactctcctg ttctctgcca ctcttccgaa gtcccttgtc 840
gagtttgccc gcgccggctt gcaggaacct acactagtcc gtctggatac cgagagcaag 900
atctcgcccg atcttcagaa tgctttcttc tctgtgaaat cctcagagaa ggaaggggcc 960
ttgctctaca tccttcatga ggtcatcaag atgccgactg ggccaaccga ggtttcgcaa 1020
caaaggaaag aagaagatgc aagcgccaag aacttgaaga acaagaagag gaagagagcg 1080
gaaatggaga aggctgtcaa tacgagagag tctccaacca agcattcgac aatcgttttt 1140
gccgcgacga agcaccatgt cgattatctt tactcactac tctgcgaggc gggattcgcc 1200
gtctcctacg tttacggctc tttagatcaa accgcccgaa agatccaggt tcaaaacttt 1260
agaacgggca tgaccaatat cctcgttgtc accgacgttg cggctagagg tattgatatt 1320
cctatcctgg caaatgtcat taattacgat ttcccctccc agccaaagat ttttgttcac 1380
cgtgtcggcc gaactgctcg tgccgggcgc aagggctgga gttacagtct ggtccgcgat 1440
gcagacgctc cttatttact tgatttgcaa ctattcctag gaagaaggct ggttgttggc 1500
cgcgaattcg gggatcaagt gaacttcgcc gaagacgttg taacgggcag tctacctagg 1560
gatgggctct ctcaaagctg tgagtgggta accaaggtcc tggacgataa tgcagacctt 1620
gcagcccaac gcacggtcgc tgctaagggt gagaagctct atatgcgaac ccggaacgcg 1680
gcatctcttg agagtgcgaa gcgatcgaaa caggtggttt cttccgacaa ttggacaagc 1740
gtacacccgc tcttccaaga tgaaactagt aatctggagg ccgagcggga gaagatgctc 1800
gcccgtattg gtggttaccg gccgccagag acaatctttg aggtcaacaa ccggcggatg 1860
ggcaaacatg agaacgtcga cgctctcgat acgattaaga gagttcgtag cactctagag 1920
tccaagaaga agcgtgcaca agcgaatgaa aagtccgagt tcctcgaaga cggtcccgac 1980
gacggaaagg cagtcaacga agccaaagaa accgagagcg agggagcatt ttctgatgag 2040
gacgacgacg ttcccaccgg cgtggcagat aacatgtcga tggcatccga ttcagagctg 2100
gaagtcacct tctcgtcata ttcaaaatca aaggacaaca aggcgaagaa agcgagcgcg 2160
gcatctttcc agaaccctga atacttcatg tcctataccc cgaataacac ctccctggcg 2220
gaggaccgag cttatggtgt gcattccggt accaactcca acttcgccca ggcctcacgc 2280
agcgcgacca tggatctggc aggcgacgat ggaggccgcg ggttcggcga ggctcgtacg 2340
ctgatgcgct gggacaagcg acacaagaag tacgtggctc gacagaatga cgaggacggc 2400
tccaagggca cacgcctcgt ccgcggtgag agcggggcga agatcgcagc cagcttccgg 2460
agcggacggt tcgacgcttg gaagagagaa aatcgtctcg gccgcttgcc tcgggtgggt 2520
gaggccgaag ctgctaatct cgctgctggt ctcaacgcag ccatctcagg caagcggttc 2580
aggcatcgca aggagcaggc gcccaagaag gctgatcctc tccgtggtga ttacgagaag 2640
atgaagaaga aggctgaact cgccaaggag cgagcaatgt ctaaggctgg cggcgctgca 2700
ccacgtggca agagcgagct gaagagtacg gacgatatcc ggattgcgcg caaattgaag 2760
cagaagagac gagagaagaa tgctcgtccc tcgaggaaga agtaa 2805

<210> 106
<211> 934
<212> PRT
<213> Aspergillus fumigatus

<220>
<223> Phylum CEA231; clone 10-80; contig 4940 region 54154-50350
      Protein sequence

<400> 106

Met Pro His Arg Ala Ala Ser Pro Ala Val Ser Glu Asn Glu Phe Asp
1               5                   10                  15

Ile Thr Gly Ala Leu Phe Gln Asn Asp Ser Asp Ser Asp Asn Glu Gln
            20                  25                  30

Pro Ser Ala Lys Ser Lys Arg Gln Pro Pro Lys Lys Val Pro Ser Gln
        35                  40                  45

Ala Leu Asp Phe Leu Gly Asp Val Asn Glu Asp Asp Asn Asp Asp Glu
    50                  55                  60

Ala Phe Ile Ala Glu Gln Gln Thr Ser Ala Asn Arg Lys Ala Ser Asn
65                  70                  75                  80

Leu Lys Gly Arg Thr Val Lys Lys Gly Gly Gly Phe Gln Ala Met Gly
                85                  90                  95

Leu Ser Ala Asn Leu Leu Lys Ala Ile Ala Arg Lys Gly Phe Ser Val
            100                 105                 110

Pro Thr Pro Ile Gln Arg Lys Thr Ile Pro Val Ile Met Asp Asp Gln
        115                 120                 125

Asp Val Val Gly Met Ala Arg Thr Gly Ser Gly Lys Thr Ala Ala Phe
    130                 135                 140

Val Ile Pro Met Ile Glu Lys Leu Lys Ser His Ser Thr Lys Val Gly
145                 150                 155                 160

Ala Arg Gly Leu Val Leu Ser Pro Ser Arg Glu Leu Ala Leu Gln Thr
                165                 170                 175

Leu Lys Val Val Lys Glu Leu Gly Arg Gly Thr Asp Leu Lys Ser Val
            180                 185                 190

Leu Leu Val Gly Gly Asp Ser Leu Glu Glu Gln Phe Ala Met Ile Ala
        195                 200                 205

Gly Asn Pro Asp Ile Ile Ile Ala Thr Pro Gly Arg Phe Leu His Leu
    210                 215                 220

Lys Val Glu Met Asn Leu Asp Leu Ser Ser Ile Arg Tyr Val Val Phe
225                 230                 235                 240

Asp Glu Ala Asp Arg Leu Phe Glu Met Gly Phe Ala Ala Gln Leu Thr
                245                 250                 255

Glu Ile Leu His Gly Leu Pro Ala Asn Arg Gln Thr Leu Leu Phe Ser
            260                 265                 270

Ala Thr Leu Pro Lys Ser Leu Val Glu Phe Ala Arg Ala Gly Leu Gln
        275                 280                 285

Glu Pro Thr Leu Val Arg Leu Asp Thr Glu Ser Lys Ile Ser Pro Asp
    290                 295                 300

Leu Gln Asn Ala Phe Phe Ser Val Lys Ser Ser Glu Lys Glu Gly Ala
305                 310                 315                 320

Leu Leu Tyr Ile Leu His Glu Val Ile Lys Met Pro Thr Gly Pro Thr
                325                 330                 335

Glu Val Ser Gln Gln Arg Lys Glu Glu Asp Ala Ser Ala Lys Asn Leu
            340                 345                 350

Lys Asn Lys Lys Arg Lys Arg Ala Glu Met Glu Lys Ala Val Asn Thr
        355                 360                 365

Arg Glu Ser Pro Thr Lys His Ser Thr Ile Val Phe Ala Ala Thr Lys
    370                 375                 380

His His Val Asp Tyr Leu Tyr Ser Leu Leu Cys Glu Ala Gly Phe Ala
385                 390                 395                 400

Val Ser Tyr Val Tyr Gly Ser Leu Asp Gln Thr Ala Arg Lys Ile Gln
                405                 410                 415

Val Gln Asn Phe Arg Thr Gly Met Thr Asn Ile Leu Val Val Thr Asp
            420                 425                 430

Val Ala Ala Arg Gly Ile Asp Ile Pro Ile Leu Ala Asn Val Ile Asn
        435                 440                 445

Tyr Asp Phe Pro Ser Gln Pro Lys Ile Phe Val His Arg Val Gly Arg
    450                 455                 460

Thr Ala Arg Ala Gly Arg Lys Gly Trp Ser Tyr Ser Leu Val Arg Asp
465                 470                 475                 480

Ala Asp Ala Pro Tyr Leu Leu Asp Leu Gln Leu Phe Leu Gly Arg Arg
                485                 490                 495

Leu Val Val Gly Arg Glu Phe Gly Asp Gln Val Asn Phe Ala Glu Asp
            500                 505                 510

Val Val Thr Gly Ser Leu Pro Arg Asp Gly Leu Ser Gln Ser Cys Glu
        515                 520                 525

Trp Val Thr Lys Val Leu Asp Asp Asn Ala Asp Leu Ala Ala Gln Arg
    530                 535                 540

Thr Val Ala Ala Lys Gly Glu Lys Leu Tyr Met Arg Thr Arg Asn Ala
545                 550                 555                 560

Ala Ser Leu Glu Ser Ala Lys Arg Ser Lys Gln Val Val Ser Ser Asp
                565                 570                 575

Asn Trp Thr Ser Val His Pro Leu Phe Gln Asp Glu Thr Ser Asn Leu
            580                 585                 590

Glu Ala Glu Arg Glu Lys Met Leu Ala Arg Ile Gly Gly Tyr Arg Pro
        595                 600                 605

Pro Glu Thr Ile Phe Glu Val Asn Asn Arg Arg Met Gly Lys His Glu
    610                 615                 620

Asn Val Asp Ala Leu Asp Thr Ile Lys Arg Val Arg Ser Thr Leu Glu
625                 630                 635                 640

Ser Lys Lys Lys Arg Ala Gln Ala Asn Glu Lys Ser Glu Phe Leu Glu
                645                 650                 655

Asp Gly Pro Asp Asp Gly Lys Ala Val Asn Glu Ala Lys Glu Thr Glu
            660                 665                 670

Ser Glu Gly Ala Phe Ser Asp Glu Asp Asp Asp Val Pro Thr Gly Val
        675                 680                 685

Ala Asp Asn Met Ser Met Ala Ser Asp Ser Glu Leu Glu Val Thr Phe

690                 695                 700

Ser Ser Tyr Ser Lys Ser Lys Asp Asn Lys Ala Lys Lys Ala Ser Ala
705                 710                 715                 720

Ala Ser Phe Gln Asn Pro Glu Tyr Phe Met Ser Tyr Thr Pro Asn Asn
                725                 730                 735

Thr Ser Leu Ala Glu Asp Arg Ala Tyr Gly Val His Ser Gly Thr Asn
            740                 745                 750

Ser Asn Phe Ala Gln Ala Ser Arg Ser Ala Thr Met Asp Leu Ala Gly
        755                 760                 765

Asp Asp Gly Gly Arg Gly Phe Gly Glu Ala Arg Thr Leu Met Arg Trp
    770                 775                 780

Asp Lys Arg His Lys Lys Tyr Val Ala Arg Gln Asn Asp Glu Asp Gly
785                 790                 795                 800

Ser Lys Gly Thr Arg Leu Val Arg Gly Glu Ser Gly Ala Lys Ile Ala
                805                 810                 815

Ala Ser Phe Arg Ser Gly Arg Phe Asp Ala Trp Lys Arg Glu Asn Arg
            820                 825                 830

Leu Gly Arg Leu Pro Arg Val Gly Glu Ala Glu Ala Ala Asn Leu Ala
        835                 840                 845

Ala Gly Leu Asn Ala Ala Ile Ser Gly Lys Arg Phe Arg His Arg Lys
    850                 855                 860

Glu Gln Ala Pro Lys Lys Ala Asp Pro Leu Arg Gly Asp Tyr Glu Lys
865                 870                 875                 880

Met Lys Lys Lys Ala Glu Leu Ala Lys Glu Arg Ala Met Ser Lys Ala
                885                 890                 895

Gly Gly Ala Ala Pro Arg Gly Lys Ser Glu Leu Lys Ser Thr Asp Asp
            900                 905                 910

Ile Arg Ile Ala Arg Lys Leu Lys Gln Lys Arg Arg Glu Lys Asn Ala
        915                 920                 925

Arg Pro Ser Arg Lys Lys
    930


<210> 107
<211> 2413
<212> DNA
<213> Aspergillus fumigatus

<220>
<223> Phylum CEA232; clone 10-175; contig 4938 region 211008-213420
      Genomic sequence containing 3' and 5'-ends and the coding region

<400> 107
cctcctcttc ttccactgcc tcatcatcac ggtatggatt gtcatcagcc atagtgagaa      60
tagtgaatgg gtttagaagt tacagcctag aaatgagagg gaaagagagg tacattgaaa     120
gcaagaagat atggtcggta tttacatcac ctccttctct gccttgagtg cctaccgctc     180

tgctgcctgc gtgcccaccg acttcaccag tgacgcgatc tcgtcatcgg agcaacgcgg 240
gggaaacacg aacatcacgt gaatgcgcca agacttgatg accccaaatt attactgatt 300
ggtcaaactt ccagcactgt tccgtcatca accacctaag ggcctagata tgttctccag 360
ttacagatcc ttcgtgccac gattctttca ttgagtggtc aaatactact cgacgtattt 420
ttgtgggctt cagtttgtgg ctaatgttag accgatagac gacggccaac ctttttaata 480
cactatcatc gcacctcccc atggctctcc gccggccatt aacacttccg aggcacattc 540
tcaatggagc ttgtttaggc ttgcgaccag ctgtgtctcg cgccgctctg gcttatgggc 600
aggagcagag gaaagggctt gcaacagcag ttcccccggt cactcaaaat gcggctgggt 660
ccaaaggccc cacggcaatg gtcttcctca acatgggtgg gccatcgaag attgacgaag 720
tggaagattt tctgagcaga ttatttgtat gcattcctca atatgcccga tgctaccacc 780
atgtatgagt ctgacaaact ctctcttcct ataccaggcc gatggcgatc tgattcctct 840
cggacgactt caatcatacc tcggccctct catcgctaag cgcagaaccc caaagatcca 900
acggcaatac tcggatattg gtggagggtc accgatcagg aaatggtccg agtatcagtg 960
cgaggaaatg tgcagattgc tagacaaaat caatcccgaa acggctcctc acaagcctta 1020
cgtcgcgttc cggtacgccg accctctgac ggaagaaatg tacacaaagt tgctggaaga 1080
tggattcggc aacgggaaag gcgggcgcgc tgtcgcgttc acacagtacc cccaatattc 1140
gtgctccacc acgggtagct cgctgaacga gttgtggaaa tggagaacca ggcttgaggg 1200
taagcgtgca aatggcaaca tggaccccgc tggtgccatc cagtggagtg tcattgatcg 1260
atggccaacg caccctggcc tcgtggaggc tttcgcccgg aacattgagg agcagctgaa 1320
gacataccca gaggagaagc gaaacggtgt cgttctcttg ttctcagccc acagtctgcc 1380
catgagtgtt gtcaacagag gtgagactca tcttcttacc gaacaacaag atttgctcgc 1440
taacacattt cctaggcgac ccatatcctg ctgaagttgc tgcaactgtg catgctgtca 1500
tgcaaagatt gaatttcagc aatccttacc gactgtgctg gcagtcccaa gtgggaccgt 1560
cagcttggct tggagcccaa actagcgata cggtcgaaaa ctatgtcaaa cgtggacaga 1620
ccgatattat tctagttccc attgccttca ccagcgacca tattgagact ctgtacgagt 1680
tggatctgga agtgataaag gaagcaaact ccccgggagt caagagagcc gagagtttga 1740
atggtaaccc cattttcatt caggcattag cagacattgc ccaagagcac ctccgtaagg 1800
gagagaagtg ctcactacag atgactctgc gctgtcaagg ctgtaagagc gaacggtgcc 1860
tggaacagaa gaaattcttt gctggcgacc gattttcttc tcttgtagtt tagtgtattt 1920
ctatatggct gttctctgcc gttgtataca atactagggg ccatttcttt tcttcagggc 1980
gttaagttgt gttgtcaacg aggtgaccca ttcacggagc acagcagagc aatgtatata 2040
ttgttgaaaa agtccaagca tataatctca tttcgttgtt ggtcttcaat ggttgagaat 2100
atataaaatc attaggaatg aaggatttcc gggtcaacct ttgacaatta ttttgccacc 2160
atttaacgtt gcacatatgc aacgataaag cacgacccag tcgtagtgtt ggtgtccctt 2220
atcgagaaat tgcatgagat tactccaacc gctcaaaatc tcaaaccctg ataagaaaaa 2280
ttgattattg aacaaactct catagcgtcg tttcgtaatt actggttggt cggaatcggg 2340
ctgacgcaaa gcccaacggc cacgaagctg ctccagctcc gttcccagtt ctcgtgaaga 2400
attggtaatc atg 2413

<210> 108
<211> 1413
<212> DNA
<213> Aspergillus fumigatus

<220>
<223> Phylum CEA232; clone 10-175; contig 4938 region 211008-213420
      Genomic sequence containing the coding region

<400> 108
atggctctcc gccggccatt aacacttccg aggcacattc tcaatggagc ttgtttaggc 60
ttgcgaccag ctgtgtctcg cgccgctctg gcttatgggc aggagcagag gaaagggctt 120
gcaacagcag ttcccccggt cactcaaaat gcggctgggt ccaaaggccc cacggcaatg 180
gtcttcctca acatgggtgg gccatcgaag attgacgaag tggaagattt tctgagcaga 240
ttatttgtat gcattcctca atatgcccga tgctaccacc atgtatgagt ctgacaaact 300
ctctcttcct ataccaggcc gatggcgatc tgattcctct cggacgactt caatcatacc 360
tcggccctct catcgctaag cgcagaaccc caaagatcca acggcaatac tcggatattg 420
gtggagggtc accgatcagg aaatggtccg agtatcagtg cgaggaaatg tgcagattgc 480
tagacaaaat caatcccgaa acggctcctc acaagcctta cgtcgcgttc cggtacgccg 540
accctctgac ggaagaaatg tacacaaagt tgctggaaga tggattcggc aacgggaaag 600
gcgggcgcgc tgtcgcgttc acacagtacc cccaatattc gtgctccacc acgggtagct 660

cgctgaacga gttgtggaaa tggagaacca ggcttgaggg taagcgtgca aatggcaaca 720
tggaccccgc tggtgccatc cagtggagtg tcattgatcg atggccaacg caccctggcc 780
tcgtggaggc tttcgcccgg aacattgagg agcagctgaa gacataccca gaggagaagc 840
gaaacggtgt cgttctcttg ttctcagccc acagtctgcc catgagtgtt gtcaacagag 900
gtgagactca tcttcttacc gaacaacaag atttgctcgc taacacattt cctaggcgac 960
ccatatcctg ctgaagttgc tgcaactgtg catgctgtca tgcaaagatt gaatttcagc 1020
aatccttacc gactgtgctg gcagtcccaa gtgggaccgt cagcttggct tggagcccaa 1080
actagcgata cggtcgaaaa ctatgtcaaa cgtggacaga ccgatattat tctagttccc 1140
attgccttca ccagcgacca tattgagact ctgtacgagt tggatctgga agtgataaag 1200
gaagcaaact ccccgggagt caagagagcc gagagtttga atggtaaccc cattttcatt 1260
caggcattag cagacattgc ccaagagcac ctccgtaagg gagagaagtg ctcactacag 1320
atgactctgc gctgtcaagg ctgtaagagc gaacggtgcc tggaacagaa gaaattcttt 1380
gctggcgacc gattttcttc tcttgtagtt tag 1413

<210> 109
<211> 1287
<212> DNA
<213> Aspergillus fumigatus

<220>
<223> Phylum CEA232; clone 10-175; contig 4938 region 211008-213420
      Coding region without introns

<400> 109
atggctctcc gccggccatt aacacttccg aggcacattc tcaatggagc ttgtttaggc 60
ttgcgaccag ctgtgtctcg cgccgctctg gcttatgggc aggagcagag gaaagggctt 120
gcaacagcag ttcccccggt cactcaaaat gcggctgggt ccaaaggccc cacggcaatg 180
gtcttcctca acatgggtgg gccatcgaag attgacgaag tggaagattt tctgagcaga 240
ttatttgccg atggcgatct gattcctctc ggacgacttc aatcatacct cggccctctc 300
atcgctaagc gcagaacccc aaagatccaa cggcaatact cggatattgg tggagggtca 360
ccgatcagga aatggtccga gtatcagtgc gaggaaatgt gcagattgct agacaaaatc 420
aatcccgaaa cggctcctca caagccttac gtcgcgttcc ggtacgccga ccctctgacg 480
gaagaaatgt acacaaagtt gctggaagat ggattcggca acgggaaagg cgggcgcgct 540
gtcgcgttca cacagtaccc ccaatattcg tgctccacca cgggtagctc gctgaacgag 600
ttgtggaaat ggagaaccag gcttgagggt aagcgtgcaa atggcaacat ggaccccgct 660
ggtgccatcc agtggagtgt cattgatcga tggccaacgc accctggcct cgtggaggct 720
ttcgcccgga acattgagga gcagctgaag acatacccag aggagaagcg aaacggtgtc 780
gttctcttgt tctcagccca cagtctgccc atgagtgttg tcaacagagg cgacccatat 840
cctgctgaag ttgctgcaac tgtgcatgct gtcatgcaaa gattgaattt cagcaatcct 900
taccgactgt gctggcagtc ccaagtggga ccgtcagctt ggcttggagc ccaaactagc 960
gatacggtcg aaaactatgt caaacgtgga cagaccgata ttattctagt tcccattgcc 1020
ttcaccagcg accatattga gactctgtac gagttggatc tggaagtgat aaaggaagca 1080
aactccccgg gagtcaagag agccgagagt ttgaatggta accccatttt cattcaggca 1140
ttagcagaca ttgcccaaga gcacctccgt aagggagaga agtgctcact acagatgact 1200
ctgcgctgtc aaggctgtaa gagcgaacgg tgcctggaac agaagaaatt ctttgctggc 1260
gaccgatttt cttctcttgt agtttag 1287

<210> 110
<211> 428
<212> PRT
<213> Aspergillus fumigatus

<220>
<223> Phylum CEA232; clone 10-175; contig 4938 region 211008-213420
      Protein sequence

<400> 110
Met Ala Leu Arg Arg Pro Leu Thr Leu Pro Arg His Ile Leu Asn Gly
1               5                   10                  15

Ala Cys Leu Gly Leu Arg Pro Ala Val Ser Arg Ala Ala Leu Ala Tyr
            20                  25                  30

Gly Gln Glu Gln Arg Lys Gly Leu Ala Thr Ala Val Pro Pro Val Thr
        35                  40                  45

Gln Asn Ala Ala Gly Ser Lys Gly Pro Thr Ala Met Val Phe Leu Asn
    50                  55                  60

Met Gly Gly Pro Ser Lys Ile Asp Glu Val Glu Asp Phe Leu Ser Arg
65                  70                  75                  80

Leu Phe Ala Asp Gly Asp Leu Ile Pro Leu Gly Arg Leu Gln Ser Tyr
                85                  90                  95

Leu Gly Pro Leu Ile Ala Lys Arg Arg Thr Pro Lys Ile Gln Arg Gln
            100                 105                 110

Tyr Ser Asp Ile Gly Gly Gly Ser Pro Ile Arg Lys Trp Ser Glu Tyr
        115                 120                 125

Gln Cys Glu Glu Met Cys Arg Leu Leu Asp Lys Ile Asn Pro Glu Thr
    130                 135                 140

Ala Pro His Lys Pro Tyr Val Ala Phe Arg Tyr Ala Asp Pro Leu Thr
145                 150                 155                 160

Glu Glu Met Tyr Thr Lys Leu Leu Glu Asp Gly Phe Gly Asn Gly Lys
                165                 170                 175

Gly Gly Arg Ala Val Ala Phe Thr Gln Tyr Pro Gln Tyr Ser Cys Ser
            180                 185                 190

Thr Thr Gly Ser Ser Leu Asn Glu Leu Trp Lys Trp Arg Thr Arg Leu
        195                 200                 205

Glu Gly Lys Arg Ala Asn Gly Asn Met Asp Pro Ala Gly Ala Ile Gln
    210                 215                 220

Trp Ser Val Ile Asp Arg Trp Pro Thr His Pro Gly Leu Val Glu Ala
225                 230                 235                 240

Phe Ala Arg Asn Ile Glu Glu Gln Leu Lys Thr Tyr Pro Glu Glu Lys
                245                 250                 255

Arg Asn Gly Val Val Leu Leu Phe Ser Ala His Ser Leu Pro Met Ser
            260                 265                 270

Val Val Asn Arg Gly Asp Pro Tyr Pro Ala Glu Val Ala Ala Thr Val
        275                 280                 285

His Ala Val Met Gln Arg Leu Asn Phe Ser Asn Pro Tyr Arg Leu Cys
    290                 295                 300

Trp Gln Ser Gln Val Gly Pro Ser Ala Trp Leu Gly Ala Gln Thr Ser
305                 310                 315                 320

Asp Thr Val Glu Asn Tyr Val Lys Arg Gly Gln Thr Asp Ile Ile Leu
                325                 330                 335

Val Pro Ile Ala Phe Thr Ser Asp His Ile Glu Thr Leu Tyr Glu Leu
            340                 345                 350

Asp Leu Glu Val Ile Lys Glu Ala Asn Ser Pro Gly Val Lys Arg Ala
        355                 360                 365

Glu Ser Leu Asn Gly Asn Pro Ile Phe Ile Gln Ala Leu Ala Asp Ile
    370                 375                 380

Ala Gln Glu His Leu Arg Lys Gly Glu Lys Cys Ser Leu Gln Met Thr
385                 390                 395                 400

Leu Arg Cys Gln Gly Cys Lys Ser Glu Arg Cys Leu Glu Gln Lys Lys
                405                 410                 415

Phe Phe Ala Gly Asp Arg Phe Ser Ser Leu Val Val
            420                 425


<210> 111
<211> 2865
<212> DNA
<213> Aspergillus fumigatus

<220>
<223> Phylum CEA233; clone 10-290; contig 4865 region 3495-6359
      Genomic sequence containing 3' and 5'-ends and the coding region

<400> 111
ccttgatcga atcccaagcc tgtagcctgg cagaacagag caaagcaaac tggcatctcc      60
tgctgaagcc taaggtcact agacaaagga acggcagaac cgcatgaacg aggagcataa     120
acaaggcggg atcgtctttc aacacgcggg cggtgagctt atcagcgccg attgcggggt     180
ggtgcagcaa atcaagtcag tcgccacttt cggcgaccat gacaaagcgc aagattggct     240
ttactaaacc gcctactttt tttttatcaa agagacttgg gtttgtcagc ttttctttat     300
cttctgaaag agcgcttctc tggtcaagct gttcaccaaa tccccatcac tactgttccc     360
tttgtcgtta ttttcgtcgc attgcatcta caacaaagaa aacgggctcg acgaaccctg     420
cgagatccat acttcctggg gtggcggtct tcttagtcct tatcgcatag cggggtgctc     480
gaccagaagt ccctgccacg atgagtgcaa tcctttctgc agacgatttg aacgatttca     540
tttctcccgg ggttgcttgc atcaagcccg ttgagagtct accacaaaaa gaatcccagt     600
cggaggtatc tttcctgtct taccagtcat ctgttgatat cagccaatag gctaacgctc     660
atttccaatt caatagaatc cctatgaggt gacaaaggaa gacaaagttc aaccggaaaa     720
ccttcccccg gctcagattt cattgactga ttgccttgca tgctccggat gtgtcacgtc     780
tgcggaagca gtgttgatat ccttgcaatc acatacggag gttctcaata ctcttgattc     840
gtaccccgaa ttgccgcttg gttctacaag ctaccaaaga ggcacacaaa aagttggatc     900
agcagacagc gatggtcgca tctttgttgc tagcgtcagc cctcaagtca gggcgagctt     960
ggcagccaca tacggaatca ccgagcggga ggcgaaatat atgattgacc aatttcttat    1020
gggccctcac ggtctcagag ctggtggaaa acatggcaat gggtttacat gggttgtgga    1080
cacgaacgtt atgcgtgaag cagtgttggc tctgacagcg gacgaggtca cgagctcttt    1140
attatcaact ggatcgggca gccttcccaa gagtccaatt ctttcgtccg cttgccccgg    1200
ctggatatgt tatgctgaaa aacacacccc ttttatcctt ccgcatttat ctcgcctcaa    1260
gtctcctcag gcgttgagcg gcacatttct gaagtcagtg ctaagcaagg cacttggggt    1320
cccgccttct cagatatggc atttagctat catgccatgc ttcgacaaga agctggaagc    1380
tagccgggaa gagctgacag acattgcatg ggcttcaacc ttcacccagt cacagacaac    1440
acccgtccgc gacgttgact gtgtcataac cacccgtgag ctactaactt tagccactgc    1500
tagggggctt tctctaccca atttgccgct caaaccattg cccgcgtcat gtttaactcc    1560
atttccagat caagccctag aatcattttt gttctctaag agctcgtcgg gccaaacagt    1620
cgaatcaggg acatctggag gctatcttca tcacgtcctc caaatcttcc aagccagaaa    1680
ccccggcagc aagattgtca cccagcgtgg gcgcaacgcc gatgttgtgg aatatgtgct    1740
catgtcgtct ggggatgagc ctctttttag ggcggctcgg tattatggct tcaggaatat    1800
acaaaatctc gtcagaaaac ttaaacccgc acgcgtgtca agactgccag gcgccaagcc    1860
gcaagcggtc tcttcaagtg caaatcgacg acagcccatg tcaaggaacg cagctccggc    1920

tggaacaggc gctgattatg catatgttga agtcatggct tgtcctggcg gctgtaccaa 1980
tggtggtggg caaataagga ttgaagatgc ccgggaggct gttccgaacg cactaaaaga 2040
gacatcgact gaaactcctg tggctgcacc gaaacccacg ccgcatgagc agcgtgcctg 2100
gctagcccgg gtagatgaag cgtactactc tgcggactcg gatagcgagg gatctgtcac 2160
gacggagccg gtttctgtcc tgtcaaggga taaccagatt catgagtttt tgaactattg 2220
gtcagagaag gttgatatac ccctttcccg gctcgcgtac acgtcctatc gcgaagtgga 2280
gagcgacgtg ggtaagacga agaatgcgcc caacgaaact gctcgtgttg tggaattggc 2340
aggaaagatc ggaggtggtt ggtgatatcg cactcgctgt ctggaaaaca ttgcatatat 2400
ccattatacc cttcaatttg ttgtggatga gaacgctctt tgcatcactg gttggttgca 2460
tgctagtacg catttggaga gatacccctt tattatagat ttctcatttg ttgggcattt 2520
gagaggattt ataagtcatg agattgctac aggaagcccc atagacgtat agagatcagg 2580
gtagagcatg ccaccttagc ttttacacta ctccgtacgg tcatgatcaa actcttgaat 2640
ttaatatttc gattgcggac aatcaaagca atgtatggag taaacatgtg gtagagcagg 2700
tacctgaggc ataacgtgct agtccagaat aggctagctc catcaaaccc cgaatcttcc 2760
atcgggatga caaagcagcc tagagcattt ggcagaaaaa gtgctctacc ccaagtctga 2820
attctctcca acaactcgcc ttcaatcacc cacgccccag tttat 2865

<210> 112
<211> 1865
<212> DNA
<213> Aspergillus fumigatus

<220>
<223> Phylum CEA233; clone 10-290; contig 4865 region 3495-6359
      Genomic sequence containing the coding region

<400> 112
atgagtgcaa tcctttctgc agacgatttg aacgatttca tttctcccgg ggttgcttgc 60
atcaagcccg ttgagagtct accacaaaaa gaatcccagt cggaggtatc tttcctgtct 120
taccagtcat ctgttgatat cagccaatag gctaacgctc atttccaatt caatagaatc 180
cctatgaggt gacaaaggaa gacaaagttc aaccggaaaa ccttcccccg gctcagattt 240
cattgactga ttgccttgca tgctccggat gtgtcacgtc tgcggaagca gtgttgatat 300
ccttgcaatc acatacggag gttctcaata ctcttgattc gtaccccgaa ttgccgcttg 360
gttctacaag ctaccaaaga ggcacacaaa aagttggatc agcagacagc gatggtcgca 420
tctttgttgc tagcgtcagc cctcaagtca gggcgagctt ggcagccaca tacggaatca 480
ccgagcggga ggcgaaatat atgattgacc aatttcttat gggccctcac ggtctcagag 540
ctggtggaaa acatggcaat gggtttacat gggttgtgga cacgaacgtt atgcgtgaag 600
cagtgttggc tctgacagcg gacgaggtca cgagctcttt attatcaact ggatcgggca 660
gccttcccaa gagtccaatt ctttcgtccg cttgccccgg ctggatatgt tatgctgaaa 720
aaacacaccc ttttatcctt ccgcatttat ctcgcctcaa gtctcctcag gcgttgagcg 780
gcacatttct gaagtcagtg ctaagcaagg cacttggggt cccgccttct cagatatggc 840
atttagctat catgccatgc ttcgacaaga agctggaagc tagccgggaa gagctgacag 900
acattgcatg ggcttcaacc ttcacccagt cacagacaac acccgtccgc gacgttgact 960
gtgtcataac cacccgtgag ctactaactt tagccactgc tagggggctt tctctaccca 1020
atttgccgct caaaccattg cccgcgtcat gtttaactcc atttccagat caagccctag 1080
aatcattttt gttctctaag agctcgtcgg gccaaacagt cgaatcaggg acatctggag 1140
gctatcttca tcacgtcctc caaatcttcc aagccagaaa ccccggcagc aagattgtca 1200
cccagcgtgg gcgcaacgcc gatgttgtgg aatatgtgct catgtcgtct ggggatgagc 1260
ctctttttag ggcggctcgg tattatggct tcaggaatat acaaaatctc gtcagaaaac 1320
ttaaacccgc acgcgtgtca agactgccag gcgccaagcc gcaagcggtc tcttcaagtg 1380
caaatcgacg acagcccatg tcaaggaacg cagctccggc tggaacaggc gctgattatg 1440
catatgttga agtcatggct tgtcctggcg gctgtaccaa tggtggtggg caaataagga 1500
ttgaagatgc ccgggaggct gttccgaacg cactaaaaga gacatcgact gaaactcctg 1560
tggctgcacc gaaacccacg ccgcatgagc agcgtgcctg gctagcccgg gtagatgaag 1620
cgtactactc tgcggactcg gatagcgagg gatctgtcac gacggagccg gtttctgtcc 1680
tgtcaaggga taaccagatt catgagtttt tgaactattg gtcagagaag gttgatatac 1740
ccctttcccg gctcgcgtac acgtcctatc gcgaagtgga gagcgacgtg ggtaagacga 1800
agaatgcgcc caacgaaact gctcgtgttg tggaattggc aggaaagatc ggaggtggtt 1860
ggtga 1865

<210> 113
<211> 1725
<212> DNA
<213> Aspergillus fumigatus

<220>
<223> Phylum CEA233; clone 10-290; contig 4865 region 3495-6359
      Coding region without introns

<400> 113
atgagtgcaa tcctttctgc agacgatttg aacgatttca tttctcccgg ggttgcttgc      60
atcaagcccg ttgagagtct accacaaaaa gaatcccagt cggagaatcc ctatgaggtg     120
acaaaggaag acaaagttca accggaaaac cttcccccgg ctcagatttc attgactgat     180
tgccttgcat gctccggatg tgtcacgtct gcggaagcag tgttgatatc cttgcaatca     240
catacggagg ttctcaatac tcttgattcc gatggtcgca tctttgttgc tagcgtcagc     300
cctcaagtca gggcgagctt ggcagccaca tacggaatca ccgagcggga ggcgaaatat     360
atgattgacc aatttcttat gggccctcac ggtctcagag ctggtggaaa acatggcaat     420
gggtttacat gggttgtgga cacgaacgtt atgcgtgaag cagtgttggc tctgacagcg     480
gacgaggtca cgagctcttt attatcaact ggatcgggca gccttcccaa gagtccaatt     540
ctttcgtccg cttgccccgg ctggatatgt tatgctgaaa aaacacaccc ttttatcctt     600
ccgcatttat ctcgcctcaa gtctcctcag gcgttgagcg gcacatttct gaagtcagtg     660
ctaagcaagg cacttggggt cccgccttct cagatatggc atttagctat catgccatgc     720
ttcgacaaga agctggaagc tagccgggaa gagctgacag acattgcatg ggcttcaacc     780
ttcacccagt cacagacaac acccgtccgc gacgttgact gtgtcataac cacccgtgag     840
ctactaactt tagccactgc tagggggctt tctctaccca atttgccgct caaaccattg     900
cccgcgtcat gtttaactcc atttccagat caagccctag aatcattttt gttctctaag     960
agctcgtcgg gccaaacagt cgaatcaggg acatctggag gctatcttca tcacgtcctc    1020
caaatcttcc aagccagaaa ccccggcagc aagattgtca cccagcgtgg gcgcaacgcc    1080
gatgttgtgg aatatgtgct catgtcgtct ggggatgagc ctctttttag ggcggctcgg    1140
tattatggct tcaggaatat acaaaatctc gtcagaaaac ttaaacccgc acgcgtgtca    1200
agactgccag gcgccaagcc gcaagcggtc tcttcaagtg caaatcgacg acagcccatg    1260
tcaaggaacg cagctccggc tggaacaggc gctgattatg catatgttga agtcatggct    1320
tgtcctggcg gctgtaccaa tggtggtggg caaataagga ttgaagatgc ccgggaggct    1380
gttccgaacg cactaaaaga gacatcgact gaaactcctg tggctgcacc gaaacccacg    1440
ccgcatgagc agcgtgcctg gctagcccgg gtagatgaag cgtactactc tgcggactcg    1500
gatagcgagg gatctgtcac gacggagccg gtttctgtcc tgtcaaggga taaccagatt    1560
catgagtttt tgaactattg gtcagagaag gttgatatac ccctttcccg gctcgcgtac    1620
acgtcctatc gcgaagtgga gagcgacgtg ggtaagacga agaatgcgcc caacgaaact    1680
gctcgtgttg tggaattggc aggaaagatc ggaggtggtt ggtga                    1725


<210> 114
<211> 574
<212> PRT
<213> Aspergillus fumigatus

<220>
<223> Phylum CEA233; clone 10-290; contig 4865 region 3495-6359
      Protein sequence

<400> 114

Met Ser Ala Ile Leu Ser Ala Asp Asp Leu Asn Asp Phe Ile Ser Pro
1               5                   10                  15

Gly Val Ala Cys Ile Lys Pro Val Glu Ser Leu Pro Gln Lys Glu Ser
            20                  25                  30

Gln Ser Glu Asn Pro Tyr Glu Val Thr Lys Glu Asp Lys Val Gln Pro
        35                  40                  45

Glu Asn Leu Pro Pro Ala Gln Ile Ser Leu Thr Asp Cys Leu Ala Cys
    50                  55                  60

Ser Gly Cys Val Thr Ser Ala Glu Ala Val Leu Ile Ser Leu Gln Ser
65                  70                  75                  80

His Thr Glu Val Leu Asn Thr Leu Asp Ser Asp Gly Arg Ile Phe Val
                85                  90                  95

Ala Ser Val Ser Pro Gln Val Arg Ala Ser Leu Ala Ala Thr Tyr Gly
            100                 105                 110

Ile Thr Glu Arg Glu Ala Lys Tyr Met Ile Asp Gln Phe Leu Met Gly
        115                 120                 125

Pro His Gly Leu Arg Ala Gly Gly Lys His Gly Asn Gly Phe Thr Trp
    130                 135                 140

Val Val Asp Thr Asn Val Met Arg Glu Ala Val Leu Ala Leu Thr Ala
145                 150                 155                 160

Asp Glu Val Thr Ser Ser Leu Leu Ser Thr Gly Ser Gly Ser Leu Pro
                165                 170                 175

Lys Ser Pro Ile Leu Ser Ser Ala Cys Pro Gly Trp Ile Cys Tyr Ala
            180                 185                 190

Glu Lys Thr His Pro Phe Ile Leu Pro His Leu Ser Arg Leu Lys Ser
        195                 200                 205

Pro Gln Ala Leu Ser Gly Thr Phe Leu Lys Ser Val Leu Ser Lys Ala
    210                 215                 220

Leu Gly Val Pro Pro Ser Gln Ile Trp His Leu Ala Ile Met Pro Cys
225                 230                 235                 240

Phe Asp Lys Lys Leu Glu Ala Ser Arg Glu Glu Leu Thr Asp Ile Ala
                245                 250                 255

Trp Ala Ser Thr Phe Thr Gln Ser Gln Thr Thr Pro Val Arg Asp Val
            260                 265                 270

Asp Cys Val Ile Thr Thr Arg Glu Leu Leu Thr Leu Ala Thr Ala Arg
        275                 280                 285

Gly Leu Ser Leu Pro Asn Leu Pro Leu Lys Pro Leu Pro Ala Ser Cys
    290                 295                 300

Leu Thr Pro Phe Pro Asp Gln Ala Leu Glu Ser Phe Leu Phe Ser Lys
305                 310                 315                 320

Ser Ser Ser Gly Gln Thr Val Glu Ser Gly Thr Ser Gly Gly Tyr Leu
                325                 330                 335

His His Val Leu Gln Ile Phe Gln Ala Arg Asn Pro Gly Ser Lys Ile
            340                 345                 350

Val Thr Gln Arg Gly Arg Asn Ala Asp Val Val Glu Tyr Val Leu Met
        355                 360                 365

Ser Ser Gly Asp Glu Pro Leu Phe Arg Ala Ala Arg Tyr Tyr Gly Phe
    370                 375                 380

Arg Asn Ile Gln Asn Leu Val Arg Lys Leu Lys Pro Ala Arg Val Ser
385                 390                 395                 400

Arg Leu Pro Gly Ala Lys Pro Gln Ala Val Ser Ser Ser Ala Asn Arg
                405                 410                 415

Arg Gln Pro Met Ser Arg Asn Ala Ala Pro Ala Gly Thr Gly Ala Asp
            420                 425                 430

Tyr Ala Tyr Val Glu Val Met Ala Cys Pro Gly Gly Cys Thr Asn Gly
        435                 440                 445

Gly Gly Gln Ile Arg Ile Glu Asp Ala Arg Glu Ala Val Pro Asn Ala
    450                 455                 460

Leu Lys Glu Thr Ser Thr Glu Thr Pro Val Ala Ala Pro Lys Pro Thr
465                 470                 475                 480

Pro His Glu Gln Arg Ala Trp Leu Ala Arg Val Asp Glu Ala Tyr Tyr
                485                 490                 495

Ser Ala Asp Ser Asp Ser Glu Gly Ser Val Thr Thr Glu Pro Val Ser
            500                 505                 510

Val Leu Ser Arg Asp Asn Gln Ile His Glu Phe Leu Asn Tyr Trp Ser
        515                 520                 525

Glu Lys Val Asp Ile Pro Leu Ser Arg Leu Ala Tyr Thr Ser Tyr Arg
    530                 535                 540

Glu Val Glu Ser Asp Val Gly Lys Thr Lys Asn Ala Pro Asn Glu Thr
545                 550                 555                 560

Ala Arg Val Val Glu Leu Ala Gly Lys Ile Gly Gly Gly Trp
                565                 570


<210> 115
<211> 1510
<212> DNA
<213> Aspergillus fumigatus

<220>
<223> Phylum CEA234; clone 10-304; contig 4899 region 443110-444619
      Genomic sequence containing 3' and 5'-ends and the coding region

<400> 115
taagagttgc aggtatgagc ctggtaaatc aagtcagtca cggatagcac aacagaacct      60
cattttgtcc tgaaagaatg aaccaaaagg ccaactcaga ctctttgcaa atgcaaggaa     120
gaggtaatga gaatgttttg ggagaagctt aaatgtagct ttgccggaac ggagaattga     180
gtaaagccgg tcatgaggcg ccaagacccc agcgaaaaag cagccctagg ccgcacgcaa     240
ccccgttcgg cgagttgcta ctggctgtta agcgagactc ttgtgggcga agaccgcaac     300
acccgaaatt cgcgatccag tagcccagag cgacttgtgt gcgtttcgga cgactttgac     360
aatcccgact cttcgacaac aaattcccat caccgccctc ccggagtctg tcgaccgtga     420
gtttgaaacc tacgccctat cgaatttctg gactgtcact gaagaatccg tttttgtcgt     480
ttttttagga agccttcgcc atggccgata tcgatgtcaa ggttgctcaa tggaagcttg     540
ttgaggttgg ccgtgttgtg ctgatccgca gcggtcctta caccggcaag cttgctgcca     600
ttgtcgagat catcgaccac aagcgtgtac gtttttcaac ggagaaattc tgagcgcagg     660

acggaaagat catggtcgga tgtgatattg acaaagaggc gcgatcatag gtcctggttg 720
acggtccttc caccgaggag aacaagatcg ttccccgtca cgctcttcct ctcgctcacg 780
ccactctcac ccccttcgtc attcccaaac tcccccgcgc tgccggcact ggccccgtca 840
agaagctctg ggagaagaac gagatcgatg gaaagtgggc taagagcacc attgctcaga 900
agactgagcg cgctgagcgg aggaagaacc ttaccgactt cgagcgcttc aaggtcctca 960
gactcaagaa gcaggtacgt tcagtttgcg aaactatggg agaattgtga tggcacattg 1020
gagggcattc ttggcaactc tgcactcgct tttcgcgaga gggaagagga gcaattactt 1080
gtattatgat ttgcgactgg ttactgacat ctggtgattt aacaggctcg ctacgaggtc 1140
cagaaggctc acgccaaggt cagggctgct gctcctaagt catagatgtt ttcatgaggc 1200
tcggtgcata gtatgaaggg gtaccttggg acggttttac atggctgagg gttttattct 1260
atttcagcaa aaattaagct gtatccacta caatgacagc caaaaaatga ttcaaaacct 1320
tgatatcctg acacgggtca tcctgctatg tcatcagatt cgcgcacccg attagtactt 1380
ggctctggtt tatagccgtc tccttagaca ttaattggga attaaacatt ttagactcaa 1440
gatcacggaa tatgtaagaa agtatcgtta tgtacattac tgagttggat tggctcgtta 1500
tgactcgtat 1510

<210> 116
<211> 685
<212> DNA
<213> Aspergillus fumigatus

<220>
<223> Phylum CEA234; clone 10-304; contig 4899 region 443110-444619
      Genomic sequence containing the coding region

<400> 116
atggccgata tcgatgtcaa ggttgctcaa tggaagcttg ttgaggttgg ccgtgttgtg 60
ctgatccgca gcggtcctta caccggcaag cttgctgcca ttgtcgagat catcgaccac 120
aagcgtgtac gtttttcaac ggagaaattc tgagcgcagg acggaaagat catggtcgga 180
tgtgatattg acaaagaggc gcgatcatag gtcctggttg acggtccttc caccgaggag 240
aacaagatcg ttccccgtca cgctcttcct ctcgctcacg ccactctcac ccccttcgtc 300
attcccaaac tcccccgcgc tgccggcact ggccccgtca agaagctctg ggagaagaac 360
gagatcgatg gaaagtgggc taagagcacc attgctcaga agactgagcg cgctgagcgg 420
aggaagaacc ttaccgactt cgagcgcttc aaggtcctca gactcaagaa gcaggtacgt 480
tcagtttgcg aaactatggg agaattgtga tggcacattg gagggcattc ttggcaactc 540
tgcactcgct tttcgcgaga gggaagagga gcaattactt gtattatgat ttgcgactgg 600
ttactgacat ctggtgattt aacaggctcg ctacgaggtc cagaaggctc acgccaaggt 660
cagggctgct gctcctaagt catag 685


<210> 117
<211> 465
<212> DNA
<213> Aspergillus fumigatus

<220>
<223> Phylum CEA234; clone 10-304; contig 4899 region 443110-444619
      Coding region without introns

<400> 117
atggccgata tcgatgtcaa ggttgctcaa tggaagcttg ttgaggttgg ccgtgttgtg 60
ctgatccgca gcggtcctta caccggcaag cttgctgcca ttgtcgagat catcgaccac 120
aagcgtgtcc tggttgacgg tccttccacc gaggagaaca agatcgttcc ccgtcacgct 180
cttcctctcg ctcacgccac tctcaccccc ttcgtcattc ccaaactccc ccgcgctgcc 240
ggcactggcc ccgtcaagaa gctctgggag aagaacgaga tcgatggaaa gtgggctaag 300
agcaccattg ctcagaagac tgagcgcgct gagcggagga agaaccttac cgacttcgag 360
cgcttcaagg tcctcagact caagaagcag gctcgctacg aggtccagaa ggctcacgcc 420
aaggtcaggg ctgctgctcc taagtcatag atgttttcat gaggc 465


<210> 118

<211> 149
<212> PRT
<213> Aspergillus fumigatus

<220>
<223> Phylum CEA234; clone 10-304; contig 4899 region 443110-444619
      Protein sequence

<400> 118

Met Ala Asp Ile Asp Val Lys Val Ala Gln Trp Lys Leu Val Glu Val
1               5                   10                  15

Gly Arg Val Val Leu Ile Arg Ser Gly Pro Tyr Thr Gly Lys Leu Ala
            20                  25                  30

Ala Ile Val Glu Ile Ile Asp His Lys Arg Val Leu Val Asp Gly Pro
        35                  40                  45

Ser Thr Glu Glu Asn Lys Ile Val Pro Arg His Ala Leu Pro Leu Ala
    50                  55                  60

His Ala Thr Leu Thr Pro Phe Val Ile Pro Lys Leu Pro Arg Ala Ala
65                  70                  75                  80

Gly Thr Gly Pro Val Lys Lys Leu Trp Glu Lys Asn Glu Ile Asp Gly
                85                  90                  95

Lys Trp Ala Lys Ser Thr Ile Ala Gln Lys Thr Glu Arg Ala Glu Arg
            100                 105                 110

Arg Lys Asn Leu Thr Asp Phe Glu Arg Phe Lys Val Leu Arg Leu Lys
        115                 120                 125

Lys Gln Ala Arg Tyr Glu Val Gln Lys Ala His Ala Lys Val Arg Ala
    130                 135                 140

Ala Ala Pro Lys Ser
145

<210> 119
<211> 1942
<212> DNA
<213> Aspergillus fumigatus

<220>
<223> Phylum CEA254; clone 7-1-10; contig 4911 region 43163-41221
      Genomic sequence containing 3' and 5'-ends and the coding region

<400> 119
tacacccagc acacctaccc tagccacaga tttcttggac cacgggaacc cgaacaaaga      60
gtctctggcc gaactctggt ctaatttcct agagcaggag tcacaggcca gtgggaatcc     120
agaatcgccg aaaccatgaa gagtttcaaa aggtggctct gttctgagcc gtatagaaaa     180
ctagcatctt ctctaagata cggtggttcc acatttatat atgcttgccg actggctttg     240
gtctcctctt cttttccatt ctagacttgc ttcgtagtaa taacactgat atcacccgcg     300
tgtttgcgac ttttgcatca aaccagcttc cccaccgctt tctttctgcc accatagcgg     360
gggacctcgt tattgagcgg acaagtcgtc gttggctttt tctgcacgtt tggcctatgc     420
ttcgtttatt cagctctggt acagctggga agttgactga tacactctcc tctctgattt     480
cttggtactc cagattgaca atgactaccg gggctggtac gatctctcat tccaacacct     540
atcatcgtat tcctcgccgt taactgacca atccaccagt gcaaaggttc cgtccagtgg     600
tggtatcggg tccctctggg actgggaagt cgaccttgct caagagactc ttcgctgaat     660

accccgatac tttcgattta tccgtgtctc gtacgtctaa ccccttgcca accctcattg 720
actatgcctg cgaattgttt cttttggtgg aattgcgctg aacggtgttt gttatattta 780
gataccactc gagctccccg tcccggggaa gaaaatggac gtgagtatta cttcacaact 840
aaagaagatt tcctggatct tgtgagcaag aatgccttta tcgagcatgc gcagtttggt 900
ggcaattact acggtactac tgtgcaggca gtgaaggatg ttgcgcagaa gggcaagatc 960
tgcgttctcg acattgagat gaggtaataa tagtcctgca acgtgaactg atatgaccgg 1020
agaagcagag gaaatccatc atcaaatgga ttgtagttca acccaaacaa cagctgacga 1080
ctgaattgca atagggcgtg aaacaagtca agcgcaccga tcttgatgct cgattcttat 1140
ttttagcacc cccgtccctt gaagaactag agaaaagact gcgtgggaga gcaaccgaga 1200
ctgaggagag cttgacggta tggctgtcct ccacattcct tcacttcccc aactcgccag 1260
actgtcccgc tggaattcta actttgcgtc agaaacgcct tgcccaagct aaaaatgaat 1320
tggaatatgc ggcgcagcct ggctctcatg ataagattgt cgtgaacgat gacctggaga 1380
aggcttataa ggaactgcgg gattggattg tcgacggtgg taactttgga gcgcgtcaat 1440
gatttattgg gcatgtctcg gcgtgtttta tttatcagcg ctgctgtata ctttagcgcc 1500
cgtagatact gtcggttgcg atactgaaaa caatgcatca tctgccttgg taacttcggt 1560
ccacagaaac ccataatcaa ggaggtcctt tcgtcgtcga cgaacataag agagattaat 1620
tacatgaaca tcaagactat gctaacaatt cgaatgttgg tctcttttct gtctggagac 1680
gacaaatcta ggaaaggtgg tagactcagt cactctcttg aacggagagg agaaaattaa 1740
gcaaaactaa aaaagagaac aaagtctgat gagcaatatg agggctgaaa aggatatctg 1800
taaagaggct gctagaataa aatggaagat gccgattgag aaggcaatgg aggaagagaa 1860
ggggtcattt atcgcagttt gggcgtggac cagaaatgac tgcagtatgt ttatggacca 1920
tgccagccgg agctattgga ct 1942

<210> 120
<211> 943
<212> DNA
<213> Aspergillus fumigatus

<220>
<223> Phylum CEA254; clone 7-1-10; contig 4911 region 43163-41221
      Genomic sequence containing the coding region

<400> 120
atgactaccg gggctggtac gatctctcat tccaacacct atcatcgtat tcctcgccgt 60
taactgacca atccaccagt gcaaaggttc cgtccagtgg tggtatcggg tccctctggg 120
actgggaagt cgaccttgct caagagactc ttcgctgaat accccgatac tttcgattta 180
tccgtgtctc gtacgtctaa ccccttgcca accctcattg actatgcctg cgaattgttt 240
cttttggtgg aattgcgctg aacggtgttt gttatattta gataccactc gagctccccg 300
tcccggggaa gaaaatggac gtgagtatta cttcacaact aaagaagatt tcctggatct 360
tgtgagcaag aatgccttta tcgagcatgc gcagtttggt ggcaattact acggtactac 420
tgtgcaggca gtgaaggatg ttgcgcagaa gggcaagatc tgcgttctcg acattgagat 480
ggaggtaata atagtcctgc aacgtgaact gatatgaccg gagaagcaga ggaaatccat 540
catcaaatgg attgtagttc aacccaaaca acagctgacg actgaattgc aatagggcgt 600
gaaacaagtc aagcgcaccg atcttgatgc tcgattctta tttttagcac ccccgtccct 660
tgaagaacta gagaaaagac tgcgtgggag agcaaccgag actgaggaga gcttgacggt 720
atggctgtcc tccacattcc ttcacttccc caactcgcca gactgtcccg ctggaattct 780
aactttgcgt cagaaacgcc ttgcccaagc taaaaatgaa ttggaatatg cggcgcagcc 840
tggctctcat gataagattg tcgtgaacga tgacctggag aaggcttata aggaactgcg 900
ggattggatt gtcgacggtg gtaactttgg agcgcgtcaa tga 943

<210> 121
<211> 603
<212> DNA
<213> Aspergillus fumigatus

<220>
<223> Phylum CEA254; clone 7-1-10; contig 4911 region 43163-41221
      Coding region without introns

<400> 121
atgactaccg gggctgtgca aaggttccgt ccagtggtgg tatcgggtcc ctctgggact      60
gggaagtcga ccttgctcaa gagactcttc gctgaatacc ccgatacttt cgatttatcc     120
gtgtctcata ccactcgagc tccccgtccc ggggaagaaa atggacgtga gtattacttc     180
acaactaaag aagatttcct ggatcttgtg agcaagaatg cctttatcga gcatgcgcag     240
tttggtggca attactacgg tactactgtg caggcagtga aggatgttgc gcagaagggc     300
aagatctgcg ttctcgacat tgagatggag ggcgtgaaac aagtcaagcg caccgatctt     360
gatgctcgat tcttattttt agcacccccg tcccttgaag aactagagaa aagactgcgt     420
gggagagcaa ccgagactga ggagagcttg acgaaacgcc ttgcccaagc taaaaatgaa     480
ttggaatatg cggcgcagcc tggctctcat gataagattg tcgtgaacga tgacctggag     540
aaggcttata aggaactgcg ggattggatt gtcgacggtg gtaactttgg agcgcgtcaa     600
tga                                                                   603

<210> 122
<211> 200
<212> PRT
<213> Aspergillus fumigatus

<220>
<223> Phylum CEA254; clone 7-1-10; contig 4911 region 43163-41221
      Protein sequence

<400> 122

Met Thr Thr Gly Ala Val Gln Arg Phe Arg Pro Val Val Val Ser Gly
1               5                   10                  15

Pro Ser Gly Thr Gly Lys Ser Thr Leu Leu Lys Arg Leu Phe Ala Glu
            20                  25                  30

Tyr Pro Asp Thr Phe Asp Leu Ser Val Ser His Thr Thr Arg Ala Pro
        35                  40                  45

Arg Pro Gly Glu Glu Asn Gly Arg Glu Tyr Tyr Phe Thr Thr Lys Glu
    50                  55                  60

Asp Phe Leu Asp Leu Val Ser Lys Asn Ala Phe Ile Glu His Ala Gln
65                  70                  75                  80

Phe Gly Gly Asn Tyr Tyr Gly Thr Thr Val Gln Ala Val Lys Asp Val
                85                  90                  95

Ala Gln Lys Gly Lys Ile Cys Val Leu Asp Ile Glu Met Glu Gly Val
            100                 105                 110

Lys Gln Val Lys Arg Thr Asp Leu Asp Ala Arg Phe Leu Phe Leu Ala
        115                 120                 125

Pro Pro Ser Leu Glu Glu Leu Glu Lys Arg Leu Arg Gly Arg Ala Thr
    130                 135                 140

Glu Thr Glu Glu Ser Leu Thr Lys Arg Leu Ala Gln Ala Lys Asn Glu
145                 150                 155                 160

Leu Glu Tyr Ala Ala Gln Pro Gly Ser His Asp Lys Ile Val Val Asn
                165                 170                 175

Asp Asp Leu Glu Lys Ala Tyr Lys Glu Leu Arg Asp Trp Ile Val Asp
            180                 185                 190

Gly Gly Asn Phe Gly Ala Arg Gln
        195                 200

<210> 123
<211> 3108
<212> DNA
<213> Aspergillus fumigatus

<220>
<223> Phylum CEA255; clone 10-3-7; contig 4899 region 441274-438167
      Genomic sequence containing 3' and 5'-ends and the coding region

<400> 123
aaaccaggcg catatgttcc gctgccgccc tgcgcaggag agggctgata aggattgcca      60
tatccagacg acgggggctg ctgggatgct gggggaggag gcgagcgcat catgggaacg     120
gtagatacat gctgaggcac aggatggtgg agtggaggtg actgcgcagg cggtggtgcg     180
aaagactggc ggtacatgtt gatttttttt tcccttgtta caataagtga gaagctagtg     240
atgaacaaaa gacttgcgac tattctgtct cgtcttcttg tcttctacca accgaagagg     300
ggggatgttg gaaatcggac agtttgagta tgagtgatgt tgaagtgtgt ttatacgtgg     360
ctggactcgg tctgatcgcc ggagagctct caccttttcc gcccacggtt ccccaccata     420
gtggccacta cacacacttg tccgttctcc aaaacccacg ctgctcgcac tgaataatat     480
acacaagaag tgcttacaac atgttagaag ccttcgaagt cttgacaaca tctggggtgg     540
tgctgtggtc gaagtcgtat gcgccggtcg gagcgcatgt tgtcaacagc ctaatcaacg     600
atgtcttcat tgaggagaag gttcgagcgc agaatcaggc agcgagcagt gcagctccta     660
tctacaagaa ggaaaagtat actctgaaat ggaagcaagt aaaggatttc aatctgatat     720
ttgtggtatg ttcacgccgc tcgttgattc aatggcgcca ctgaccgatt ccataggctg     780
tatatcaatc tctgctacat cttggttgga tcgacaaact cttggataat gtttcgacca     840
tattcatcga cttatataag gatgagctaa ggagcacacg ggctaggatt attgagtacc     900
cattcgataa gtacttcgac cagcaggtgc gagagcttga ggacaatgct ggggctccta     960
catcagaatc tctcgtagta gagatcaacg agagaaagga ccctcttgtc tcatcagata    1020
acggcgggcc acctccgcca cccgtgcctg gtctgctgaa aggtatctga cgtcgataat    1080
ttttctctgc tagtgatcat attgctaact acctccgaag cgcaacgtcc agttgcgcag    1140
ggcgtggcga cctcggacga gggttcgcca ccccaaaccc cagatctttc tcgatcgtca    1200
acgcccattt caggtcatct attgaccgcg aaaggagggc ctgctggccg cgcctctcgt    1260
cgcgcacgca aagcggccaa cgcgagcgct accgcttctt ctggagatga aagcattcgg    1320
aaggggaaaa cattgaaaag tggaaaaaag atgcgcaagt gggatgctga tggctttgcg    1380
gatgaggacg acggcaaggt cctcgattac tccgcccccg cagatggtga ggacgcaccg    1440
gctcctgtag tcgaggctgt tgcgcaggaa tcctggggac gccgaacagg caagggccaa     1500
tttgtgctga aagatctagg ggatgaagtc cattccattc ttgagaatgc tgatcatgaa    1560
aagacaaagt cttcctcgtc cacgggcttt gttgggtctg gagtcaacgc acttggtgga    1620
ttcttccgta atattgtcgg cggcaaggtc cttactgagg ctgacttgga gaaacccttg    1680
aaagccatgg aagaccattt gctgaagaag aacgttgcgc gcgaagcggc cgtccgtcta    1740
tgtcaaggcg tccagcgcga attagttggc aagaagacag gcaactttca aagtgttgat    1800
gcagcactgc gctccgcaat ggagtcctcg ttgcgcaaaa tattgacgcc aacgtcatct    1860
ctcgatctac tgcgtgagat cgatgctgtt agatctccga cgagcaaagg acaggctcct    1920
cgcccatatg tcatttccat cgtgggcgtg aacggtgttg ggaagtcgac aaatctgggc    1980
aaaatttgtt acttccttct ccagaataac tatcgtgttc tgattgcagc ctgtgacacc    2040
ttccgctctg gagccgtgga gcagttacga gtccatgctc gcaatttgaa ggaacttagt    2100
acccgggaga atgctggaga ggttgaactc tacgagaagg gatatggaaa ggatgcagcg    2160
aatgtagcga aggatgcagt ggagtacggt gcggcgaatc atttcgacgt tgtgttgatt    2220
gatactgccg gtcgccgtca taacgaccaa cgccttatgt cttcgctcga gaagttcgcc    2280
aagttcgcca aaccagataa gatcttcatg gtcggtgaag ctctggtcgg tacggacagc    2340
gtgatgcagg ctcgcaactt caaccaagct ttcggcactg ggagaaacct cgatgggttc    2400
atcatcagta aatgtgatac cgttggtgac atggtaggta cgcttgtcag catggtgcat    2460
gctacaggca ttcctattgt ttttctgggt gtaggccagc actatggtga tttgaggggc    2520
ctaagtgttc cttgggctgt caatctgctg atgaagtgag cgcgcagctt ttgccttgta    2580
atagagaatt atattatctc gcttctgatc acgctgggtc ttggatggcc ttcttcaaaa    2640
ataagttact gcgattgtac atctcgcgct tcgctaaaga tagagaaacg aaagatagac    2700
aaagaacggg ggaaaaaatt gacaaaaaga atgacctacg caggattcga acctgcaatc    2760
tcttgatccg tagtcaagcg ccttaccatt gggccagcag gccttcttga aggttttcac    2820

ccagaaatat gctttatcag gaatctggga acatccgcat aaactccaat aataagcttg 2880
tgcctgtcat tgttactacg ctaaagtgac tctctagtgt ggctcttatg caggagaaac 2940
catacaggat attgtaacgg tgaatgcatt ttttctgcct tgaggtatga caccattttg 3000
ttttggaggt ggactcgatc acgagctcac tatcccggcc tcatgggagg attatgacac 3060
ccttggttcc tgaatacttg gtcttggctg gaattacttc aatgacac 3108

<210> 124
<211> 2059
<212> DNA
<213> Aspergillus fumigatus

<220>
<223> Phylum CEA255; clone 10-3-7; contig 4899 region 441274-438167
      Genomic sequence containing the coding region

<400> 124
atgttagaag ccttcgaagt cttgacaaca tctggggtgg tgctgtggtc gaagtcgtat 60
gcgccggtcg gagcgcatgt tgtcaacagc ctaatcaacg atgtcttcat tgaggagaag 120
gttcgagcgc agaatcaggc agcgagcagt gcagctccta tctacaagaa ggaaaagtat 180
actctgaaat ggaagcaagt aaaggatttc aatctgatat ttgtggtatg ttcacgccgc 240
tcgttgattc aatggcgcca ctgaccgatt ccataggctg tatatcaatc tctgctacat 300
cttggttgga tcgacaaact cttggataat gtttcgacca tattcatcga cttatataag 360
gatgagctaa ggagcacacg ggctaggatt attgagtacc cattcgataa gtacttcgac 420
cagcaggtgc gagagcttga ggacaatgct ggggctccta catcagaatc tctcgtagta 480
gagatcaacg agagaaagga ccctcttgtc tcatcagata acggcgggcc acctccgcca 540
cccgtgcctg gtctgctgaa aggtatctga cgtcgataat ttttctctgc tagtgatcat 600
attgctaact acctccgaag cgcaacgtcc agttgcgcag ggcgtggcga cctcggacga 660
gggttcgcca ccccaaaccc cagatctttc tcgatcgtca acgcccattt caggtcatct 720
attgaccgcg aaaggagggc ctgctggccg cgcctctcgt cgcgcacgca aagcggccaa 780
cgcgagcgct accgcttctt ctggagatga aagcattcgg aaggggaaaa cattgaaaag 840
tggaaaaaag atgcgcaagt gggatgctga tggctttgcg gatgaggacg acggcaaggt 900
cctcgattac tccgcccccg cagatggtga ggacgcaccg gctcctgtag tcgaggctgt 960
tgcgcaggaa tcctggggac gccgaacagg caagggccaa tttgtgctga aagatctagg 1020
ggatgaagtc cattccattc ttgagaatgc tgatcatgaa aagacaaagt cttcctcgtc 1080
cacgggcttt gttgggtctg gagtcaacgc acttggtgga ttcttccgta atattgtcgg 1140
cggcaaggtc cttactgagg ctgacttgga gaaacccttg aaagccatgg aagaccattt 1200
gctgaagaag aacgttgcgc gcgaagcggc cgtccgtcta tgtcaaggcg tccagcgcga 1260
attagttggc aagaagacag gcaactttca aagtgttgat gcagcactgc gctccgcaat 1320
ggagtcctcg ttgcgcaaaa tattgacgcc aacgtcatct ctcgatctac tgcgtgagat 1380
cgatgctgtt agatctccga cgagcaaagg acaggctcct cgcccatatg tcatttccat 1440
cgtgggcgtg aacggtgttg ggaagtcgac aaatctgggc aaaatttgtt acttccttct 1500
ccagaataac tatcgtgttc tgattgcagc ctgtgacacc ttccgctctg gagccgtgga 1560
gcagttacga gtccatgctc gcaatttgaa ggaacttagt acccgggaga atgctggaga 1620
ggttgaactc tacgagaagg gatatggaaa ggatgcagcg aatgtagcga aggatgcagt 1680
ggagtacggt gcggcgaatc atttcgacgt tgtgttgatt gatactgccg gtcgccgtca 1740
taacgaccaa cgccttatgt cttcgctcga gaagttcgcc aagttcgcca aaccagataa 1800
gatcttcatg gtcggtgaag ctctggtcgg tacggacagc gtgatgcagg ctcgcaactt 1860
caaccaagct ttcggcactg ggagaaacct cgatgggttc atcatcagta aatgtgatac 1920
cgttggtgac atggtaggta cgcttgtcag catggtgcat gctacaggca ttcctattgt 1980
ttttctgggt gtaggccagc actatggtga tttgaggggc ctaagtgttc cttgggctgt 2040
caatctgctg atgaagtga 2059

<210> 125
<211> 1884
<212> DNA
<213> Aspergillus fumigatus

<220>
<223> Phylum CEA255; clone 10-3-7; contig 4899 region 441274-438167

Coding region without introns

<400> 125
atgttagaag ccttcgaagt cttgacaaca tctggggtgg tgctgtggtc gaagtcgtat      60
gcgccggtcg gagcgcatgt tgtcaacagc ctaatcaacg atgtcttcat tgaggagaag     120
gttcgagcgc agaatcaggc agcgagcagt gcagctccta tctacaagaa ggaaaagtat     180
actctgaaat ggaagcaagt aaaggatttc aatctgatat ttgtgcttgg ttggatcgac     240
aaactcttgg ataatgtttc gaccatattc atcgacttat ataaggatga gctaaggagc     300
acacggctta ggattattga gtacccattc gataagtact tcgaccagca ggtgcgagag     360
cttgaggaca atgctggggc tcctacatca gaatctctcg tagtagagat caacgagaga     420
aaggaccctc ttgtctcatc agataacggc gggccacctc cgccacccgt gcctgcctcg     480
gacgagggtt cgccacccca aaccccagat ctttctcgat cgtcaacgcc catttcaggt     540
catctattga ccgcgaaagg agggcctgct ggccgcgcct ctcgtcgcgc acgcaaagcg     600
gccaacgcga gcgctaccgc ttcttctgga gatgaaagca ttcggaaggg gaaaacattg     660
aaaagtggaa aaaagatgcg caagtgggat gctgatggct ttgcggatga ggacgacggc     720
aaggtcctcg attactccgc ccccgcagat ggtgaggacg caccggctcc tgtagtcgag     780
gctgttgcgc aggaatcctg gggacgccga acaggcaagg gccaatttgt gctgaaagat     840
ctaggggatg aagtccattc cattcttgag aatgctgatc atgaaaagac aaagtcttcc     900
tcgtccacgg gctttgttgg gtctggagtc aacgcacttg gtggattctt ccgtaatatt     960
gtcggcggca aggtccttac tgaggctgac ttggagaaac ccttgaaagc catggaagac    1020
catttgctga agaagaacgt tgcgcgcgaa gcggccgtcc gtctatgtca aggcgtccag    1080
cgcgaattag ttggcaagaa gacaggcaac tttcaaagtg ttgatgcagc actgcgctcc    1140
gcaatggagt cctcgttgcg caaaatattg acgccaacgt catctctcga tctactgcgt    1200
gagatcgatg ctgttagatc tccgacgagc aaaggacagg ctcctcgccc atatgtcatt    1260
tccatcgtgg gcgtgaacgg tgttgggaag tcgacaaatc tgggcaaaat ttgttacttc    1320
cttctccaga ataactatcg tgttctgatt gcagcctgtg acaccttccg ctctggagcc    1380
gtggagcagt tacgagtcca tgctcgcaat ttgaaggaac ttagtacccg ggagaatgct    1440
ggagaggttg aactctacga gaagggatat ggaaaggatg cagcgaatgt agcgaaggat    1500
gcagtggagt acggtgcggc gaatcatttc gacgttgtgt tgattgatac tgccggtcgc    1560
cgtcataacg accaacgcct tatgtcttcg ctcgagaagt tcgccaagtt cgccaaacca    1620
gataagatct tcatggtcgg tgaagctctg gtcggtacgg acagcgtgat gcaggctcgc    1680
aacttcaacc aagctttcgg cactgggaga aacctcgatg ggttcatcat cagtaaatgt    1740
gataccgttg gtgacatggt aggtacgctt gtcagcatgg tgcatgctac aggcattcct    1800
attgtttttc tgggtgtagg ccagcactat ggtgatttga ggggcctaag tgttccttgg    1860
gctgtcaatc tgctgatgaa gtga                                           1884


<210> 126
<211> 641
<212> PRT
<213> Aspergillus fumigatus

<220>
<223> Phylum CEA255; clone 10-3-7; contig 4899 region 441274-438167
      Protein sequence

<400> 126

Met Leu Glu Ala Phe Glu Val Leu Thr Thr Ser Gly Val Val Leu Trp
1               5                   10                  15

Ser Lys Ser Tyr Ala Pro Val Gly Ala His Val Val Asn Ser Leu Ile
            20                  25                  30

Asn Asp Val Phe Ile Glu Glu Lys Val Arg Ala Gln Asn Gln Ala Ala
        35                  40                  45

Ser Ser Ala Ala Pro Ile Tyr Lys Lys Glu Lys Tyr Thr Leu Lys Trp
    50                  55                  60

Lys Gln Val Lys Asp Phe Asn Leu Ile Phe Val Ala Val Tyr Gln Ser

65                  70                  75                  80

Leu Leu His Leu Gly Trp Ile Asp Lys Leu Leu Asp Asn Val Ser Thr
                85                  90                  95

Ile Phe Ile Asp Leu Tyr Lys Asp Glu Leu Arg Ser Thr Arg Ala Arg
            100                 105                 110

Ile Ile Glu Tyr Pro Phe Asp Lys Tyr Phe Asp Gln Gln Val Arg Glu
        115                 120                 125

Leu Glu Asp Asn Ala Gly Ala Pro Thr Ser Glu Ser Leu Val Val Glu
    130                 135                 140

Ile Asn Glu Arg Lys Asp Pro Leu Val Ser Ser Asp Asn Gly Gly Pro
145                 150                 155                 160

Pro Pro Pro Pro Val Pro Val Ala Gln Gly Val Ala Thr Ser Asp Glu
                165                 170                 175

Gly Ser Pro Pro Gln Thr Pro Asp Leu Ser Arg Ser Ser Thr Pro Ile
            180                 185                 190

Ser Gly His Leu Leu Thr Ala Lys Gly Gly Pro Ala Gly Arg Ala Ser
        195                 200                 205

Arg Arg Ala Arg Lys Ala Ala Asn Ala Ser Ala Thr Ala Ser Ser Gly
    210                 215                 220

Asp Glu Ser Ile Arg Lys Gly Lys Thr Leu Lys Ser Gly Lys Lys Met
225                 230                 235                 240

Arg Lys Trp Asp Ala Asp Gly Phe Ala Asp Glu Asp Asp Gly Lys Val
                245                 250                 255

Leu Asp Tyr Ser Ala Pro Ala Asp Gly Glu Asp Ala Pro Ala Pro Val
            260                 265                 270

Val Glu Ala Val Ala Gln Glu Ser Trp Gly Arg Arg Thr Gly Lys Gly
        275                 280                 285

Gln Phe Val Leu Lys Asp Leu Gly Asp Glu Val His Ser Ile Leu Glu
    290                 295                 300

Asn Ala Asp His Glu Lys Thr Lys Ser Ser Ser Ser Thr Gly Phe Val
305                 310                 315                 320

Gly Ser Gly Val Asn Ala Leu Gly Gly Phe Phe Arg Asn Ile Val Gly
                325                 330                 335

Gly Lys Val Leu Thr Glu Ala Asp Leu Glu Lys Pro Leu Lys Ala Met
            340                 345                 350

Glu Asp His Leu Leu Lys Lys Asn Val Ala Arg Glu Ala Ala Val Arg
        355                 360                 365

Leu Cys Gln Gly Val Gln Arg Glu Leu Val Gly Lys Lys Thr Gly Asn
    370                 375                 380

Phe Gln Ser Val Asp Ala Ala Leu Arg Ser Ala Met Glu Ser Ser Leu
385                 390                 395                 400

Arg Lys Ile Leu Thr Pro Thr Ser Ser Leu Asp Leu Leu Arg Glu Ile
                405                 410                 415

Asp Ala Val Arg Ser Pro Thr Ser Lys Gly Gln Ala Pro Arg Pro Tyr
            420                 425                 430

Val Ile Ser Ile Val Gly Val Asn Gly Val Gly Lys Ser Thr Asn Leu
        435                 440                 445

Gly Lys Ile Cys Tyr Phe Leu Leu Gln Asn Asn Tyr Arg Val Leu Ile
    450                 455                 460

Ala Ala Cys Asp Thr Phe Arg Ser Gly Ala Val Glu Gln Leu Arg Val
465                 470                 475                 480

His Ala Arg Asn Leu Lys Glu Leu Ser Thr Arg Glu Asn Ala Gly Glu
                485                 490                 495

Val Glu Leu Tyr Glu Lys Gly Tyr Gly Lys Asp Ala Ala Asn Val Ala
            500                 505                 510

Lys Asp Ala Val Glu Tyr Gly Ala Ala Asn His Phe Asp Val Val Leu
        515                 520                 525

Ile Asp Thr Ala Gly Arg Arg His Asn Asp Gln Arg Leu Met Ser Ser
    530                 535                 540

Leu Glu Lys Phe Ala Lys Phe Ala Lys Pro Asp Lys Ile Phe Met Val
545                 550                 555                 560

Gly Glu Ala Leu Val Gly Thr Asp Ser Val Met Gln Ala Arg Asn Phe
                565                 570                 575

Asn Gln Ala Phe Gly Thr Gly Arg Asn Leu Asp Gly Phe Ile Ile Ser
            580                 585                 590

Lys Cys Asp Thr Val Gly Asp Met Val Gly Thr Leu Val Ser Met Val
        595                 600                 605

His Ala Thr Gly Ile Pro Ile Val Phe Leu Gly Val Gly Gln His Tyr
    610                 615                 620

Gly Asp Leu Arg Gly Leu Ser Val Pro Trp Ala Val Asn Leu Leu Met
625                 630                 635                 640

Lys

<210> 127
<211> 2564
<212> DNA
<213> Aspergillus fumigatus

<220>
<223> Phylum CEA256; clone 2-6-4; contig 4938 region 582107-579544
      Genomic sequence containing 3' and 5'-ends and the coding region

<400> 127
cagcgaaggt atcggagagg agttgaccag aactggggag tagtcatggt tatgagacga      60

aaggtcgacg gttcagaagg atttttgcgg gacttgattg agagttgtga gcgcgacaga    120
tggattggat caaggattca attgatcaga gctcggctca gatcatgtat ccagtcttcc    180
gatgattccg attattgtga aactgggtta aatcgcgcca gaggggcagg tatcgtgacg    240
gagagggggt atatcgtcga atggaggttg tgagtgacga cggccgacga ttgcgcagtt    300
caaagcggcg aagaggtctt ggcactctcg gtccaaacac gttgcccgtt ctctccaact    360
aaacggaact gacgaagctc cttcagagga cactctcctc taccgattca tttccttaat    420
ctattccttc tctttctccg gactgcagtg acttcccttt cagccaattg cccgctccac    480
tgtgcggcat tcgatatacc atgcggtggt gcctcactct tctggcattc tgcttcttgg    540
cagttgtacg tgcattaagt agctccggca gtcgtctgtt ggttgttttg gaagatgcca    600
cagaaaagga attatactcg aaattatggg ctgacctaga aggtgctcta acctactgaa    660
cttctacgtt aatatgctaa tattaattgg tagctcgagg atataacctc gacttcgaat    720
cccccaagaa tgacaagctc agcctgttcg aactcggaga ccgagtctac gaccacatgc    780
ttctcctgcc tcccaagtca aagggttagc gttaccctta gacatgtcca tatgctctgc    840
tttgtacatc tcaattgacc tcttggccag gctatggacc ctcccttacc cccaagaata    900
tcattgattt catgaacaag gacggtaacg tcctcctcgc cttgtcgggc aagtccacaa    960
ccgccagcgc tatcagctcg ctgctattgg agctcgatct ccatctccct gtcgatcgtt   1020
cctctgtcac cgtcgatcac ttcaactacg atacactttc tgcctccgat aagcatgatg   1080
ttctgctact ccaccgacca ggcaagttga ggtccgatac caaggctttc tttgatggcg   1140
agggcgttgt agcatttccc agagccgtcc cccacaccct gggcgatgca aaccctctca   1200
ttgcgcctat tctgcgagcg cccgccactg cgtatagtta caaccccaag gaggacgcgt   1260
cgtcagttga ggatgttgca gctacgggtt cgcagttggc tctggtctcg gccatgcagg   1320
ctagaaactc cgctcggttc actctactgg gatccgtgga gagtctgcag gatcagtggt   1380
tttctgcgac tgtcaaggct cctggtgatg ggaagcagat gaagacggtc aaccaggaat   1440
tcgccaagca gcttactgcg tggacattca aggaaaccgg agtcctcaag gtcggaaaga   1500
tcgagcatca tctggctgaa gatggtgaaa tcactcccga gaagctgaac cctaagatct   1560
atcgaataaa gaatgaaact gtaagtgaca gccatctgag gttccattgc ctatttgcat   1620
gctcaccctt ctcaacaggt ctttagcatt gaactttccg aatacaacta tgatcgttac   1680
gcgcccttcg aggttccaac tggcgatgcc gtccagctcg agtttaccat gctgtctccc   1740
ttccatcgcc tgaacttgga acccgtccgt cgaacagata acagtacagt ttacagcaca   1800
cgattcacca cccccgatca gcatggaatc ttctccttcc gagtgaacta caagcgcccg   1860
ttcctcacga acatcgaaga aaaacttgag gtgaccgttc gtcatttcgc tcataacgag   1920
tacccccgaa gctggaaaat cagcggtgga tgggtctgga ttgcgggtct gtggtccgtc   1980
atcgctggct tcttagtatt cgttgttgca tggctttact cagcgccttc tgccgccgca   2040
ctgaacacaa agaagacaca ataatcctct atagaatctc aacgaatgat acttcggaat   2100
gaagcgtgca cttacgccgg aagcccataa ttcaaaagta tgtacagtca tatgccataa   2160
gctagtagtg ttacatgaaa tccggaatct caagacattc cgccaactgg gaactccaaa   2220
accatgccag gagatgagat aaaaagccgc acaataactc agaacagcgg atctaggcat   2280
ctggccagcc tagtgagccg cgagcttgaa aatttatagt cctctagtct tctcagggtc   2340
catctgaact tcattcccgc tgatctcctt aacggtttcg aaggcgggct tgccggcgta   2400
tagttgctgg tgggattggg atttgcctcc ttcgagctcc ctggaaaccg agataggctg   2460
atttccacgg aatcgctttc gcatgtcgat caacccacgc ctagattgtg cccgttgtca   2520
gcactatcca atgtaattaa agcacgcata tccgttccac ccac                    2564


<210> 128
<211> 1564
<212> DNA
<213> Aspergillus fumigatus

<220>
<223> Phylum CEA256; clone 2-6-4; contig 4938 region 582107-579544
      Genomic sequence containing the coding region

<400> 128
atgcggtggt gcctcactct tctggcattc tgcttcttgg cagttgtacg tgcattaagt     60
agctccggca gtcgtctgtt ggttgttttg gaagatgcca cagaaaagga attatactcg    120
aaattatggg ctgacctaga aggtgctcta acctactgaa cttctacgtt aatatgctaa    180
tattaattgg tagctcgagg atataacctc gacttcgaat cccccaagaa tgacaagctc    240
agcctgttcg aactcggaga ccgagtctac gaccacatgc ttctcctgcc tcccaagtca    300
aagggttagc gttaccctta gacatgtcca tatgctctgc tttgtacatc tcaattgacc    360
tcttggccag gctatggacc ctcccttacc cccaagaata tcattgattt catgaacaag    420

gacggtaacg tcctcctcgc cttgtcgggc aagtccacaa ccgccagcgc tatcagctcg 480
ctgctattgg agctcgatct ccatctccct gtcgatcgtt cctctgtcac cgtcgatcac 540
ttcaactacg atacactttc tgcctccgat aagcatgatg ttctgctact ccaccgacca 600
ggcaagttga ggtccgatac caaggctttc tttgatggcg agggcgttgt agcatttccc 660
agagccgtcc cccacaccct gggcgatgca aaccctctca ttgcgcctat tctgcgagcg 720
cccgccactg cgtatagtta caaccccaag gaggacgcgt cgtcagttga ggatgttgca 780
gctacgggtt cgcagttggc tctggtctcg gccatgcagg ctagaaactc cgctcggttc 840
actctactgg gatccgtgga gagtctgcag gatcagtggt tttctgcgac tgtcaaggct 900
cctggtgatg ggaagcagat gaagacggtc aaccaggaat tcgccaagca gcttactgcg 960
tggacattca aggaaaccgg agtcctcaag gtcggaaaga tcgagcatca tctggctgaa 1020
gatggtgaaa tcactcccga gaagctgaac cctaagatct atcgaataaa gaatgaaact 1080
gtaagtgaca gccatctgag gttccattgc ctatttgcat gctcaccctt ctcaacaggt 1140
ctttagcatt gaactttccg aatacaacta tgatcgttac gcgcccttcg aggttccaac 1200
tggcgatgcc gtccagctcg agtttaccat gctgtctccc ttccatcgcc tgaacttgga 1260
acccgtccgt cgaacagata acagtacagt ttacagcaca cgattcacca cccccgatca 1320
gcatggaatc ttctccttcc gagtgaacta caagcgcccg ttcctcacga acatcgaaga 1380
aaaacttgag gtgaccgttc gtcatttcgc tcataacgag tacccccgaa gctggaaaat 1440
cagcggtgga tgggtctgga ttgcgggtct gtggtccgtc atcgctggct tcttagtatt 1500
cgttgttgca tggctttact cagcgccttc tgccgccgca ctgaacacaa agaagacaca 1560
ataa 1564

<210> 129
<211> 1383
<212> DNA
<213> Aspergillus fumigatus

<220>
<223> Phylum CEA256; clone 2-6-4; contig 4938 region 582107-579544
      Coding region without introns

<400> 129
atgcggtggt gcctcactct tctggcattc tgcttcttgg cagttgtacg tgcattaagt 60
agctccggca gtcgtctgtt ggttgttttg gaagatgcca cagaaaagga attatactcg 120
aaattatggg ctgacctaga aggatataac ctcgacttcg aatcccccaa gaatgacaag 180
ctcagcctgt tcgaactcgg agaccgagtc tacgaccaca tgcttctcct gcctcccaag 240
tcaaagggct atggaccctc ccttaccccc aagaatatca ttgatttcat gaacaaggac 300
ggtaacgtcc tcctcgcctt gtcgggcaag tccacaaccg ccagcgctat cagctcgctg 360
ctattggagc tcgatctcca tctccctgtc gatcgttcct ctgtcaccgt cgatcacttc 420
aactacgata cactttctgc ctccgataag catgatgttc tgctactcca ccgaccaggc 480
aagttgaggt ccgataccaa ggctttcttt gatggcgagg gcgttgtagc atttcccaga 540
gccgtccccc acaccctggg cgatgcaaac cctctcattg cgcctattct gcgagcgccc 600
gccactgcgt atagttacaa ccccaaggag gacgcgtcgt cagttgagga tgttgcagct 660
acgggttcgc agttggctct ggtctcggcc atgcaggcta gaaactccgc tcggttcact 720
ctactgggat ccgtggagag tctgcaggat cagtggtttt ctgcgactgt caaggctcct 780
ggtgatggga agcagatgaa gacggtcaac caggaattcg ccaagcagct tactgcgtgg 840
acattcaagg aaaccggagt cctcaaggtc ggaaagatcg agcatcatct ggctgaagat 900
ggtgaaatca ctcccgagaa gctgaaccct aagatctatc gaataaagaa tgaaactgtc 960
tttagcattg aactttccga atacaactat gatcgttacg cgcccttcga ggttccaact 1020
ggcgatgccg tccagctcga gtttaccatg ctgtctccct tccatcgcct gaacttggaa 1080
cccgtccgtc gaacagataa cagtacagtt tacagcacac gattcaccac ccccgatcag 1140
catggaatct tctccttccg agtgaactac aagcgcccgt tcctcacgaa catcgaagaa 1200
aaacttgagg tgaccgttcg tcatttcgct cataacgagt acccccgaag ctggaaaatc 1260
agcggtggat gggtctggat tgcgggtctg tggtccgtca tcgctggctt cttagtattc 1320
gttgttgcat ggctttactc agcgccttct gccgccgcac tgaacacaaa gaagacacaa 1380
taa 1383

<210> 130
<211> 460
<212> PRT

<213> Aspergillus fumigatus

<220>
<223> Phylum CEA256; clone 2-6-4; contig 4938 region 582107-579544
      Protein sequence

<400> 130
Met Arg Trp Cys Leu Thr Leu Leu Ala Phe Cys Phe Leu Ala Val Val
1               5                   10                  15

Arg Ala Leu Ser Ser Ser Gly Ser Arg Leu Leu Val Val Leu Glu Asp
            20                  25                  30

Ala Thr Glu Lys Glu Leu Tyr Ser Lys Leu Trp Ala Asp Leu Glu Gly
        35                  40                  45

Tyr Asn Leu Asp Phe Glu Ser Pro Lys Asn Asp Lys Leu Ser Leu Phe
    50                  55                  60

Glu Leu Gly Asp Arg Val Tyr Asp His Met Leu Leu Leu Pro Pro Lys
65                  70                  75                  80

Ser Lys Gly Tyr Gly Pro Ser Leu Thr Pro Lys Asn Ile Ile Asp Phe
                85                  90                  95

Met Asn Lys Asp Gly Asn Val Leu Leu Ala Leu Ser Gly Lys Ser Thr
            100                 105                 110

Thr Ala Ser Ala Ile Ser Ser Leu Leu Leu Glu Leu Asp Leu His Leu
        115                 120                 125

Pro Val Asp Arg Ser Ser Val Thr Val Asp His Phe Asn Tyr Asp Thr
    130                 135                 140

Leu Ser Ala Ser Asp Lys His Asp Val Leu Leu Leu His Arg Pro Gly
145                 150                 155                 160

Lys Leu Arg Ser Asp Thr Lys Ala Phe Phe Asp Gly Glu Gly Val Val
                165                 170                 175

Ala Phe Pro Arg Ala Val Pro His Thr Leu Gly Asp Ala Asn Pro Leu
            180                 185                 190

Ile Ala Pro Ile Leu Arg Ala Pro Ala Thr Ala Tyr Ser Tyr Asn Pro
        195                 200                 205

Lys Glu Asp Ala Ser Ser Val Glu Asp Val Ala Ala Thr Gly Ser Gln
    210                 215                 220

Leu Ala Leu Val Ser Ala Met Gln Ala Arg Asn Ser Ala Arg Phe Thr
225                 230                 235                 240

Leu Leu Gly Ser Val Glu Ser Leu Gln Asp Gln Trp Phe Ser Ala Thr
                245                 250                 255

Val Lys Ala Pro Gly Asp Gly Lys Gln Met Lys Thr Val Asn Gln Glu
            260                 265                 270

Phe Ala Lys Gln Leu Thr Ala Trp Thr Phe Lys Glu Thr Gly Val Leu
        275                 280                 285

Lys Val Gly Lys Ile Glu His His Leu Ala Glu Asp Gly Glu Ile Thr
    290                 295                 300

Pro Glu Lys Leu Asn Pro Lys Ile Tyr Arg Ile Lys Asn Glu Thr Val
305                 310                 315                 320

Phe Ser Ile Glu Leu Ser Glu Tyr Asn Tyr Asp Arg Tyr Ala Pro Phe
                325                 330                 335

Glu Val Pro Thr Gly Asp Ala Val Gln Leu Glu Phe Thr Met Leu Ser
            340                 345                 350

Pro Phe His Arg Leu Asn Leu Glu Pro Val Arg Arg Thr Asp Asn Ser
        355                 360                 365

Thr Val Tyr Ser Thr Arg Phe Thr Thr Pro Asp Gln His Gly Ile Phe
    370                 375                 380

Ser Phe Arg Val Asn Tyr Lys Arg Pro Phe Leu Thr Asn Ile Glu Glu
385                 390                 395                 400

Lys Leu Glu Val Thr Val Arg His Phe Ala His Asn Glu Tyr Pro Arg
                405                 410                 415

Ser Trp Lys Ile Ser Gly Gly Trp Val Trp Ile Ala Gly Leu Trp Ser
            420                 425                 430

Val Ile Ala Gly Phe Leu Val Phe Val Val Ala Trp Leu Tyr Ser Ala
        435                 440                 445

Pro Ser Ala Ala Ala Leu Asn Thr Lys Lys Thr Gln
    450                 455                 460


<210> 131

<211> 3376
<212> DNA
<213> Aspergillus fumigatus

<220>
<223> Phylum CEA257; clone 2-1-1; contig 4951 region 8362-11737
      Genomic sequence containing 3' and 5'-ends and the coding region

<400> 131
gcccaggcaa catctacctt atggcatcgc agagcgagaa ttgctcaaca tctttgaggt      60
ctttctgctt gaattcccgg ggacccttct tctccttctt gctcttcgcc ttggcctttt     120
tatttcgggc ctgagcattg ctcttggctc ctcggacctg ttggtgaaag ctcgaaagca     180
aaggcgccat tgttctttgt cgaagaggca attgacatgt tgagagaatg ctccttgccg     240
ccgacggcag gatcgagccg aaggaggaca tggtttgaag agtggattga tgcgagcttt     300
cagactaact cctgggagac ggagatgttt tcttctccga aagctctctc tcagtgatgc     360
gggcaggaga aacgtaacgc cggcggagtc ccttttgagt cagatgcccc tctgtactat     420
tcaattttcg gggaattcaa cagcccactt gttacgctct cgcaggtcga tttcactcga     480
ggcggatttt gagggccgca atgtctcagt atcagcttac tgtggccacc agggccaatc     540
agccctatgt acttcctgtc ctactggtcg caacttccat caacgaggca cgaccaagcc     600
cagtgatatc gatcacctat gaggatactg cggttcttcg tgaaggagac aaggccgtcg     660
tgcaatacac tggagctagc ggtaatccta tctttggcct tatcaatgct gttcaggaac     720
tccgcaaaga cttccccttc cttaacagca aggatgagaa gctggtaaga ggcgccatgg     780
agccttactg ctgatgagca ctgataagtg atactaaccc tccttttata ggagaatgaa     840
tggctgtctc agttggaagc atttgctcct ctagatttca aggcccttga ccctgaattg     900
cagcgcctcg atacccacct cctgctgaga tctttcgtcg tcggttacgc tctctcgacg     960

gccgacattg ccctttgggg tgccatccga ggcaaccgtg tcgcagttgc cgcgatcaag 1020
aagggctcac ttgtcaatgt gactcgttgg ttctatttct tggaggatct gtgcccgtgg 1080
gccacatcta cactggaggt cttgaaccag gctgtgcgag agaagaaggc cgccaaggcg 1140
aaggagggag ctagctacga catcgctctt ctcaacactg aaaaaggcgt ggtgacaagg 1200
tttcctcccg agccttcagg ttatcttcac atcggtcacg caaaagctgc gctgctcaac 1260
gactactttg cccacgagaa gtataatggc acccttcttg tccgctttga cgacacaaat 1320
ccttcgaacg agaagctcga gttccaggac gcgatcattg aagatcttgc tctcatgggc 1380
atcaagcccg acaagatgag ctacaccagt gactactttg acgagcttta ccagtacgcc 1440
cttcaaatca tcaaggacgg taacgcctac gccgacgata ccgagaagga ggtcatggct 1500
gagcagagaa tgaatggaaa acccagcaag cgtcgtgatg catccgtcga ggagaacctt 1560
gcccgcttcg aggagatgaa gaagggtacc cctgagggtc tccgttggtg tatccgagcc 1620
aagatgtctg tcgataaccc caacaaggcc atgcgtgatc ctgtcattta ccgctgcaac 1680
cctgcccctc accaccgcac tgggacgaag tggaagatct atcctaccta tgacttcgcc 1740
tgccctatcg tcgattcaat tgagggtgtg actcatgccc tcagaaccat tgaataccgc 1800
gatcgcaacc ctcagtacca gtggttcttg gacacgctca agcttcgcca tgtccaaatc 1860
tgggattttg ctcgcatgaa cttcattcgc accttgctgt ctaagagaaa acttaccaag 1920
ctcgttaacc aaggtgtcgt ctggggatgg gatgagtaag tttacctttg cttgcaaacg 1980
gattcttgtc ttactaacga tgtcagtcct cgtttcccca ccatccgagt aagtaacatg 2040
cctagtcatg cctgcgaatc cccttattca tccggcattt tttaccatct cacctacttc 2100
ccatgtactg ttaacttccc acgctaatcc tttcataggg catccgacga aggggaatga 2160
ctatccctgc tctgagagaa ttcattctta agcagggacc cagcaagaac atcaccaacc 2220
ttgactggac cctgatctgg gcgaccaaca agaagtacat tgatcctgtc gcacctcgtc 2280
acactgccat tctcaagaag gatatggtca aggcgatcgt caagggaggc ccggctacac 2340
cttacacgga agagaaacct aagcacggca agaaccctgc agttggtatg aagaaggtgg 2400
tttttggtaa cacggtcatt ttcgaccaga aagatgccaa gagcttcaag caagatgaag 2460
agatcacctt gatgagctgg ggtaatgcca ttgtccgtaa gatcgagacc gatcctacct 2520
caggcatcgt caaggagctg gagctggagc tccacctgga aggtgacttc aaaaagaccg 2580
agaagaaggt cacgtggctc tctactgagg gacaggacct aattcccgtt gaattggtcg 2640
atttcgacta tctcctcaac aaggacaccc tgcaggagga cgacgtcctt gaggatgtcc 2700
tgaacaagaa caccgagttc agagaggacg ctgttgctga ctgcaacgtc gctgaactga 2760
aagaaggtga catcatccag tttgagcgca agggctatta ccgtgttgac cgggcctatg 2820
taccgggcaa gccggctgtt ttgttcaaca ttcccacggg caagacgggc aaatagatga 2880
atagttcgat atttgggact taacgaagcc acgcatgtgt ctacaacgct gtagatatta 2940
gaagcactga aagagaattc aatttacatt gttaggatgc taaatcgctg atttacgaca 3000
ggtgtaagtc ttgttaattg caagaatacc agtacttata gttatttccc ttcttctttg 3060
cagcaacgat accttgatgc tccctacaag gaagcttaca caatatggtc taaggtgtgc 3120
tctatctata cgccatacta gctgccgcgg tctgcgctac ccgcgccatc gcgccatctt 3180
ctactccgta caatctccac aatttctccc agctgcattg agatgtgatt ccatgagaag 3240
cgaaagatga tcttcatgac gtcgaagact aaactacctt acctcttggc cggacacgag 3300
ccatccggcg agaaccgaca gcgttgttcc atggcttact agttggcctg tggtcatcgt 3360
ctacttgatc atcttt 3376

<210> 132
<211> 2376
<212> DNA
<213> Aspergillus fumigatus

<220>
<223> Phylum CEA257; clone 2-1-1; contig 4951 region 8362-11737
      Genomic sequence containing the coding region

<400> 132
atgtctcagt atcagcttac tgtggccacc agggccaatc agccctatgt acttcctgtc 60
ctactggtcg caacttccat caacgaggca cgaccaagcc cagtgatatc gatcacctat 120
gaggatactg cggttcttcg tgaaggagac aaggccgtcg tgcaatacac tggagctagc 180
ggtaatccta tctttggcct tatcaatgct gttcaggaac tccgcaaaga cttccccttc 240
cttaacagca aggatgagaa gctggtaaga ggcgccatgg agccttactg ctgatgagca 300
ctgataagtg atactaaccc tccttttata ggagaatgaa tggctgtctc agttggaagc 360
atttgctcct ctagatttca aggcccttga ccctgaattg cagcgcctcg atacccacct 420
cctgctgaga tctttcgtcg tcggttacgc tctctcgacg gccgacattg ccctttgggg 480

tgccatccga ggcaaccgtg tcgcagttgc cgcgatcaag aagggctcac ttgtcaatgt 540
gactcgttgg ttctatttct tggaggatct gtgcccgtgg gccacatcta cactggaggt 600
cttgaaccag gctgtgcgag agaagaaggc cgccaaggcg aaggagggag ctagctacga 660
catcgctctt ctcaacactg aaaaaggcgt ggtgacaagg tttcctcccg agccttcagg 720
ttatcttcac atcggtcacg caaaagctgc gctgctcaac gactactttg cccacgagaa 780
gtataatggc acccttcttg tccgctttga cgacacaaat ccttcgaacg agaagctcga 840
gttccaggac gcgatcattg aagatcttgc tctcatgggc atcaagcccg acaagatgag 900
ctacaccagt gactactttg acgagcttta ccagtacgcc cttcaaatca tcaaggacgg 960
taacgcctac gccgacgata ccgagaagga ggtcatggct gagcagagaa tgaatggaaa 1020
acccagcaag cgtcgtgatg catccgtcga ggagaacctt gcccgcttcg aggagatgaa 1080
gaagggtacc cctgagggtc tccgttggtg tatccgagcc aagatgtctg tcgataaccc 1140
caacaaggcc atgcgtgatc ctgtcattta ccgctgcaac cctgcccctc accaccgcac 1200
tgggacgaag tggaagatct atcctaccta tgacttcgcc tgccctatcg tcgattcaat 1260
tgagggtgtg actcatgccc tcagaaccat tgaataccgc gatcgcaacc ctcagtacca 1320
gtggttcttg gacacgctca agcttcgcca tgtccaaatc tgggattttg ctcgcatgaa 1380
cttcattcgc accttgctgt ctaagagaaa acttaccaag ctcgttaacc aaggtgtcgt 1440
ctggggatgg gatgagtaag tttacctttg cttgcaaacg gattcttgtc ttactaacga 1500
tgtcagtcct cgtttcccca ccatccgagt aagtaacatg cctagtcatg cctgcgaatc 1560
cccttattca tccggcattt tttaccatct cacctacttc ccatgtactg ttaacttccc 1620
acgctaatcc tttcataggg catccgacga aggggaatga ctatccctgc tctgagagaa 1680
ttcattctta agcagggacc cagcaagaac atcaccaacc ttgactggac cctgatctgg 1740
gcgaccaaca agaagtacat tgatcctgtc gcacctcgtc acactgccat tctcaagaag 1800
gatatggtca aggcgatcgt caagggaggc ccggctacac cttacacgga agagaaacct 1860
aagcacggca agaaccctgc agttggtatg aagaaggtgg tttttggtaa cacggtcatt 1920
ttcgaccaga aagatgccaa gagcttcaag caagatgaag agatcacctt gatgagctgg 1980
ggtaatgcca ttgtccgtaa gatcgagacc gatcctacct caggcatcgt caaggagctg 2040
gagctggagc tccacctgga aggtgacttc aaaaagaccg agaagaaggt cacgtggctc 2100
tctactgagg gacaggacct aattcccgtt gaattggtcg atttcgacta tctcctcaac 2160
aaggacaccc tgcaggagga cgacgtcctt gaggatgtcc tgaacaagaa caccgagttc 2220
agagaggacg ctgttgctga ctgcaacgtc gctgaactga aagaaggtga catcatccag 2280
tttgagcgca agggctatta ccgtgttgac cgggcctatg taccgggcaa gccggctgtt 2340
ttgttcaaca ttcccacggg caagacgggc aaatag 2376

<210> 133
<211> 2148
<212> DNA
<213> Aspergillus fumigatus

<220>
<223> Phylum CEA257; clone 2-1-1; contig 4951 region 8362-11737
      Coding region without introns

<400> 133
atgtctcagt atcagcttac tgtggccacc agggccaatc agccctatgt acttcctgtc 60
ctactggtcg caacttccat caacgaggca cgaccaagcc cagtgatatc gatcacctat 120
gaggatactg cggttcttcg tgaaggagac aaggccgtcg tgcaatacac tggagctagc 180
ggtaatccta tctttggcct tatcaatgct gttcaggaac tccgcaaaga cttccccttc 240
cttaacagca aggatgagaa gctggagaat gaatggctgt ctcagttgga agcatttgct 300
cctctagatt tcaaggccct tgaccctgaa ttgcagcgcc tcgataccca cctcctgctg 360
agatctttcg tcgtcggtta cgctctctcg acggccgaca ttgccctttg gggtgccatc 420
cgaggcaacc gtgtcgcagt tgccgcgatc aagaagggct cacttgtcaa tgtgactcgt 480
tggttctatt tcttggagga tctgtgcccg tgggccacat ctacactgga ggtcttgaac 540
caggctgtgc gagagaagaa ggccgccaag gcgaaggagg gagctagcta cgacatcgct 600
cttctcaaca ctgaaaaagg cgtggtgaca aggtttcctc ccgagccttc aggttatctt 660
cacatcggtc acgcaaaagc tgcgctgctc aacgactact ttgcccacga gaagtataat 720
ggcacccttc ttgtccgctt tgacgacaca aatccttcga acgagaagct cgagttccag 780
gacgcgatca ttgaagatct tgctctcatg ggcatcaagc ccgacaagat gagctacacc 840
agtgactact ttgacgagct ttaccagtac gcccttcaaa tcatcaagga cggtaacgcc 900
tacgccgacg ataccgagaa ggaggtcatg gctgagcaga gaatgaatgg aaaacccagc 960
aagcgtcgtg atgcatccgt cgaggagaac cttgcccgct tcgaggagat gaagaagggt 1020

acccctgagg gtctccgttg gtgtatccga gccaagatgt ctgtcgataa ccccaacaag 1080
gccatgcgtg atcctgtcat ttaccgctgc aaccctgccc ctcaccaccg cactgggacg 1140
aagtggaaga tctatcctac ctatgacttc gcctgcccta tcgtcgattc aattgagggt 1200
gtgactcatg ccctcagaac cattgaatac cgcgatcgca accctcagta ccagtggttc 1260
ttggacacgc tcaagcttcg ccatgtccaa atctgggatt ttgctcgcat gaacttcatt 1320
cgcaccttgc tgtctaagag aaaacttacc aagctcgtta accaaggtgt cgtctgggga 1380
tgggatgatc ctcgtttccc caccatccga ggcatccgac gaaggggaat gactatccct 1440
gctctgagag aattcattct taagcaggga cccagcaaga acatcaccaa ccttgactgg 1500
accctgatct gggcgaccaa caagaagtac attgatcctg tcgcacctcg tcacactgcc 1560
attctcaaga aggatatggt caaggcgatc gtcaagggag gcccggctac accttacacg 1620
gaagagaaac ctaagcacgg caagaaccct gcagttggta tgaagaaggt ggtttttggt 1680
aacacggtca ttttcgacca gaaagatgcc aagagcttca agcaagatga agagatcacc 1740
ttgatgagct ggggtaatgc cattgtccgt aagatcgaga ccgatcctac ctcaggcatc 1800
gtcaaggagc tggagctgga gctccacctg gaaggtgact tcaaaaagac cgagaagaag 1860
gtcacgtggc tctctactga gggacaggac ctaattcccg ttgaattggt cgatttcgac 1920
tatctcctca acaaggacac cctgcaggag gacgacgtcc ttgaggatgt cctgaacaag 1980
aacaccgagt tcagagagga cgctgttgct gactgcaacg tcgctgaact gaaagaaggt 2040
gacatcatcc agtttgagcg caagggctat taccgtgttg accgggccta tgtaccgggc 2100
aagccggctg ttttgttcaa cattcccacg ggcaagacgg gcaaatag 2148

<210> 134
<211> 715
<212> PRT
<213> Aspergillus fumigatus

<220>
<223> Phylum CEA257; clone 2-1-1; contig 4951 region 8362-11737
      Protein sequence

<400> 134

Met Ser Gln Tyr Gln Leu Thr Val Ala Thr Arg Ala Asn Gln Pro Tyr
1               5                   10                  15

Val Leu Pro Val Leu Leu Val Ala Thr Ser Ile Asn Glu Ala Arg Pro
            20                  25                  30

Ser Pro Val Ile Ser Ile Thr Tyr Glu Asp Thr Ala Val Leu Arg Glu
        35                  40                  45

Gly Asp Lys Ala Val Val Gln Tyr Thr Gly Ala Ser Gly Asn Pro Ile
    50                  55                  60

Phe Gly Leu Ile Asn Ala Val Gln Glu Leu Arg Lys Asp Phe Pro Phe
65                  70                  75                  80

Leu Asn Ser Lys Asp Glu Lys Leu Glu Asn Glu Trp Leu Ser Gln Leu
                85                  90                  95

Glu Ala Phe Ala Pro Leu Asp Phe Lys Ala Leu Asp Pro Glu Leu Gln
            100                 105                 110

Arg Leu Asp Thr His Leu Leu Leu Arg Ser Phe Val Val Gly Tyr Ala
        115                 120                 125

Leu Ser Thr Ala Asp Ile Ala Leu Trp Gly Ala Ile Arg Gly Asn Arg
    130                 135                 140

Val Ala Val Ala Ala Ile Lys Lys Gly Ser Leu Val Asn Val Thr Arg

145                 150                 155                 160

Trp Phe Tyr Phe Leu Glu Asp Leu Cys Pro Trp Ala Thr Ser Thr Leu
                165                 170                 175

Glu Val Leu Asn Gln Ala Val Arg Glu Lys Lys Ala Ala Lys Ala Lys
            180                 185                 190

Glu Gly Ala Ser Tyr Asp Ile Ala Leu Leu Asn Thr Glu Lys Gly Val
        195                 200                 205

Val Thr Arg Phe Pro Pro Glu Pro Ser Gly Tyr Leu His Ile Gly His
    210                 215                 220

Ala Lys Ala Ala Leu Leu Asn Asp Tyr Phe Ala His Glu Lys Tyr Asn
225                 230                 235                 240

Gly Thr Leu Leu Val Arg Phe Asp Asp Thr Asn Pro Ser Asn Glu Lys
                245                 250                 255

Leu Glu Phe Gln Asp Ala Ile Ile Glu Asp Leu Ala Leu Met Gly Ile
            260                 265                 270

Lys Pro Asp Lys Met Ser Tyr Thr Ser Asp Tyr Phe Asp Glu Leu Tyr
        275                 280                 285

Gln Tyr Ala Leu Gln Ile Ile Lys Asp Gly Asn Ala Tyr Ala Asp Asp
    290                 295                 300

Thr Glu Lys Glu Val Met Ala Glu Gln Arg Met Asn Gly Lys Pro Ser
305                 310                 315                 320

Lys Arg Arg Asp Ala Ser Val Glu Glu Asn Leu Ala Arg Phe Glu Glu
                325                 330                 335

Met Lys Lys Gly Thr Pro Glu Gly Leu Arg Trp Cys Ile Arg Ala Lys
            340                 345                 350

Met Ser Val Asp Asn Pro Asn Lys Ala Met Arg Asp Pro Val Ile Tyr
        355                 360                 365

Arg Cys Asn Pro Ala Pro His His Arg Thr Gly Thr Lys Trp Lys Ile
    370                 375                 380

Tyr Pro Thr Tyr Asp Phe Ala Cys Pro Ile Val Asp Ser Ile Glu Gly
385                 390                 395                 400

Val Thr His Ala Leu Arg Thr Ile Glu Tyr Arg Asp Arg Asn Pro Gln
                405                 410                 415

Tyr Gln Trp Phe Leu Asp Thr Leu Lys Leu Arg His Val Gln Ile Trp
            420                 425                 430

Asp Phe Ala Arg Met Asn Phe Ile Arg Thr Leu Leu Ser Lys Arg Lys
        435                 440                 445

Leu Thr Lys Leu Val Asn Gln Gly Val Val Trp Gly Trp Asp Asp Pro
    450                 455                 460

Arg Phe Pro Thr Ile Arg Gly Ile Arg Arg Arg Gly Met Thr Ile Pro
465                 470                 475                 480

Ala Leu Arg Glu Phe Ile Leu Lys Gln Gly Pro Ser Lys Asn Ile Thr
                485                 490                 495

Asn Leu Asp Trp Thr Leu Ile Trp Ala Thr Asn Lys Lys Tyr Ile Asp
            500                 505                 510

Pro Val Ala Pro Arg His Thr Ala Ile Leu Lys Lys Asp Met Val Lys
        515                 520                 525

Ala Ile Val Lys Gly Gly Pro Ala Thr Pro Tyr Thr Glu Glu Lys Pro
    530                 535                 540

Lys His Gly Lys Asn Pro Ala Val Gly Met Lys Lys Val Val Phe Gly
545                 550                 555                 560

Asn Thr Val Ile Phe Asp Gln Lys Asp Ala Lys Ser Phe Lys Gln Asp
                565                 570                 575

Glu Glu Ile Thr Leu Met Ser Trp Gly Asn Ala Ile Val Arg Lys Ile
            580                 585                 590

Glu Thr Asp Pro Thr Ser Gly Ile Val Lys Glu Leu Glu Leu Glu Leu
        595                 600                 605

His Leu Glu Gly Asp Phe Lys Lys Thr Glu Lys Lys Val Thr Trp Leu
    610                 615                 620

Ser Thr Glu Gly Gln Asp Leu Ile Pro Val Glu Leu Val Asp Phe Asp
625                 630                 635                 640

Tyr Leu Leu Asn Lys Asp Thr Leu Gln Glu Asp Asp Val Leu Glu Asp
                645                 650                 655

Val Leu Asn Lys Asn Thr Glu Phe Arg Glu Asp Ala Val Ala Asp Cys
            660                 665                 670

Asn Val Ala Glu Leu Lys Glu Gly Asp Ile Ile Gln Phe Glu Arg Lys
        675                 680                 685

Gly Tyr Tyr Arg Val Asp Arg Ala Tyr Val Pro Gly Lys Pro Ala Val
    690                 695                 700

Leu Phe Asn Ile Pro Thr Gly Lys Thr Gly Lys
705                 710                 715


<210> 135
<211> 3639
<212> DNA
<213> Aspergillus fumigatus

<220>
<223> Phylum CEA258; clone 2-10-16; contig 4912 region 46084-42446
      Genomic sequence containing 3' and 5'-ends and the coding region

<400> 135
acgcttttcg cttgtcatac tcaactctag accggttttc atctcccgat atgccttttt      60
gatttccctt ggaggctctt gctttgcctg gagggcgtca cgagtcctgg acgtcatcca     120
ttcactttgg cttcgacacg gcctcacctc ggagtcgaaa tctggaattc acaacgtgag     180
ggtggccaga ccgatgaaag gatattgtaa cgctgcaatt gcttactcca aaccgtagct     240

ctttgtgccg tatcctttgt tgtgctggac gtggactact agtctccgca ttaaaaggga 300
aacttttgcg tcgtcgtttc ctcaccttgt ttgtcgacac gtccgagact taatcatccg 360
aaaagaacgg cctgcgatca cggccgacct catgctcgcc tatcgctcga gactatcccc 420
tcaccgactc tgacccgcgt accctggcct cacaacacca acacttctcc tctagtttcg 480
cgtacctaga acacgcaaac atgtcgccat caatatccta catttcaggc cagcttaggc 540
agctaatata ctatcatctc gataacaatt tgtgccgtaa tgcgctgttc ctcgccggtc 600
gtttacatgc ttacgagccc cgaacggcgg aagcgtcgta tttactcgct ctctgccatc 660
ttcagaacgg gcaagtcaaa gccgcatacg attacagcag gaattttgga tcgagaggca 720
cccacctcgg ctgctcctat gtcttcgcgc aagcgtgctt ggacctagga aagtatctgg 780
aaggtatcac agcgttagag cggagtaaag gcctttgggc ttcgaagaac cactggagta 840
agtattccat gcgctgatac ttgcagtcga cctgcattta tttcgttgct ttctgttact 900
gacacatttc caccttgtgc tctagataag cacagtgaga cgcgaagaca acatctgccg 960
gatgcagccg cagtattctg cctgttaggt aaattatggc atgcgcataa ggacatcaac 1020
aaagctgtgg aatgctatgt tgaatctctg aagctgaatc ccttcatgtg ggatgcgttc 1080
caagggttgt gcgacaccgg taagctttgg agaataaacc taatacatct agccatggct 1140
aattgtgttc taccgccaag gagtcaatgt ccgcgtgtca aacatctaca agttgaattc 1200
tgaattgctg gccgtattgt cttcatcgcc acaggcggat gctgagccaa tatccgataa 1260
gtctgcacac acgaatgggc cactgcaagc gcaggcgaat gttaatccaa gttccgatcc 1320
ttttgcctcg actacttctc gcagtgattc aggtaccagc catgggagct ctgccttgtg 1380
ggaaaaacta aatggaagca cagtaagcgt ggcgtcatcg ggagtgccag catcaatcgt 1440
gcatgaagga gccgaaacgc cgagtggtca aagcagcgga tctgatgagt tccggttagc 1500
taacggaatg aacggcgcgg atgcttcttg ggaccctcct ttagctcctg caaggaaaaa 1560
cagaacgatc caggcaataa gcggcgagta tccaatggac cctcctccca agatgaaacc 1620
cactgggatc cgaccaagga caaggaccag gactgagccg gaggaccaaa tttcagccca 1680
gatagaccgg gaggcaacaa atgcgcccag ggtcggggac cgcaaacgaa ctgtttctgg 1740
tcaggtagcg catccaccga cgtcacaacc cacagaacca ggagcacccc agcggcggag 1800
tgtgcgactc ttcaaccaga ttaaacccac gaccagcaaa ttgtcggcgt ccgcgctggg 1860
agtcaaggat gctagagaag tcaagaaagc gaaagccaca ggtacgaagg ggcgtacgac 1920
aaccaccacc atgggacgag tagtgagtgg cagccgaaaa catgccagcg aacatcatga 1980
tgcagatggt aaagacggac ggtcggtacc gtccgcccac actcacgcca tctccaaagg 2040
cgctgctcaa gaaagatcga aagaaatcga ggcgttgacc tggctgctgg agctattctc 2100
gaaacttgct tctggattct ttgccttgtg tcgctaccga tgcccagagt caatccagat 2160
cttcaattcg ctctctcaag gccaacggga aacaccgtgg gttctcgctc agattggacg 2220
agcgtactat gagcaggcta tgtattccga ggcagaaaag tacttctacc gtgtgaagac 2280
catggcaccc tcgcgcttgg aagacatgga gatctactcg actgtccttt ggcatctgaa 2340
gaacgatgtt gagttagcct atttggcgca tgagttgatg gaaacagacc gcctgtcgcc 2400
acaggcgtgg tgcgccatcg gtaattcgtt ttcccaccag cgagatcatg accaggcctt 2460
gaagtgcttt aagcgggcaa cccagctgga tcctcagttt gcctacgggt ttactcttca 2520
agggcacgag tatgttgcca acgaagaata cgacaaggcg cttgatgcat accgtcacgg 2580
tatcagcgcg gatagtcggc attacaatgc ttggtacgga ctgggcacgg tttatgacaa 2640
aatgggcaaa ctggactttg ccgaacaaca cttccggaat gcggcaagca ttaacccgac 2700
caacgcagtt ttgatctgct gcattggatt ggtactggaa aaaatgaaca accctaaagc 2760
ggctctcgtg caatatggtc gcgcttgttc cttggcacct cattccgtac tcgcgcgatt 2820
ccgcaaggcc cgcgcattga tgaagctcca ggagctcaaa ttagctctgt ctgagttgaa 2880
gattctcaaa gacatggctc cagacgaagc taacgtgcat tatctgttgg gtaagctcta 2940
caaaatgctt cacgacaaag ccaatgccat taagcacttc acaactgctt tgaacttgga 3000
tccaaaggta tgcctatcac ttccattccg tcaaattacc agacaatact aacggattcg 3060
gttacaggca gcacaataca tcaaggatgc catggaatct cttgacgatg acgaggagga 3120
tgatgaggac atgagctgat ttgcagtcca cttcccacac atcgatccaa ttctggatct 3180
tacttgctac ccccttcttc accaggcgcc tgctattcgg gcttgcattt atgagagtcg 3240
gcgttaaatt tgatcttgga ttttcgcagc attagcatta gcattataag ggcgttttac 3300
aggccgggtt ggttagaggt gcatttaccc tgcatcaggt tttccaaaac ggagctgagg 3360
ctcgtggatc ttgtttcttg tatattagcg tgggttggct ttgtcggggc tggtcattgg 3420
ctgcatatga gagacgaaga cgcctgaacg acagatccat ggacatagat gtctgtcgaa 3480
taaggcactt gcataatctg cgatgtcatt cttatgccta ttgttatcct tccggaaata 3540
tattccatcg tatttgagcc tgaaccacgg atttactcca gaataactta taccactcta 3600
ctgcgcacga ctgtagaagt aattccagaa gctgcaggc 3639

<210> 136
<211> 2639

<212> DNA
<213> Aspergillus fumigatus

<220>
<223> Phylum CEA258; clone 2-10-16; contig 4912 region 46084-42446
      Genomic sequence containing the coding region

<400> 136
atgtcgccat caatatccta catttcaggc cagcttaggc agctaatata ctatcatctc      60
gataacaatt tgtgccgtaa tgcgctgttc ctcgccggtc gtttacatgc ttacgagccc     120
cgaacggcgg aagcgtcgta tttactcgct ctctgccatc ttcagaacgg gcaagtcaaa     180
gccgcatacg attacagcag gaattttgga tcgagaggca cccacctcgg ctgctcctat     240
gtcttcgcgc aagcgtgctt ggacctagga aagtatctgg aaggtatcac agcgttagag     300
cggagtaaag gcctttgggc ttcgaagaac cactggagta agtattccat gcgctgatac     360
ttgcagtcga cctgcattta tttcgttgct ttctgttact gacacatttc caccttgtgc     420
tctagataag cacagtgaga cgcgaagaca acatctgccg gatgcagccg cagtattctg     480
cctgttaggt aaattatggc atgcgcataa ggacatcaac aaagctgtgg aatgctatgt     540
tgaatctctg aagctgaatc ccttcatgtg ggatgcgttc caagggttgt gcgacaccgg     600
taagctttgg agaataaacc taatacatct agccatggct aattgtgttc taccgccaag     660
gagtcaatgt ccgcgtgtca aacatctaca agttgaattc tgaattgctg gccgtattgt     720
cttcatcgcc acaggcggat gctgagccaa tatccgataa gtctgcacac acgaatggcc     780
cactgcaagc gcaggcgaat gttaatccaa gttccgatcc ttttgcctcg actacttctc     840
gcagtgattc aggtaccagc catgggagct ctgccttgtg ggaaaaacta aatggaagca     900
cagtaagcgt ggcgtcatcg ggagtgccag catcaatcgt gcatgaagga gccgaaacgc     960
cgagtggtca aagcagcgga tctgatgagt tccggttagc taacggaatg aacggcgcgg    1020
atgcttcttg ggaccctcct ttagctcctg caaggaaaaa cagaacgatc caggcaataa    1080
gcggcgagta tccaatggac cctcctccca agatgaaacc cactgggatc cgaccaagga    1140
caaggaccag gactgagccg gaggaccaaa tttcagccca gatagaccgg gaggcaacaa    1200
atgcgcccag ggtcggggac cgcaaacgaa ctgtttctgg tcaggtagcg catccaccga    1260
cgtcacaacc cacagaacca ggagcacccc agcggcggag tgtgcgactc ttcaaccaga    1320
ttaaacccac gaccagcaaa ttgtcggcgt ccgcgctggg agtcaaggat gctagagaag    1380
tcaagaaagc gaaagccaca ggtacgaagg ggcgtacgac aaccaccacc atgggacgag    1440
tagtgagtgg cagccgaaaa catgccagcg aacatcatga tgcagatggt aaagacggac    1500
ggtcggtacc gtccgcccac actcacgcca tctccaaagg cgctgctcaa gaaagatcga    1560
aagaaatcga ggcgttgacc tggctgctgg agctattctc gaaacttgct tctggattct    1620
ttgccttgtg tcgctaccga tgcccagagt caatccagat cttcaattcg ctctctcaag    1680
gccaacggga aacaccgtgg gttctcgctc agattggacg agcgtactat gagcaggcta    1740
tgtattccga ggcagaaaag tacttctacc gtgtgaagac catggcaccc tcgcgcttgg    1800
aagacatgga gatctactcg actgtccttt ggcatctgaa gaacgatgtt gagttagcct    1860
atttggcgca tgagttgatg gaaacagacc gcctgtcgcc acaggcgtgg tgcgccatcg    1920
gtaattcgtt ttcccaccag cgagatcatg accaggcctt gaagtgcttt aagcgggcaa    1980
cccagctgga tcctcagttt gcctacgggt ttactcttca agggcacgag tatgttgcca    2040
acgaagaata cgacaaggcg cttgatgcat accgtcacgg tatcagcgcg gatagtcggc    2100
attacaatgc ttggtacgga ctgggcacgg tttatgacaa aatgggcaaa ctggactttg    2160
ccgaacaaca cttccggaat gcggcaagca ttaacccgac caacgcagtt ttgatctgct    2220
gcattggatt ggtactggaa aaaatgaaca accctaaagc ggctctcgtg caatatggtc    2280
gcgcttgttc cttggcacct cattccgtac tcgcgcgatt ccgcaaggcc cgcgcattga    2340
tgaagctcca ggagctcaaa ttagctctgt ctgagttgaa gattctcaaa gacatggctc    2400
cagacgaagc taacgtgcat tatctgttgg gtaagctcta caaaatgctt cacgacaaag    2460
ccaatgccat taagcacttc acaactgctt tgaacttgga tccaaaggta tgcctatcac    2520
ttccattccg tcaaattacc agacaatact aacggattcg gttacaggca gcacaataca    2580
tcaaggatgc catggaatct cttgacgatg acgaggagga tgatgaggac atgagctga     2639


<210> 137
<211> 2430
<212> DNA
<213> Aspergillus fumigatus

<220>
<223> Phylum CEA258; clone 2-10-16; contig 4912 region 46084-42446

Coding region without introns

<400> 137
atgtcgccat caatatccta catttcaggc cagcttaggc agctaatata ctatcatctc 60
gataacaatt tgtgccgtaa tgcgctgttc ctcgccggtc gtttacatgc ttacgagccc 120
cgaacggcgg aagcgtcgta tttactcgct ctctgccatc ttcagaacgg gcaagtcaaa 180
gccgcatacg attacagcag gaattttgga tcgagaggca cccacctcgg ctgctcctat 240
gtcttcgcgc aagcgtgctt ggacctagga aagtatctgg aaggtatcac agcgttagag 300
cggagtaaag gcctttgggc ttcgaagaac cactggaata agcacagtga gacgcgaaga 360
caacatctgc cggatgcagc cgcagtattc tgcctgttag gtaaattatg gcatgcgcat 420
aaggacatca acaaagctgt ggaatgctat gttgaatctc tgaagctgaa tcccttcatg 480
tgggatgcgt tccaagggtt gtgcgacacc ggagtcaatg tccgcgtgtc aaacatctac 540
aagttgaatt ctgaattgct ggccgtattg tcttcatcgc cacaggcgga tgctgagcca 600
atatccgata agtctgcaca cacgaatggg ccactgcaag cgcaggcgaa tgttaatcca 660
agttccgatc cttttgcctc gactacttct cgcagtgatt caggtaccag ccatgggagc 720
tctgccttgt gggaaaaact aaatggaagc acagtaagcg tggcgtcatc gggagtgcca 780
gcatcaatcg tgcatgaagg agccgaaacg ccgagtggtc aaagcagcgg atctgatgag 840
ttccggttag ctaacggaat gaacggcgcg gatgcttctt gggaccctcc tttagctcct 900
gcaaggaaaa acagaacgat ccaggcaata agcggcgagt atccaatgga ccctcctccc 960
aagatgaaac ccactgggat ccgaccaagg acaaggacca ggactgagcc ggaggaccaa 1020
atttcagccc agatagaccg ggaggcaaca aatgcgccca gggtcgggga ccgcaaacga 1080
actgtttctg gtcaggtagc gcatccaccg acgtcacaac ccacagaacc aggagcaccc 1140
cagcggcgga gtgtgcgact cttcaaccag attaaaccca cgaccagcaa attgtcggcg 1200
tccgcgctgg gagtcaagga tgctagagaa gtcaagaaag cgaaagccac aggtacgaag 1260
gggcgtacga caaccaccac catggacgga gtagtgagtg gcagccgaaa acatgccagc 1320
gaacatcatg atgcagatgg taaagacgga cggtcggtac cgtccgccca cactcacgcc 1380
atctccaaag gcgctgctca agaaagatcg aaagaaatcg aggcgttgac ctggctgctg 1440
gagctattct cgaaacttgc ttctggattc tttgccttgt gtcgctaccg atgcccagag 1500
tcaatccaga tcttcaattc gctctctcaa ggccaacggg aaacaccgtg ggttctcgct 1560
cagattggac gagcgtacta tgagcaggct atgtattccg aggcagaaaa gtacttctac 1620
cgtgtgaaga ccatggcacc ctcgcgcttg gaagacatgg agatctactc gactgtcctt 1680
tggcatctga agaacgatgt tgagttagcc tatttggcgc atgagttgat ggaaacagac 1740
cgcctgtcgc cacaggcgtg gtgcgccatc ggtaattcgt tttcccacca gcgagatcat 1800
gaccaggcct tgaagtgctt taagcgggca acccagctgg atcctcagtt tgcctacggg 1860
tttactcttc aagggcacga gtatgttgcc aacgaagaat acgacaaggc gcttgatgca 1920
taccgtcacg gtatcagcgc ggatagtcgg cattacaatg cttggtacgg actgggcacg 1980
gtttatgaca aaatgggcaa actggacttt gccgaacaac acttccggaa tgcggcaagc 2040
attaacccga ccaacgcagt tttgatctgc tgcattggat tggtactgga aaaaatgaac 2100
aaccctaaag cggctctcgt gcaatatggt cgcgcttgtt ccttggcacc tcattccgta 2160
ctcgcgcgat tccgcaaggc ccgcgcattg atgaagctcc aggagctcaa attagctctg 2220
tctgagttga agattctcaa agacatggct ccagacgaag ctaacgtgca ttatctgttg 2280
ggtaagctct acaaaatgct tcacgacaaa gccaatgcca ttaagcactt cacaactgct 2340
ttgaacttgg atccaaaggc agcacaatac atcaaggatg ccatggaatc tcttgacgat 2400
gacgaggagg atgatgagga catgagctga 2430

<210> 138
<211> 809
<212> PRT
<213> Aspergillus fumigatus

<220>
<223> Phylum CEA258; clone 2-10-16; contig 4912 region 46084-42446
      Protein sequence

<400> 138

Met Ser Pro Ser Ile Ser Tyr Ile Ser Gly Gln Leu Arg Gln Leu Ile
1               5                   10                  15

Tyr Tyr His Leu Asp Asn Asn Leu Cys Arg Asn Ala Leu Phe Leu Ala
            20                  25                  30

Gly Arg Leu His Ala Tyr Glu Pro Arg Thr Ala Glu Ala Ser Tyr Leu
        35                  40                  45

Leu Ala Leu Cys His Leu Gln Asn Gly Gln Val Lys Ala Ala Tyr Asp
    50                  55                  60

Tyr Ser Arg Asn Phe Gly Ser Arg Gly Thr His Leu Gly Cys Ser Tyr
65                  70                  75                  80

Val Phe Ala Gln Ala Cys Leu Asp Leu Gly Lys Tyr Leu Glu Gly Ile
                85                  90                  95

Thr Ala Leu Glu Arg Ser Lys Gly Leu Trp Ala Ser Lys Asn His Trp
            100                 105                 110

Asn Lys His Ser Glu Thr Arg Arg Gln His Leu Pro Asp Ala Ala Ala
        115                 120                 125

Val Phe Cys Leu Leu Gly Lys Leu Trp His Ala His Lys Asp Ile Asn
    130                 135                 140

Lys Ala Val Glu Cys Tyr Val Glu Ser Leu Lys Leu Asn Pro Phe Met
145                 150                 155                 160

Trp Asp Ala Phe Gln Gly Leu Cys Asp Thr Gly Val Asn Val Arg Val
                165                 170                 175

Ser Asn Ile Tyr Lys Leu Asn Ser Glu Leu Leu Ala Val Leu Ser Ser
            180                 185                 190

Ser Pro Gln Ala Asp Ala Glu Pro Ile Ser Asp Lys Ser Ala His Thr
        195                 200                 205

Asn Gly Pro Leu Gln Ala Gln Ala Asn Val Asn Pro Ser Ser Asp Pro
    210                 215                 220

Phe Ala Ser Thr Thr Ser Arg Ser Asp Ser Gly Thr Ser His Gly Ser
225                 230                 235                 240

Ser Ala Leu Trp Glu Lys Leu Asn Gly Ser Thr Val Ser Val Ala Ser
                245                 250                 255

Ser Gly Val Pro Ala Ser Ile Val His Glu Gly Ala Glu Thr Pro Ser
            260                 265                 270

Gly Gln Ser Ser Gly Ser Asp Glu Phe Arg Leu Ala Asn Gly Met Asn
        275                 280                 285

Gly Ala Asp Ala Ser Trp Asp Pro Pro Leu Ala Pro Ala Arg Lys Asn
    290                 295                 300

Arg Thr Ile Gln Ala Ile Ser Gly Glu Tyr Pro Met Asp Pro Pro Pro
305                 310                 315                 320

Lys Met Lys Pro Thr Gly Ile Arg Pro Arg Thr Arg Thr Arg Thr Glu
                325                 330                 335

Pro Glu Asp Gln Ile Ser Ala Gln Ile Asp Arg Glu Ala Thr Asn Ala
            340                 345                 350

Pro Arg Val Gly Asp Arg Lys Arg Thr Val Ser Gly Gln Val Ala His
        355                 360                 365

Pro Pro Thr Ser Gln Pro Thr Glu Pro Gly Ala Pro Gln Arg Arg Ser
    370                 375                 380

Val Arg Leu Phe Asn Gln Ile Lys Pro Thr Thr Ser Lys Leu Ser Ala
385                 390                 395                 400

Ser Ala Leu Gly Val Lys Asp Ala Arg Glu Val Lys Lys Ala Lys Ala
                405                 410                 415

Thr Gly Thr Lys Gly Arg Thr Thr Thr Thr Thr Met Gly Arg Val Val
            420                 425                 430

Ser Gly Ser Arg Lys His Ala Ser Glu His His Asp Ala Asp Gly Lys
        435                 440                 445

Asp Gly Arg Ser Val Pro Ser Ala His Thr His Ala Ile Ser Lys Gly
    450                 455                 460

Ala Ala Gln Glu Arg Ser Lys Glu Ile Glu Ala Leu Thr Trp Leu Leu
465                 470                 475                 480

Glu Leu Phe Ser Lys Leu Ala Ser Gly Phe Phe Ala Leu Cys Arg Tyr
                485                 490                 495

Arg Cys Pro Glu Ser Ile Gln Ile Phe Asn Ser Leu Ser Gln Gly Gln
            500                 505                 510

Arg Glu Thr Pro Trp Val Leu Ala Gln Ile Gly Arg Ala Tyr Tyr Glu
        515                 520                 525

Gln Ala Met Tyr Ser Glu Ala Glu Lys Tyr Phe Tyr Arg Val Lys Thr
    530                 535                 540

Met Ala Pro Ser Arg Leu Glu Asp Met Glu Ile Tyr Ser Thr Val Leu
545                 550                 555                 560

Trp His Leu Lys Asn Asp Val Glu Leu Ala Tyr Leu Ala His Glu Leu
                565                 570                 575

Met Glu Thr Asp Arg Leu Ser Pro Gln Ala Trp Cys Ala Ile Gly Asn
            580                 585                 590

Ser Phe Ser His Gln Arg Asp His Asp Gln Ala Leu Lys Cys Phe Lys
        595                 600                 605

Arg Ala Thr Gln Leu Asp Pro Gln Phe Ala Tyr Gly Phe Thr Leu Gln
    610                 615                 620

Gly His Glu Tyr Val Ala Asn Glu Glu Tyr Asp Lys Ala Leu Asp Ala
625                 630                 635                 640

Tyr Arg His Gly Ile Ser Ala Asp Ser Arg His Tyr Asn Ala Trp Tyr
                645                 650                 655

Gly Leu Gly Thr Val Tyr Asp Lys Met Gly Lys Leu Asp Phe Ala Glu
            660                 665                 670

Gln His Phe Arg Asn Ala Ala Ser Ile Asn Pro Thr Asn Ala Val Leu

675                 680                 685

Ile Cys Cys Ile Gly Leu Val Leu Glu Lys Met Asn Asn Pro Lys Ala
    690                 695                 700

Ala Leu Val Gln Tyr Gly Arg Ala Cys Ser Leu Ala Pro His Ser Val
705                 710                 715                 720

Leu Ala Arg Phe Arg Lys Ala Arg Ala Leu Met Lys Leu Gln Glu Leu
                725                 730                 735

Lys Leu Ala Leu Ser Glu Leu Lys Ile Leu Lys Asp Met Ala Pro Asp
            740                 745                 750

Glu Ala Asn Val His Tyr Leu Leu Gly Lys Leu Tyr Lys Met Leu His
        755                 760                 765

Asp Lys Ala Asn Ala Ile Lys His Phe Thr Thr Ala Leu Asn Leu Asp
    770                 775                 780

Pro Lys Ala Ala Gln Tyr Ile Lys Asp Ala Met Glu Ser Leu Asp Asp
785                 790                 795                 800

Asp Glu Glu Asp Asp Glu Asp Met Ser
                805

<210> 139
<211> 2684
<212> DNA
<213> Aspergillus fumigatus

<220>
<223> Phylum CEA259; clone 5-4-21; contig 4963 region 373462-376145
      Genomic sequence containing 3' and 5'-ends and the coding region

<400> 139
   acctgttcga cgctcacact caaatgcgca ccagtcgccg cagccaaccg gagcacatac      60
   acattccacc ggctcaatgg ggtcgaatgg tccacaagga atgtccgaaa attgggaagg     120
   aagagatcaa agacaagttc caatgtccac gcgtgaggta gcaacgcccg ggaacagacc     180
   tggccttttt gcgaccctgc acgatcgagg gaaggtggct aagcaacccg atttctatga     240
   tccttacttt ccgctcaaat atctggaggt accgaaatgt acgtgagccc tgccgtcaaa     300
   ttactaccgc ctgcttactt gtcatggcct ataactgacc ctgccctgca gccgatcata     360
   tttataaaag agcccactac ggtttacaat cgggaattcc cgatgaggtg gactttgcat     420
   tgtatcacct tgttcagatt tcgaatcaac gatgggataa attcaagttt gagggtttcc     480
   ccttgcttgc ggagaacctc atggcaaagg ccctggatat ctcccttgtc acaaccgggg     540
   tgaagtggga gcttcagtat gatgttcttc aactcagtga tcgcgtcaat gagctgaact     600
   cgctacatgg cacacgagat ctgttggaga agatcaaaca aatgccagtt acattgccgg     660
   aagacaccct cgagacgtac gaattcaacc accttctgcg caacgttaaa gaagcgaccc     720
   tggtactacg caatatggtc cttctgaaag agaatgccta ctatgtgtca cggtacgcga     780
   aaggcctgct ccgagacttc ctcgtcatta tgatcaactt gcccaatcag cctcgtctca     840
   acgagatcaa gaacgacgct ttggacattg cagaggaggt caccaagttt atgaagaccg     900
   atccggaaga tccactttgg atctcacttc tcaattgtct cgggtcgtca gatcgtgctc     960
   acgtggtccg cgcactctgg gctctcaccc atttctccac tgaattagac gagccagagg    1020
   cgaaccgggc aatggaacgg ataccaaaag agactttgca gcagctctac tttcacactc    1080
   ttctcgactt ggacaaagat attctcagtg gtgcattgga cttctggtac cagtatacac    1140
   tttcgtccga gaacattgag actttgattg aggtcttcaa cttgcctacc gtcttcgtcc    1200
   cccggatggt cgcactgtta acgcacgaag gccgaccgaa caagaaggaa actgtgttgc    1260
   aagaagaaaa ggtggccccc ccaccgtcgg atatccctcg tgtaccgccc gagctcatga    1320
   aagagctgat ggagctttcg gagcctgaac ggagctcgcg ttggctccgg tgctgctttg    1380
   tggaggacct cgagtgcgag atcacccaaa ttgccttgtg gcaggcgtat cagagcagat    1440
   ttgcagaccc tcgccttcct ggtggcggtg ttctccctgc ggctgaattt atcaaaaatg 1500
   tcagtacgac tttcacgaac gcgcaagcac aggtgatcaa tggccctggt gcagccacga 1560
   aattcatcat caaaggcatt cggcccctgg agaccgccta taccttcgag ggctttccct 1620
   acatttactg caagtgggcg gacaactcga agccaagcaa gacgtgtcag cgtgctttca 1680
   agtcgccggc agaacttcgc catcacgtct tcacggaaca catgaacctc aagcctactg 1740
   aaacgccggg acactataac ctggagaagg cggagtcgcc cgttcatacc tgcctttggg 1800
   acaactgcac gaaattccgg tcgtctggtc cgagtgccaa tactgcaatg gtcgctgggc 1860
   acgtctccgc acatctgccc gaggaacgtg cgccagatgc ggagccgccg acatccaaac 1920
   gtgcggttct ccaagagcgc atcgtccgca aatggtacta cctggacact ccagtcaatg 1980
   agcgaggcga gccgtttgga gtggcctaca aggcggcgtt agtattgcgt aaccttgccc 2040
   gaaacctgcc tacgggtatt gcaccgcagt acaacgggct ttcatggaag aaagccgtct 2100
   tccttagtca tcgtccaaag atcatcgaag catgggaccg caaccgctca ttgcgcaagg 2160
   aacttaccga gttgatcatg gtaatagaaa aagaggatta ttactgaact tggggcgcga 2220
   gagagacgag atgaacagta aatgatcctc ttcaatgacg gctgttcgtt cttctttcaa 2280
   actcacggtc ctcgtcctta ttcttctgca tggccattga tgtacaataa tacccttttt 2340
   cttccacatg atatctttcg ataatctttt cttgcttttt cttctttcat ttctcatgag 2400
   gaactcctca cctgaatgaa aaggtggttg ttttgctcct accgttcgcc cttcaagcta 2460
   ggtgagggtt cactcgcaat ctaaaacaca cggcgtctgg gtttgggggg gtaggaatct 2520
   gctgggtgat gcacggtttc ctttaacgat gtactatagg tagatcgcag ccaggagaaa 2580
   agaaaagata ctaatacgct aggtgctcaa tgattccagg tcacttggaa aattgatcgg 2640
   agcgtgagag ctcctatcaa ttttatccct taagccgctt caaa 2684

<210> 140
<211> 1707
<212> DNA
<213> Aspergillus fumigatus

<220>
<223> Phylum CEA259; clone 5-4-21; contig 4963 region 373462-376145
      Genomic sequence containing the coding region

<400> 140
atggcaaagg ccctggatat ctcccttgtc acaaccgggg tgaagtggga gcttcagtat 60
gatgttcttc aactcagtga tcgcgtcaat gagctgaact cgctacatgg cacacgagat 120
ctgttggaga agatcaaaca aatgccagtt acattgccgg aagacaccct cgagacgtac 180
gaattcaacc accttctgcg caacgttaaa gaagcgaccc tggtactacg caatatggtc 240
cttctgaaag agaatgccta ctatgtgtca cggtacgcga aaggcctgct ccgagacttc 300
ctcgtcatta tgatcaactt gcccaatcag cctcgtctca acgagatcaa gaacgacgct 360
ttggacattg cagaggaggt caccaagttt atgaagaccg atccggaaga tccactttgg 420
atctcacttc tcaattgtct cgggtcgtca gatcgtgctc acgtggtccg cgcactctgg 480
gctctcaccc atttctccac tgaattagac gagccagagg cgaaccgggc aatggaacgg 540
ataccaaaag agactttgca gcagctctac tttcacactc ttctcgactt ggacaaagat 600
attctcagtg gtgcattgga cttctggtac cagtatacac tttcgtccga gaacattgag 660
actttgattg aggtcttcaa cttgcctacc gtcttcgtcc cccggatggt cgcactgtta 720
acgcacgaag gccgaccgaa caagaaggaa actgtgttgc aagaagaaaa ggtggccccc 780
ccaccgtcgg atatccctcg tgtaccgccc gagctcatga aagagctgat ggagctttcg 840
gagcctgaac ggagctcgcg ttggctccgg tgctgctttg tggaggacct cgagtgcgag 900
atcacccaaa ttgccttgtg gcaggcgtat cagagcagat ttgcagaccc tcgccttcct 960
ggtggcggtg ttctccctgc ggctgaattt atcaaaaatg tcagtacgac tttcacgaac 1020
gcgcaagcac aggtgatcaa tggccctggt gcagccacga aattcatcat caaaggcatt 1080
cggcccctgg agaccgccta taccttcgag ggctttccct acatttactg caagtgggcg 1140
gacaactcga agccaagcaa gacgtgtcag cgtgctttca agtcgccggc agaacttcgc 1200
catcacgtct tcacggaaca catgaacctc aagcctactg aaacgccggg acactataac 1260
ctggagaagg cggagtcgcc cgttcatacc tgcctttggg acaactgcac gaaattccgg 1320
tcgtctggtc cgagtgccaa tactgcaatg gtcgctgggc acgtctccgc acatctgccc 1380
gaggaacgtg cgccagatgc ggagccgccg acatccaaac gtgcggttct ccaagagcgc 1440
atcgtccgca aatggtacta cctggacact ccagtcaatg agcgaggcga gccgtttgga 1500
gtggcctaca aggcggcgtt agtattgcgt aaccttgccc gaaacctgcc tacgggtatt 1560
gcaccgcagt acaacgggct ttcatggaag aaagccgtct tccttagtca tcgtccaaag 1620
atcatcgaag catgggaccg caaccgctca ttgcgcaagg aacttaccga gttgatcatg 1680

gtaatagaaa aagaggatta ttactga                                          1707

<210> 141
<211> 1707
<212> DNA
<213> Aspergillus fumigatus

<220>
<223> Phylum CEA259; clone 5-4-21; contig 4963 region 373462-376145
      Coding region without introns

<400> 141
atggcaaagg ccctggatat ctcccttgtc acaaccgggg tgaagtggga gcttcagtat      60
gatgttcttc aactcagtga tcgcgtcaat gagctgaact cgctacatgg cacacgagat     120
ctgttggaga agatcaaaca aatgccagtt acattgccgg aagacaccct cgagacgtac     180
gaattcaacc accttctgcg caacgttaaa gaagcgaccc tggtactacg caatatggtc     240
cttctgaaag agaatgccta ctatgtgtca cggtacgcga aaggcctgct ccgagacttc     300
ctcgtcatta tgatcaactt gcccaatcag cctcgtctca acgagatcaa gaacgacgct     360
ttggacattg cagaggaggt caccaagttt atgaagaccg atccggaaga tccactttgg     420
atctcacttc tcaattgtct cgggtcgtca gatcgtgctc acgtggtccg cgcactctgg     480
gctctcaccc atttctccac tgaattagac gagccagagg cgaaccgggc aatggaacgg     540
ataccaaaag agactttgca gcagctctac tttcacactc ttctcgactt ggacaaagat     600
attctcagtg gtgcattgga cttctggtac cagtatacac tttcgtccga gaacattgag     660
actttgattg aggtcttcaa cttgcctacc gtcttcgtcc cccggatggt cgcactgtta     720
acgcacgaag gccgaccgaa caagaaggaa actgtgttgc aagaagaaaa ggtggccccc     780
ccaccgtcgg atatccctcg tgtaccgccc gagctcatga aagagctgat ggagctttcg     840
gagcctgaac ggagctcgcg ttggctccgg tgctgctttg tggaggacct cgagtgcgag     900
atcacccaaa ttgccttgtg gcaggcgtat cagagcagat ttgcagaccc tcgccttcct     960
ggtggcggtg ttctccctgc ggctgaattt atcaaaaatg tcagtacgac tttcacgaac    1020
gcgcaagcac aggtgatcaa tggccctggt gcagccacga aattcatcat caaaggcatt    1080
cggcccctgg agaccgccta taccttcgag ggctttccct acatttactg caagtgggcg    1140
gacaactcga agccaagcaa gacgtgtcag cgtgctttca agtcgccggc agaacttcgc    1200
catcacgtct tcacggaaca catgaacctc aagcctactg aaacgccggg acactataac    1260
ctggagaagg cggagtcgcc cgttcatacc tgcctttggg acaactgcac gaaattccgg    1320
tcgtctggtc cgagtgccaa tactgcaatg gtcgctgggc acgtctccgc acatctgccc    1380
gaggaacgtg cgccagatgc ggagccgccg acatccaaac gtgcggttct ccaagagcgc    1440
atcgtccgca aatggtacta cctggacact ccagtcaatg agcgaggcga gccgtttgga    1500
gtggcctaca aggcggcgtt agtattgcgt aaccttgccc gaaacctgcc tacgggtatt    1560
gcaccgcagt acaacgggct ttcatggaag aaagccgtct tccttagtca tcgtccaaag    1620
atcatcgaag catgggaccg caaccgctca ttgcgcaagg aacttaccga gttgatcatg    1680
gtaatagaaa aagaggatta ttactga                                        1707

<210> 142
<211> 568
<212> PRT
<213> Aspergillus fumigatus

<220>
<223> Phylum CEA259; clone 5-4-21; contig 4963 region 373462-376145
      Protein sequence

<400> 142

Met Ala Lys Ala Leu Asp Ile Ser Leu Val Thr Thr Gly Val Lys Trp
1               5                   10                  15

Glu Leu Gln Tyr Asp Val Leu Gln Leu Ser Asp Arg Val Asn Glu Leu
            20                  25                  30

Asn Ser Leu His Gly Thr Arg Asp Leu Leu Glu Lys Ile Lys Gln Met
        35                  40                  45

Pro Val Thr Leu Pro Glu Asp Thr Leu Glu Thr Tyr Glu Phe Asn His
    50                  55                  60

Leu Leu Arg Asn Val Lys Glu Ala Thr Leu Val Leu Arg Asn Met Val
65                  70                  75                  80

Leu Leu Lys Glu Asn Ala Tyr Tyr Val Ser Arg Tyr Ala Lys Gly Leu
                85                  90                  95

Leu Arg Asp Phe Leu Val Ile Met Ile Asn Leu Pro Asn Gln Pro Arg
            100                 105                 110

Leu Asn Glu Ile Lys Asn Asp Ala Leu Asp Ile Ala Glu Glu Val Thr
        115                 120                 125

Lys Phe Met Lys Thr Asp Pro Glu Asp Pro Leu Trp Ile Ser Leu Leu
    130                 135                 140

Asn Cys Leu Gly Ser Ser Asp Arg Ala His Val Val Arg Ala Leu Trp
145                 150                 155                 160

Ala Leu Thr His Phe Ser Thr Glu Leu Asp Glu Pro Glu Ala Asn Arg
                165                 170                 175

Ala Met Glu Arg Ile Pro Lys Glu Thr Leu Gln Gln Leu Tyr Phe His
            180                 185                 190

Thr Leu Leu Asp Leu Asp Lys Asp Ile Leu Ser Gly Ala Leu Asp Phe
        195                 200                 205

Trp Tyr Gln Tyr Thr Leu Ser Ser Glu Asn Ile Glu Thr Leu Ile Glu
    210                 215                 220

Val Phe Asn Leu Pro Thr Val Phe Val Pro Arg Met Val Ala Leu Leu
225                 230                 235                 240

Thr His Glu Gly Arg Pro Asn Lys Lys Glu Thr Val Leu Gln Glu Glu

                245                 250                 255

Lys Val Ala Pro Pro Pro Ser Asp Ile Pro Arg Val Pro Pro Glu Leu
            260                 265                 270

Met Lys Glu Leu Met Glu Leu Ser Glu Pro Glu Arg Ser Ser Arg Trp
        275                 280                 285

Leu Arg Cys Cys Phe Val Glu Asp Leu Glu Cys Glu Ile Thr Gln Ile
    290                 295                 300

Ala Leu Trp Gln Ala Tyr Gln Ser Arg Phe Ala Asp Pro Arg Leu Pro
305                 310                 315                 320

Gly Gly Gly Val Leu Pro Ala Ala Glu Phe Ile Lys Asn Val Ser Thr
                325                 330                 335

Thr Phe Thr Asn Ala Gln Ala Gln Val Ile Asn Gly Pro Gly Ala Ala
            340                 345                 350

Thr Lys Phe Ile Ile Lys Gly Ile Arg Pro Leu Glu Thr Ala Tyr Thr
        355                 360                 365

Phe Glu Gly Phe Pro Tyr Ile Tyr Cys Lys Trp Ala Asp Asn Ser Lys
    370                 375                 380

Pro Ser Lys Thr Cys Gln Arg Ala Phe Lys Ser Pro Ala Glu Leu Arg
385                 390                 395                 400

His His Val Phe Thr Glu His Met Asn Leu Lys Pro Thr Glu Thr Pro
                405                 410                 415

Gly His Tyr Asn Leu Glu Lys Ala Glu Ser Pro Val His Thr Cys Leu
            420                 425                 430

Trp Asp Asn Cys Thr Lys Phe Arg Ser Ser Gly Pro Ser Ala Asn Thr
        435                 440                 445

Ala Met Val Ala Gly His Val Ser Ala His Leu Pro Glu Glu Arg Ala
    450                 455                 460

Pro Asp Ala Glu Pro Pro Thr Ser Lys Arg Ala Val Leu Gln Glu Arg
465                 470                 475                 480

Ile Val Arg Lys Trp Tyr Tyr Leu Asp Thr Pro Val Asn Glu Arg Gly
                485                 490                 495

Glu Pro Phe Gly Val Ala Tyr Lys Ala Ala Leu Val Leu Arg Asn Leu
            500                 505                 510

Ala Arg Asn Leu Pro Thr Gly Ile Ala Pro Gln Tyr Asn Gly Leu Ser
        515                 520                 525

Trp Lys Lys Ala Val Phe Leu Ser His Arg Pro Lys Ile Ile Glu Ala
    530                 535                 540

Trp Asp Arg Asn Arg Ser Leu Arg Lys Glu Leu Thr Glu Leu Ile Met
545                 550                 555                 560

Val Ile Glu Lys Glu Asp Tyr Tyr
                565


<210> 143
<211> 2542
<212> DNA
<213> Aspergillus fumigatus

<220>
<223> Phylum CEA260; clone 2-10-21; contig 4849 region 12560-15101
      Genomic sequence containing 3' and 5'-ends and the coding region

<400> 143
aggcacacag tgcactgtac tgactcagtt gttcctccag gcctcgataa cacgacttac      60
gtctagtgtc atctccttcc acgtttccaa ttatcttctc ttttccttcc aaaaaaatta     120
aaaagcagat actctcgtct gctagacccg atactttgac cggtatcggt atcttccagt     180
acgagggtgc tcggcatata gtcacaattc ctttttcaag aggctttttc ctcctctccc     240
tatcctttcg ccctccctag catccctttc gagcttgcct taatttcgtc catctggcct     300
gtgtgccatt ctcttcattg cgatcaaggg ccttctctct caggcggaca cagcccccct     360
gttcgtctgg gcagcagata gtgcttccta ggcttctgtg tctacggtca actacatata     420
caccactgcg ttgctcctca tctcaataat cggtcttgca ccagcatacg tcacacaatt     480

cattacgtca atcagcggaa atggtctaca tcggcatccc caagaactac acggcttcgc 540
cgtcttcctt tgccggaact ccgtccttga cgatcaatta cgaggcaacg caggatcttg 600
attctaccaa tgcttttgaa ggtttgttga cgccggtgac acgtgtgaga gcagcgctgg 660
acagaggctg acagtctaca ggtccagaga aactcttgga ggtgtggttc gcgccttccg 720
ctcaggaatt aggtccagcg cagcccgccg gtctgaaggc tgttccggag gagatctgga 780
aggacatgtt ggatctcgtc aattgccagg tcctctcgat tgtttcgtca gaggatgtgg 840
acgcctacct gctctccgag tctagcatgt tcgtttggcc tcacaaactc atcttgaaga 900
cttgtggtac caccactctt ctgtctggtc tcccacgcat tctcgagatt gccgctttgt 960
tcggtggctt ccccaagtct accgcccctt ctcgcggaat ctccgtcgcc gctgcgccct 1020
accgcgtctt ctacagccgc aagaacttcc tgttccccga ccgccagcgg ggccctcacc 1080
gcagctggag agatgaagtg cggactatgg ataagctctt cctcaacggc agcgcctaca 1140
tgattggcaa gatgaatggc gagcactggt acttgtacct gactgaacct cataccatgc 1200
tcaccccgcc aacgagcccg ggagccaaga ccgagtttac ggaaacggag accaaggtcc 1260
tcagtgtacc ccagggcgct gctctgcaga ctgattcgga ggatgagact ttggaagtct 1320
tgatgaccga cttggatgag gagaacgcca agcagttcta cctcgagaat gccactgccg 1380
ttgcggagaa ccgttatcgc aactcaaatt cggagaagag tggccatgtt gatgttttca 1440
gcaacacttc ctccgatatc agcgattttg actccgacgg aagccaggtt ctgcctccag 1500
agttgactac cgagggtcac gcgctcggaa ccgtggtctc tgaagcctgt ggactttcct 1560
ctgtgtatcc taaggagaag tatcccgatt cgcgcatcga tgcctacctg tttacaccat 1620
gcggcttctc cgccaacggc gtgattccgc ctcctgaggg aaaagctgga acccactact 1680
tcacagtaca cgtcactcca gagccgcact gttcatatgc gtcctttgag accaacgtac 1740
cgcactcgca gaacggccag actaccgctg gaatcatcaa gcaagtggtc gacatcttca 1800
agcctggtcg cttcagcgtg actctcttcg aggccaagcc agcgctgagc caggtcgaag 1860
acgagtggaa ggaagccaag tacctggccg ctcgtcggac cgccaaaatg gaacatgtgg 1920
agggatatcg ccgagtggac cggattgtcc acgacctcga cggctatgag cttgtcttcc 1980
gctattatga acgcctggac tggaaagggg gggcccctcg gctgggagag gagagatctt 2040
gaagaacagg ccatagcacg ttgagataat cttttgcttg cggaatttgg ttggacattc 2100
ttttgagatg gaaatggttt cattctgcat tttttctacg tctgcccaat attcgctgaa 2160
cagccctgtg ctcacttcat ttgagctcgc agagtatcct tcgacaacat gagcgttcgt 2220
ttcgtgtaca aagctcattg actccctgta ctgtcgttac tgttctgatt ttgcattgag 2280
ctacccgagc gtttgcagtg atcatgtgat tatatgattc gtattcatac gttctttcag 2340
tcgtatgcga atatttttta taacatatat ctaggatatg tatccaagtt caagaaggta 2400
gactcgtagt agaatgtgtt gatccagttg atggccgacg ccaactggta tcgattacgg 2460
attggcagac gtgccagatc agtccggagt ttcttttttg ttgggatcgc atcacagctc 2520
caacacgaca ttcaactttc aa 2542

<210> 144
<211> 1542
<212> DNA
<213> Aspergillus fumigatus

<220>
<223> Phylum CEA260; clone 2-10-21; contig 4849 region 12560-15101
      Genomic sequence containing the coding region

<400> 144
atggtctaca tcggcatccc caagaactac acggcttcgc cgtcttcctt tgccggaact 60
ccgtccttga cgatcaatta cgaggcaacg caggatcttg attctaccaa tgcttttgaa 120
ggtttgttga cgccggtgac acgtgtgaga gcagcgctgg acagaggctg acagtctaca 180
ggtccagaga aactcttgga ggtgtggttc gcgccttccg ctcaggaatt aggtccagcg 240
cagcccgccg gtctgaaggc tgttccggag gagatctgga aggacatgtt ggatctcgtc 300
aattgccagg tcctctcgat tgtttcgtca gaggatgtgg acgcctacct gctctccgag 360
tctagcatgt tcgtttggcc tcacaaactc atcttgaaga cttgtggtac caccactctt 420
ctgtctggtc tcccacgcat tctcgagatt gccgctttgt tcggtggctt ccccaagtct 480
accgcccctt ctcgcggaat ctccgtcgcc gctgcgccct accgcgtctt ctacagccgc 540
aagaacttcc tgttccccga ccgccagcgg ggccctcacc gcagctggag agatgaagtg 600
cggactatgg ataagctctt cctcaacggc agcgcctaca tgattggcaa gatgaatggc 660
gagcactggt acttgtacct gactgaacct cataccatgc tcaccccgcc aacgagcccg 720
ggagccaaga ccgagtttac ggaaacggag accaaggtcc tcagtgtacc ccagggcgct 780
gctctgcaga ctgattcgga ggatgagact ttggaagtct tgatgaccga cttggatgag 840

gagaacgcca agcagttcta cctcgagaat gccactgccg ttgcggagaa ccgttatcgc 900
aactcaaatt cggagaagag tggccatgtt gatgttttca gcaacacttc ctccgatatc 960
agcgattttg actccgacgg aagccaggtt ctgcctccag agttgactac cgagggtcac 1020
gcgctcggaa ccgtggtctc tgaagcctgt ggactttcct ctgtgtatcc taaggagaag 1080
tatcccgatt cgcgcatcga tgcctacctg tttacaccat gcggcttctc cgccaacggc 1140
gtgattccgc ctcctgaggg aaaagctgga acccactact tcacagtaca cgtcactcca 1200
gagccgcact gttcatatgc gtcctttgag accaacgtac cgcactcgca gaacggccag 1260
actaccgctg gaatcatcaa gcaagtggtc gacatcttca agcctggtcg cttcagcgtg 1320
actctcttcg aggccaagcc agcgctgagc caggtcgaag acgagtggaa ggaagccaag 1380
tacctggccg ctcgtcggac cgccaaaatg gaacatgtgg agggatatcg ccgagtggac 1440
cggattgtcc acgacctcga cggctatgag cttgtcttcc gctattatga acgcctggac 1500
tggaaagggg gggcccctcg gctgggagag gagagatctt ga 1542

<210> 145
<211> 1482
<212> DNA
<213> Aspergillus fumigatus

<220>
<223> Phylum CEA260; clone 2-10-21; contig 4849 region 12560-15101
      Coding region without introns

<400> 145
atggtctaca tcggcatccc caagaactac acggcttcgc cgtcttcctt tgccggaact 60
ccgtccttga cgatcaatta cgaggcaacg caggatcttg attctaccaa tgcttttgaa 120
ggtccagaga aactcttgga ggtgtggttc gcgccttccg ctcaggaatt aggtccagcg 180
cagcccgccg gtctgaaggc tgttccggag gagatctgga aggacatgtt ggatctcgtc 240
aattgccagg tcctctcgat tgtttcgtca gaggatgtgg acgcctacct gctctccgag 300
tctagcatgt tcgtttggcc tcacaaactc atcttgaaga cttgtggtac caccactctt 360
ctgtctggtc tcccacgcat tctcgagatt gccgctttgt tcggtggctt ccccaagtct 420
accgcccctt ctcgcggaat ctccgtcgcc gctgcgccct accgcgtctt ctacagccgc 480
aagaacttcc tgttccccga ccgccagcgg ggccctcacc gcagctggag agatgaagtg 540
cggactatgg ataagctctt cctcaacggc agcgcctaca tgattggcaa gatgaatggc 600
gagcactggt acttgtacct gactgaacct cataccatgc tcaccccgcc aacgagcccg 660
ggagccaaga ccgagtttac ggaaacggag accaaggtcc tcagtgtacc ccagggcgct 720
gctctgcaga ctgattcgga ggatgagact ttggaagtct tgatgaccga cttggatgag 780
gagaacgcca agcagttcta cctcgagaat gccactgccg ttgcggagaa ccgttatcgc 840
aactcaaatt cggagaagag tggccatgtt gatgttttca gcaacacttc ctccgatatc 900
agcgattttg actccgacgg aagccaggtt ctgcctccag agttgactac cgagggtcac 960
gcgctcggaa ccgtggtctc tgaagcctgt ggactttcct ctgtgtatcc taaggagaag 1020
tatcccgatt cgcgcatcga tgcctacctg tttacaccat gcggcttctc cgccaacggc 1080
gtgattccgc ctcctgaggg aaaagctgga acccactact tcacagtaca cgtcactcca 1140
gagccgcact gttcatatgc gtcctttgag accaacgtac cgcactcgca gaacggccag 1200
actaccgctg gaatcatcaa gcaagtggtc gacatcttca agcctggtcg cttcagcgtg 1260
actctcttcg aggccaagcc agcgctgagc caggtcgaag acgagtggaa ggaagccaag 1320
tacctggccg ctcgtcggac cgccaaaatg gaacatgtgg agggatatcg ccgagtggac 1380
cggattgtcc acgacctcga cggctatgag cttgtcttcc gctattatga acgcctggac 1440
tggaaagggg gggcccctcg gctgggagag gagagatctt ga 1482

<210> 146
<211> 493
<212> PRT
<213> Aspergillus fumigatus

<220>
<223> Phylum CEA260; clone 2-10-21; contig 4849 region 12560-15101
      Protein sequence

<400> 146

Met Val Tyr Ile Gly Ile Pro Lys Asn Tyr Thr Ala Ser Pro Ser Ser
1               5                   10                  15

Phe Ala Gly Thr Pro Ser Leu Thr Ile Asn Tyr Glu Ala Thr Gln Asp
            20                  25                  30

Leu Asp Ser Thr Asn Ala Phe Glu Gly Pro Glu Lys Leu Leu Glu Val
        35                  40                  45

Trp Phe Ala Pro Ser Ala Gln Glu Leu Gly Pro Ala Gln Pro Ala Gly
    50                  55                  60

Leu Lys Ala Val Pro Glu Glu Ile Trp Lys Asp Met Leu Asp Leu Val
65                  70                  75                  80

Asn Cys Gln Val Leu Ser Ile Val Ser Ser Glu Asp Val Asp Ala Tyr
                85                  90                  95

Leu Leu Ser Glu Ser Ser Met Phe Val Trp Pro His Lys Leu Ile Leu
            100                 105                 110

Lys Thr Cys Gly Thr Thr Thr Leu Leu Ser Gly Leu Pro Arg Ile Leu
        115                 120                 125

Glu Ile Ala Ala Leu Phe Gly Gly Phe Pro Lys Ser Thr Ala Pro Ser
    130                 135                 140

Arg Gly Ile Ser Val Ala Ala Ala Pro Tyr Arg Val Phe Tyr Ser Arg
145                 150                 155                 160

Lys Asn Phe Leu Phe Pro Asp Arg Gln Arg Gly Pro His Arg Ser Trp
                165                 170                 175

Arg Asp Glu Val Arg Thr Met Asp Lys Leu Phe Leu Asn Gly Ser Ala
            180                 185                 190

Tyr Met Ile Gly Lys Met Asn Gly Glu His Trp Tyr Leu Tyr Leu Thr
        195                 200                 205

Glu Pro His Thr Met Leu Thr Pro Pro Thr Ser Pro Gly Ala Lys Thr
    210                 215                 220

Glu Phe Thr Glu Thr Glu Thr Lys Val Leu Ser Val Pro Gln Gly Ala
225                 230                 235                 240

Ala Leu Gln Thr Asp Ser Glu Asp Glu Thr Leu Glu Val Leu Met Thr
                245                 250                 255

Asp Leu Asp Glu Glu Asn Ala Lys Gln Phe Tyr Leu Glu Asn Ala Thr
            260                 265                 270

Ala Val Ala Glu Asn Arg Tyr Arg Asn Ser Asn Ser Glu Lys Ser Gly
        275                 280                 285

His Val Asp Val Phe Ser Asn Thr Ser Ser Asp Ile Ser Asp Phe Asp
    290                 295                 300

Ser Asp Gly Ser Gln Val Leu Pro Pro Glu Leu Thr Thr Glu Gly His
305                 310                 315                 320

Ala Leu Gly Thr Val Val Ser Glu Ala Cys Gly Leu Ser Ser Val Tyr
                325                 330                 335

Pro Lys Glu Lys Tyr Pro Asp Ser Arg Ile Asp Ala Tyr Leu Phe Thr
            340                 345                 350

Pro Cys Gly Phe Ser Ala Asn Gly Val Ile Pro Pro Pro Glu Gly Lys
        355                 360                 365

Ala Gly Thr His Tyr Phe Thr Val His Val Thr Pro Glu Pro His Cys
    370                 375                 380

Ser Tyr Ala Ser Phe Glu Thr Asn Val Pro His Ser Gln Asn Gly Gln
385                 390                 395                 400

Thr Thr Ala Gly Ile Ile Lys Gln Val Val Asp Ile Phe Lys Pro Gly
                405                 410                 415

Arg Phe Ser Val Thr Leu Phe Glu Ala Lys Pro Ala Leu Ser Gln Val
            420                 425                 430

Glu Asp Glu Trp Lys Glu Ala Lys Tyr Leu Ala Ala Arg Arg Thr Ala
        435                 440                 445

Lys Met Glu His Val Glu Gly Tyr Arg Arg Val Asp Arg Ile Val His
    450                 455                 460

Asp Leu Asp Gly Tyr Glu Leu Val Phe Arg Tyr Tyr Glu Arg Leu Asp
465                 470                 475                 480

Trp Lys Gly Gly Ala Pro Arg Leu Gly Glu Glu Arg Ser
                485                 490


<210> 147
<211> 1637
<212> DNA
<213> Aspergillus fumigatus

<220>
<223> Phylum CEA261; clone 7-5-9; contig 4857 region 164191-165827
      Genomic sequence containing 3' and 5'-ends and the coding region

<400> 147
aaagatagag aagacgttgc gcggaacctt ttgaggaccc tcagctctat ttgagtgtct      60
gtcacgaggt ccatatctgg cgatacggag ggctggctgt acaattaggt tactcttatt     120
tcctactgat ctagtgatat aaagtttgga tgcattatgt aaattaaatc tcgggggcaa     180
atgaacattt cgaatatcgt tataaaactc acagaaagtg ctgtcaatgg cacaaattta     240
gcaatcaata ctcatctgag tatgttgttg ataagtccga aaacaaccta aatatttacc     300
tcattaggaa aggtgcactc cgtagttacc tcgactcgcg gttagtctgg tgactaagtt     360
cttggcgttg tgatgagggc aagtcctatc atgtgatcat agttagggtt tccacacacc     420
aggctctcca atatagcaag aaaatagaag gattaggtct cgtctccgaa catccatccc     480
gccagcacac aaccgccaaa atgggtcgcg ttagaaccaa ggtaagttac agatgaagca     540
tcatgagtta tcttcaaaaa agccccaaaa gagtatcatt tctgacgaaa tgggtttttc     600
ttcaatagac agtcaagagg tccgccaagg tcatcatcga gcgctactac cccaagttga     660
cgctcgactt tgagaccaac aagcgtcttt gcgatgagat cgctatcatt gcctccaagc     720
gccttcgcaa caaggtgggc aatccatcac tgagccgtac aacagtcgga atttgacttg     780
ctgacgaaaa ctagattgct ggttacacca cccaccttat gaagcgtatc cagcgtggcc     840
ctgtccgcgg tatctctttc aagctgcagg aggaggagcg tgagcgcaag gatcagtacg     900
ttcctgaggt ttccgctctg gatgtttccc agaccgagtc cggccagctc gatgtcgatg     960
ccgacaccaa ggaccttctc aagtccatgg gcgtaagttc tgttctcaac gcggttgttc    1020

gtggttttaa agcagtccgt taacttatat tgcccactac agttcgacaa tctcaaggtc   1080
aacgttgtca acgtctccca acatcaggtt caggagcgcc cccgccgctt ccggtagatg   1140
cgcgcacccc tcgagcctcg aaaaaaaagt taccgattgt cttcggtcga tctatggcgt   1200
gctcaatcac acttgctctg gctgacttcg cagctatgat gtagcctaga gacacaggaa   1260
tgaacataat tctctctgag aaaggtgtcg ctgattctcc tggtggagat gacgcttgat   1320
tgccaaaatt tctccttttg cttactgtcc gtttcagctc gggcgctgcg tagaaggttc   1380
tctctgcatg atgcgcagga tgtcatcaga gagtcgaaac ctttggtgcg aactgcacca   1440
tcaactgcac cgcattggat cagatccata ttaatcagtc tatctacaga agtaaattgg   1500
gtatcgtcat aagcacaaag acgccgtaga accacaaatc gaaccacccc atcgaattct   1560
gtcgtgacca ggctcacgcc aaacccgctg agattgaagc atatcatgat cagatttcct   1620
ttggcacgta gccttca                                                  1637

<210> 148
<211> 637
<212> DNA
<213> Aspergillus fumigatus

<220>
<223> Phylum CEA261; clone 7-5-9; contig 4857 region 164191-165827
      Genomic sequence containing the coding region

<400> 148
atgggtcgcg ttagaaccaa ggtaagttac agatgaagca tcatgagtta tcttcaaaaa     60
agccccaaaa gagtatcatt tctgacgaaa tgggtttttc ttcaatagac agtcaagagg    120
tccgccaagg tcatcatcga gcgctactac cccaagttga cgctcgactt tgagaccaac    180
aagcgtcttt gcgatgagat cgctatcatt gcctccaagc gccttcgcaa caaggtgggc    240
aatccatcac tgagccgtac aacagtcgga atttgacttg ctgacgaaaa ctagattgct    300
ggttacacca cccaccttat gaagcgtatc cagcgtggcc ctgtccgcgg tatctctttc    360
aagctgcagg aggaggagcg tgagcgcaag gatcagtacg ttcctgaggt ttccgctctg    420
gatgtttccc agaccgagtc cggccagctc gatgtcgatg ccgacaccaa ggaccttctc    480
aagtccatgg gcgtaagttc tgttctcaac gcggttgttc gtggttttaa agcagtccgt    540
taacttatat tgcccactac agttcgacaa tctcaaggtc aacgttgtca acgtctccca    600
acatcaggtt caggagcgcc cccgccgctt ccggtag                             637

<210> 149
<211> 420
<212> DNA
<213> Aspergillus fumigatus

<220>
<223> Phylum CEA261; clone 7-5-9; contig 4857 region 164191-165827
      Coding region without introns

<400> 149
atgggtcgcg ttagaaccaa gacagtcaag aggtccgcca aggtcatcat cgagcgctac     60
taccccaagt tgacgctcga ctttgagacc aacaagcgtc tttgcgatga gatcgctatc    120
attgcctcca agcgccttcg caacaagatt gctggttaca ccacccacct tatgaagcgt    180
atccagcgtg gccctgtccg cggtatctct ttcaagctgc aggaggagga gcgtgagcgc    240
aaggatcagt acgttcctga ggtttccgct ctggatgttt cccagaccga gtccggccag    300
ctcgatgtcg atgccgacac caaggacctt ctcaagtcca tgggcttcga caatctcaag    360
gtcaacgttg tcaacgtctc ccaacatcag gttcaggagc gcccccgccg cttccggtag    420

<210> 150
<211> 139
<212> PRT
<213> Aspergillus fumigatus

<220>
<223> Phylum CEA261; clone 7-5-9; contig 4857 region 164191-165827

Protein sequence

<400> 150

Met Gly Arg Val Arg Thr Lys Thr Val Lys Arg Ser Ala Lys Val Ile
1               5                   10                  15

Ile Glu Arg Tyr Tyr Pro Lys Leu Thr Leu Asp Phe Glu Thr Asn Lys
            20                  25                  30

Arg Leu Cys Asp Glu Ile Ala Ile Ile Ala Ser Lys Arg Leu Arg Asn
        35                  40                  45

Lys Ile Ala Gly Tyr Thr Thr His Leu Met Lys Arg Ile Gln Arg Gly
    50                  55                  60

Pro Val Arg Gly Ile Ser Phe Lys Leu Gln Glu Glu Glu Arg Glu Arg
65                  70                  75                  80

Lys Asp Gln Tyr Val Pro Glu Val Ser Ala Leu Asp Val Ser Gln Thr
                85                  90                  95

Glu Ser Gly Gln Leu Asp Val Asp Ala Asp Thr Lys Asp Leu Leu Lys
            100                 105                 110

Ser Met Gly Phe Asp Asn Leu Lys Val Asn Val Val Asn Val Ser Gln
        115                 120                 125

His Gln Val Gln Glu Arg Pro Arg Arg Phe Arg
    130                 135


<210> 151
<211> 2037
<212> DNA
<213> Aspergillus fumigatus

<220>
<223> Phylum CEA262; clone 10-2-18
      Genomic sequence containing 3' and 5'-ends and the coding region

<400> 151
aaggtagtag gtgcagatat tgttgataga catttcaaaa tgtattagtt acatgattac      60
ttacttagat gtaatctttc gataatactt tctagtcttg ttgagttcag aaggccagtg     120
tgtgctgaaa atgacagcga cctatgcggt gcccgtgtag cgaagagcac tggctggaaa     180
taagaagttt attagaggag cctcatgatg cataatcatt gtaagcgcac gatgcacaat     240
aatatatccg aatttctcca gatgacacta agataataac gaaaatatca catgacgttg     300
tgggcaggta tgtattatgt aatctgatcg gtagggccga tgtctcgctt agcggacttt     360
tctgtgggat tgcaatttca acttattatt ccgccgacca gcaacaaagc ggttactcga     420
ctcgactccc tccaccagag cccgtggtgt gatatacctg tctgtctttg atcctcgcaa     480
gatagacttg agtcgcagtt atggcggttg gaaagtatgc caattcactt ctattattgt     540
tctgaacgct tttagcatgt gtctggatac ggtggtttac aggtactgat ccgggaacag     600
gaacaagcgc ttgtcgaagg gcaagaaggg tgttaagaag aggaccgttg atcctttctc     660
caggaaggac gaatactctg ttaaggtatg tcgacgtgga ctgtgtaagt cgaccgcagc     720
taatctatat caggcgcctt ccactttcca gatcagagag tatgttgcac gcatatgatg     780
tcgaattgca ggataaaggc gattcacaat ggtagtggag attatgctga ctgaattata     840
gtgtcgggaa gactctggtc aaccgcacca gtggtctcaa gaacgccaat gactccctga     900
agggtcgaat tttcgaggtc tcgctggctg acctgcagaa tgatgaagac catgctttcc     960
gcaaggttaa gcttcgtgtg gacgaggttc agggcaagaa ctgtttgacc aacttccacg    1020
gtcttgattt cacaaccgac aaattgcgat ccctcgtgcg caagtggcag tcgctgatcg    1080
aagccaatgt cactgtgaag acgaccgatg attatctcct tcggcttttt gctatcgcct    1140

tcaccaagag acgcccgaac cagattaaga agaccacata tgctcgttct tctcaaatcc 1200
gtgccatccg caagaagatg attgaaatca tgcagaggga ggcagccagc tgctctctcg 1260
ctcagctcac tcacaagctc attcctgagg tcattggtcg tgagatcgag aaggctaccc 1320
agggaatcta tcctttgcag aatgtgtgtg accctgttat tcttactcgg gatgaagact 1380
aactgcaatc taggtccata ttcgcaaggt caagcttctt aaggctccca agttcgacct 1440
gggtgcactg ctgaatctgc acggtgaatc tacaaccgat gataagggcc acaaggtcga 1500
gagagagttc aaggagcagg ttctcgaaag cgtttaagtg gactgaatta ccagtatgct 1560
ggttattcgg gacattgatt tgtacctacc tgtatgcttg gattcttttt ttatgagtta 1620
aaatgggaaa agaacttttg tcgcggcatc atgtctttat tgactggtgg tgtcgttaac 1680
ttctatgtcc tttgagaatg gagcttgcaa agaaaacttt gcccttattc aaatatttaa 1740
ttggacaatt ccgatcaaag tttagcagta gaatacctgc tataccagtg atgtgctgat 1800
gcaacggcca cctgcagttt actttcagtt gattcaaatt ctatattaac agagcccttt 1860
taccacacca ctgacctggt attagtatag tgtctcgccc taggagacta aagaattgct 1920
agaagtatgg ttatacataa tgttgaatag ttagtatgat ttattaatat tattttcagt 1980
gcactgatat atatcataat gctactaaat atagctaccc taagatttat atagaga 2037

<210> 152
<211> 1037
<212> DNA
<213> Aspergillus fumigatus

<220>
<223> Phylum CEA262; clone 10-2-18
      Genomic sequence containing the coding region

<400> 152
atggcggttg gaaagtatgc caattcactt ctattattgt tctgaacgct tttagcatgt 60
gtctggatac ggtggtttac aggtactgat ccgggaacag gaacaagcgc ttgtcgaagg 120
gcaagaaggg tgttaagaag aggaccgttg atcctttctc caggaaggac gaatactctg 180
ttaaggtatg tcgacgtgga ctgtgtaagt cgaccgcagc taatctatat caggcgcctt 240
ccactttcca gatcagagag tatgttgcac gcatatgatg tcgaattgca ggataaaggc 300
gattcacaat ggtagtggag attatgctga ctgaattata gtgtcgggaa gactctggtc 360
aaccgcacca gtggtctcaa gaacgccaat gactccctga agggtcgaat tttcgaggtc 420
tcgctggctg acctgcagaa tgatgaagac catgctttcc gcaaggttaa gcttcgtgtg 480
gacgaggttc agggcaagaa ctgtttgacc aacttccacg gtcttgattt cacaaccgac 540
aaattgcgat ccctcgtgcg caagtggcag tcgctgatcg aagccaatgt cactgtgaag 600
acgaccgatg attatctcct tcggcttttt gctatcgcct tcaccaagag acgcccgaac 660
cagattaaga agaccacata tgctcgttct tctcaaatcc gtgccatccg caagaagatg 720
attgaaatca tgcagaggga ggcagccagc tgctctctcg ctcagctcac tcacaagctc 780
attcctgagg tcattggtcg tgagatcgag aaggctaccc agggaatcta tcctttgcag 840
aatgtgtgtg accctgttat tcttactcgg gatgaagact aactgcaatc taggtccata 900
ttcgcaaggt caagcttctt aaggctccca agttcgacct gggtgcactg ctgaatctgc 960
acggtgaatc tacaaccgat gataagggcc acaaggtcga gagagagttc aaggagcagg 1020
ttctcgaaag cgtttaa 1037

<210> 153
<211> 771
<212> DNA
<213> Aspergillus fumigatus

<220>
<223> Phylum CEA262; clone 10-2-18
      Coding region without introns

<400> 153
atggcggttg gaaagaacaa gcgcttgtcg aagggcaaga agggtgttaa gaagaggacc 60
gttgatcctt tctccaggaa ggacgaatac tctgttaagg cgccttccac tttccagatc 120
agagatgtcg ggaagactct ggtcaaccgc accagtggtc tcaagaacgc caatgactcc 180
ctgaagggtc gaattttcga ggtctcgctg gctgacctgc agaatgatga agaccatgct 240

ttccgcaagg ttaagcttcg tgtggacgag gttcagggca agaactgttt gaccaacttc 300
cacggtcttg atttcacaac cgacaaattg cgatccctcg tgcgcaagtg gcagtcgctg 360
atcgaagcca atgtcactgt gaagacgacc gatgattatc tccttcggct ttttgctatc 420
gccttcacca agagacgccc gaaccagatt aagaagacca catatgctcg ttcttctcaa 480
atccgtgcca tccgcaagaa gatgattgaa atcatgcaga gggaggcagc cagctgctct 540
ctcgctcagc tcactcacaa gctcattcct gaggtcattg gtcgtgagat cgagaaggct 600
acccagggaa tctatccttt gcagaatgtc catattcgca aggtcaagct tcttaaggct 660
cccaagttcg acctgggtgc actgctgaat ctgcacggtg aatctacaac cgatgataag 720
ggccacaagg tcgagagaga gttcaaggag caggttctcg aaagcgttta a 771

<210> 154
<211> 256
<212> PRT
<213> Aspergillus fumigatus

<220>
<223> Phylum CEA262; clone 10-2-18
      Protein sequence

<400> 154

Met Ala Val Gly Lys Asn Lys Arg Leu Ser Lys Gly Lys Lys Gly Val
1               5                   10                  15

Lys Lys Arg Thr Val Asp Pro Phe Ser Arg Lys Asp Glu Tyr Ser Val
            20                  25                  30

Lys Ala Pro Ser Thr Phe Gln Ile Arg Asp Val Gly Lys Thr Leu Val
        35                  40                  45

Asn Arg Thr Ser Gly Leu Lys Asn Ala Asn Asp Ser Leu Lys Gly Arg
    50                  55                  60

Ile Phe Glu Val Ser Leu Ala Asp Leu Gln Asn Asp Glu Asp His Ala
65                  70                  75                  80

Phe Arg Lys Val Lys Leu Arg Val Asp Glu Val Gln Gly Lys Asn Cys
                85                  90                  95

Leu Thr Asn Phe His Gly Leu Asp Phe Thr Thr Asp Lys Leu Arg Ser
            100                 105                 110

Leu Val Arg Lys Trp Gln Ser Leu Ile Glu Ala Asn Val Thr Val Lys
        115                 120                 125

Thr Thr Asp Asp Tyr Leu Leu Arg Leu Phe Ala Ile Ala Phe Thr Lys
    130                 135                 140

Arg Arg Pro Asn Gln Ile Lys Lys Thr Thr Tyr Ala Arg Ser Ser Gln
145                 150                 155                 160

Ile Arg Ala Ile Arg Lys Lys Met Ile Glu Ile Met Gln Arg Glu Ala
                165                 170                 175

Ala Ser Cys Ser Leu Ala Gln Leu Thr His Lys Leu Ile Pro Glu Val
            180                 185                 190

Ile Gly Arg Glu Ile Glu Lys Ala Thr Gln Gly Ile Tyr Pro Leu Gln
        195                 200                 205

Asn Val His Ile Arg Lys Val Lys Leu Leu Lys Ala Pro Lys Phe Asp
    210                 215                 220

Leu Gly Ala Leu Leu Asn Leu His Gly Glu Ser Thr Thr Asp Asp Lys
225                 230                 235                 240

Gly His Lys Val Glu Arg Glu Phe Lys Glu Gln Val Leu Glu Ser Val
                245                 250                 255

<210> 155
<211> 1819
<212> DNA
<213> Aspergillus fumigatus

<220>
<223> Phylum CEA263; clone 4-3-3; contig 4944 region 159432-161250
      Genomic sequence containing 3' and 5'-ends and the coding region

<400> 155
aattcatcag cataacgaac ccccaacgac ttcgaaaaaa aagcccgatt cgaaagaatt      60
gcgcattcaa cataccatgg tgggcggctt cgtgtcgtgt cgtacgggta ttgtcgacaa     120
tgaggattga agatgggcca ggtcaatttg ggatgttcgt tgtgggacta gggttttttt     180
ctgtgttggt gcggtcacgc tgcggctggg ctaagcgggc acgtgactgt ggctgactgc     240
ctggtgacgc cccccccggg aggaaccccc aaccggcagc cagataggct cgggaggatc     300
atcgtcgaat gatggcattg ttcttggtct cagtggatgg gttattaatg actgcctgga     360
cggctggatg actccgtcgc tgatttagca ttgtgatcca cgatttatgt ttcatttctg     420
gggcgcgggt ttactaccat cacttttgtc actaccatca cttttatact gagtttctga     480
ccccgacccc gaaccagact atggcaactt cgactgggac cggatgggct cagctccggc     540
agcaagcccg ttcgcttgag actcaggtac ggaactcgaa actacgctat aatgaggctt     600
tactcgtgat ttggatgttg acaataatgt tcctagaccg agagtctgtt tcacacctat     660
gcgcagtatg catcgatgac gaagctgcct ccgaaaccct cagaagaaga acaacggatt     720
gaatcgcaac tgaaggatct tcttgaaaag gtgtgcactt tgaggccctc tagtccagcc     780
caacagacga tcatgctgac acgatccgat catagcgtga agccctcatc tcccagctct     840
cccgtctcct tgactccgaa gccactctta ccgcatctgc cctgaaacag agcaatcttg     900
cccgcaatcg cgaagtcctc caggatcatc gccgcgaatt gcagcgcctg aacgccgcaa     960
tcgccgagtc ccgcgaccga gccaatcttc tgtctaacgt ccgctccgac attgatgcct    1020
accgcaattc aaaccccgcc gcggctgagg cagactacat gctcgaggag cggggtcgta    1080
tagatgaaag ccataacatg atagatggtg tcctaagcca ggcgtatgca atcaacgaga    1140
gttttgggct acaacgtgaa accctggcca gcatcaatcg ccgtatcgtc ggtgctgcca    1200
ataaggtacc aggaatgaat gcattgattg gtaagattgg gacgaagagg agacgtgacg    1260
caatcatctt gggggctttc atcggctttt gtttcttgat ggtgttcttc ttccgatgag    1320
atgctggtgc tccgtatacc gccgatcttc ctgtgttata attccttgct caacgttatc    1380
tacatcggag accgcacggc gttcgggtgt tttcatgtac tccttttctg catgcaagca    1440
ctaatcacaa tggtcatggc gtttcaggtt gtctatttca catttatgta catacagggt    1500
cagactgctg tagccctagg gctcaccgca tgatcactct tggtttcgga cttgcggatt    1560
caccttggtt tcttcccgcc cattcctcag ccggtagctt cgactcgaga ctgattcttc    1620
tctcctggat taatttgcga accccgttgt tcaatccgtc tagctcgcct tcctctgccg    1680
gcccgctacc cgcccatcgg atgcgacagt tctcgtccag cagatagaca taaccaactt    1740
tactgttcat cattccgatt gcttctttca acccatccgt aagacctttg cgcacaagga    1800
aataccgttc gtgctgctc                                                 1819

<210> 156
<211> 819
<212> DNA
<213> Aspergillus fumigatus

<220>
<223> Phylum CEA263; clone 4-3-3; contig 4944 region 159432-161250
      Genomic sequence containing the coding region

<400> 156
atggcaactt cgactgggac cggatgggct cagctccggc agcaagcccg ttcgcttgag      60
actcaggtac ggaactcgaa actacgctat aatgaggctt tactcgtgat ttggatgttg     120
acaataatgt tcctagaccg agagtctgtt tcacacctat gcgcagtatg catcgatgac     180
gaagctgcct ccgaaaccct cagaagaaga acaacggatt gaatcgcaac tgaaggatct     240
tcttgaaaag gtgtgcactt tgaggccctc tagtccagcc caacagacga tcatgctgac     300
acgatccgat catagcgtga agccctcatc tcccagctct cccgtctcct tgactccgaa     360
gccactctta ccgcatctgc cctgaaacag agcaatcttg cccgcaatcg cgaagtcctc     420
caggatcatc gccgcgaatt gcagcgcctg aacgccgcaa tcgccgagtc ccgcgaccga     480
gccaatcttc tgtctaacgt ccgctccgac attgatgcct accgcaattc aaaccccgcc     540
gcggctgagg cagactacat gctcgaggag cggggtcgta tagatgaaag ccataacatg     600
atagatggtg tcctaagcca ggcgtatgca atcaacgaga gttttgggct acaacgtgaa     660
accctggcca gcatcaatcg ccgtatcgtc ggtgctgcca ataaggtacc aggaatgaat     720
gcattgattg gtaagattgg gacgaagagg agacgtgacg caatcatctt gggggctttc     780
atcggctttt gtttcttgat ggtgttcttc ttccgatga                            819


<210> 157
<211> 684
<212> DNA
<213> Aspergillus fumigatus

<220>
<223> Phylum CEA263; clone 4-3-3; contig 4944 region 159432-161250
      Coding region without introns

<400> 157
atggcaactt cgactgggac cggatgggct cagctccggc agcaagcccg ttcgcttgag      60
actcagaccg agagtctgtt tcacacctat gcgcagtatg catcgatgac gaagctgcct     120
ccgaaaccct cagaagaaga acaacggatt gaatcgcaac tgaaggatct tcttgaaaag     180
cgtgaagccc tcatctccca gctctcccgt ctccttgact ccgaagccac tcttaccgca     240
tctgccctga aacagagcaa tcttgcccgc aatcgcgaag tcctccagga tcatcgccgc     300
gaattgcagc gcctgaacgc cgcaatcgcc gagtcccgcg accgagccaa tcttctgtct     360
aacgtccgct ccgacattga tgcctaccgc aattcaaacc ccgccgcggc tgaggcagac     420
tacatgctcg aggagcgggg tcgtatagat gaaagccata acatgataga tggtgtccta     480
agccaggcgt atgcaatcaa cgagagtttt gggctacaac gtgaaaccct ggccagcatc     540
aatcgccgta tcgtcggtgc tgccaataag gtaccaggaa tgaatgcatt gattggtaag     600
attgggacga agaggagacg tgacgcaatc atcttggggg ctttcatcgg cttttgtttc     660
ttgatggtgt tcttcttccg atga                                            684


<210> 158
<211> 227
<212> PRT
<213> Aspergillus fumigatus

<220>
<223> Phylum CEA263; clone 4-3-3; contig 4944 region 159432-161250
      Protein sequence

<400> 158

Met Ala Thr Ser Thr Gly Thr Gly Trp Ala Gln Leu Arg Gln Gln Ala
1               5                   10                  15

Arg Ser Leu Glu Thr Gln Thr Glu Ser Leu Phe His Thr Tyr Ala Gln
            20                  25                  30

Tyr Ala Ser Met Thr Lys Leu Pro Pro Lys Pro Ser Glu Glu Glu Gln
        35                  40                  45

Arg Ile Glu Ser Gln Leu Lys Asp Leu Leu Glu Lys Arg Glu Ala Leu
    50                  55                  60

Ile Ser Gln Leu Ser Arg Leu Leu Asp Ser Glu Ala Thr Leu Thr Ala
65                  70                  75                  80

Ser Ala Leu Lys Gln Ser Asn Leu Ala Arg Asn Arg Glu Val Leu Gln
                85                  90                  95

Asp His Arg Arg Glu Leu Gln Arg Leu Asn Ala Ala Ile Ala Glu Ser
            100                 105                 110

Arg Asp Arg Ala Asn Leu Leu Ser Asn Val Arg Ser Asp Ile Asp Ala
        115                 120                 125

Tyr Arg Asn Ser Asn Pro Ala Ala Ala Glu Ala Asp Tyr Met Leu Glu
    130                 135                 140

Glu Arg Gly Arg Ile Asp Glu Ser His Asn Met Ile Asp Gly Val Leu
145                 150                 155                 160

Ser Gln Ala Tyr Ala Ile Asn Glu Ser Phe Gly Leu Gln Arg Glu Thr
                165                 170                 175

Leu Ala Ser Ile Asn Arg Arg Ile Val Gly Ala Ala Asn Lys Val Pro
            180                 185                 190

Gly Met Asn Ala Leu Ile Gly Lys Ile Gly Thr Lys Arg Arg Arg Asp
        195                 200                 205

Ala Ile Ile Leu Gly Ala Phe Ile Gly Phe Cys Phe Leu Met Val Phe
    210                 215                 220

Phe Phe Arg
225


<210> 159
<211> 2601
<212> DNA
<213> Aspergillus fumigatus

<220>
<223> Phylum CEA264; contig 4899 region 65039-62439
      Genomic sequence containing 3' and 5'-ends and the coding region

<400> 159
tataatttct gaactgtaga ctccaaattg caaatcctct cgcctttcaa gtgattccac      60
tcctgcctcc ttttcgtctt ctttttttcc cctttttatc ttttcatttt atcatctttg      120
ttactgtttt cccaaacgaa tccattatta tttctttccc ttggaggttc cctactgtgg      180
tcttgtgtct gcactcttgc caagcccatt ggtccttgct ctgagcctat cacttgcgat      240
tcgcccgccc ataagtccgc ctctctcaac ctttccatct cacgcgcacc tccactcaac      300
atccaccatt cggatattcc gcccatccaa agcgaacacc cctccttctg ctccaccatc      360
gattgcagtc tgcccaaaac ggacttcaga actcccttct acgctatttt ccgccattca      420
ctgttgaagt gcagccctcc atactctcga tagcaactgc ccaacccccc tcttactgcc      480
aaccccacaa gttgcccgtg atgtcacaaa atcgacctgg ggtgttctcg aatctgcgca      540
tgggtggtaa ggaacatcca aatgctgagt ccaattgttc agaaaacatt acccaggagc      600
ctgtggaact aactgctttg ctttccgacc atacagaagt cgtccgcgag aaggtccagg      660
atggactgac aggggaaact aaggagattt cgtactcaca atgtaaaatc gtcggcaatg      720
gatcgtttgg tgtcgtcttt cagacgaaaa tgatgccaag cggcgaggat gctgccatta      780
agagggtcct tcaagacaag cgcttcaaag tatgtgtaca ttataagggc aattgccctc      840

gctgcccaac ccaaagatac tgtcgctgac gagataccag aatcgagaac tgcagattat 900
gcggattgtt cgccatccta acatcgtaga attgaaagcc ttctattact cgaacggcga 960
gagggtatgc gactctcctt tgtctcccca ttcgttctag tttgccgttt gctgactacc 1020
ctaccattgt ctttcacaga aggatgaagt gtacctaaac ctcgttctcg aatacgtacc 1080
agaaaccgtg tatcgggcgt cgcggtactt taataaactc aaaacgacta tgccaatgtt 1140
ggaagtcaag ctgtatatct atcaattgtt ccgttccctg gcatacatcc attcacaagg 1200
catctgccac cgtgacatca agccccagaa tctcttactt gatccatcca ccggcatcct 1260
caaactctgc gactttggtt cggccaagat tctggtagag aatgagccca acgtttccta 1320
tatctgttcc cgctactatc gtgcgccgga attgatcttt ggcgccacta attacacaac 1380
aaagatcggt aagtcttgac tgattcctcc ttcaagtttg gtactgtcat gctgacgatc 1440
gtcaagacgt gtggtccacg ggttgtgtga tggctgaact catgcttggt cagccattgt 1500
tccctggaga gtcgggaatt gaccaactgg tggaaatcat caaggttctt ggaaccccta 1560
ctcgggagca gatccgcacc atgaacccaa actatatgga gcacaaattc cctcaaatca 1620
agccacaccc attcaacaag gtgaccacgc tcttaaagaa cttcttgcga atatgcactg 1680
acttgatgac cccaggtttt ccggagagct cctcacgagg ccattgatct gatctcagct 1740
ttgctagaat acacgccgac acaacgtctc tccgctatcg aggcgatgtg ccacccgttc 1800
ttcgacgaac tcagagatcc caatacgcga ctgcccgact ctcggcaccc tggtggcgct 1860
gctagagacc tccccaatct ctttgatttc tccagacatg gtttgttgtc acttgaggcc 1920
caaattcatt cttccagatg gcttattcgc tgatcactct tttgtagaac tttctattgc 1980
acctgcattg aacagccggc tggttccccc tcatgcacgc gccgctctcg aggcccgggg 2040
gctagacatt gacaacttca ctcctctcac gaaggaggag atgatggcac gtctcgactg 2100
agtcgatacg tttctcgtta cgaatcaaac gccccctcga tagtgcttat atccgcacaa 2160
ctgcgcgagg atatgatgtc atgcgatgca ttttgctttg tgaactggga tgtccttgga 2220
ccagaagtgg cttttatgga acttgtcgac cagagtcgct gactagacac ccgggagttg 2280
gcatcttttc cgaacgtatg aggatacaag gcaaatcccc atatttaaaa gaaaaaggag 2340
aaaacaggag aaaaaaaagg aatgggaaat acagaaacat cgcattctgg tcacaacata 2400
agcggttttc gtgaggcggt ggtgcatcat gacaatatca tgcaacgggg ttgaggaatt 2460
gtcatagtct ggagttttcg aggtgtctga gacagctctt gggaaaggaa aaaaagtgat 2520
accctttagt gtgcgagtct gccttcgggg atttaagttt gcatagcttc attctcgttt 2580
gaaggaggac atgaccattc c 2601

<210> 160
<211> 1601
<212> DNA
<213> Aspergillus fumigatus

<220>
<223> Phylum CEA264; contig 4899 region 65039-62439
      Genomic sequence containing the coding region

<400> 160
atgtcacaaa atcgacctgg ggtgttctcg aatctgcgca tgggtggtaa ggaacatcca 60
aatgctgagt ccaattgttc agaaaacatt acccaggagc ctgtggaact aactgctttg 120
ctttccgacc atacagaagt cgtccgcgag aaggtccagg atggactgac aggggaaact 180
aaggagattt cgtactcaca atgtaaaatc gtcggcaatg gatcgtttgg tgtcgtcttt 240
cagacgaaaa tgatgccaag cggcgaggat gctgccatta agagggtcct tcaagacaag 300
cgcttcaaag tatgtgtaca ttataagggc aattgccctc gctgcccaac ccaaagatac 360
tgtcgctgac gagataccag aatcgagaac tgcagattat gcggattgtt cgccatccta 420
acatcgtaga attgaaagcc ttctattact cgaacggcga gagggtatgc gactctcctt 480
tgtctcccca ttcgttctag tttgccgttt gctgactacc ctaccattgt ctttcacaga 540
aggatgaagt gtacctaaac ctcgttctcg aatacgtacc agaaaccgtg tatcgggcgt 600
cgcggtactt taataaactc aaaacgacta tgccaatgtt ggaagtcaag ctgtatatct 660
atcaattgtt ccgttccctg gcatacatcc attcacaagg catctgccac cgtgacatca 720
agccccagaa tctcttactt gatccatcca ccggcatcct caaactctgc gactttggtt 780
cggccaagat tctggtagag aatgagccca acgtttccta tatctgttcc cgctactatc 840
gtgcgccgga attgatcttt ggcgccacta attacacaac aaagatcggt aagtcttgac 900
tgattcctcc ttcaagtttg gtactgtcat gctgacgatc gtcaagacgt gtggtccacg 960
ggttgtgtga tggctgaact catgcttggt cagccattgt tccctggaga gtcgggaatt 1020
gaccaactgg tggaaatcat caaggttctt ggaaccccta ctcgggagca gatccgcacc 1080
atgaacccaa actatatgga gcacaaattc cctcaaatca agccacaccc attcaacaag 1140

gtgaccacgc tcttaaagaa cttcttgcga atatgcactg acttgatgac cccaggtttt    1200
ccggagagct cctcacgagg ccattgatct gatctcagct ttgctagaat acacgccgac    1260
acaacgtctc tccgctatcg aggcgatgtg ccacccgttc ttcgacgaac tcagagatcc    1320
caatacgcga ctgcccgact ctcggcaccc tggtggcgct gctagagacc tccccaatct    1380
ctttgatttc tccagacatg gtttgttgtc acttgaggcc caaattcatt cttccagatg    1440
gcttattcgc tgatcactct tttgtagaac tttctattgc acctgcattg aacagccggc    1500
tggttccccc tcatgcacgc gccgctctcg aggcccgggg gctagacatt gacaacttca    1560
ctcctctcac gaaggaggag atgatggcac gtctcgactg a                        1601


<210> 161
<211> 1185
<212> DNA
<213> Aspergillus fumigatus

<220>
<223> Phylum CEA264; contig 4899 region 65039-62439
      Coding region without introns

<400> 161
atgtcacaaa atcgacctgg ggtgttctcg aatctgcgca tgggtgaagt cgtccgcgag      60
aaggtccagg atggactgac aggggaaact aaggagattt cgtactcaca atgtaaaatc     120
gtcggcaatg gatcgtttgg tgtcgtcttt cagacgaaaa tgatgccaag cggcgaggat     180
gctgccatta agagggtcct tcaagacaag cgcttcaaaa atcgagaact gcagattatg     240
cggattgttc gccatcctaa catcgtagaa ttgaaagcct tctattactc gaacggcgag     300
aggaaggatg aagtgtacct aaacctcgtt ctcgaatacg taccagaaac cgtgtatcgg     360
gcgtcgcggt actttaataa actcaaaacg actatgccaa tgttggaagt caagctgtat     420
atctatcaat tgttccgttc cctggcatac atccattcac aaggcatctg ccaccgtgac     480
atcaagcccc agaatctctt acttgatcca tccaccggca tcctcaaact ctgcgacttt     540
ggttcggcca agattctggt agagaatgag cccaacgttt cctatatctg ttcccgctac     600
tatcgtgcgc cggaattgat ctttggcgcc actaattaca caacaaagat cgacgtgtgg     660
tccacgggtt gtgtgatggc tgaactcatg cttggtcagc cattgttccc tggagagtcg     720
ggaattgacc aactggtgga aatcatcaag gttcttggaa cccctactcg ggagcagatc     780
cgcaccatga acccaaacta tatggagcac aaattccctc aaatcaagcc acacccattc     840
aacaaggttt tccggagagc tcctcacgag gccattgatc tgatctcagc tttgctagaa     900
tacacgccga cacaacgtct ctccgctatc gaggcgatgt gccacccgtt cttcgacgaa     960
ctcagagatc ccaatacgcg actgcccgac tctcggcacc ctggtggcgc tgctagagac    1020
ctccccaatc tctttgattt ctccagacat gaactttcta ttgcacctgc attgaacagc    1080
cggctggttc cccctcatgc acgcgccgct ctcgaggccc gggggctaga cattgacaac    1140
ttcactcctc tcacgaagga ggagatgatg gcacgtctcg actga                    1185


<210> 162
<211> 394
<212> PRT
<213> Aspergillus fumigatus

<220>
<223> Phylum CEA264; contig 4899 region 65039-62439
      Protein sequence

<400> 162

Met Ser Gln Asn Arg Pro Gly Val Phe Ser Asn Leu Arg Met Gly Glu
1               5                   10                  15

Val Val Arg Glu Lys Val Gln Asp Gly Leu Thr Gly Glu Thr Lys Glu
            20                  25                  30

Ile Ser Tyr Ser Gln Cys Lys Ile Val Gly Asn Gly Ser Phe Gly Val
        35                  40                  45

Val Phe Gln Thr Lys Met Met Pro Ser Gly Glu Asp Ala Ala Ile Lys
    50                  55                  60

Arg Val Leu Gln Asp Lys Arg Phe Lys Asn Arg Glu Leu Gln Ile Met
65                  70                  75                  80

Arg Ile Val Arg His Pro Asn Ile Val Glu Leu Lys Ala Phe Tyr Tyr
                85                  90                  95

Ser Asn Gly Glu Arg Lys Asp Glu Val Tyr Leu Asn Leu Val Leu Glu
            100                 105                 110

Tyr Val Pro Glu Thr Val Tyr Arg Ala Ser Arg Tyr Phe Asn Lys Leu
        115                 120                 125

Lys Thr Thr Met Pro Met Leu Glu Val Lys Leu Tyr Ile Tyr Gln Leu
    130                 135                 140

Phe Arg Ser Leu Ala Tyr Ile His Ser Gln Gly Ile Cys His Arg Asp
145                 150                 155                 160

Ile Lys Pro Gln Asn Leu Leu Leu Asp Pro Ser Thr Gly Ile Leu Lys
                165                 170                 175

Leu Cys Asp Phe Gly Ser Ala Lys Ile Leu Val Glu Asn Glu Pro Asn
            180                 185                 190

Val Ser Tyr Ile Cys Ser Arg Tyr Tyr Arg Ala Pro Glu Leu Ile Phe
        195                 200                 205

Gly Ala Thr Asn Tyr Thr Thr Lys Ile Asp Val Trp Ser Thr Gly Cys
    210                 215                 220

Val Met Ala Glu Leu Met Leu Gly Gln Pro Leu Phe Pro Gly Glu Ser
225                 230                 235                 240

Gly Ile Asp Gln Leu Val Glu Ile Ile Lys Val Leu Gly Thr Pro Thr
                245                 250                 255

Arg Glu Gln Ile Arg Thr Met Asn Pro Asn Tyr Met Glu His Lys Phe
            260                 265                 270

Pro Gln Ile Lys Pro His Pro Phe Asn Lys Val Phe Arg Arg Ala Pro
        275                 280                 285

His Glu Ala Ile Asp Leu Ile Ser Ala Leu Leu Glu Tyr Thr Pro Thr
    290                 295                 300

Gln Arg Leu Ser Ala Ile Glu Ala Met Cys His Pro Phe Phe Asp Glu
305                 310                 315                 320

Leu Arg Asp Pro Asn Thr Arg Leu Pro Asp Ser Arg His Pro Gly Gly
                325                 330                 335

Ala Ala Arg Asp Leu Pro Asn Leu Phe Asp Phe Ser Arg His Glu Leu
            340                 345                 350

Ser Ile Ala Pro Ala Leu Asn Ser Arg Leu Val Pro Pro His Ala Arg
        355                 360                 365

Ala Ala Leu Glu Ala Arg Gly Leu Asp Ile Asp Asn Phe Thr Pro Leu
    370                 375                 380

Thr Lys Glu Glu Met Met Ala Arg Leu Asp
385                 390


<210> 163
<211> 2539
<212> DNA
<213> Aspergillus fumigatus

<220>
<223> Phylum CEA265; clone 11-4-9; contig 4826 region 355652-358190
      Genomic sequence containing 3' and 5'-ends and the coding region

<400> 163
   ttgaggattt gcggaacctt cacgatggca cttgcaccca caatagcaat acccagagcc      60
   ttggagatag caagagacgt gcaggcagca tccttggtga catccagatc gaggaggagg     120
   gcgctgtggc atggcgatcc gatcagggag atgatgacat cgtggacagg ttgtgggaga     180
   gcggacgaga taggacggag aacgggttga agagggtcaa taacgaccct ttgaagcgta     240
   tccatggcga gagtctacag ggcaaaagac ggctcagggc cttggttgaa gaggagtttg     300
   ccaacggagc acggtggttc caaaagatca gcaacaacca tcgggacgga atggtgcatt     360
   cattcctgcg gttgtggtct ctgtgcctcg ccggggaatt tctgccctgt caatcgcgac     420
   tcttccgaga ctcactatct catgatctag atatcgtcct atcgtgattt caatatccct     480
   ccgtccatgt tccttccgcc atgatttatc tccggtcctc gttgctgagg tctggattgg     540
   ctcgagatcc tgctcgcctg tgttcacaat gcttctcacg actctcacca tcacgacgac     600
   ctgtcgcagt tcgcagcttc ttctcctcat ctcggctgcg ggctggcatt gccgatcatg     660
   aatcaactcc ctcgactgtc caaaagacct atttttctgc caatcggacc gcagatggct     720
   tacttgcatc cttatccgcc gtcaatagct cccctcgaag tattgccgac aatgcgttat     780
   cacagggtgc agccagttcg gagtcgatta cttcacagtc tacttcacaa gagttacctc     840
   atcgccggag gaagcggtta aaggaagagg cggccaagaa taatgctgca gaaaccgaac     900
   tccctcctga tgcctcgtct caattgtcca ccctctcatc agccctccct gcgacttccc     960
   tgcgccgcaa gctggctgcg tttctcgccc tcacaaagcc tcgtctctcg ttcctgatcg    1020
   tgttgacgac tacctccgct tatgggatgt acccgatctc ctctcttctc acacttgacc    1080
   cttcaatgac tcccctaccg accctctcga cctcaacctt gacctttctc tacctgacca    1140
   caggaacctt cttgtcttca tgcagcgcca ataccttgaa tatgctcctt gaacctaaat    1200
   acgatgccct catgtcacgg acacggaacc ggccgttagt gcgggggcta ctctcacgcc    1260
   gtgctgcggt attgtttgcg attgcgactg ctgctgcagg tctcggtttg ttatacattg    1320
   gaacgaaccc tacgactact gcgctctccg ccagtaatat ctgtctctat gcctttgtgt    1380
   atacgccgct gaagcgtata tcagtgatca acacctgggt aggcgccgtg gtaggaggca    1440
   ttcctccgtt gatgggttgg accgctgcag caggccagac agcgaccact ggccacgaca    1500
   gctggcggga catgttgttc agcaaggata gcatcggtgg ttggctcctg ggtggcattc    1560
   tctttgcatg gcagtttcct catttcaatg ctttgtccta catgatccgt gaagagtaca    1620
   aggcagccgg gtacaggatg ctcgcatgga ctaatcccgc cgcaaatgcc cgtgtcgcac    1680
   tacgatattc tcttctcatg tttcctttct ccgtcggtct ctggtgggta ggagttgtcg    1740
   gtaatggttt cctggttgga agcacggcgg ccaatggctg gctagtcaaa gaggcctaca    1800
   aattctggcg gcaccaaggc gccaacggca gtgctcgacg cctcttctgg gccagtattt    1860
   ggcagctgcc aatcctcctt gtcggtggtc tggtcacgaa gaaaggtctc tgggatggtg    1920
   tctggaacaa tgttttcggt cagcctgtgg aagacgagga tgactatctc tgggaggatg    1980
   aggatgaagt ggcagaggcg gagcgcaaga tgatacctgc gaagacgagt agctcgtgat    2040
   tcttactttt gtttatcagc gcaattcgac actgatattg ttttgtttac agcactttat    2100
   catactagac tgcctttttg gcatgggagg ctggcgtcgt tgtataacaa attcttttta    2160
   aacttcactc atgatgatcc gcaacacgta gatatagctt caacatgaat tgattcaggt    2220
   agttttctgt atagcacctt aaacgggggg ttcttgctat atcgaacctc taccccaact    2280
   tataagattg agttctgtat cacaatctac ttgcacttaa accaagacta tatcggtgtt    2340
   tctatgagac aactatttac agatcgcccc cgtgagaaga gattgggtat catgtctatc    2400
   ctaatgtaca attcagctga gcggcttgcg gcaactgcat cttcatacac tcattcatcc    2460
   tttaaacgaa gatggtaggt ttctaatgct tctttcgtca tcatgctagg agccttgacc    2520
   atggcatcat cgccaccct                                                2539

<210> 164
<211> 1539
<212> DNA
<213> Aspergillus fumigatus

<220>
<223> Phylum CEA265; clone 11-4-9; contig 4826 region 355652-358190
      Genomic sequence containing the coding region

<400> 164
atgatttatc tccggtcctc gttgctgagg tctggattgg ctcgagatcc tgctcgcctg      60
tgttcacaat gcttctcacg actctcacca tcacgacgac ctgtcgcagt tcgcagcttc     120
ttctcctcat ctcggctgcg ggctggcatt gccgatcatg aatcaactcc ctcgactgtc     180
caaaagacct atttttctgc caatcggacc gcagatggct tacttgcatc cttatccgcc     240
gtcaatagct cccctcgaag tattgccgac aatgcgttat cacagggtgc agccagttcg     300
gagtcgatta cttcacagtc tacttcacaa gagttacctc atcgccggag gaagcggtta     360
aaggaagagg cggccaagaa taatgctgca gaaaccgaac tccctcctga tgcctcgtct     420
caattgtcca ccctctcatc agccctccct gcgacttccc tgcgccgcaa gctggctgcg     480
tttctcgccc tcacaaagcc tcgtctctcg ttcctgatcg tgttgacgac tacctccgct     540
tatgggatgt acccgatctc ctctcttctc acacttgacc cttcaatgac tcccctaccg     600
accctctcga cctcaacctt gacctttctc tacctgacca caggaacctt cttgtcttca     660
tgcagcgcca ataccttgaa tatgctcctt gaacctaaat acgatgccct catgtcacgg     720
acacggaacc ggccgttagt gcgggggcta ctctcacgcc gtgctgcggt attgtttgcg     780
attgcgactg ctgctgcagg tctcggtttg ttatacattg gaacgaaccc tacgactact     840
gcgctctccg ccagtaatat ctgtctctat gcctttgtgt atacgccgct gaagcgtata     900
tcagtgatca acacctgggt aggcgccgtg gtaggaggca ttcctccgtt gatgggttgg     960
accgctgcag caggccagac agcgaccact ggccacgaca gctggcggga catgttgttc    1020
agcaaggata gcatcggtgg ttggctcctg ggtggcattc tctttgcatg gcagtttcct    1080
catttcaatg ctttgtccta catgatccgt gaagagtaca aggcagccgg gtacaggatg    1140
ctcgcatgga ctaatcccgc cgcaaatgcc cgtgtcgcac tacgatattc tcttctcatg    1200
tttcctttct ccgtcggtct ctggtgggta ggagttgtcg gtaatggttt cctggttgga    1260
agcacggcgg ccaatggctg gctagtcaaa gaggcctaca aattctggcg gcaccaaggc    1320
gccaacggca gtgctcgacg cctcttctgg gccagtattt ggcagctgcc aatcctcctt    1380
gtcggtggtc tggtcacgaa gaaaggtctc tgggatggtg tctggaacaa tgttttcggt    1440
cagcctgtgg aagacgagga tgactatctc tgggaggatg aggatgaagt ggcagaggcg    1500
gagcgcaaga tgatacctgc gaagacgagt agctcgtga                           1539


<210> 165
<211> 1539
<212> DNA
<213> Aspergillus fumigatus

<220>
<223> Phylum CEA265; clone 11-4-9; contig 4826 region 355652-358190
      Coding region without introns

<400> 165
atgatttatc tccggtcctc gttgctgagg tctggattgg ctcgagatcc tgctcgcctg      60
tgttcacaat gcttctcacg actctcacca tcacgacgac ctgtcgcagt tcgcagcttc     120
ttctcctcat ctcggctgcg ggctggcatt gccgatcatg aatcaactcc ctcgactgtc     180
caaaagacct atttttctgc caatcggacc gcagatggct tacttgcatc cttatccgcc     240
gtcaatagct cccctcgaag tattgccgac aatgcgttat cacagggtgc agccagttcg     300
gagtcgatta cttcacagtc tacttcacaa gagttacctc atcgccggag gaagcggtta     360
aaggaagagg cggccaagaa taatgctgca gaaaccgaac tccctcctga tgcctcgtct     420
caattgtcca ccctctcatc agccctccct gcgacttccc tgcgccgcaa gctggctgcg     480
tttctcgccc tcacaaagcc tcgtctctcg ttcctgatcg tgttgacgac tacctccgct     540
tatgggatgt acccgatctc ctctcttctc acacttgacc cttcaatgac tcccctaccg     600
accctctcga cctcaacctt gacctttctc tacctgacca caggaacctt cttgtcttca     660
tgcagcgcca ataccttgaa tatgctcctt gaacctaaat acgatgccct catgtcacgg     720

acacggaacc ggccgttagt gcgggggcta ctctcacgcc gtgctgcggt attgtttgcg 780
attgcgactg ctgctgcagg tctcggtttg ttatacattg gaacgaaccc tacgactact 840
gcgctctccg ccagtaatat ctgtctctat gcctttgtgt atacgccgct gaagcgtata 900
tcagtgatca acacctgggt aggcgccgtg gtaggaggca ttcctccgtt gatgggttgg 960
accgctgcag caggccagac agcgaccact ggccacgaca gctggcggga catgttgttc 1020
agcaaggata gcatcggtgg ttggctcctg ggtggcattc tctttgcatg gcagtttcct 1080
catttcaatg ctttgtccta catgatccgt gaagagtaca aggcagccgg gtacaggatg 1140
ctcgcatgga ctaatcccgc cgcaaatgcc cgtgtcgcac tacgatattc tcttctcatg 1200
tttcctttct ccgtcggtct ctggtgggta ggagttgtcg gtaatggttt cctggttgga 1260
agcacggcgg ccaatggctg gctagtcaaa gaggcctaca aattctggcg gcaccaaggc 1320
gccaacggca gtgctcgacg cctcttctgg gccagtattt ggcagctgcc aatcctcctt 1380
gtcggtggtc tggtcacgaa gaaaggtctc tgggatggtg tctggaacaa tgttttcggt 1440
cagcctgtgg aagacgagga tgactatctc tgggaggatg aggatgaagt ggcagaggcg 1500
gagcgcaaga tgatacctgc gaagacgagt agctcgtga 1539

<210> 166
<211> 512
<212> PRT
<213> Aspergillus fumigatus

<220>
<223> Phylum CEA265; clone 11-4-9; contig 4826 region 355652-358190
      Protein sequence

<400> 166

Met Ile Tyr Leu Arg Ser Ser Leu Leu Arg Ser Gly Leu Ala Arg Asp
1               5                   10                  15

Pro Ala Arg Leu Cys Ser Gln Cys Phe Ser Arg Leu Ser Pro Ser Arg
            20                  25                  30

Arg Pro Val Ala Val Arg Ser Phe Phe Ser Ser Ser Arg Leu Arg Ala
        35                  40                  45

Gly Ile Ala Asp His Glu Ser Thr Pro Ser Thr Val Gln Lys Thr Tyr
    50                  55                  60

Phe Ser Ala Asn Arg Thr Ala Asp Gly Leu Leu Ala Ser Leu Ser Ala
65                  70                  75                  80

Val Asn Ser Ser Pro Arg Ser Ile Ala Asp Asn Ala Leu Ser Gln Gly
                85                  90                  95

Ala Ala Ser Ser Glu Ser Ile Thr Ser Gln Ser Thr Ser Gln Glu Leu
            100                 105                 110

Pro His Arg Arg Arg Lys Arg Leu Lys Glu Glu Ala Ala Lys Asn Asn
        115                 120                 125

Ala Ala Glu Thr Glu Leu Pro Pro Asp Ala Ser Ser Gln Leu Ser Thr
    130                 135                 140

Leu Ser Ser Ala Leu Pro Ala Thr Ser Leu Arg Arg Lys Leu Ala Ala
145                 150                 155                 160

Phe Leu Ala Leu Thr Lys Pro Arg Leu Ser Phe Leu Ile Val Leu Thr
                165                 170                 175

Thr Thr Ser Ala Tyr Gly Met Tyr Pro Ile Ser Ser Leu Leu Thr Leu

            180                 185                 190

Asp Pro Ser Met Thr Pro Leu Pro Thr Leu Ser Thr Ser Thr Leu Thr
        195                 200                 205

Phe Leu Tyr Leu Thr Thr Gly Thr Phe Leu Ser Ser Cys Ser Ala Asn
    210                 215                 220

Thr Leu Asn Met Leu Leu Glu Pro Lys Tyr Asp Ala Leu Met Ser Arg
225                 230                 235                 240

Thr Arg Asn Arg Pro Leu Val Arg Gly Leu Leu Ser Arg Arg Ala Ala
                245                 250                 255

Val Leu Phe Ala Ile Ala Thr Ala Ala Ala Gly Leu Gly Leu Leu Tyr
            260                 265                 270

Ile Gly Thr Asn Pro Thr Thr Thr Ala Leu Ser Ala Ser Asn Ile Cys
        275                 280                 285

Leu Tyr Ala Phe Val Tyr Thr Pro Leu Lys Arg Ile Ser Val Ile Asn
    290                 295                 300

Thr Trp Val Gly Ala Val Val Gly Gly Ile Pro Pro Leu Met Gly Trp
305                 310                 315                 320

Thr Ala Ala Ala Gly Gln Thr Ala Thr Thr Gly His Asp Ser Trp Arg
                325                 330                 335

Asp Met Leu Phe Ser Lys Asp Ser Ile Gly Gly Trp Leu Leu Gly Gly
            340                 345                 350

Ile Leu Phe Ala Trp Gln Phe Pro His Phe Asn Ala Leu Ser Tyr Met
        355                 360                 365

Ile Arg Glu Glu Tyr Lys Ala Ala Gly Tyr Arg Met Leu Ala Trp Thr
    370                 375                 380

Asn Pro Ala Ala Asn Ala Arg Val Ala Leu Arg Tyr Ser Leu Leu Met
385                 390                 395                 400

Phe Pro Phe Ser Val Gly Leu Trp Trp Val Gly Val Val Gly Asn Gly
                405                 410                 415

Phe Leu Val Gly Ser Thr Ala Ala Asn Gly Trp Leu Val Lys Glu Ala
            420                 425                 430

Tyr Lys Phe Trp Arg His Gln Gly Ala Asn Gly Ser Ala Arg Arg Leu
        435                 440                 445

Phe Trp Ala Ser Ile Trp Gln Leu Pro Ile Leu Leu Val Gly Gly Leu
    450                 455                 460

Val Thr Lys Lys Gly Leu Trp Asp Gly Val Trp Asn Asn Val Phe Gly
465                 470                 475                 480

Gln Pro Val Glu Asp Glu Asp Asp Tyr Leu Trp Glu Asp Glu Asp Glu
                485                 490                 495

Val Ala Glu Ala Glu Arg Lys Met Ile Pro Ala Lys Thr Ser Ser Ser
            500                 505                 510

<210> 167
<211> 2679
<212> DNA
<213> Aspergillus fumigatus

<220>
<223> Phylum CEA266; clone 2-10-18; contig 4898 region 329309-331987
      Genomic sequence containing 3' and 5'-ends and the coding region

<400> 167
tcacattcac tgcaggtcgt tgtactccgg agttatgcaa tgatcgtccc gtttaattat      60
cttccgaact ttggactcgt atattttagc tgcgtgtcac ggtgatcatg atcttcattc     120
atctctttat ctgattcgat caataccggg agtactgcaa ggaggacaag tagacaggca     180
tctcgagaat tcggtgagaa agcaaggtac agaagagaac tactccgtac tctgtactct     240
gtagagaaag gcaggaggtt caaacatgat tggcccgtgg agaataagaa aatatcatgc     300
cttaggtcca aggctagtgc ctcacatgac cttatcagtt gagtcaggtg atcttatcgt     360
tgtcccagag agatgtgaag aattattgca ccggggagca cgcaaggaaa ccattctatc     420
ctatctcgtc cctttagatt accacaggac atctacatct tgaaccttac cattccaaat     480
tacagactgc ctctgagtac atgctcaacg ccgcggttgc tgccccgcga tgttttgtat     540
atcccactga tcgcgcagca atgcgcttgg gctttgctct tcgtctctcc tctcctgcac     600
ctctcttctc aacagcacct ttccgtcgac agttgcatgc ttccggcgtc cgatcaattg     660
aacctgttat ctttcgaaat agccttgaaa agactcttga ggctcatcga tcctccaatc     720
gagccagtct gatccgcaag gtgattaacc acgattgtcc tgctgaaacg ccccctccaa     780
ttttaccact tgagaatcgt gctggtcatg atcaatcatc tcaaaaggcc tcctccgtgt     840
caaatgcaga gtcagagtcc ccccggtctt ctgcgcctgc gagacgagcg cagaggaagg     900
cccgttcgcc cagccaagta gccaccccgc agccccagac aacagaatat ccacaactgc     960
aatggcatgc agatgaaacc aagggccgac cggcacaaag tccttggctg aagtacttga    1020
ctaccgattg gaaaacgccc gatgccgttt cgcgtctcga cgcggagatc cgcgctcttg    1080
agctctacat gacaccgacc ccgtcggagc ggactgagat agatcggctg gttgcagata    1140
tgggtaggtt gctagcggga atcgtcccca gcccgcccca ggtaaccggt tcatggcgga    1200
cgcgatttgc cttgagccac tcgggtctcg attttgtctt acctgtcccg gattcagacc    1260
gatccacccg tgacgttcgc aagccgagtg ccacacggcc caaggtgctc cagacttaca    1320
aaaagctctt acatgaagtg ggacatgcgc ttcagcagtc cccctcgttc gcggagcgag    1380
tccgcatcat aggcagccgt ttccccgtcc tctcagccat ccatcgcccc acgggccgcc    1440
tgctgcagtt ccactgcggt gaagggctac cggcctctgt cgaatacatc atggattacc    1500
aggccgagta tccctcgatc cggccgctct acgtgaccgc tcgcctgatc ctggaggcgc    1560
ggggtaggta tggccgtact cagatgtcta ttgaatccga tgccctcgta atgcttctcg    1620
tggccttcct caaaatgaac cacgggcgtt ttcagcggcc cgactgtctc ggcgagcagc    1680
tgatcgcgtt tctgcgcgcc tacggcagcg atattgacct gaccaccacc ggtgtgtccg    1740
tcgatccccc cagttggttc aatgctagta cggtcaaacg cgccagcgcc ctgtacgcgc    1800
ccgatgatct acccgcgcat ctgcgcggcc agcgctccct catcagcctc aagagaacag    1860
cagccgccag acgcaatctg cctgccgcca gccggctgtg cgtgcaggac cccaccaatt    1920
acatgaatga tctgggccgc agctgcgtgc gtacgttgga actccagcac acgttctcgc    1980
ttgctcatga ccgtctcggc gcaagtctca agcgctggga tgacagtgaa ccggccgcga    2040
acgttagtat cctgacacgg gccctgcaag caaacttttc tgattttgaa aatctacgcg    2100
ccaaatcgct taagctcaac gcgacctagc aatgaactgg gccagagcct tggagcttgg    2160
gacattgcag cctatcttat ttctcacttc cattactgat agtaaattat atatgagata    2220
atgtggagtc cggcaacgtg tttcgtcacc tgggctaatc cctgtcactg gccactatgt    2280
agatgcagtt gactcaatag agggtcgcta taatcaaaca tcagacaatg cacgagtaga    2340
acagatggaa tatgcttgta agagaacgcc cgcttcctag tcaagcagct ctgaagagca    2400
aaacaatata tccaaacccg ctctattcga acgaaaatgc ggaaataacc atgacagaca    2460
aagaagagac aagccaggcc agggcaaaag tagtctatga catgaattca acgatcaaga    2520
tcacgccggt gtatgagaac tccagggtta gcaataccaa caatcgacat gtatggcacc    2580
gcaaggaaaa gcaccgcgga accatacgag gatatacgat cgatcaattg ctgcccctct    2640
gtcagcagct atttccattg ctgacgggat gctgatggg                           2679


<210> 168
<211> 1629

<212> DNA
<213> Aspergillus fumigatus

<220>
<223> Phylum CEA266; clone 2-10-18; contig 4898 region 329309-331987
      Genomic sequence containing the coding region

<400> 168
atgctcaacg ccgcggttgc tgccccgcga tgttttgtat atcccactga tcgcgcagca      60
atgcgcttgg gctttgctct tcgtctctcc tctcctgcac ctctcttctc aacagcacct     120
ttccgtcgac agttgcatgc ttccggcgtc cgatcaattg aacctgttat ctttcgaaat     180
agccttgaaa agactcttga ggctcatcga tcctccaatc gagccagtct gatccgcaag     240
gtgattaacc acgattgtcc tgctgaaacg ccccctccaa ttttaccact tgagaatcgt     300
gctggtcatg atcaatcatc tcaaaaggcc tcctccgtgt caaatgcaga gtcagagtcc     360
ccccggtctt ctgcgcctgc gagacgagcg cagaggaagg cccgttcgcc cagccaagta     420
gccaccccgc agccccagac aacagaatat ccacaactgc aatggcatgc agatgaaacc     480
aagggccgac cggcacaaag tccttggctg aagtacttga ctaccgattg gaaaacgccc     540
gatgccgttt cgcgtctcga cgcggagatc cgcgctcttg agctctacat gacaccgacc     600
ccgtcggagc ggactgagat agatcggctg gttgcagata tgggtaggtt gctagcggga     660
atcgtcccca gcccgcccca ggtaaccggt tcatggcgga cgcgatttgc cttgagccac     720
tcgggtctcg attttgtctt acctgtcccg gattcagacc gatccacccg tgacgttcgc     780
aagccgagtg ccacacggcc caaggtgctc cagacttaca aaaagctctt acatgaagtg     840
ggacatgcgc ttcagcagtc cccctcgttc gcggagcgag tccgcatcat aggcagccgt     900
ttccccgtcc tctcagccat ccatcgcccc acgggccgcc tgctgcagtt ccactgcggt     960
gaagggctac cggcctctgt cgaatacatc atggattacc aggccgagta tccctcgatc    1020
cggccgctct acgtgaccgc tcgcctgatc ctggaggcgc ggggtaggta tggccgtact    1080
cagatgtcta ttgaatccga tgccctcgta atgcttctcg tggccttcct caaaatgaac    1140
cacgggcgtt ttcagcggcc cgactgtctc ggcgagcagc tgatcgcgtt tctgcgcgcc    1200
tacggcagcg atattgacct gaccaccacc ggtgtgtccg tcgatccccc cagttggttc    1260
aatgctagta cggtcaaacg cgccagcgcc ctgtacgcgc ccgatgatct acccgcgcat    1320
ctgcgcggcc agcgctccct catcagcctc aagagaacag cagccgccag acgcaatctg    1380
cctgccgcca gccggctgtg cgtgcaggac cccaccaatt acatgaatga tctgggccgc    1440
agctgcgtgc gtacgttgga actccagcac acgttctcgc ttgctcatga ccgtctcggc    1500
gcaagtctca agcgctggga tgacagtgaa ccggccgcga acgttagtat cctgacacgg    1560
gccctgcaag caaacttttc tgattttgaa aatctacgcg ccaaatcgct taagctcaac    1620
gcgacctag                                                            1629


<210> 169
<211> 1629
<212> DNA
<213> Aspergillus fumigatus

<220>
<223> Phylum CEA266; clone 2-10-18; contig 4898 region 329309-331987
      Coding region without introns

<400> 169
atgctcaacg ccgcggttgc tgccccgcga tgttttgtat atcccactga tcgcgcagca      60
atgcgcttgg gctttgctct tcgtctctcc tctcctgcac ctctcttctc aacagcacct     120
ttccgtcgac agttgcatgc ttccggcgtc cgatcaattg aacctgttat ctttcgaaat     180
agccttgaaa agactcttga ggctcatcga tcctccaatc gagccagtct gatccgcaag     240
gtgattaacc acgattgtcc tgctgaaacg ccccctccaa ttttaccact tgagaatcgt     300
gctggtcatg atcaatcatc tcaaaaggcc tcctccgtgt caaatgcaga gtcagagtcc     360
ccccggtctt ctgcgcctgc gagacgagcg cagaggaagg cccgttcgcc cagccaagta     420
gccaccccgc agccccagac aacagaatat ccacaactgc aatggcatgc agatgaaacc     480
aagggccgac cggcacaaag tccttggctg aagtacttga ctaccgattg gaaaacgccc     540
gatgccgttt cgcgtctcga cgcggagatc cgcgctcttg agctctacat gacaccgacc     600
ccgtcggagc ggactgagat agatcggctg gttgcagata tgggtaggtt gctagcggga     660
atcgtcccca gcccgcccca ggtaaccggt tcatggcgga cgcgatttgc cttgagccac     720
tcgggtctcg attttgtctt acctgtcccg gattcagacc gatccacccg tgacgttcgc     780

aagccgagtg ccacacggcc caaggtgctc cagacttaca aaaagctctt acatgaagtg     840
ggacatgcgc ttcagcagtc cccctcgttc gcggagcgag tccgcatcat aggcagccgt     900
ttccccgtcc tctcagccat ccatcgcccc acgggccgcc tgctgcagtt ccactgcggt     960
gaagggctac cggcctctgt cgaatacatc atggattacc aggccgagta tccctcgatc    1020
cggccgctct acgtgaccgc tcgcctgatc ctggaggcgc ggggtaggta tggccgtact    1080
cagatgtcta ttgaatccga tgccctcgta atgcttctcg tggccttcct caaaatgaac    1140
cacgggcgtt ttcagcggcc cgactgtctc ggcgagcagc tgatcgcgtt tctgcgcgcc    1200
tacggcagcg atattgacct gaccaccacc ggtgtgtccg tcgatccccc cagttggttc    1260
aatgctagta cggtcaaacg cgccagcgcc ctgtacgcgc ccgatgatct acccgcgcat    1320
ctgcgcggcc agcgctccct catcagcctc aagagaacag cagccgccag acgcaatctg    1380
cctgccgcca gccggctgtg cgtgcaggac cccaccaatt acatgaatga tctgggccgc    1440
agctgcgtgc gtacgttgga actccagcac acgttctcgc ttgctcatga ccgtctcggc    1500
gcaagtctca agcgctggga tgacagtgaa ccggccgcga acgttagtat cctgacacgg    1560
gccctgcaag caaacttttc tgattttgaa aatctacgcg ccaaatcgct taagctcaac    1620
gcgacctag                                                            1629

<210> 170
<211> 542
<212> PRT
<213> Aspergillus fumigatus

<220>
<223> Phylum CEA266; clone 2-10-18; contig 4898 region 329309-331987
      Protein sequence

<400> 170
Met Leu Asn Ala Ala Val Ala Ala Pro Arg Cys Phe Val Tyr Pro Thr
1               5                   10                  15

Asp Arg Ala Ala Met Arg Leu Gly Phe Ala Leu Arg Leu Ser Ser Pro
            20                  25                  30

Ala Pro Leu Phe Ser Thr Ala Pro Phe Arg Arg Gln Leu His Ala Ser
        35                  40                  45

Gly Val Arg Ser Ile Glu Pro Val Ile Phe Arg Asn Ser Leu Glu Lys
    50                  55                  60

Thr Leu Glu Ala His Arg Ser Ser Asn Arg Ala Ser Leu Ile Arg Lys
65                  70                  75                  80

Val Ile Asn His Asp Cys Pro Ala Glu Thr Pro Pro Pro Ile Leu Pro
                85                  90                  95

Leu Glu Asn Arg Ala Gly His Asp Gln Ser Ser Gln Lys Ala Ser Ser
            100                 105                 110

Val Ser Asn Ala Glu Ser Glu Ser Pro Arg Ser Ser Ala Pro Ala Arg
        115                 120                 125

Arg Ala Gln Arg Lys Ala Arg Ser Pro Ser Gln Val Ala Thr Pro Gln
    130                 135                 140

Pro Gln Thr Thr Glu Tyr Pro Gln Leu Gln Trp His Ala Asp Glu Thr
145                 150                 155                 160

Lys Gly Arg Pro Ala Gln Ser Pro Trp Leu Lys Tyr Leu Thr Thr Asp
                165                 170                 175

Trp Lys Thr Pro Asp Ala Val Ser Arg Leu Asp Ala Glu Ile Arg Ala

180                 185                 190

Leu Glu Leu Tyr Met Thr Pro Thr Pro Ser Glu Arg Thr Glu Ile Asp
        195                 200                 205

Arg Leu Val Ala Asp Met Gly Arg Leu Leu Ala Gly Ile Val Pro Ser
    210                 215                 220

Pro Pro Gln Val Thr Gly Ser Trp Arg Thr Arg Phe Ala Leu Ser His
225                 230                 235                 240

Ser Gly Leu Asp Phe Val Leu Pro Val Pro Asp Ser Asp Arg Ser Thr
                245                 250                 255

Arg Asp Val Arg Lys Pro Ser Ala Thr Arg Pro Lys Val Leu Gln Thr
            260                 265                 270

Tyr Lys Lys Leu Leu His Glu Val Gly His Ala Leu Gln Gln Ser Pro
        275                 280                 285

Ser Phe Ala Glu Arg Val Arg Ile Ile Gly Ser Arg Phe Pro Val Leu
    290                 295                 300

Ser Ala Ile His Arg Pro Thr Gly Arg Leu Leu Gln Phe His Cys Gly
305                 310                 315                 320

Glu Gly Leu Pro Ala Ser Val Glu Tyr Ile Met Asp Tyr Gln Ala Glu
                325                 330                 335

Tyr Pro Ser Ile Arg Pro Leu Tyr Val Thr Ala Arg Leu Ile Leu Glu
            340                 345                 350

Ala Arg Gly Arg Tyr Gly Arg Thr Gln Met Ser Ile Glu Ser Asp Ala
        355                 360                 365

Leu Val Met Leu Leu Val Ala Phe Leu Lys Met Asn His Gly Arg Phe
    370                 375                 380

Gln Arg Pro Asp Cys Leu Gly Glu Gln Leu Ile Ala Phe Leu Arg Ala
385                 390                 395                 400

Tyr Gly Ser Asp Ile Asp Leu Thr Thr Thr Gly Val Ser Val Asp Pro
                405                 410                 415

Pro Ser Trp Phe Asn Ala Ser Thr Val Lys Arg Ala Ser Ala Leu Tyr
            420                 425                 430

Ala Pro Asp Asp Leu Pro Ala His Leu Arg Gly Gln Arg Ser Leu Ile
        435                 440                 445

Ser Leu Lys Arg Thr Ala Ala Ala Arg Arg Asn Leu Pro Ala Ala Ser
    450                 455                 460

Arg Leu Cys Val Gln Asp Pro Thr Asn Tyr Met Asn Asp Leu Gly Arg
465                 470                 475                 480

Ser Cys Val Arg Thr Leu Glu Leu Gln His Thr Phe Ser Leu Ala His
                485                 490                 495

Asp Arg Leu Gly Ala Ser Leu Lys Arg Trp Asp Asp Ser Glu Pro Ala
            500                 505                 510

Ala Asn Val Ser Ile Leu Thr Arg Ala Leu Gln Ala Asn Phe Ser Asp
        515                 520                 525

Phe Glu Asn Leu Arg Ala Lys Ser Leu Lys Leu Asn Ala Thr
    530                 535                 540


<210> 171
<211> 1573
<212> DNA
<213> Aspergillus fumigatus

<220>
<223> Phylum CEA280; clone 6-8-13; contig 4925 region 997952-996381
      Genomic sequence containing 3' and 5'-ends and the coding region

<220>
<223> misc_feature
<223> (683)..(683)
<223> n is a, c, g, or t

<400> 171
tggtgcttag ggacctgtga gtttgtgaca caacccaccc cggaaccagg gattctccac      60
cgcgcccccg accgaccatt ttggacttgg ttagtaagct ccagtgacca gaaagcttac     120
cagctagctt cacttgagat atcacaagat gctttggtcg gcctgccacg aggtcttgct     180
ccatgtcaac cacgaggatt cagtctctgc ctcatctcag tccaggcgag gtttctttgc     240
tggatcttgc ggctgatgac cctcgcgatg tggtgtccct gtccgacaag gaagcgttga     300
ttttgcagct ctacaatcaa atccaggaac tggaactgga aaaggcactt cttgaacaag     360
gtacgcgtca aatttatttt ttttgtaatt ttcttttttt tgtttctctg atgtagcttc     420
ttgggccctc caaatctgtc agcccaggtt actgattccc actagagctg gaaccggctt     480
ctggggacaa tctggatgag caacttgcaa tcgcagaacg tgagcttctc gaggcaaggg     540
ccacgtacac ggtcaggaga aaggccacca gtactgtcct gatgactgat ccaacattaa     600
aagctgttca cttgaaagct atatcacctg ttgaaaggtt ttacctcctt tcagtttatg     660
cgagcatttc aattctgcat tcncctatac taacgcttca tagagctctt ctacccctgg     720
tcaaccggcg tgatgtgttg tctttggcac atgagaacct aatgaatgcg cacaacgcga     780
ctttgaggga actatccaat ttagaagtac aaaatctaga gctacaccag aggaatcaag     840
agctagcgcg gcagcttctt gagtccgcga aggatgatga ttcatggaga gaagcactgg     900
atgatgacga cctcaaggca caacttgagc agctagaggc cgatcgcaaa aagagcaaat     960
caagatggga agtcatgaaa agcgttgcaa gtgctattgt tgtgggaagt ggagtgaact    1020
gggctgaaga cgatgagctt acagctctag tcattgatga atctgatgat taaataatcg    1080
cctcgaaatt aatgatttcg aacaatttgg tagtattgac ttctccgacc ggcgtactac    1140
aggatatgac ttccatttat gactagtaga gtaaacctca ttcattattt ccaactaggg    1200
cggtatatac acagtatcgt cttggtcgaa tcagcagaac ggctgaggaa gctcggttag    1260
gtaccgtaag tgtcgcccag actgccgata acctgaagac gcgccagcgc ccactagaac    1320
aatcttcagt ggccgtgaga cccacgtgac atcatcgtcc atccacacta acaagacttg    1380
actgtcagac catatgattt ctgctgggtg tcccaaataa taatcttcat acatacattg    1440
gccctgaagc cctggaatca tggagaaaat tcgaatacag atggaaggca attagaaccc    1500
gataaggtgg gcgctaattg atggatgcgt ctaagaatgt tatgcattaa tcagcgattt    1560
acgggtaacc atg                                                       1573


<210> 172
<211> 573
<212> DNA
<213> Aspergillus fumigatus

<220>
<223> Phylum CEA280; clone 6-8-13; contig 4925 region 997952-996381
      Genomic sequence containing the coding region

<220>
<223> misc_feature
<223> (183)..(183)
<223> n is a, c, g, or t

<400> 172
caacttgcaa tcgcagaacg tgagcttctc gaggcaaggg ccacgtacac ggtcaggaga      60
aaggccacca gtactgtcct gatgactgat ccaacattaa aagctgttca cttgaaagct     120
atatcacctg ttgaaaggtt ttacctcctt tcagtttatg cgagcatttc aattctgcat     180
tcncctatac taacgcttca tagagctctt ctacccctgg tcaaccggcg tgatgtgttg     240
tctttggcac atgagaacct aatgaatgcg cacaacgcga ctttgaggga actatccaat     300
ttagaagtac aaaatctaga gctacaccag aggaatcaag agctagcgcg gcagcttctt     360
gagtccgcga aggatgatga ttcatggaga gaagcactgg atgatgacga cctcaaggca     420
caacttgagc agctagaggc cgatcgcaaa aagagcaaat caagatggga agtcatgaaa     480
agcgttgcaa gtgctattgt tgtgggaagt ggagtgaact gggctgaaga cgatgagctt     540
acagctctag tcattgatga atctgatgat taa                                  573


<210> 173
<211> 573
<212> DNA
<213> Aspergillus fumigatus

<220>
<223> Phylum CEA280; clone 6-8-13; contig 4925 region 997952-996381
      Coding region without introns

<220>
<223> misc_feature
<223> (183)..(183)
<223> n is a, c, g, or t

<400> 173
caacttgcaa tcgcagaacg tgagcttctc gaggcaaggg ccacgtacac ggtcaggaga      60
aaggccacca gtactgtcct gatgactgat ccaacattaa aagctgttca cttgaaagct     120
atatcacctg ttgaaaggtt ttacctcctt tcagtttatg cgagcatttc aattctgcat     180
tcncctatac taacgcttca tagagctctt ctacccctgg tcaaccggcg tgatgtgttg     240
tctttggcac atgagaacct aatgaatgcg cacaacgcga ctttgaggga actatccaat     300
ttagaagtac aaaatctaga gctacaccag aggaatcaag agctagcgcg gcagcttctt     360
gagtccgcga aggatgatga ttcatggaga gaagcactgg atgatgacga cctcaaggca     420
caacttgagc agctagaggc cgatcgcaaa aagagcaaat caagatggga agtcatgaaa     480
agcgttgcaa gtgctattgt tgtgggaagt ggagtgaact gggctgaaga cgatgagctt     540
acagctctag tcattgatga atctgatgat taa                                  573


<210> 174
<211> 190
<212> PRT
<213> Aspergillus fumigatus

<220>
<223> Phylum CEA280; clone 6-8-13; contig 4925 region 997952-996381
      Protein sequence

<400> 174

Gln Leu Ala Ile Ala Glu Arg Glu Leu Leu Glu Ala Arg Ala Thr Tyr
1               5                   10                  15

Thr Val Arg Arg Lys Ala Thr Ser Thr Val Leu Met Thr Asp Pro Thr
            20                  25                  30

Leu Lys Ala Val His Leu Lys Ala Ile Ser Pro Val Glu Arg Phe Tyr
        35                  40                  45

Leu Leu Ser Val Tyr Ala Ser Ile Ser Ile Leu His Ser Pro Ile Leu
    50                  55                  60

Thr Leu His Arg Ala Leu Leu Pro Leu Val Asn Arg Arg Asp Val Leu
65                  70                  75                  80

Ser Leu Ala His Glu Asn Leu Met Asn Ala His Asn Ala Thr Leu Arg
                85                  90                  95

Glu Leu Ser Asn Leu Glu Val Gln Asn Leu Glu Leu His Gln Arg Asn
            100                 105                 110

Gln Glu Leu Ala Arg Gln Leu Leu Glu Ser Ala Lys Asp Asp Asp Ser
        115                 120                 125

Trp Arg Glu Ala Leu Asp Asp Asp Asp Leu Lys Ala Gln Leu Glu Gln
    130                 135                 140

Leu Glu Ala Asp Arg Lys Lys Ser Lys Ser Arg Trp Glu Val Met Lys
145                 150                 155                 160

Ser Val Ala Ser Ala Ile Val Val Gly Ser Gly Val Asn Trp Ala Glu
                165                 170                 175

Asp Asp Glu Leu Thr Ala Leu Val Ile Asp Glu Ser Asp Asp
            180                 185                 190

<210> 175
<211> 2593
<212> DNA
<213> Aspergillus fumigatus

<220>
<223> Phylum CEA281.1; clone 5-3-11; contig 4839 region 10030-12622
      Genomic sequence containing 3' and 5'-ends and the coding region

<400> 175
tgtcaaagaa agtggatata tatgctgctt gttctcacct ggtaatccat cttgaatcac      60
gtccactaag aatcagaaaa actgagcgag tggacaaaaa cgggtgatac gcatcgataa     120
gcttcgactg gaaattcgga gatcgccata tatttgagtt atgtaagcac cgccgcaggc     180
gtctcattgg gctggggaag ctatcaacaa ccacccagag cttcttgaac ttaactccgg     240
gggtgcatga ctaatagttt caataatgga cgtcggatgc tttgtaaatc aacggcggtc     300
ctacaatggg gatctatgca cagttcgtta cataggtaaa gttgagggca ccaccggcga     360
gtggctcgga gtggaatggg atgaccccac gcgggggaag cattctggag aacacaacgg     420
agtgagatat tttacatgta tgaaagtatt ttcaagactg gatagagcgg attgactgac     480
ttgaacggaa ggtagaagga aacaccccac ggctggttcg ttcgtgcgcc cttcgcgacg     540
gaccgacaga cctcgaggct tccttgaggc agtgcgtcac aagtatgctt ctgagttcca     600
agaagaactc gcaagacagc agtcaggcga agtctctgct gcgcgggaaa tcatcaaatt     660
tagtagcaaa gtagtggaag aggtcggctt cgacaagatc cggaagaaac ttgcagagct     720
ccaggaattg aaaatcgtgc tcctggatcg cctatgcatc gcaggagttc tccctcatag     780
agcgagtcta catgagcttg cagaggcttg caaggagata gaacagacat gtcctaagat     840
cgttgacctc gatctgagtt acaacttact ggaaagctgg gttgacattg caaacatatg     900
tcaacagctg aagcgcttga agacattgaa gctgatgttg gtcattcagt acatctgtga     960
gaagcatgct gacagttggc agcggaaatc gtctaggtcc tcgacaggag ggtctgatat    1020
tcgacggtat cacaacacta cacttggacg agactctact cgaatgggac gaggtatgct    1080
gcacaaagct actgcttggt taccagtgag ctgactgact cttggtcccc tttagatttc    1140
agctttgaca tatcaattcc cgtcactctc tgctctgtct gcctccgcaa atcagattac    1200

ccagatcttg acacctatca cggataccat cacgaccttg acactggaaa acaatgacat 1260
ctcttcgcta tcctcattag catgtctgac ctctttgagc aagctcgagc acctctcgct 1320
gagagagaat cgtatcggga aagtctatgc gtctggcatg gaaggaaact ctcttcagtt 1380
ttccgaaaat ctcagatcgg tggacctatc cagaaacaat atcgattctt ggctgtttgt 1440
gaatgaactt caacgcgtat ttcctgggct gcaatctttg cgcatatcag gaaatcccct 1500
gtacgacaag cctgttgccc cctcgaacgt cacaaattta ccggagaagc caatgacggt 1560
ggacgaggcc tatatgctaa cactctctcg actcgcttcc atccaaacgc tcaactacag 1620
caagataact tcccaagacc gaagtaatgg cgaactctat tacctttccc tcattggcaa 1680
ggagttatcc gcgtatccgg aaagcgcaga acgcgagatt cttgctacac atccccgcta 1740
tcaggagctt tgtgagaaat acggagcgcc cacaatcagg agagccgagc tggcaggcgc 1800
tgccgtgaat ccgcgctctg ttgccgcccg agtagtgaag ttggcttttt gcttgcactc 1860
atcagttagt tccggtgcaa accaagaaca atttcgagtt cagaagatcc cgagatcctt 1920
taatacatat caagtcaagg caatcgcctc ccgcctgttc aatttgccgc cttaccagtg 1980
ccgactagtc tgggagacca acgagttaga ccctattcat caggagaaaa aggacgatgg 2040
agacgattgg gatagtgatg aggatgaagc cacagctatt ggattggggg agagtaacaa 2100
gctcacaccg gcgacggagg atggaaagtt catcagaaga gaggttgagc tcctggattc 2160
aacgcgagac ataggctttt ggttccaacc cgatactgtt gaggcaagga tcagagtaga 2220
agttgcaaca tcgaattgac aactatttcg acataggagt tgtgaaggag ttccttaacc 2280
tacattgtcg cgggtggggt aatatatata gttgagcacg actcgtgcgt taggatacaa 2340
cgaggtcaac gacagaaaag tagaacctct tacgcaagcg cctgtggcct ctgttacccg 2400
aatggattta gaggccttct tggttattct taaacaatat gatatgtgta gtgtatattc 2460
aagctggagg gcaacactga ctgctgtttt aggactaggt cggttttggg tggcagatta 2520
tcaccgcaac tgttttgtga taacagtgat atcatttccc tttcatataa caatttaata 2580
ctcagacatc gtc 2593

<210> 176
<211> 1974
<212> DNA
<213> Aspergillus fumigatus

<220>
<223> Phylum CEA281.1; clone 5-3-11; contig 4839 region 10030-12622
      Genomic sequence containing the coding region

<400> 176
atggacgtcg gatgctttgt aaatcaacgg cggtcctaca atggggatct atgcacagtt 60
cgttacatag gtaaagttga gggcaccacc ggcgagtggc tcggagtgga atgggatgac 120
cccacgcggg ggaagcattc tggagaacac aacggagtga gatattttac atgtatgaaa 180
gtattttcaa gactggatag agcggattga ctgacttgaa cggaaggtag aaggaaacac 240
cccacggctg gttcgttcgt gcgcccttcg cgacggaccg acagacctcg aggcttcctt 300
gaggcagtgc gtcacaagta tgcttctgag ttccaagaag aactcgcaag acagcagtca 360
ggcgaagtct ctgctgcgcg ggaaatcatc aaatttagta gcaaagtagt ggaagaggtc 420
ggcttcgaca agatccggaa gaaacttgca gagctccagg aattgaaaat cgtgctcctg 480
gatcgcctat gcatcgcagg agttctccct catagagcga gtctacatga gcttgcagag 540
gcttgcaagg agatagaaca gacatgtcct aagatcgttg acctcgatct gagttacaac 600
ttactggaaa gctgggttga cattgcaaac atatgtcaac agctgaagcg cttgaagaca 660
ttgaagctga tgttggtcat tcagtacatc tgtgagaagc atgctgacag ttggcagcgg 720
aaatcgtcta ggtcctcgac aggagggtct gatattcgac ggtatcacaa cactacactt 780
ggacgagact ctactcgaat gggacgaggt atgctgcaca aagctactgc ttggttacca 840
gtgagctgac tgactcttgg tcccctttag atttcagctt tgacatatca attcccgtca 900
ctctctgctc tgtctgcctc cgcaaatcag attacccaga tcttgacacc tatcacggat 960
accatcacga ccttgacact ggaaaacaat gacatctctt cgctatcctc attagcatgt 1020
ctgacctctt tgagcaagct cgagcacctc tcgctgagag agaatcgtat cgggaaagtc 1080
tatgcgtctg gcatggaagg aaactctctt cagttttccg aaaatctcag atcggtggac 1140
ctatccagaa acaatatcga ttcttggctg tttgtgaatg aacttcaacg cgtatttcct 1200
gggctgcaat ctttgcgcat atcaggaaat cccctgtacg acaagcctgt tgccccctcg 1260
aacgtcacaa atttaccgga gaagccaatg acggtggacg aggcctatat gctaacactc 1320
tctcgactcg cttccatcca aacgctcaac tacagcaaga taacttccca agaccgaagt 1380
aatggcgaac tctattacct ttccctcatt ggcaaggagt tatccgcgta tccggaaagc 1440
gcagaacgcg agattcttgc tacacatccc cgctatcagg agctttgtga gaaatacgga 1500

gcgcccacaa tcaggagagc cgagctggca ggcgctgccg tgaatccgcg ctctgttgcc 1560
gcccgagtag tgaagttggc tttttgcttg cactcatcag ttagttccgg tgcaaaccaa 1620
gaacaatttc gagttcagaa gatcccgaga tcctttaata catatcaagt caaggcaatc 1680
gcctcccgcc tgttcaattt gccgccttac cagtgccgac tagtctggga gaccaacgag 1740
ttagacccta ttcatcagga gaaaaaggac gatggagacg attgggatag tgatgaggat 1800
gaagccacag ctattggatt gggggagagt aacaagctca caccggcgac ggaggatgga 1860
aagttcatca gaagagaggt tgagctcctg gattcaacgc gagacatagg cttttggttc 1920
caacccgata ctgttgaggc aaggatcaga gtagaagttg caacatcgaa ttga 1974

<210> 177
<211> 1830
<212> DNA
<213> Aspergillus fumigatus

<220>
<223> Phylum CEA281.1; clone 5-3-11; contig 4839 region 10030-12622
      Coding region without introns

<400> 177

atggacgtcg gatgctttgt aaatcaacgg cggtcctaca atggggatct atgcacagtt 60
cgttacatag gtaaagttga gggcaccacc ggcgagtggc tcggagtgga atgggatgac 120
cccacgcggg ggaagcattc tggagaacac aacggagtga gatattttac atgtagaagg 180
aaacacccca cggctggttc gttcgtgcgc ccttcgcgac ggaccgacag acctcgaggc 240
ttccttgagg cagtgcgtca caagtatgct tctgagttcc aagaagaact cgcaagacag 300
cagtcaggcg aagtctctgc tgcgcgggaa atcatcaaat ttagtagcaa agtagtggaa 360
gaggtcggct tcgacaagat ccggaagaaa cttgcagagc tccaggaatt gaaaatcgtg 420
ctcctggatc gcctatgcat cgcaggagtt ctccctcata gagcgagtct acatgagctt 480
gcagaggctt gcaaggagat agaacagaca tgtcctaaga tcgttgacct cgatctgagt 540
tacaacttac tggaaagctg ggttgacatt gcaaacatat gtcaacagct gaagcgcttg 600
aagacattga agctgatgtt ggtcattcag tacatctgtg agaagcatgc tgacagttgg 660
cagcggaaat cgtctaggtc ctcgacagga gggtctgata ttcgacggta tcacaacact 720
acacttggac gagactctac tcgaatggga cgaggtatgc tgcacaaagc tactgcttgg 780
ttaccaatta cccagatctt gacacctatc acggatacca tcacgacctt gacactggaa 840
aacaatgaca tctcttcgct atcctcatta gcatgtctga cctctttgag caagctcgag 900
cacctctcgc tgagagagaa tcgtatcggg aaagtctatg cgtctggcat ggaaggaaac 960
tctcttcagt tttccgaaaa tctcagatcg gtggacctat ccagaaacaa tatcgattct 1020
tggctgtttg tgaatgaact tcaacgcgta tttcctgggc tgcaatcttt gcgcatatca 1080
ggaaatcccc tgtacgacaa gcctgttgcc ccctcgaacg tcacaaattt accggagaag 1140
ccaatgacgg tggacgaggc ctatatgcta acactctctc gactcgcttc catccaaacg 1200
ctcaactaca gcaagataac ttcccaagac cgaagtaatg gcgaactcta ttacctttcc 1260
ctcattggca aggagttatc cgcgtatccg gaaagcgcag aacgcgagat tcttgctaca 1320
catccccgct atcaggagct ttgtgagaaa tacggagcgc ccacaatcag gagagccgag 1380
ctggcaggcg ctgccgtgaa tccgcgctct gttgccgccc gagtagtgaa gttggctttt 1440
tgcttgcact catcagttag ttccggtgca aaccaagaac aatttcgagt tcagaagatc 1500
ccgagatcct ttaatacata tcaagtcaag gcaatcgcct cccgcctgtt caatttgccg 1560
ccttaccagt gccgactagt ctgggagacc aacgagttag accctattca tcaggagaaa 1620
aaggacgatg gagacgattg ggatagtgat gaggatgaag ccacagctat tggattgggg 1680
gagagtaaca agctcacacc ggcgacggag gatggaaagt tcatcagaag agaggttgag 1740
ctcctggatt caacgcgaga cataggcttt tggttccaac ccgatactgt tgaggcaagg 1800
atcagagtag aagttgcaac atcgaattga 1830

<210> 178
<211> 609
<212> PRT
<213> Aspergillus fumigatus

<220>
<223> Phylum CEA281.1; clone 5-3-11; contig 4839 region 10030-12622

Protein sequence

<400> 178

Met Asp Val Gly Cys Phe Val Asn Gln Arg Arg Ser Tyr Asn Gly Asp
1               5                   10                  15

Leu Cys Thr Val Arg Tyr Ile Gly Lys Val Glu Gly Thr Thr Gly Glu
            20                  25                  30

Trp Leu Gly Val Glu Trp Asp Asp Pro Thr Arg Gly Lys His Ser Gly
        35                  40                  45

Glu His Asn Gly Val Arg Tyr Phe Thr Cys Arg Arg Lys His Pro Thr
    50                  55                  60

Ala Gly Ser Phe Val Arg Pro Ser Arg Arg Thr Asp Arg Pro Arg Gly
65                  70                  75                  80

Phe Leu Glu Ala Val Arg His Lys Tyr Ala Ser Glu Phe Gln Glu Glu
                85                  90                  95

Leu Ala Arg Gln Gln Ser Gly Glu Val Ser Ala Ala Arg Glu Ile Ile
            100                 105                 110

Lys Phe Ser Ser Lys Val Val Glu Glu Val Gly Phe Asp Lys Ile Arg
        115                 120                 125

Lys Lys Leu Ala Glu Leu Gln Glu Leu Lys Ile Val Leu Leu Asp Arg
    130                 135                 140

Leu Cys Ile Ala Gly Val Leu Pro His Arg Ala Ser Leu His Glu Leu
145                 150                 155                 160

Ala Glu Ala Cys Lys Glu Ile Glu Gln Thr Cys Pro Lys Ile Val Asp
                165                 170                 175

Leu Asp Leu Ser Tyr Asn Leu Leu Glu Ser Trp Val Asp Ile Ala Asn
            180                 185                 190

Ile Cys Gln Gln Leu Lys Arg Leu Lys Thr Leu Lys Leu Met Leu Val
        195                 200                 205

Ile Gln Tyr Ile Cys Glu Lys His Ala Asp Ser Trp Gln Arg Lys Ser
    210                 215                 220

Ser Arg Ser Ser Thr Gly Gly Ser Asp Ile Arg Arg Tyr His Asn Thr
225                 230                 235                 240

Thr Leu Gly Arg Asp Ser Thr Arg Met Gly Arg Gly Met Leu His Lys
                245                 250                 255

Ala Thr Ala Trp Leu Pro Ile Thr Gln Ile Leu Thr Pro Ile Thr Asp
            260                 265                 270

Thr Ile Thr Thr Leu Thr Leu Glu Asn Asn Asp Ile Ser Ser Leu Ser
        275                 280                 285

Ser Leu Ala Cys Leu Thr Ser Leu Ser Lys Leu Glu His Leu Ser Leu
    290                 295                 300

Arg Glu Asn Arg Ile Gly Lys Val Tyr Ala Ser Gly Met Glu Gly Asn
305                 310                 315                 320

Ser Leu Gln Phe Ser Glu Asn Leu Arg Ser Val Asp Leu Ser Arg Asn
                325                 330                 335

Asn Ile Asp Ser Trp Leu Phe Val Asn Glu Leu Gln Arg Val Phe Pro
            340                 345                 350

Gly Leu Gln Ser Leu Arg Ile Ser Gly Asn Pro Leu Tyr Asp Lys Pro
        355                 360                 365

Val Ala Pro Ser Asn Val Thr Asn Leu Pro Glu Lys Pro Met Thr Val
    370                 375                 380

Asp Glu Ala Tyr Met Leu Thr Leu Ser Arg Leu Ala Ser Ile Gln Thr
385                 390                 395                 400

Leu Asn Tyr Ser Lys Ile Thr Ser Gln Asp Arg Ser Asn Gly Glu Leu
                405                 410                 415

Tyr Tyr Leu Ser Leu Ile Gly Lys Glu Leu Ser Ala Tyr Pro Glu Ser
            420                 425                 430

Ala Glu Arg Glu Ile Leu Ala Thr His Pro Arg Tyr Gln Glu Leu Cys
        435                 440                 445

Glu Lys Tyr Gly Ala Pro Thr Ile Arg Arg Ala Glu Leu Ala Gly Ala
    450                 455                 460

Ala Val Asn Pro Arg Ser Val Ala Ala Arg Val Val Lys Leu Ala Phe
465                 470                 475                 480

Cys Leu His Ser Ser Val Ser Ser Gly Ala Asn Gln Glu Gln Phe Arg
                485                 490                 495

Val Gln Lys Ile Pro Arg Ser Phe Asn Thr Tyr Gln Val Lys Ala Ile
            500                 505                 510

Ala Ser Arg Leu Phe Asn Leu Pro Pro Tyr Gln Cys Arg Leu Val Trp
        515                 520                 525

Glu Thr Asn Glu Leu Asp Pro Ile His Gln Glu Lys Lys Asp Asp Gly
    530                 535                 540

Asp Asp Trp Asp Ser Asp Glu Asp Glu Ala Thr Ala Ile Gly Leu Gly
545                 550                 555                 560

Glu Ser Asn Lys Leu Thr Pro Ala Thr Glu Asp Gly Lys Phe Ile Arg
                565                 570                 575

Arg Glu Val Glu Leu Leu Asp Ser Thr Arg Asp Ile Gly Phe Trp Phe
            580                 585                 590

Gln Pro Asp Thr Val Glu Ala Arg Ile Arg Val Glu Val Ala Thr Ser
        595                 600                 605

Asn

<210> 179

<211> 1867
<212> DNA
<213> Aspergillus fumigatus

<220>
<223> Phylum CEA281.2; clone 5-3-11; contig 4839 region 12269-14135
      Genomic sequence containing 3' and 5'-ends and the coding region

<400> 179
caactatttc gacataggag ttgtgaagga gttccttaac ctacattgtc gcgggtgggg      60
taatatatat agttgagcac gactcgtgcg ttaggataca acgaggtcaa cgacagaaaa     120
gtagaacctc ttacgcaagc gcctgtggcc tctgttaccc gaatggattt agaggccttc     180
ttggttattc ttaaacaata tgatatgtgt agtgtatatt caagctggag ggcaacactg     240
actgctgttt taggactagg tcggttttgg gtggcagatt atcaccgcaa ctgttttgtg     300
ataacagtga tatcatttcc ctttcatata acaatttaat actcagacat cgtcatggca     360
gaatactgga aatcagctgt aagtgccctt cattcagttc cgcagacttc ttcgtgataa     420
tctttacgtg gggaagtccg gcatcaactg acagcaatat tctagccccg gttctggtgc     480
aaacaatgca agatattcat tcgggataca cccttcgaga aaacccagca tgaagcgagt     540
gccaaacacc agggaaacct taagcgtttc ctacgagata tccaccggga aaatgaacgg     600
aagcaaagag aaactcagaa ggcgaaggat gaagtcgagc gattaaggca aactgtcgca     660
ggaaaaccag gtgcaaaaga cagcggcgca acagcttgga aacacgcctc ggctgcccct     720
ccaccggcag aacgacctgt gtccctggaa gagagaaaga agcagatagc gcagctggca     780
gagatgggaa ttgctatccc ggacgaatac cgtggtgaac tcgcgctcgc tggcgaatgg     840
cagacggtat ccgaacgagt tattcgacca gatgacgata cagaggaagg aaagcctggt     900
agctctatcg gcgttcggaa acgcaagatg gaaggcgatg aggaggagca ggaggcgcga     960
caggaggccg agagattcgt gagtcagggt tggggctcga ggactcggca gtatcctggg    1020
gagcagagcg atgcagacct ggatgcactt ctaaattcta ccaaggatgt aaagaaggtc    1080
aagttgtcgg cgccggatga agggtcgaaa gagaaggcta gcaaagaggg tgctacacca    1140
agcaacgata cggaccaggc tgcggctcag gagtcagaac taccatcagt caagtctgag    1200
ggtaaagaag cggcgcagct tgctacaaca gataccccag cggtgaagca ggaagaggag    1260
gcggcaccta caggagttgt ttttaagaag cgcaagccga aggtcctgag gaaatagtcg    1320
aatttgcagc tgctggatat ctattatcta ccatgcgcat aaatgtacag atgatgcgtt    1380
atggttgcgc acggtccaat atgcctcgcc tgccggtgct cacatgaagc gatcatgggt    1440
tcttgtctca ctgcgctcca gtgttcgaaa accgggatga tgcctctggc ctccagtcgt    1500
ccgtcgccga agcaacccag ctcatagaag atctggattc tcgattcgct tgcgcggaaa    1560
gcctagtctg gtcttgcgtt agcctatatc cagattgagt gtatcgattc gagcttatgc    1620
gggttgtctg ataatattct gccttacatt ggcagagaga cgttggagca catagggtgg    1680
atggagaatg atcagttctt acgtatgtaa gcatgatgtc tagctcagaa aagagtccca    1740
tataccatgc gactcgttgg cagcatccac ctcttccttt ggagtgcaat ctacaatagc    1800
atgcatacga aacaaatttc gttgacaagg agacccaggg cgagaagagt aatatagcaa    1860
gccagct                                                              1867


<210> 180
<211> 963
<212> DNA
<213> Aspergillus fumigatus

<220>
<223> Phylum CEA281.2; clone 5-3-11; contig 4839 region 12269-14135
      Genomic sequence containing the coding region

<400> 180
atggcagaat actggaaatc agctgtaagt gcccttcatt cagttccgca gacttcttcg      60
tgataatctt tacgtgggga agtccggcat caactgacag caatattcta gccccggttc     120
tggtgcaaac aatgcaagat attcattcgg gatacaccct tcgagaaaac ccagcatgaa     180
gcgagtgcca aacaccaggg aaaccttaag cgtttcctac gagatatcca ccgggaaaat     240
gaacggaagc aaagagaaac tcagaaggcg aaggatgaag tcgagcgatt aaggcaaact     300
gtcgcaggaa aaccaggtgc aaaagacagc ggcgcaacag cttggaaaca cgcctcggct     360
gcccctccac cggcagaacg acctgtgtcc ctggaagaga gaaagaagca gatagcgcag     420
ctggcagaga tgggaattgc tatcccggac gaataccgtg gtgaactcgc gctcgctggc     480

gaatggcaga cggtatccga acgagttatt cgaccagatg acgatacaga ggaaggaaag     540
cctggtagct ctatcggcgt tcggaaacgc aagatggaag gcgatgagga ggagcaggag     600
gcgcgacagg aggccgagag attcgtgagt caggttgggg gctcgaggac tcggcagtat     660
cctggggagc agagcgatgc agacctggat gcacttctaa attctaccaa ggatgtaaag     720
aaggtcaagt tgtcggcgcc ggatgaaggg tcgaaagaga aggctagcaa agagggtgct     780
acaccaagca acgatacgga ccaggctgcg gctcaggagt cagaactacc atcagtcaag     840
tctgagggta aagaagcggc gcagcttgct acaacagata ccccagcggt gaagcaggaa     900
gaggaggcgg cacctacagg agttgttttt aagaagcgca agccgaaggt cctgaggaaa     960
tag                                                                   963


<210> 181
<211> 876
<212> DNA
<213> Aspergillus fumigatus

<220>
<223> Phylum CEA281.2; clone 5-3-11; contig 4839 region 12269-14135
      Coding region without introns

<400> 181
atggcagaat actggaaatc agctccccgg ttctggtgca aacaatgcaa gatattcatt      60
cgggatacac ccttcgagaa aacccagcat gaagcgagtg ccaaacacca gggaaacctt     120
aagcgtttcc tacgagatat ccaccgggaa aatgaacgga agcaaagaga aactcagaag     180
gcgaaggatg aagtcgagcg attaaggcaa actgtcgcag gaaaaccagg tgcaaaagac     240
agcggcgcaa cagcttggaa acacgcctcg gctgcccctc caccggcaga acgacctgtg     300
tccctggaag agagaaagaa gcagatagcg cagctggcag agatgggaat tgctatcccg     360
gacgaatacc gtggtgaact cgcgctcgct ggcgaatggc agacggtatc cgaacgagtt     420
attcgaccag atgacgatac agaggaagga aagcctggta gctctatcgg cgttcggaaa     480
cgcaagatgg aaggcgatga ggaggagcag gaggcgcgac aggaggccga gagattcgtg     540
agtcaggttg ggggctcgag gactcggcag tatcctgggg agcagagcga tgcagacctg     600
gatgcacttc taaattctac caaggatgta aagaaggtca agttgtcggc gccggatgaa     660
gggtcgaaag agaaggctag caaagagggt gctacaccaa gcaacgatac ggaccaggct     720
gcggctcagg agtcagaact accatcagtc aagtctgagg gtaaagaagc ggcgcagctt     780
gctacaacag ataccccagc ggtgaagcag gaagaggagg cggcacctac aggagttgtt     840
tttaagaagc gcaagccgaa ggtcctgagg aaatag                               876


<210> 182
<211> 291
<212> PRT
<213> Aspergillus fumigatus

<220>
<223> Phylum CEA281.2; clone 5-3-11; contig 4839 region 12269-14135
      Protein sequence

<400> 182

Met Ala Glu Tyr Trp Lys Ser Ala Pro Arg Phe Trp Cys Lys Gln Cys
1               5                   10                  15

Lys Ile Phe Ile Arg Asp Thr Pro Phe Glu Lys Thr Gln His Glu Ala
            20                  25                  30

Ser Ala Lys His Gln Gly Asn Leu Lys Arg Phe Leu Arg Asp Ile His
        35                  40                  45

Arg Glu Asn Glu Arg Lys Gln Arg Glu Thr Gln Lys Ala Lys Asp Glu
    50                  55                  60

Val Glu Arg Leu Arg Gln Thr Val Ala Gly Lys Pro Gly Ala Lys Asp
65                  70                  75                  80

Ser Gly Ala Thr Ala Trp Lys His Ala Ser Ala Ala Pro Pro Pro Ala
                85                  90                  95

Glu Arg Pro Val Ser Leu Glu Glu Arg Lys Lys Gln Ile Ala Gln Leu
            100                 105                 110

Ala Glu Met Gly Ile Ala Ile Pro Asp Glu Tyr Arg Gly Glu Leu Ala
        115                 120                 125

Leu Ala Gly Glu Trp Gln Thr Val Ser Glu Arg Val Ile Arg Pro Asp
    130                 135                 140

Asp Asp Thr Glu Glu Gly Lys Pro Gly Ser Ser Ile Gly Val Arg Lys
145                 150                 155                 160

Arg Lys Met Glu Gly Asp Glu Glu Glu Gln Glu Ala Arg Gln Glu Ala
                165                 170                 175

Glu Arg Phe Val Ser Gln Gly Trp Gly Ser Arg Thr Arg Gln Tyr Pro
            180                 185                 190

Gly Glu Gln Ser Asp Ala Asp Leu Asp Ala Leu Leu Asn Ser Thr Lys
        195                 200                 205

Asp Val Lys Lys Val Lys Leu Ser Ala Pro Asp Glu Gly Ser Lys Glu
    210                 215                 220

Lys Ala Ser Lys Glu Gly Ala Thr Pro Ser Asn Asp Thr Asp Gln Ala
225                 230                 235                 240

Ala Ala Gln Glu Ser Glu Leu Pro Ser Val Lys Ser Glu Gly Lys Glu
                245                 250                 255

Ala Ala Gln Leu Ala Thr Thr Asp Thr Pro Ala Val Lys Gln Glu Glu
            260                 265                 270

Glu Ala Ala Pro Thr Gly Val Val Phe Lys Lys Arg Lys Pro Lys Val
        275                 280                 285

Leu Arg Lys
    290


<210> 183
<211> 2193
<212> DNA
<213> Aspergillus fumigatus

<220>
<223> Phylum CEA282.1; clone 10-4-20; contig 4929 region 328110-325663
      Genomic sequence containing 3' and 5'-ends and the coding region

<400> 183
gagcaactgc cagaaatcca gacgtgcaac tcctccgcaa aaaaaagcgt tgtctcccaa      60
aacggaggct gttaagttat cgccacgggg aaatagccca aaaggaactc gtcacagctg     120
gaatcaacac caagtaccga agaaacaagc gagcagcggc tgtttggctt ctgcagctgc     180
acaaaaaatg ggaacgaagt gaatgaggtt agatagagat gaggatggat caagaagcgc     240
cctccagatg tagcaatgaa gagatgatgt tgcaagaaga ggtgaaacaa gctggcggca     300

cgggatcagg ctaggctaga tagggttagc aacgagggtg acatcacgtg agaacgggca 360
tcgtgatatg gatgacaatt aacatcataa acactcttcg ttcagttgct gtgactcctg 420
acgcgtaagg ggatctgggg tgaagtcaag caatagactc tctgacagat ttgactttag 480
agaaagtaaa taacaccact atggacatct cgcaagaaac cgttgataaa atacgacgtt 540
tcgcgcaaaa gcgccaaaaa gcggaggagt tctacgagga acactcggta aatccagcta 600
attttgacgc ttacaatcgc aagttggatg agacgttggc agagctgcag gctcaagtca 660
aacgtcatga ggatgagctc cgcaaggtac gtcaacaagt tgcctagaat ataagccgac 720
tgtcacaaga gatttcatgc atgaattagg aatactgaca agaggaacag ctacgcatga 780
ccaccacgat cgagttcgct caaattgggg cagatccttg ggcccgcatc tcagaagtgc 840
gcagagccaa gaaagcgtat gattctcttc tgcaatcgga aacgcgactg ccgagtccag 900
gctcgccctt gccttcatta cttgcggttg acgaggcgtc tcgtctcgtc aaggagagca 960
agacctcaat ctcactgacg gcggagaaac tgtctgcgga tcgtcagcgc ttgaaagcgg 1020
aagaagccaa tttgcgcgat gcgcaactga tcaaagacgg gttggagaaa aggattgagc 1080
ggctgaacgc agaaaaatcg agtcaagtcc agaaaactcc tgcgcagctt gcgtatgatc 1140
tcgtcaagga gcagcaggaa aagatcgaga gacttgatac taccacagaa gagctaaagt 1200
cctctctcta taaatttgtc gaagacacac ttgccccaat gcttgctgca gaaaatctgg 1260
gcggtcccac tgtcggagat gcgttggaaa tttcggacac taccttaaaa gcgggctaca 1320
ctagccatgg gaagcctaag aaaccaaaaa ctccggccgt ggggacttct gacagtggcc 1380
aacagcggat tgacgagctt gttcgtcgcc aaactgcgca ggagggcaac gagcaggcaa 1440
cccttttgaa caaaagagag gcggccgccg ctgaaatgcg agctcttctt actgctctgt 1500
tagatgcgga ttactcctat gtcgaccttc cgcacgagtc agcggcctcg cgctttctag 1560
taagagcgaa ggtagctcaa ttccatccgc gcgatgccag gaagcttcgg ttaattgatt 1620
ttgggcgctc attagtcgat tgaggtggct acatgtaccg tactacatct cccagcttac 1680
aaatggtatc acatttcacc aaacatctgg gaaaagacaa acagacgcca tccccacgga 1740
tatatagcga ctcaaccgaa agccagtaag atatctagag ccggcgaaaa ccacgtgttt 1800
caacgaagaa gcggccccga aagccactgg tagcataacg ccttgagaat gcgagagata 1860
catcaaaagc ttatcagaaa gttcaatgct cgaggtcaaa aatataccgt taatgccata 1920
caagaaacat ggaagaagaa agaccgtagc cgggtatcag atcggcatca ttccgatgct 1980
ggtagaagta ctcttggccg tattctttgc tttggagcca gttcgggaac ccgccgagcg 2040
cttgttgact tgatcgctgg gctcccttct aggtcgcggc gtttttattt ttgaactcga 2100
ccctgtagcg ttcttgcggt ggaagcgctt cttggacgaa gtctttcttt tcttggaacg 2160
gctagtctcg gtgtcttcat actcttcgga tga 2193

<210> 184
<211> 1448
<212> DNA
<213> Aspergillus fumigatus

<220>
<223> Phylum CEA282.1; clone 10-4-20; contig 4929 region 328110-325663
      Genomic sequence containing the coding region

<400> 184
atgtctgctt ctccatccgc actgcaatcg accaagcggc ccttggagga cccttcttcg 60
ccgtccggac caaatgatca gccagaagct aaacgtcctg ccttggacaa agtagtaaag 120
ggaaacgagt cggagaccta tacggatgcc aaggctgagc cttccgctgc gccaagtgct 180
actgctgatg gccagggcga cactgttgtt cctgatgctc caaatggtaa gggtgcatcc 240
acggagacgc agccaattca gtcgaccgcg tctcatggcg agcgcgctac ttctcagccc 300
gaacagcagc gcccacaaga tgaatccagc tggattcaca ttcgcgctgt aatttctagc 360
caggaagctg ccacagtcat tggcaagggt ggagaaaacg tatctcagat tcgtcgtttg 420
tctggagcaa agtgcactgt cagcgactac tcccgtggtg cagtcgaacg tattttgacc 480
gtgagcggcc cacaggatgc cgttgccaag gttggttttt tgatctatcc ttcgctgttt 540
gaaagattgc taattcagag taggcgtttg gtttgatcat ccgtacattg aacaatgaac 600
ctcttgatgc cccctctacc gcccaatcca agacataccc tctgcgtttg ctgatccccc 660
atctccttat tggctccatc attggcaaag gtggttcacg cattcgcgaa attcaggaag 720
cttctggtgc ccgactgaat gcatccgatt cgtgccttcc cttgtcctct gagcggtcac 780
ttgtaattct cggcgttgcc gattctgtcc acatcgctac ctactacgtc gccgtaaccc 840
tcgttgagca gctcactgag cgctttggag gtcctgcagc ctcagcttat gccactcgca 900
gcggtggccc tgctggagca gtgcctggcg gtatgcaggt tgtcccgtat gttccacagc 960
ccgctggtgg tcaatatggc catccagaac atttcaagag acaccatcac caccccaatc 1020

gcgctgctgc aggcgcctat ggggtccctt accttcacgg tcagcctgct cccgcaccag   1080
tggcccagcc ggctttgcat tatggagctg ctccccatgc cccttacgca ggagctggcc   1140
cccatcagcc tgctccatac ggcgcaccgc agcccgctca ggcacgcggc gctcctaccc   1200
ctgccacacc cgttggaggt gtcatgcctg gtcagccatt gactcagcag atctacatcc   1260
ccaacgacat ggttggtgcc atcatcggaa agggcggtgc gaagatcaat gagattcgac   1320
acctcagtgg cagtgtgatc aagattaatg agcctcaaga gaacagcaat gagcgtttgg   1380
tgactattac tggaacccag gaatgcaacc aaatggctct gtacatgctt tactcgcgac   1440
ttggttag                                                            1448

<210> 185
<211> 1395
<212> DNA
<213> Aspergillus fumigatus

<220>
<223> Phylum CEA282.1; clone 10-4-20; contig 4929 region 328110-325663
      Coding region without introns

<400> 185
atgtctgctt ctccatccgc actgcaatcg accaagcggc ccttggagga cccttcttcg     60
ccgtccggac caaatgatca gccagaagct aaacgtcctg ccttggacaa agtagtaaag    120
ggaaacgagt cggagaccta tacggatgcc aaggctgagc cttccgctgc gccaagtgct    180
actgctgatg gccagggcga cactgttgtt cctgatgctc caaatggtaa gggtgcatcc    240
acggagacgc agccaattca gtcgaccgcg tctcatggcg agcgcgctac ttctcagccc    300
gaacagcagc gcccacaaga tgaatccagc tggattcaca ttcgcgctgt aatttctagc    360
caggaagctg ccacagtcat tggcaagggt ggagaaaacg tatctcagat tcgtcgtttg    420
tctggagcaa agtgcactgt cagcgactac tcccgtggtg cagtcgaacg tattttgacc    480
gtgagcggcc cacaggatgc cgttgccaag gcgtttggtt tgatcatccg tacattgaac    540
aatgaacctc ttgatgcccc ctctaccgcc caatccaaga cataccctct gcgtttgctg    600
atcccccatc tccttattgg ctccatcatt ggcaaaggtg gttcacgcat tcgcgaaatt    660
caggaagctt ctggtgcccg actgaatgca tccgattcgt gccttccctt gtcctctgag    720
cggtcacttg taattctcgg cgttgccgat tctgtccaca tcgctaccta ctacgtcgcc    780
gtaaccctcg ttgagcagct cactgagcgc tttggaggtc ctgcagcctc agcttatgcc    840
actcgcagcg gtggccctgc tggagcagtg cctggcggta tgcaggttgt cccgtatgtt    900
ccacagcccg ctggtggtca atatggccat ccagaacatt tcaagagaca ccatcaccac    960
cccaatcgcg ctgctgcagg cgcctatggg gtcccttacc ttcacggtca gcctgctccc   1020
gcaccagtgg cccagccggc tttgcattat ggagctgctc cccatgcccc ttacgcagga   1080
gctggccccc atcagcctgc tccatacggc gcaccgcagc ccgctcaggc acgcggcgct   1140
cctacccctg ccacacccgt tggaggtgtc atgcctggtc agccattgac tcagcagatc   1200
tacatcccca acgacatggt tggtgccatc atcggaaagg gcggtgcgaa gatcaatgag   1260
attcgacacc tcagtggcag tgtgatcaag attaatgagc ctcaagagaa cagcaatgag   1320
cgtttggtga ctattactgg aacccaggaa tgcaaccaaa tggctctgta catgctttac   1380
tcgcgacttg gttag                                                    1395

<210> 186
<211> 464
<212> PRT
<213> Aspergillus fumigatus

<220>
<223> Phylum CEA282.1; clone 10-4-20; contig 4929 region 328110-325663
      Protein sequence

<400> 186

Met Ser Ala Ser Pro Ser Ala Leu Gln Ser Thr Lys Arg Pro Leu Glu
1               5                   10                  15

Asp Pro Ser Ser Pro Ser Gly Pro Asn Asp Gln Pro Glu Ala Lys Arg

            20                  25                  30

Pro Ala Leu Asp Lys Val Val Lys Gly Asn Glu Ser Glu Thr Tyr Thr
        35                  40                  45

Asp Ala Lys Ala Glu Pro Ser Ala Ala Pro Ser Ala Thr Ala Asp Gly
    50                  55                  60

Gln Gly Asp Thr Val Val Pro Asp Ala Pro Asn Gly Lys Gly Ala Ser
65                  70                  75                  80

Thr Glu Thr Gln Pro Ile Gln Ser Thr Ala Ser His Gly Glu Arg Ala
                85                  90                  95

Thr Ser Gln Pro Glu Gln Gln Arg Pro Gln Asp Glu Ser Ser Trp Ile
            100                 105                 110

His Ile Arg Ala Val Ile Ser Ser Gln Glu Ala Ala Thr Val Ile Gly
        115                 120                 125

Lys Gly Gly Glu Asn Val Ser Gln Ile Arg Arg Leu Ser Gly Ala Lys
    130                 135                 140

Cys Thr Val Ser Asp Tyr Ser Arg Gly Ala Val Glu Arg Ile Leu Thr
145                 150                 155                 160

Val Ser Gly Pro Gln Asp Ala Val Ala Lys Ala Phe Gly Leu Ile Ile
                165                 170                 175

Arg Thr Leu Asn Asn Glu Pro Leu Asp Ala Pro Ser Thr Ala Gln Ser
            180                 185                 190

Lys Thr Tyr Pro Leu Arg Leu Leu Ile Pro His Leu Leu Ile Gly Ser
        195                 200                 205

Ile Ile Gly Lys Gly Gly Ser Arg Ile Arg Glu Ile Gln Glu Ala Ser
    210                 215                 220

Gly Ala Arg Leu Asn Ala Ser Asp Ser Cys Leu Pro Leu Ser Ser Glu
225                 230                 235                 240

Arg Ser Leu Val Ile Leu Gly Val Ala Asp Ser Val His Ile Ala Thr
                245                 250                 255

Tyr Tyr Val Ala Val Thr Leu Val Glu Gln Leu Thr Glu Arg Phe Gly
            260                 265                 270

Gly Pro Ala Ala Ser Ala Tyr Ala Thr Arg Ser Gly Gly Pro Ala Gly
        275                 280                 285

Ala Val Pro Gly Gly Met Gln Val Val Pro Tyr Val Pro Gln Pro Ala
    290                 295                 300

Gly Gly Gln Tyr Gly His Pro Glu His Phe Lys Arg His His His His
305                 310                 315                 320

Pro Asn Arg Ala Ala Ala Gly Ala Tyr Gly Val Pro Tyr Leu His Gly
                325                 330                 335

Gln Pro Ala Pro Ala Pro Val Ala Gln Pro Ala Leu His Tyr Gly Ala
            340                 345                 350

Ala Pro His Ala Pro Tyr Ala Gly Ala Gly Pro His Gln Pro Ala Pro
        355                 360                 365

Tyr Gly Ala Pro Gln Pro Ala Gln Ala Arg Gly Ala Pro Thr Pro Ala
    370                 375                 380

Thr Pro Val Gly Gly Val Met Pro Gly Gln Pro Leu Thr Gln Gln Ile
385                 390                 395                 400

Tyr Ile Pro Asn Asp Met Val Gly Ala Ile Ile Gly Lys Gly Gly Ala
                405                 410                 415

Lys Ile Asn Glu Ile Arg His Leu Ser Gly Ser Val Ile Lys Ile Asn
            420                 425                 430

Glu Pro Gln Glu Asn Ser Asn Glu Arg Leu Val Thr Ile Thr Gly Thr
        435                 440                 445

Gln Glu Cys Asn Gln Met Ala Leu Tyr Met Leu Tyr Ser Arg Leu Gly
    450                 455                 460

<210> 187
<211> 2121
<212> DNA
<213> Aspergillus fumigatus

<220>
<223> Phylum CEA282.2; clone 10-4-20; contig 4839 region 328075-330267
      Genomic sequence containing 3' and 5'-ends and the coding region

<400> 187
   taagttatcg ccacggggaa atagcccaaa aggaactcgt cacagctgga atcaacacca      60
   agtaccgaag aaacaagcga gcagcggctg tttggcttct gcagctgcac aaaaaatggg     120
   aacgaagtga atgaggttag atagagatga ggatggatca agaagcgccc tccagatgta     180
   gcaatgaaga gatgatgttg caagaagagg tgaaacaagc tggcggcacg ggatcaggct     240
   aggctagata gggttagcaa cgagggtgac atcacgtgag aacgggcatc gtgatatgga     300
   tgacaattaa catcataaac actcttcgtt cagttgctgt gactcctgac gcgtaagggg     360
   atctggggtg aagtcaagca atagactctc tgacagattt gactttagag aaagtaaata     420
   acaccactat ggacatctcg caagaaaccg ttgataaaat acgacgtttc gcgcaaaagc     480
   gccaaaaagc ggaggagttc tacgaggaac actcggtaaa tccagctaat tttgacgctt     540
   acaatcgcaa gttggatgag acgttggcag agctgcaggc tcaagtcaaa cgtcatgagg     600
   atgagctccg caaggtacgt caacaagttg cctagaatat aagccgactg tcacaagaga     660
   tttcatgcat gaattaggaa tactgacaag aggaacagct acgcatgacc accacgatcg     720
   agttcgctca aattggggca gatccttggg cccgcatctc agaagtgcgc agagccaaga     780
   aagcgtatga ttctcttctg caatcggaaa cgcgactgcc gagtccaggc tcgcccttgc     840
   cttcattact tgcggttgac gaggcgtctc gtctcgtcaa ggagagcaag acctcaatct     900
   cactgacggc ggagaaactg tctgcggatc gtcagcgctt gaaagcggaa gaagccaatt     960
   tgcgcgatgc gcaactgatc aaagacgggt tggagaaaag gattgagcgg ctgaacgcag    1020
   aaaaatcgag tcaagtccag aaaactcctg cgcagcttgc gtatgatctc gtcaaggagc    1080
   agcaggaaaa gatcgagaga cttgatacta ccacagaaga gctaaagtcc tctctctata    1140
   aatttgtcga agacacactt gccccaatgc ttgctgcaga aaatctgggc ggtcccactg    1200
   tcggagatgc gttggaaatt tcggacacta ccttaaaagc gggctacact agccatggga    1260
   agcctaagaa accaaaaact ccggccgtgg ggacttctga cagtggccaa cagcggattg    1320
   acgagcttgt tcgtcgccaa actgcgcagg agggcaacga gcaggcaacc cttttgaaca    1380
   aaagagaggc ggccgccgct gaaatgcgag ctcttcttac tgctctgtta gatgcggatt    1440
   actcctatgt cgaccttccg cacgagtcag cggcctcgcg ctttctagta agagcgaagg    1500
   tagctcaatt ccatccgcgc gatgccagga agcttcggtt aattgatttt gggcgctcat    1560
   tagtcgattg aggtggctac atgtaccgta ctacatctcc cagcttacaa atggtatcac    1620
   atttcaccaa acatctggga aaagacaaac agacgccatc cccacggata tatagcgact    1680
   caaccgaaag ccagtaagat atctagagcc ggcgaaaacc acgtgtttca acgaagaagc    1740
   ggccccgaaa gccactggta gcataacgcc ttgagaatgc gagagataca tcaaaagctt 1800
   atcagaaagt tcaatgctcg aggtcaaaaa tataccgtta atgccataca agaaacatgg 1860
   aagaagaaag accgtagccg ggtatcagat cggcatcatt ccgatgctgg tagaagtact 1920
   cttggccgta ttctttgctt tggagccagt tcgggaaccc gccgagcgct tgttgacttg 1980
   atcgctgggc tcccttctag gtcgcggcgt ttttattttt gaactcgacc ctgtagcgtt 2040
   cttgcggtgg aagcgcttct tggacgaagt ctttcttttc ttggaacggc tagtctcggt 2100
   gtcttcatac tcttcggatg a 2121

<210> 188
<211> 1143
<212> DNA
<213> Aspergillus fumigatus

<220>
<223> Phylum CEA282.2; clone 10-4-20; contig 4839 region 328075-330267
      Genomic sequence containing the coding region

<400> 188
atggacatct cgcaagaaac cgttgataaa atacgacgtt tcgcgcaaaa gcgccaaaaa 60
gcggaggagt tctacgagga acactcggta aatccagcta attttgacgc ttacaatcgc 120
aagttggatg agacgttggc agagctgcag gctcaagtca aacgtcatga ggatgagctc 180
cgcaaggtac gtcaacaagt tgcctagaat ataagccgac tgtcacaaga gatttcatgc 240
atgaattagg aatactgaca agaggaacag ctacgcatga ccaccacgat cgagttcgct 300
caaattgggg cagatccttg ggcccgcatc tcagaagtgc gcagagccaa gaaagcgtat 360
gattctcttc tgcaatcgga aacgcgactg ccgagtccag gctcgccctt gccttcatta 420
cttgcggttg acgaggcgtc tcgtctcgtc aaggagagca agacctcaat ctcactgacg 480
gcggagaaac tgtctgcgga tcgtcagcgc ttgaaagcgg aagaagccaa tttgcgcgat 540
gcgcaactga tcaaagacgg gttggagaaa aggattgagc ggctgaacgc agaaaaatcg 600
agtcaagtcc agaaaactcc tgcgcagctt gcgtatgatc tcgtcaagga gcagcaggaa 660
aagatcgaga gacttgatac taccacagaa gagctaaagt cctctctcta taaatttgtc 720
gaagacacac ttgccccaat gcttgctgca gaaaatctgg gcggtcccac tgtcggagat 780
gcgttggaaa tttcggacac taccttaaaa gcgggctaca ctagccatgg gaagcctaag 840
aaaccaaaaa ctccggccgt ggggacttct gacagtggcc aacagcggat tgacgagctt 900
gttcgtcgcc aaactgcgca ggagggcaac gagcaggcaa cccttttgaa caaaagagag 960
gcggccgccg ctgaaatgcg agctcttctt actgctctgt tagatgcgga ttactcctat 1020
gtcgaccttc cgcacgagtc agcggcctcg cgctttctag taagagcgaa ggtagctcaa 1080
ttccatccgc gcgatgccag gaagcttcgg ttaattgatt ttgggcgctc attagtcgat 1140
tga 1143

<210> 189
<211> 1035
<212> DNA
<213> Aspergillus fumigatus

<220>
<223> Phylum CEA282.2; clone 10-4-20; contig 4839 region 328075-330267
      Coding region without introns

<400> 189
atggacatct cgcaagaaac cgttgataaa atacgacgtt tcgcgcaaaa gcgccaaaaa 60
gcggaggagt tctacgagga acactcggta aatccagcta attttgacgc ttacaatcgc 120
aagttggatg agacgttggc agagctgcag gctcaagtca aacgtcatga ggatgagctc 180
cgcaagttcg ctcaaattgg ggcagatcct tgggcccgca tctcagaagt gcgcagagcc 240
aagaaagcgt atgattctct tctgcaatcg gaaacgcgac tgccgagtcc aggctcgccc 300
ttgccttcat tacttgcggt tgacgaggcg tctcgtctcg tcaaggagag caagacctca 360
atctcactga cggcggagaa actgtctgcg gatcgtcagc gcttgaaagc ggaagaagcc 420
aatttgcgcg atgcgcaact gatcaaagac gggttggaga aaaggattga gcggctgaac 480
gcagaaaaat cgagtcaagt ccagaaaact cctgcgcagc ttgcgtatga tctcgtcaag 540
gagcagcagg aaaagatcga gagacttgat actaccacag aagagctaaa gtcctctctc 600

tataaatttg tcgaagacac acttgcccca atgcttgctg cagaaaatct gggcggtccc    660
actgtcggag atgcgttgga aatttcggac actaccttaa aagcgggcta cactagccat    720
gggaagccta agaaaccaaa aactccggcc gtggggactt ctgacagtgg ccaacagcgg    780
attgacgagc ttgttcgtcg ccaaactgcg caggagggca acgagcaggc aacccttttg    840
aacaaaagag aggcggccgc cgctgaaatg cgagctcttc ttactgctct gttagatgcg    900
gattactcct atgtcgacct tccgcacgag tcagcggcct cgcgctttct agtaagagcg    960
aaggtagctc aattccatcc gcgcgatgcc aggaagcttc ggttaattga ttttgggcgc   1020
tcattagtcg attga                                                    1035

<210> 190
<211> 344
<212> PRT
<213> Aspergillus fumigatus

<220>
<223> Phylum CEA282.2; clone 10-4-20; contig 4839 region 328075-330267
      Protein sequence

<400> 190
Met Asp Ile Ser Gln Glu Thr Val Asp Lys Ile Arg Arg Phe Ala Gln
1               5                   10                  15

Lys Arg Gln Lys Ala Glu Glu Phe Tyr Glu Glu His Ser Val Asn Pro
            20                  25                  30

Ala Asn Phe Asp Ala Tyr Asn Arg Lys Leu Asp Glu Thr Leu Ala Glu
        35                  40                  45

Leu Gln Ala Gln Val Lys Arg His Glu Asp Glu Leu Arg Lys Phe Ala
    50                  55                  60

Gln Ile Gly Ala Asp Pro Trp Ala Arg Ile Ser Glu Val Arg Arg Ala
65                  70                  75                  80

Lys Lys Ala Tyr Asp Ser Leu Leu Gln Ser Glu Thr Arg Leu Pro Ser
                85                  90                  95

Pro Gly Ser Pro Leu Pro Ser Leu Leu Ala Val Asp Glu Ala Ser Arg
            100                 105                 110

Leu Val Lys Glu Ser Lys Thr Ser Ile Ser Leu Thr Ala Glu Lys Leu
        115                 120                 125

Ser Ala Asp Arg Gln Arg Leu Lys Ala Glu Glu Ala Asn Leu Arg Asp
    130                 135                 140

Ala Gln Leu Ile Lys Asp Gly Leu Glu Lys Arg Ile Glu Arg Leu Asn
145                 150                 155                 160

Ala Glu Lys Ser Ser Gln Val Gln Lys Thr Pro Ala Gln Leu Ala Tyr
                165                 170                 175

Asp Leu Val Lys Glu Gln Gln Glu Lys Ile Glu Arg Leu Asp Thr Thr
            180                 185                 190

Thr Glu Glu Leu Lys Ser Ser Leu Tyr Lys Phe Val Glu Asp Thr Leu
        195                 200                 205

Ala Pro Met Leu Ala Ala Glu Asn Leu Gly Gly Pro Thr Val Gly Asp
    210                 215                 220

Ala Leu Glu Ile Ser Asp Thr Thr Leu Lys Ala Gly Tyr Thr Ser His
225                 230                 235                 240

Gly Lys Pro Lys Lys Pro Lys Thr Pro Ala Val Gly Thr Ser Asp Ser
                245                 250                 255

Gly Gln Gln Arg Ile Asp Glu Leu Val Arg Arg Gln Thr Ala Gln Glu
            260                 265                 270

Gly Asn Glu Gln Ala Thr Leu Leu Asn Lys Arg Glu Ala Ala Ala Ala
        275                 280                 285

Glu Met Arg Ala Leu Leu Thr Ala Leu Leu Asp Ala Asp Tyr Ser Tyr
    290                 295                 300

Val Asp Leu Pro His Glu Ser Ala Ala Ser Arg Phe Leu Val Arg Ala
305                 310                 315                 320

Lys Val Ala Gln Phe His Pro Arg Asp Ala Arg Lys Leu Arg Leu Ile
                325                 330                 335

Asp Phe Gly Arg Ser Leu Val Asp
            340

<210> 191
<211> 2000
<212> DNA
<213> Aspergillus fumigatus

<220>
<223> Phylum CEA283; clone 11-6-20; contig 4910 region 9638-11637
      Genomic sequence containing 3' and 5'-ends and the coding region

<400> 191
tgaacctatg taccatccac tcctgagtcc tgctcagggg aaatgcatgt gagcaggtgc      60
cttctcacct ctgctccttt aatacttcat tgcggggata ttattccgtg ctggacagct     120
tctgcaagat cagtaattat gtaggaacac gaaaacaatg ttctacacat tagttttctc     180
ctgatgacca tcttctcttt gatcaatgca gcagacaaca aatcctgtcc aatcatccca     240
acatactgtt tcctaccttg attggcgcaa atccggcgac aataatcaag ggtagagtga     300
tggactaggc tcaggcggtt tgactttgca aatccaattg aattctgatc agtgctaagc     360
cgatgatggc gttgagcgac caaggacaat ttccgactga gctcagcatt tccctcgctc     420
gcgactcgcg aggcgcggtc caggacggga ggtcttggaa aggctgaacg gtcaatcaat     480
catcaaatgg gtaggatggt gcgtaaatag cccacatgcg cataaaagtg gatgagtgcc     540
gcatcggtat cttcgttctt gtttcctttt ccgtctcacc ccaagatcat gggtcatgaa     600
ttgataatag tatcgtcaac tggcttagcg tgatgactac gtatcttttt gagcgtcaag     660
ttcggtcgtt tcaatcggag tggttttctc cgccaacgcc tagtccattg ctcccaagag     720
aggggctgat aaatgacggc gctcaggggc ccaggggctt catgtaggca caatggaagt     780
aaaacgtatg tacgaagaat ggatcatctc tcaaagtact atactccgta ctcttgcttg     840
tattctgata tttcagttca ataatcgaaa aatagtgacg cgcggtatgg tgcagatctt     900
atgcagatag gacagtcagc ccgccagtat tacggagaac ctaaggtact catagtaaag     960
tatgatgata cggccgcata cgacctctaa ttcatcgaat actccagaag cggctagcca    1020
ctttcctgaa atcgaggtct atccatggac tcagcatctt gtacggagta caaactcctg    1080
ccagcttatt ccaactgatg accctggccc ttatatgagc aagcaggata tgattgggag    1140
acttgagttg tgtttggaac cagctggtca gccacggaat gatattaggc ttactaaggt    1200
agctattgtc tcacacaggt atgcactgcg gaacatggat taattgatac ttatgaaccc    1260
taactgcttt ggaggggagg tgatactcta cctgagggaa aggcgaacaa ttacagagta    1320
atgataatgc ataaataata tctcatgatg ttaccctgcc tcgtcaaaca tggagtctcg    1380
ctttcgattg tgaatccact tctccatttt tacgtcagac taattccatt taatcaatac    1440
ctctcgtatg ggtaaatcaa atgcaccgga aatttacata tgatatcaaa agtagtagtt    1500
ctccgactag ggaggatgga gcaaggtaat ctatatttat tggctgctcg tttgcaggcc    1560

aaaccaaaac caacatgctc tgtatcttag cctccgtatc tgatctcata tctttgagga   1620
cattgattgg attgtcgatc ccaatgacgg cgtgctgaga ctgttctaaa gagagctcaa   1680
aagaaataga gcaaaagtaa gatcatctac ggactatcta cgtcctatag gagtttcact   1740
ctgtgataat ggctcacgcc cacataaact gtccatgacg aaatcgatac agcctcaaga   1800
aagccaacaa ttttagtttg gaagtcgaat cacaccacat aggttaacca gcaaaagcgc   1860
agacctccaa taaaggttcg ctcatcgtcc ccttctgcgt ctactttgga gctaaagctg   1920
cgcgtctgta catcttgacc tccttcggcc tctcctttgc gctgtgctca gttttccctt   1980
cttttgtctc ttggactgga                                                2000

<210> 192
<211> 4336
<212> DNA
<213> Aspergillus fumigatus

<220>
<223> ATPase; Phylum CEA284.1; clone 4-3-4; contig 4899
      region 472441-476776
      Genomic sequence containing 3' and 5'-ends and the coding region

<400> 192
   acgattggcc aatcgtttgc tgtaatgctt ggcggcttct tggcattgcc cagcccaagg     60
   tgctcacgtg ggtgggatga tgggcatgtc gactgtcgtg tgcgtcatcg ggctgggagg    120
   cggcaaatgc tcgcgcaccg acccacggaa ttcggccaag attctggatc gattgcccct    180
   tatgtatgcg aaggtttggg gatgtgattg gtgtgcagcg taaacgagta cggattgata    240
   agagtgccaa gattggaacc actgttcacg tcatggaggg gggtttccgg cacgccggtt    300
   aggagtgaga cagagtgact taaatatgtc gcgatacccc cgcaacgacg ggagaagaaa    360
   atctggttct acaacctagg ctttaatttc caagttgatt tcaagtcgtt gtgtatggac    420
   tctcactcgc ttgaagatac gatatacatg aagcctcctt aggataactg cactggaata    480
   gaacattttc gttatataac atgggtgcgc agaagaagca atcctctgat aactctcagg    540
   ctttgtctca gcctgctcat gcgcttcgct atgaggatgt ccttcgtgag ctggccgtcg    600
   atccagacca gggtctgacc gtgggggagg cgaagcgaag actccaacaa tatggtccga    660
   atgaactgga agggggagag ggtgtttcca ttgtcaagat tgtcattagg cagatcgcaa    720
   atgccatgat gctggttagt ctctcccttt tgttagacag acaacgacag acaactgacg    780
   acagcaggtc ctgatcatcg ccatggcggt cagtttcggc attcaatcct ggattgaggg    840
   tggtgtcatt ggggctgtca ttggtctaaa cattgtggta ggtgtctatc aagactatgc    900
   cgcagagaaa acgatggatt cccttcgatc gttgagttct cccaccggaa ctgtcacgcg    960
   tgatggcaaa accagtacca ttcctgccaa cgagattgtc cccggggaca tgattgaact   1020
   caaagttgga gacacggttc ctgcggatct tcggtgcgta aagagttacc agaaaatatc   1080
   gttcccgttc actaaccgcc gataggctcg ttgatgcaat gaatttcgaa accgacgagg   1140
   ccctcctgac tggcgagtcg ctgccggtgc agaaagaggt cgataccact tttgacccag   1200
   acacgggacc tggtgatcgg ttgaatattg cgtatagttc ctccacggtg acgagaggcc   1260
   gcgctcgggg cgttgttatc agcacgggga tgcaaaccga gattggagcc atcgccgctg   1320
   ctctccgcgc cagcgactcc aagaggcggc ccgtcaagcg cggtcccgag ggagaaacca   1380
   agaagcgatg gtacgtgcag gcgtggaccc tgacttgcac ggatgccgtg ggacggttcc   1440
   tgggaatcaa cgttggcaca ccgctgcagc gcaaactctc gaaactggct ttggcccttt   1500
   tcgccattgc catcatcttc gccatcattg tcatgggcgt caatgggttc cgcaatgaca   1560
   aggaggtcat catctacgca gtcgcaacag gtcttgcaat gatcccggcc tgcctggtgg   1620
   tggtcctgac cattaccatg gcggttggaa ccaagcaaat ggttgaaaga catgtcattg   1680
   tccggaagct tgattccctt gaagccctgg gcgctgtgac taacatttgc tcagacaaaa   1740
   cgggaaccct cacccagggg cgcatggtcg ccaaacgggc gtggattccc tctgttggca   1800
   ccttctcggt ggggtcttct aataacccct tgaacccgga agagggcgac cttagcctgc   1860
   tgcctgaccc gccggtcaaa gttggccctg acgctcatgg agagccttcc cggccggagg   1920
   atctgctcaa ggacaatcct cttttggagc aatatctgaa cgtggccgcc atggcaaacc   1980
   ttgcccatgt gcacaggtcc gagcacaatg aatggcaggc tcgtggtgaa ccgaccgaca   2040
   ttgcgattca ggtgtttgcc tcgcgcttta actggggacg cgatcgctgg accaaaggcg   2100
   agaagcctgt ttggcgccaa aaagctgaat atccatttga ttctactgtg aaaaagatgt   2160
   ctgtcatctt taagaacacc aatgatgatc gggagatgat cttcaccaag ggcgcggtgg   2220
   aacgtgtcat cgaggcttgc accaccgtca cctggacagc tggttccgac ccgatcgcct   2280
   tggacgagaa cataaaggag gaaatcttgc agaacatgga agctctcgca aaggagggcc   2340
   tgcgagttct ctgcctcgcc tgtcgggaga atcacaaccc cgtcaaaggg gaagttgtcc   2400
   ctgcgcgtga ggaagtggag aaagatctca ctttctgtgg actcatcggt ctgtatgacc 2460
   ctccccgacc tgagacggcc ggcgctattg acgagtgcta ccgcgctggg atttcggttc 2520
   acatggtcac gggcgaccac ccgggtactg cgcgagcgat tgcagcgcag gttggcatca 2580
   ttccagccaa catggacagt cttgccaaag atgttgcgga cgccatggtg atgactgcca 2640
   gtcagtttga taagttgaca gacgaggaaa tcgatgcgtt gcctactctg cctgctgtga 2700
   ttgctcggtg cgctcccaac acaaaggttc ggatgattga tgcccttcat cgtcggggtc 2760
   ggtttgcggc aatgactggt gatggagtca atgactcgcc atcgttgaag cgggcagatg 2820
   ttggaattgc tatgggacaa tcggggtccg atgtggccaa agacgcttcg gaactggtgt 2880
   tgactgatga caactttgcg tcgattatca acggaattga ggagggtcgt cgtatttttg 2940
   ataatattca gaaattcgtc ctgcatcttc tggcggaaaa cgtcggtctc gccctcacgc 3000
   ttctgatagg tctgtgtttc aaagatgaca acgggcaatc tgtgtttcct attgctcctg 3060
   tcgaaattct atggatcatc atgatcacct ccgggctccc ggacatggga ctgggtatgg 3120
   agatcgcggc tccggatatc atggaccgtc ctcctcaaag cgtaagtaca tcagtcactc 3180
   tttctgggat catatactaa cgacaacaga aacaaggtat tttcacctgg gaagtcatag 3240
   tcgacaccat ggtctacggc gtctggatgg cggctctctg tctcgcctct ttctcgctcg 3300
   tcctctttgg ttggggagat ggcaacctgg cctcgggctg caacagcgac tacagccccg 3360
   aatgcgacgg tgtgttccgc gcccgagcta cgaccttcgt gtgcatgaca tggttcgctc 3420
   tcttcctcgc ctgggagatg attgatatgc gccgcagctt cttccgcatg cagcccaatt 3480
   ccaagcgcta tttcacccag tggatgtttg acgtctggcg caacaagttt ctgttcagcg 3540
   gcatcatgat tggattcgtc acgaccttcc ccatcctgta catccctgtg atcaatgacg 3600
   ttgtcttcaa gcatgtcggt atctcctggg agtggggtgt ggtgtttgtc gaagcgattc 3660
   ttttcttcgc tggctgcgag gcctggaagt ggtgcaagcg catttatttc aggcacacca 3720
   gccaaaagga aactggcaga gaaagggtac ttcgggactt tagccgttat accaccatga 3780
   gcaggtccga gacccaagca acgggtgacc tcaacgtgga aaagtcgatg gtttagatct 3840
   ggcaataggc caggatagca gcagatggag ggcagggaag cgactgcgtg tatttgcatg 3900
   catctaattg gtggccactg gttttcatta agcagaatgt acatagatat gaatagaagt 3960
   agcgatttat cttttttttc cgcctttcgg tttccccccc ttctgcaggt tcacgagtaa 4020
   ttgactttat cctcactcta gagtctagtt gatatggtat gtctttgtaa atatgcaatc 4080
   agtctcagtt tatcatattc attctgattc actataactg gattatataa ttctgtatgc 4140
   atacttatcg ccttatctaa aaaggcagta atttggcaca tccacacggt tccttgatat 4200
   atactctcgt ccatgggtac atcataggct tcataccctt gatatatatc gggaaccctt 4260
   cagcacaaca gacaagtcaa caacaaactg atgaaaatag attagatagt attacagtac 4320
   tacctatgta gttcta 4336

<210> 193
<211> 3336
<212> DNA
<213> Aspergillus fumigatus

<220>
<223> ATPase; Phylum CEA284.1; clone 4-3-4; contig 4899
      region 472441-476776
      Genomic sequence containing the coding region

<400> 193
atgggtgcgc agaagaagca atcctctgat aactctcagg ctttgtctca gcctgctcat 60
gcgcttcgct atgaggatgt ccttcgtgag ctggccgtcg atccagacca gggtctgacc 120
gtgggggagg cgaagcgaag actccaacaa tatggtccga atgaactgga agggggagag 180
ggtgtttcca ttgtcaagat tgtcattagg cagatcgcaa atgccatgat gctggttagt 240
ctctcccttt tgttagacag acaacgacag acaactgacg acagcaggtc ctgatcatcg 300
ccatggcggt cagtttcggc attcaatcct ggattgaggg tggtgtcatt ggggctgtca 360
ttggtctaaa cattgtggta ggtgtctatc aagactatgc cgcagagaaa acgatggatt 420
cccttcgatc gttgagttct cccaccggaa ctgtcacgcg tgatggcaaa accagtacca 480
ttcctgccaa cgagattgtc cccggggaca tgattgaact caaagttgga gacacggttc 540
ctgcggatct tcggtgcgta aagagttacc agaaaatatc gttcccgttc actaaccgcc 600
gataggctcg ttgatgcaat gaatttcgaa accgacgagg ccctcctgac tggcgagtcg 660
ctgccggtgc agaaagaggt cgataccact tttgacccag acacggaccc tggtgatcgg 720
ttgaatattg cgtatagttc ctccacggtg acgagaggcc gcgctcgggg cgttgttatc 780
agcacgggga tgcaaaccga gattggagcc atcgccgctg ctctccgcgc cagcgactcc 840
aagaggcggc ccgtcaagcg cggtcccgag ggagaaacca agaagcgatg gtacgtgcag 900

gcgtggaccc tgacttgcac ggatgccgtg ggacggttcc tgggaatcaa cgttggcaca 960
ccgctgcagc gcaaactctc gaaactggct ttggcccttt tcgccattgc catcatcttc 1020
gccatcattg tcatgggcgt caatgggttc cgcaatgaca aggaggtcat catctacgca 1080
gtcgcaacag gtcttgcaat gatcccggcc tgcctggtgg tggtcctgac cattaccatg 1140
gcggttggaa ccaagcaaat ggttgaaaga catgtcattg tccggaagct tgattccctt 1200
gaagccctgg gcgctgtgac taacatttgc tcagacaaaa cgggaaccct cacccagggg 1260
cgcatggtcg ccaaacgggc gtggattccc tctgttggca ccttctcggt ggggtcttct 1320
aataacccct tgaacccgga agagggcgac cttagcctgc tgcctgaccc gccggtcaaa 1380
gttggccctg acgctcatgg agagccttcc cggccggagg atctgctcaa ggacaatcct 1440
cttttggagc aatatctgaa cgtggccgcc atggcaaacc ttgcccatgt gcacaggtcc 1500
gagcacaatg aatggcaggc tcgtggtgaa ccgaccgaca ttgcgattca ggtgtttgcc 1560
tcgcgcttta actggggacg cgatcgctgg accaaaggcg agaagcctgt ttggcgccaa 1620
aaagctgaat atccatttga ttctactgtg aaaaagatgt ctgtcatctt taagaacacc 1680
aatgatgatc gggagatgat cttcaccaag ggcgcggtgg aacgtgtcat cgaggcttgc 1740
accaccgtca cctggacagc tggttccgac ccgatcgcct tggacgagaa cataaaggag 1800
gaaatcttgc agaacatgga agctctcgca aaggagggcc tgcgagttct ctgcctcgcc 1860
tgtcgggaga atcacaaccc cgtcaaaggg gaagttgtcc ctgcgcgtga ggaagtggag 1920
aaagatctca ctttctgtgg actcatcggt ctgtatgacc ctccccgacc tgagacggcc 1980
ggcgctattg acgagtgcta ccgcgctggg atttcggttc acatggtcac gggcgaccac 2040
ccgggtactg cgcgagcgat tgcagcgcag gttggcatca ttccagccaa catggacagt 2100
cttgccaaag atgttgcgga cgccatggtg atgactgcca gtcagtttga taagttgaca 2160
gacgaggaaa tcgatgcgtt gcctactctg cctgctgtga ttgctcggtg cgctcccaac 2220
acaaaggttc ggatgattga tgcccttcat cgtcggggtc ggtttgcggc aatgactggt 2280
gatggagtca atgactcgcc atcgttgaag cgggcagatg ttggaattgc tatgggacaa 2340
tcggggtccg atgtggccaa agacgcttcg gaactggtgt tgactgatga caactttgcg 2400
tcgattatca acggaattga ggagggtcgt cgtatttttg ataatattca gaaattcgtc 2460
ctgcatcttc tggcggaaaa cgtcggtctc gccctcacgc ttctgatagg tctgtgtttc 2520
aaagatgaca acgggcaatc tgtgtttcct attgctcctg tcgaaattct atggatcatc 2580
atgatcacct ccgggctccc ggacatggga ctgggtatgg agatcgcggc tccggatatc 2640
atggaccgtc ctcctcaaag cgtaagtaca tcagtcactc tttctgggat catatactaa 2700
cgacaacaga aacaaggtat tttcacctgg gaagtcatag tcgacaccat ggtctacggc 2760
gtctggatgg cggctctctg tctcgcctct ttctcgctcg tcctctttgg ttggggagat 2820
ggcaacctgg cctcgggctg caacagcgac tacagccccg aatgcgacgg tgtgttccgc 2880
gcccgagcta cgaccttcgt gtgcatgaca tggttcgctc tcttcctcgc ctgggagatg 2940
attgatatgc gccgcagctt cttccgcatg cagcccaatt ccaagcgcta tttcacccag 3000
tggatgtttg acgtctggcg caacaagttt ctgttcagcg gcatcatgat tggattcgtc 3060
acgaccttcc ccatcctgta catccctgtg atcaatgacg ttgtcttcaa gcatgtcggt 3120
atctcctggg agtggggtgt ggtgtttgtc gaagcgattc ttttcttcgc tggctgcgag 3180
gcctggaagt ggtgcaagcg catttatttc aggcacacca gccaaaagga aactggcaga 3240
gaaagggtac ttcgggactt tagccgttat accaccatga gcaggtccga gacccaagca 3300
acgggtgacc tcaacgtgga aaagtcgatg gtttag 3336

<210> 194
<211> 3180
<212> DNA
<213> Aspergillus fumigatus

<220>
<223> ATPase; Phylum CEA284.1; clone 4-3-4; contig 4899
      region 472441-476776
      Coding region without introns

<400> 194
atgggtgcgc agaagaagca atcctctgat aactctcagg ctttgtctca gcctgctcat 60
gcgcttcgct atgaggatgt ccttcgtgag ctggccgtcg atccagacca gggtctgacc 120
gtgggggagg cgaagcgaag actccaacaa tatggtccga atgaactgga agggggagag 180
ggtgtttcca ttgtcaagat tgtcattagg cagatcgcaa atgccatgat gctggtcctg 240
atcatcgcca tggcggtcag tttcggcatt caatcctgga ttgagggtgg tgtcattggg 300
gctgtcattg gtctaaacat tgtggtaggt gtctatcaag actatgccgc agagaaaacg 360
atggattccc ttcgatcgtt gagttctccc accggaactg tcacgcgtga tggcaaaacc 420

agtaccattc ctgccaacga gattgtcccc ggggacatga ttgaactcaa agttggagac 480
acggttcctg cggatcttcg gctcgttgat gcaatgaatt tcgaaaccga cgaggccctc 540
ctgactggcg agtcgctgcc ggtgcagaaa gaggtcgata ccacttttga cccagacacg 600
ggacctggtg atcggttgaa tattgcgtat agttcctcca cggtgacgag aggccgcgct 660
cggggcgttg ttatcagcac ggggatgcaa accgagattg gagccatcgc cgctgctctc 720
cgcgccagcg actccaagag gcggcccgtc aagcgcggtc ccgagggaga aaccaagaag 780
cgatggtacg tgcaggcgtg gaccctgact tgcacggatg ccgtgggacg gttcctggga 840
atcaacgttg gcacaccgct gcagcgcaaa ctctcgaaac tggctttggc ccttttcgcc 900
attgccatca tcttcgccat cattgtcatg ggcgtcaatg ggttccgcaa tgacaaggag 960
gtcatcatct acgcagtcgc aacaggtctt gcaatgatcc cggcctgcct ggtggtggtc 1020
ctgaccatta ccatggcggt tggaaccaag caaatggttg aaagacatgt cattgtccgg 1080
aagcttgatt cccttgaagc cctgggcgct gtgactaaca tttgctcaga caaaacggga 1140
accctcaccc aggggcgcat ggtcgccaaa cggcgtggaa ttccctctgt tggcaccttc 1200
tcggtggggt cttctaataa ccccttgaac ccggaagagg gcgaccttag cctgctgcct 1260
gacccgccgg tcaaagttgg ccctgacgct catggagagc cttcccggcc ggaggatctg 1320
ctcaaggaca atcctctttt ggagcaatat ctgaacgtgg ccgccatggc aaaccttgcc 1380
catgtgcaca ggtccgagca caatgaatgg caggctcgtg gtgaaccgac cgacattgcg 1440
attcaggtgt ttgcctcgcg ctttaactgg ggacgcgatc gctggaccaa aggcgagaag 1500
cctgtttggc gccaaaaagc tgaatatcca tttgattcta ctgtgaaaaa gatgtctgtc 1560
atctttaaga acaccaatga tgatcgggag atgatcttca ccaagggcgc ggtggaacgt 1620
gtcatcgagg cttgcaccac cgtcacctgg acagctggtt ccgacccgat cgccttggac 1680
gagaacataa aggaggaaat cttgcagaac atggaagctc tcgcaaagga gggcctgcga 1740
gttctctgcc tcgcctgtcg ggagaatcac aaccccgtca aaggggaagt tgtccctgcg 1800
cgtgaggaag tggagaaaga tctcactttc tgtggactca tcggtctgta tgaccctccc 1860
cgacctgaga cggccggcgc tattgacgag tgctaccgcg ctgggatttc ggttcacatg 1920
gtcacgggcg accacccggg tactgcgcga gcgattgcag cgcaggttgg catcattcca 1980
gccaacatgg acagtcttgc caaagatgtt gcggacgcca tggtgatgac tgccagtcag 2040
tttgataagt tgacagacga ggaaatcgat gcgttgccta ctctgcctgc tgtgattgct 2100
cggtgcgctc ccaacacaaa ggttcggatg attgatgccc ttcatcgtcg ggtcggtttt 2160
gcggcaatga ctggtgatgg agtcaatgac tcgccatcgt tgaagcgggc agatgttgga 2220
attgctatgg gacaatcggg gtccgatgtg gccaaagacg cttcggaact ggtgttgact 2280
gatgacaact ttgcgtcgat tatcaacgga attgaggagg gtcgtcgtat ttttgataat 2340
attcagaaat tcgtcctgca tcttctggcg gaaaacgtcg gtctcgccct cacgcttctg 2400
ataggtctgt gtttcaaaga tgacaacggg caatctgtgt ttcctattgc tcctgtcgaa 2460
attctatgga tcatcatgat cacctccggg ctcccggaca tgggactggg tatggagatc 2520
gcggctccgg atatcatgga ccgtcctcct caaagcgtaa gtattttcac ctgggaagtc 2580
atagtcgaca ccatggtcta cggcgtctgg atggcggctc tctgtctcgc ctctttctcg 2640
ctcgtcctct ttggttgggg agatggcaac ctggcctcgg gctgcaacag cgactacagc 2700
cccgaatgcg acggtgtgtt ccgcgcccga gctacgacct tcgtgtgcat gacatggttc 2760
gctctcttcc tcgcctggga gatgattgat atgcgccgca gcttcttccg catgcagccc 2820
aattccaagc gctatttcac ccagtggatg tttgacgtct ggcgcaacaa gtttctgttc 2880
agcggcatca tgattggatt cgtcacgacc ttccccatcc tgtacatccc tgtgatcaat 2940
gacgttgtct tcaagcatgt cggtatctcc tgggagtggg gtgtggtgtt tgtcgaagcg 3000
attcttttct tcgctggctg cgaggcctgg aagtggtgca agcgcattta tttcaggcac 3060
accagccaaa aggaaactgg cagagaaagg gtacttcggg actttagccg ttataccacc 3120
atgagcaggt ccgagaccca agcaacgggt gacctcaacg tggaaaagtc gatggtttag 3180

<210> 195
<211> 1059
<212> PRT
<213> Aspergillus fumigatus

<220>
<223> ATPase; Phylum CEA284.1; clone 4-3-4; contig 4899
      region 472441-476776
      Protein sequence

<400> 195

Met Gly Ala Gln Lys Lys Gln Ser Ser Asp Asn Ser Gln Ala Leu Ser
1               5                   10                  15

Gln Pro Ala His Ala Leu Arg Tyr Glu Asp Val Leu Arg Glu Leu Ala
            20                  25                  30

Val Asp Pro Asp Gln Gly Leu Thr Val Gly Glu Ala Lys Arg Arg Leu
        35                  40                  45

Gln Gln Tyr Gly Pro Asn Glu Leu Glu Gly Gly Glu Gly Val Ser Ile
    50                  55                  60

Val Lys Ile Val Ile Arg Gln Ile Ala Asn Ala Met Met Leu Val Leu
65                  70                  75                  80

Ile Ile Ala Met Ala Val Ser Phe Gly Ile Gln Ser Trp Ile Glu Gly
                85                  90                  95

Gly Val Ile Gly Ala Val Ile Gly Leu Asn Ile Val Val Gly Val Tyr
            100                 105                 110

Gln Asp Tyr Ala Ala Glu Lys Thr Met Asp Ser Leu Arg Ser Leu Ser
        115                 120                 125

Ser Pro Thr Gly Thr Val Thr Arg Asp Gly Lys Thr Ser Thr Ile Pro
    130                 135                 140

Ala Asn Glu Ile Val Pro Gly Asp Met Ile Glu Leu Lys Val Gly Asp
145                 150                 155                 160

Thr Val Pro Ala Asp Leu Arg Leu Val Asp Ala Met Asn Phe Glu Thr
                165                 170                 175

Asp Glu Ala Leu Leu Thr Gly Glu Ser Leu Pro Val Gln Lys Glu Val
            180                 185                 190

Asp Thr Thr Phe Asp Pro Asp Thr Gly Pro Gly Asp Arg Leu Asn Ile
        195                 200                 205

Ala Tyr Ser Ser Ser Thr Val Thr Arg Gly Arg Ala Arg Gly Val Val
    210                 215                 220

Ile Ser Thr Gly Met Gln Thr Glu Ile Gly Ala Ile Ala Ala Ala Leu
225                 230                 235                 240

Arg Ala Ser Asp Ser Lys Arg Arg Pro Val Lys Arg Gly Pro Glu Gly
                245                 250                 255

Glu Thr Lys Lys Arg Trp Tyr Val Gln Ala Trp Thr Leu Thr Cys Thr
            260                 265                 270

Asp Ala Val Gly Arg Phe Leu Gly Ile Asn Val Gly Thr Pro Leu Gln
        275                 280                 285

Arg Lys Leu Ser Lys Leu Ala Leu Ala Leu Phe Ala Ile Ala Ile Ile
    290                 295                 300

Phe Ala Ile Ile Val Met Gly Val Asn Gly Phe Arg Asn Asp Lys Glu
305                 310                 315                 320

Val Ile Ile Tyr Ala Val Ala Thr Gly Leu Ala Met Ile Pro Ala Cys
                325                 330                 335

Leu Val Val Val Leu Thr Ile Thr Met Ala Val Gly Thr Lys Gln Met
            340                 345                 350

Val Glu Arg His Val Ile Val Arg Lys Leu Asp Ser Leu Glu Ala Leu
        355                 360                 365

Gly Ala Val Thr Asn Ile Cys Ser Asp Lys Thr Gly Thr Leu Thr Gln
    370                 375                 380

Gly Arg Met Val Ala Lys Arg Ala Trp Ile Pro Ser Val Gly Thr Phe
385                 390                 395                 400

Ser Val Gly Ser Ser Asn Asn Pro Leu Asn Pro Glu Glu Gly Asp Leu
                405                 410                 415

Ser Leu Leu Pro Asp Pro Pro Val Lys Val Gly Pro Asp Ala His Gly
            420                 425                 430

Glu Pro Ser Arg Pro Glu Asp Leu Leu Lys Asp Asn Pro Leu Leu Glu
        435                 440                 445

Gln Tyr Leu Asn Val Ala Ala Met Ala Asn Leu Ala His Val His Arg
    450                 455                 460

Ser Glu His Asn Glu Trp Gln Ala Arg Gly Glu Pro Thr Asp Ile Ala
465                 470                 475                 480

Ile Gln Val Phe Ala Ser Arg Phe Asn Trp Gly Arg Asp Arg Trp Thr
                485                 490                 495

Lys Gly Glu Lys Pro Val Trp Arg Gln Lys Ala Glu Tyr Pro Phe Asp
            500                 505                 510

Ser Thr Val Lys Lys Met Ser Val Ile Phe Lys Asn Thr Asn Asp Asp
        515                 520                 525

Arg Glu Met Ile Phe Thr Lys Gly Ala Val Glu Arg Val Ile Glu Ala
    530                 535                 540

Cys Thr Thr Val Thr Trp Thr Ala Gly Ser Asp Pro Ile Ala Leu Asp
545                 550                 555                 560

Glu Asn Ile Lys Glu Glu Ile Leu Gln Asn Met Glu Ala Leu Ala Lys
                565                 570                 575

Glu Gly Leu Arg Val Leu Cys Leu Ala Cys Arg Glu Asn His Asn Pro
            580                 585                 590

Val Lys Gly Glu Val Val Pro Ala Arg Glu Glu Val Glu Lys Asp Leu
        595                 600                 605

Thr Phe Cys Gly Leu Ile Gly Leu Tyr Asp Pro Pro Arg Pro Glu Thr
    610                 615                 620

Ala Gly Ala Ile Asp Glu Cys Tyr Arg Ala Gly Ile Ser Val His Met
625                 630                 635                 640

Val Thr Gly Asp His Pro Gly Thr Ala Arg Ala Ile Ala Ala Gln Val
                645                 650                 655

Gly Ile Ile Pro Ala Asn Met Asp Ser Leu Ala Lys Asp Val Ala Asp

            660                 665                 670

Ala Met Val Met Thr Ala Ser Gln Phe Asp Lys Leu Thr Asp Glu Glu
        675                 680                 685

Ile Asp Ala Leu Pro Thr Leu Pro Ala Val Ile Ala Arg Cys Ala Pro
    690                 695                 700

Asn Thr Lys Val Arg Met Ile Asp Ala Leu His Arg Arg Gly Arg Phe
705                 710                 715                 720

Ala Ala Met Thr Gly Asp Gly Val Asn Asp Ser Pro Ser Leu Lys Arg
                725                 730                 735

Ala Asp Val Gly Ile Ala Met Gly Gln Ser Gly Ser Asp Val Ala Lys
            740                 745                 750

Asp Ala Ser Glu Leu Val Leu Thr Asp Asp Asn Phe Ala Ser Ile Ile
        755                 760                 765

Asn Gly Ile Glu Glu Gly Arg Arg Ile Phe Asp Asn Ile Gln Lys Phe
    770                 775                 780

Val Leu His Leu Leu Ala Glu Asn Val Gly Leu Ala Leu Thr Leu Leu
785                 790                 795                 800

Ile Gly Leu Cys Phe Lys Asp Asp Asn Gly Gln Ser Val Phe Pro Ile
                805                 810                 815

Ala Pro Val Glu Ile Leu Trp Ile Ile Met Ile Thr Ser Gly Leu Pro
            820                 825                 830

Asp Met Gly Leu Gly Met Glu Ile Ala Ala Pro Asp Ile Met Asp Arg
        835                 840                 845

Pro Pro Gln Ser Val Ser Ile Phe Thr Trp Glu Val Ile Val Asp Thr
    850                 855                 860

Met Val Tyr Gly Val Trp Met Ala Ala Leu Cys Leu Ala Ser Phe Ser
865                 870                 875                 880

Leu Val Leu Phe Gly Trp Gly Asp Gly Asn Leu Ala Ser Gly Cys Asn
                885                 890                 895

Ser Asp Tyr Ser Pro Glu Cys Asp Gly Val Phe Arg Ala Arg Ala Thr
            900                 905                 910

Thr Phe Val Cys Met Thr Trp Phe Ala Leu Phe Leu Ala Trp Glu Met
        915                 920                 925

Ile Asp Met Arg Arg Ser Phe Phe Arg Met Gln Pro Asn Ser Lys Arg
    930                 935                 940

Tyr Phe Thr Gln Trp Met Phe Asp Val Trp Arg Asn Lys Phe Leu Phe
945                 950                 955                 960

Ser Gly Ile Met Ile Gly Phe Val Thr Thr Phe Pro Ile Leu Tyr Ile
                965                 970                 975

Pro Val Ile Asn Asp Val Val Phe Lys His Val Gly Ile Ser Trp Glu
            980                 985                 990

Trp Gly Val Val Phe Val Glu Ala Ile Leu Phe Phe Ala Gly Cys Glu
        995                 1000                1005

Ala Trp Lys Trp Cys Lys Arg Ile Tyr Phe Arg His Thr Ser Gln
    1010                1015                1020

Lys Glu Thr Gly Arg Glu Arg Val Leu Arg Asp Phe Ser Arg Tyr
    1025                1030                1035

Thr Thr Met Ser Arg Ser Glu Thr Gln Ala Thr Gly Asp Leu Asn
    1040                1045                1050

Val Glu Lys Ser Met Val
    1055

<210> 196
<211> 2059
<212> DNA
<213> Aspergillus fumigatus

<220>
<223> Homologue GmZnf1; Phylum CEA284.2;
      contig 4899 region 477626-479684
      Genomic sequence containing 3' and 5'-ends and the coding region

<400> 196
cgccgaacta tggggcatat cggtagccaa caccacttta ctatactgtc acgcgagtca      60
taaatcgcag atggcccagt tccagccctg acaagagtgt cacacttgtt ctattacgga     120
atgggccgct tcccgatgta accgcacaag ccgctatctt ccctggaatg ttggcacaag     180
gctacctatt caggcgctca agaatgccga tgcctgaatt cgcagttgac tggcaagttt     240
catgagcgat gtggcttcac ttttggccag aaacattggc tgttccatct cagcgagcag     300
ggtttcaaac ttggatgcct tggtgccttt ccacatgagg ttaagcacca ttggctttgg     360
atattttgag acgcatactg tgctgtggca cagtcagtgc atttgtagcc agtggctgtg     420
ttgtcatggt cgtgaaggtg gattagagtc tgttggttgc tgaagatgta taggagaaaa     480
tagcatataa ggggactgag atgtctgtgg ttgttcttgt ttccaagcca acagcacatt     540
tctcagaatt ggcatctcat cacagagtcc gtgtaattat cacattagca aaagccacaa     600
tgtcccaccc cgatctatcg accattctcg aggtctaccc cgaatgcgaa gtcacctgct     660
acgggtacgc tcccagccaa cggcgccgct gcagaatgcg aaccaggaaa gacaaccgag     720
acagggcttc gtaccttctc gaagagggca ccagatatct tcagcgcggc ctccccgtcg     780
acggtctgct tattgagcta gccccgctag ttctctgcac acgcttccac caataccagg     840
cagacgactt ggtccgggac tggcgggcca agctgcggga gttccagcag cagactcttc     900
tgaatgctat gctgaaatcg ctccaagagc ttgtggacag tcgggcgcgt tcgcgtgctg      960
ctaggtcggc gggtcggcgt ctcccagaaa gagtttcgag tccgacacgg ctggagaggt    1020
cggctgctat tgtaacagag gaagaaccgg ctgctcctga aagagaggaa gaggaggaag    1080
aaagaggaga tagggaggac gagcctgaac cagaacccga acctgaacct gaacttaccc    1140
ccagccggtc atctacggag acttcctcgc cagccgttga ggctcatgtc gcggagccaa    1200
ccgttcctca gactgagtcc agaagagtca ctcgcaaacc aatcgaagga gactgtacta    1260
tctgcctgtg tcctctacga gaacaagaca gtgatgaaaa cggcgaggga tcagaagatc    1320
gcgatgatga gaatgaggat gatgcggcgg gcacagggtc cggcacagcg tccgacgagc    1380
acgacgcccc cgaagaacat gacgatgacg accttgtata ttgcaaaaac cagtgcggta    1440
cgaactatca caaagcctgt attgacgtgt ggcatgctac tcagcgtaca tttgaaactc    1500
cacgtgggga tcctatcggc ctgtcctgcc cgtactgtcg agcagcatgg tcctcctgat    1560
ttcttcattc tgcggggagt ctcggcgttt gttacttatt ttgtgatccc aattccttct    1620
cacgttcttg ttgcaatacg tcagatattt ccttgtacag ttatgcctgg gggtgattat    1680
gagtacataa cggccttgtt cagaattata ttaatagcag tttcctggtt ctgcaaatat    1740
agtcgtgttg gccttcaagc ttgtagctga gataaatgga tacatagatg gctctcttct    1800
atcgtgatga tcattcccac acgttgttcc tctcttcgcc tgtgtctcga tctcttgaat    1860
ctcgcctcga cctcggctcc agctagaagc atcgaataca atagctcctg acaatggact    1920
gttctctgaa ggatgagtgg tctcaatgaa tgtgaccgct tgccgcgatt ggcctcgaag    1980

acttaaagga gcaggcttct tttctgggca gagcggagtg aagcttctga accgcctcag 2040
gcaccgcgaa cctggagct 2059

<210> 197
<211> 1059
<212> DNA
<213> Aspergillus fumigatus

<220>
<223> Homologue GmZnf1; Phylum CEA284.2;
      contig 4899 region 477626-479684
      Genomic sequence containing the coding region

<400> 197
atgtctgtgg ttgttcttgt ttccaagcca acagcacatt tctcagaatt ggcatctcat 60
cacagagtcc gtgtaattat cacattagca aaagccacaa tgtcccaccc cgatctatcg 120
accattctcg aggtctaccc cgaatgcgaa gtcacctgct acgggtacgc tcccagccaa 180
cggcgccgct gcagaatgcg aaccaggaaa gacaaccgag acagggcttc gtaccttctc 240
gaagagggca ccagatatct tcagcgcggc ctccccgtcg acggtctgct tattgagcta 300
gccccgctag ttctctgcac acgcttccac caataccagg cagacgactt ggtccgggac 360
tggcgggcca agctgcggga gttccagcag cagactcttc tgaatgctat gctgaaatcg 420
ctccaagagc ttgtggacag tcgggcgcgt tcgcgtgctg ctaggtcggc gggtcggcgt 480
ctcccagaaa gagtttcgag tccgacacgg ctggagaggt cggctgctat tgtaacagag 540
gaagaaccgg ctgctcctga aagagaggaa gaggaggaag aaagaggaga tagggaggac 600
gagcctgaac cagaacccga acctgaacct gaacttaccc ccagccggtc atctacggag 660
acttcctcgc cagccgttga ggctcatgtc gcggagccaa ccgttcctca gactgagtcc 720
agaagagtca ctcgcaaacc aatcgaagga gactgtacta tctgcctgtg tcctctacga 780
gaacaagaca gtgatgaaaa cggcgaggga tcagaagatc gcgatgatga gaatgaggat 840
gatgcggcgg gcacagggtc cggcacagcg tccgacgagc acgacgcccc cgaagaacat 900
gacgatgacg accttgtata ttgcaaaaac cagtgcggta cgaactatca caaagcctgt 960
attgacgtgt ggcatgctac tcagcgtaca tttgaaactc cacgtgggga tcctatcggc 1020
ctgtcctgcc cgtactgtcg agcagcatgg tcctcctga 1059

<210> 198
<211> 1059
<212> DNA
<213> Aspergillus fumigatus

<220>
<223> Homologue GmZnf1; Phylum CEA284.2;
      contig 4899 region 477626-479684
      Coding region without introns

<400> 198
atgtctgtgg ttgttcttgt ttccaagcca acagcacatt tctcagaatt ggcatctcat 60
cacagagtcc gtgtaattat cacattagca aaagccacaa tgtcccaccc cgatctatcg 120
accattctcg aggtctaccc cgaatgcgaa gtcacctgct acgggtacgc tcccagccaa 180
cggcgccgct gcagaatgcg aaccaggaaa gacaaccgag acagggcttc gtaccttctc 240
gaagagggca ccagatatct tcagcgcggc ctccccgtcg acggtctgct tattgagcta 300
gccccgctag ttctctgcac acgcttccac caataccagg cagacgactt ggtccgggac 360
tggcgggcca agctgcggga gttccagcag cagactcttc tgaatgctat gctgaaatcg 420
ctccaagagc ttgtggacag tcgggcgcgt tcgcgtgctg ctaggtcggc gggtcggcgt 480
ctcccagaaa gagtttcgag tccgacacgg ctggagaggt cggctgctat tgtaacagag 540
gaagaaccgg ctgctcctga aagagaggaa gaggaggaag aaagaggaga tagggaggac 600
gagcctgaac cagaacccga acctgaacct gaacttaccc ccagccggtc atctacggag 660
acttcctcgc cagccgttga ggctcatgtc gcggagccaa ccgttcctca gactgagtcc 720
agaagagtca ctcgcaaacc aatcgaagga gactgtacta tctgcctgtg tcctctacga 780
gaacaagaca gtgatgaaaa cggcgaggga tcagaagatc gcgatgatga gaatgaggat 840
gatgcggcgg gcacagggtc cggcacagcg tccgacgagc acgacgcccc cgaagaacat 900

gacgatgacg accttgtata ttgcaaaaac cagtgcggta cgaactatca caaagcctgt     960
attgacgtgt ggcatgctac tcagcgtaca tttgaaactc cacgtgggga tcctatcggc    1020
ctgtcctgcc cgtactgtcg agcagcatgg tcctcctga                           1059

<210> 199
<211> 352
<212> PRT
<213> Aspergillus fumigatus

<220>
<223> Homologue GmZnf1; Phylum CEA284.2;
      contig 4899 region 477626-479684
      Protein sequence

<400> 199

Met Ser Val Val Val Leu Val Ser Lys Pro Thr Ala His Phe Ser Glu
1               5                   10                  15

Leu Ala Ser His His Arg Val Arg Val Ile Ile Thr Leu Ala Lys Ala
            20                  25                  30

Thr Met Ser His Pro Asp Leu Ser Thr Ile Leu Glu Val Tyr Pro Glu
        35                  40                  45

Cys Glu Val Thr Cys Tyr Gly Tyr Ala Pro Ser Gln Arg Arg Arg Cys
    50                  55                  60

Arg Met Arg Thr Arg Lys Asp Asn Arg Asp Arg Ala Ser Tyr Leu Leu
65                  70                  75                  80

Glu Glu Gly Thr Arg Tyr Leu Gln Arg Gly Leu Pro Val Asp Gly Leu
                85                  90                  95

Leu Ile Glu Leu Ala Pro Leu Val Leu Cys Thr Arg Phe His Gln Tyr
            100                 105                 110

Gln Ala Asp Asp Leu Val Arg Asp Trp Arg Ala Lys Leu Arg Glu Phe
        115                 120                 125

Gln Gln Gln Thr Leu Leu Asn Ala Met Leu Lys Ser Leu Gln Glu Leu
    130                 135                 140

Val Asp Ser Arg Ala Arg Ser Arg Ala Ala Arg Ser Ala Gly Arg Arg
145                 150                 155                 160

Leu Pro Glu Arg Val Ser Ser Pro Thr Arg Leu Glu Arg Ser Ala Ala
                165                 170                 175

Ile Val Thr Glu Glu Glu Pro Ala Ala Pro Glu Arg Glu Glu Glu Glu
            180                 185                 190

Glu Glu Arg Gly Asp Arg Glu Asp Glu Pro Glu Pro Glu Pro Glu Pro
        195                 200                 205

Glu Pro Glu Leu Thr Pro Ser Arg Ser Ser Thr Glu Thr Ser Ser Pro
    210                 215                 220

Ala Val Glu Ala His Val Ala Glu Pro Thr Val Pro Gln Thr Glu Ser
225                 230                 235                 240

Arg Arg Val Thr Arg Lys Pro Ile Glu Gly Asp Cys Thr Ile Cys Leu
                245                 250                 255

Cys Pro Leu Arg Glu Gln Asp Ser Asp Glu Asn Gly Glu Gly Ser Glu
            260                 265                 270

Asp Arg Asp Asp Glu Asn Glu Asp Asp Ala Ala Gly Thr Gly Ser Gly
        275                 280                 285

Thr Ala Ser Asp Glu His Asp Ala Pro Glu Glu His Asp Asp Asp Asp
    290                 295                 300

Leu Val Tyr Cys Lys Asn Gln Cys Gly Thr Asn Tyr His Lys Ala Cys
305                 310                 315                 320

Ile Asp Val Trp His Ala Thr Gln Arg Thr Phe Glu Thr Pro Arg Gly
                325                 330                 335

Asp Pro Ile Gly Leu Ser Cys Pro Tyr Cys Arg Ala Ala Trp Ser Ser
            340                 345                 350

## Description

The present invention is directed to polynucleotides encoding proteins Essential For the Growth (EFG) of filamentous fungi. The invention also deals with namely polypeptides encoded by said polynucleotides, screening assays for identifying compounds capable of inhibiting said EFG protein activities, pharmaceutical or phytosanitary compositions comprising such compounds.

### BACKGROUND

The opportunistic pathogen *Aspergillus fumigatus* is the cause of the most frequent deadly airborne fungal infection in developed countries. In order to identify novel antifungal drug targets, the inventors investigated the genome of *A. fumigatus* for genes that are necessary for efficient fungal growth.

*Aspergillus fumigatus* is a saprophytic filamentous fungus that disseminates through the release of asexual spores (conidia) into air^{1,2}. They are daily inhaled without major consequences for human health. However, in immuno-compromised hosts, *A. fumigatus* can cause a usually fatal infection, termed invasive pulmonary aspergillosis^{1,3} (IPA). With the increasing number of immuno-deficient patients and the development of severe immuno-suppressive therapies, *A. fumigatus* has become the most prevalent airborne fungal pathogen⁴. Because of a difficult diagnosis during lifetime and the lack of non-toxic efficient antifungal treatments, IPA is associated with a mortality rate as high as 85%.

Currently available drugs belong to two families: polyenes (e.g. amphotericin B) and azoles (e.g. itraconazole), both of them targeting fungal membranes^{1,5}. Relative toxicity and side effects, in addition to an often-late diagnostic, limit their use^{1,5}. Recently, new antifungal compounds of the candin family (caspofungin) which target the enzyme responsible for cell wall β(1,3)-glucan biosynthesis came on the market⁶.

Besides, *A. fumigatus* is closely related to phytopathogenic fungis. There is a high need of new fungicidal compounds against such fungi. Thus, EFG fungal genes identified by the inventors have a strong utility in the phytopathology field : the identified EFG genes are useful for identifying new fungicidal compositions in screening assays. Knowing the EFG described further, homologous genes of *A. fumigatus* can be isolated from other fungi, namely: *Botrytis cinerea, Mycosphaerella graminicola, Stagnospora nodorum, Blumeria graminis, Colleotrichum lindemuthianum, Puccincia graminis, Leptosphaeria maculans, Fusarium oxysporum, Fusarium graminearum, Venturia inaequalis,* most preferably *Magnaporthe* fungi, even more preferably *Magnaporthe grisea.*

A rational approach to increase the antifungal arsenal relies on the identification of novel targets involved in various aspects of the fungal biology^{7,8}. Although genes necessary for virulence are seen as potential candidates⁹, no genuine virulence factor has been identified in *A. fumigatus* yet^{2,10}. On the other hand, studies which have addressed the role of proposed virulence factors, e.g. adhesins, toxic secondary metabolites or secreted proteases, by direct mutagenesis have only identified genes involved in melanin biosynthesis necessary for conidia pigmentation as important for virulence^{2,37,38,39}. Therefore, it appears that the search for *A. fumigatus* virulence factors has only identified genes that protect conidia from the host response (pigment biosynthesis prevents complement binding and phagocytosis) and metabolic pathway genes (reduced level of an essential nutrient at the site of infection).

Alternative attractive antifungal targets lie among gene products that are essential for fungal growth *ex vivo*^{11,12}. Compendia of essential genes have been obtained for *Saccharomyces cerevisiae* through various approaches including systematic gene inactivation or insertional mutagenesis in a diploid background followed by the analysis of meiotic progenies^{13,14}. More recently, a set of genes critical for growth of the dimorphic yeast *Candida albicans* has also been defined using inducible expression of antisense RNA molecules¹⁵. Among the 86 *C. albicans* genes identified, 38% have no known homologues in available databases¹⁵. Differences in essential biological processes between the yeasts *S. cerevisiae* and *C. albicans* highlight the need to study the larger and more complex filamentous fungal genomes to reveal species-specific and filamentous-specific targets.

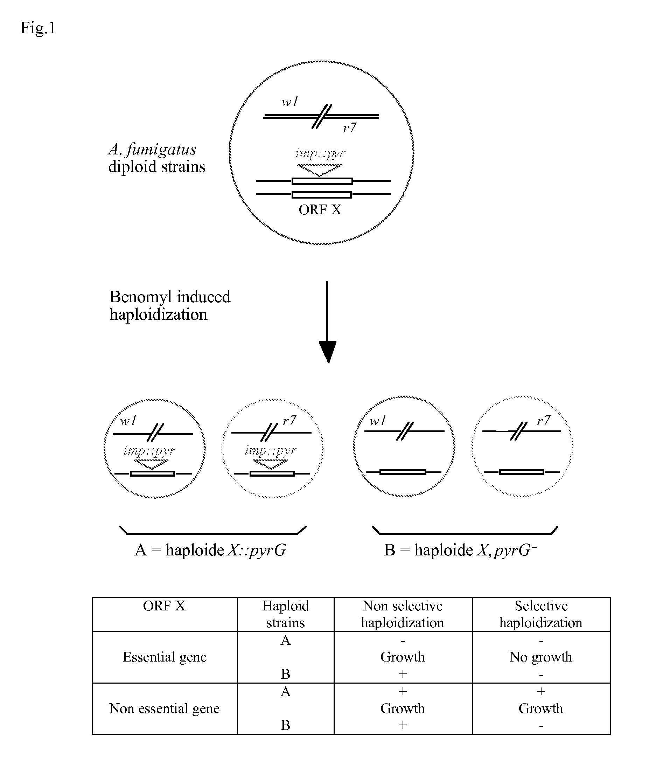

*A. fumigatus* is haploid and devoid of a sexual cycle¹⁶, preventing the application of strategies that use classical genetics to define essential genes. The inventors have now demonstrated that the parasexual genetic cycle can be used to demonstrate the essential function of *A. fumigatus* genes. The inventors have used techniques of chemical haploidization of artificial diploid strains^{17,18}. In this setting, a heterozygous *A. fumigatus* diploid is generated by targeted gene replacement or by random insertional mutagenesis and subjected to haploidization with or without the selective pressure corresponding to the introduced mutation. The absence of haploid progenies under selective condition only is indicative of the inactivation of a gene essential for *A. fumigatus* growth (Fig. 1). Using this approach, the inventors have demonstrated that the *FKS1* gene, encoding the 1,3-β-D-glucan synthase catalytic subunit, and the *smcA* gene, encoding a member of the SMC (structural maintenance of chromosome) protein family, are essential for *A. fumigatus* growth. However, their results have shown that the currently used insertional mutagenesis schemes for *A. fumigatus* which rely on integration of a heterologous DNA molecule by DNA-mediated transformation^{19,20} lead to frequent genomic rearrangements that hamper a high-throughput analysis (data not shown). So there was a need for new techniques allowing a reliable identification of EFG genes.

Transposon mutagenesis has been used widely in bacteria^{21,22} and yeasts^{23,24} to elucidate various biological questions but it was only very recently that it has been applied to the filamentous fungus kingdom^{25,26}. In particular, the *impala160* transposable element from *Fusarium oxysporum,* a Class II transposable element of the *Tcl-mariner* family²⁷, has been shown to transpose efficiently in *Fusarium* species²⁸, *A. nidulans*²⁹ and *Magnaporthe grisea*³⁰. However, transposon mutagenesis has not been used yet for a reliable identification of genes essential for growth of *Aspergillus* fungi, especially *A. fumigatus.* Such identification needs appropriated protocol settings and is absolutely not obvious, practically speaking. Furthermore, as it will be shown, the EFG genes of *A. fumigatus* identified by the inventors were until now not known, and some of them are surprisingly totally specific for Aspergilli or for filamentous ascomycetes, being not found in ascomycetous yeasts. The new EFG genes from *A. fumigatus* described in this application are as such neither described nor suggested in the prior art.

### SUMMARY OF THE INVENTION

The inventors have succeeded in the identification of EFG genes, by using an *in* vivo transposon mutagenesis system for *A. fumigatus.* The inventors have shown that *impala160* (see FR 2 791 361) and its derivatives also transpose *in A. fumigatus* and can be used to generate a collection of random heterozygous diploids. Screening by parasexual genetics of such a collection has resulted in the complete characterization without prior sequence information of 210 new *A. fumigatus* genes which are necessary for efficient fungal growth.

The present invention thus pertains, according to a first aspect, to nucleic acid molecules, including in particular the complete cDNA sequence, encoding the EFG protein, as well as the corresponding translation product. Oligonucleotide probes or primers hybridizing specifically with a EFG genomic DNA or cDNA sequence are also part of the present invention, as well as DNA amplification and detection methods using said primers and probes.

A further aspect of the invention consists of recombinant vectors comprising any of the nucleic acid sequences described above, and in particular of recombinant vectors comprising a EFG regulatory sequence or a sequence encoding a EFG protein, as well as of cell hosts comprising said nucleic acid sequences or recombinant vectors.

The invention is also directed to methods for the screening of substances or molecules that inhibit the expression of the EFG genes, as well as with methods for the screening of substances or molecules that interact with and/or inhibit the activity of a EFG polypeptide.

Another object of the invention is to develop new compositions, either pharmaceutical or phytosanitarical, capable of inhibiting or preferably completely suppressing the toxic effect of filamentous fungi.

More precisely, the invention relates, according to a first aspect, to a nucleic acid encoding an Essential For Growth (EFG) polypeptide selected from the group consisting of :
(i) a nucleic acid molecule encoding a polypeptide comprising the amino acid sequence depicted in one of SEQ ID N°3,6,9,12,15,18,21,24,27,30,33,36,39,42,45,48,51,54,57,60,94,98,102,106,110,114, 118,122,126,13 0,134,13 8,142,146,150,154,158,162,166,170 ;
(ii) a nucleic acid molecule comprising the nucleic acid sequence as depicted in one of SEQ ID N°1,4,7,10,13,16,19,22,25,28,31,34,37,40,43,46,49,52,55,58,92,96,100,104,108,112, 116,120,124,128,132,136,140,144,148,152,156,160,164,168 ;
(iii) a nucleic sequence having at least 80, 85, 90, 95, 98, 99% identity with a sequence of SEQ ID N°1,4,7,10,13,16,19,22,25,28,31,34,37,40,43,46,49,52,55,58,92,96,100,104,108,112, 116,120,124,128,132,136,140,144,148,152,156,160,164,168 ;
(iv) a nucleic acid molecule which hybridizes under stringent conditions to
   (a) a nucleic acid as defined in (i), (ii) and (iii), or
   (b) a complementary strand of (a) ;
(v) a nucleic acid the sequence of which is degenerated as a result of the genetic code to the sequence of a nucleic acid as defined in (i), (ii), (iii) and (iv).

The invention also relates to an isolated nucleic acid, said nucleic acid comprising a nucleotide sequence encoding:
i) a EFG polypeptide comprising an amino acid sequence having at least 80% identity to a sequence of SEQ ID N°3,6,9,12,15,18,21,24,27,30,33,36,39,42,45,48,51,54,57,60,94,98,10 2,106,110,114,118,122,126,130,134,138,142,146,150,154,158,162,16 6,170 ; or
ii) a biologically active fragment of said polypeptide.

The invention also relates to an isolated nucleic acid, said nucleic acid comprising a nucleotide sequence encoding:
i) a EFG polypeptide comprising an amino acid sequence which is orthologous to a sequence of SEQ ID N°3,6,9,12,15,18,21,24,27,30,33,36,39,42,45,48,51,54,57,60,94,98,10 2,106,110,114,118,122,126,130,134,13 8,142,146,150,154,158,162,16 6,170; or
ii) a biologically active fragment of said polypeptide.

The invention also relates to an isolated nucleic acid sequence mentioned above encoding a polypeptide of *A. fumigatus* exhibiting a biological function associated to fungal growth, said nucleic acid comprising a sequence of SEQ ID N°2,5,8,14,17,20,23,26,29,32,35,38,41,44,47,50,53,56,59,93,97,101,105,109,113,11 7,121,125,129,133,137,141,145,149,153,157,161,165,169. The sequences of SEQ ID N°2,5,8,14,17,20,23,26,29,32,35,38,41,44,47,50,53,56,59,93,97,101,105,109,113,11 7,121,125,129,133,137,141,145,149,153,157,161,165,169 are issued respectively from the sequences of SEQ ID N°1,4,7,10,13,16,19,22,25,28,31,34,37,40,43,46,49,52,55,58,92,96,100,104,108,112, 116,120,124,128,132,136,140,144,148,152,156,160,164,168, and are also defined as ORF N°2,5,8,14,17,20,23,26,29,32,35,38,41,44,47,50,53,56,59,93,97,101,105,109,113,11 7,121,125,129,133,137,141,145,149,153,157,161,165,169. For instance SEQ ID N°2 is ORF N°2, is issued from SEQ ID N°1, and encodes for the protein of SEQ ID N°3 ; SEQ ID N°5 is ORF N°5, is issued from SEQ ID N°4, and encodes for the protein of SEQ ID N°6.

ORF (open reading frame) are representative fragments of the EFG genes of the invention, between a start codon and a stop codon or between two stop codons encoding the EFG polypeptides of the invention.

The biological function associated to fungal growth is preferably chosen in the group consisting of protein synthesis, protein maturation, protein transport, nuclear architecture, RNA processing, nucleotide metabolism, chromatine structure, cell cycle control.

The invention also relates to said nucleic acid operably linked to a promoter, to an expression cassette comprising said nucleic acid, to a host cell comprising said expression cassette.

According to another aspect the invention relates to a biologically active polypeptide encoded by a nucleic acid described above.

The invention also relates to a polypeptide comprising an amino acid sequence of at least 80% amino acid sequence identity to a sequence of SEQ ID N°3,6,9,12,15,18,21,24,27,30,33,36,39,42,45,48,51,54,57,60,94,98,102,106,110,114, 118,122,126,13 0,134,13 8,142,146,150,154,15 8,162,166,170.

According to a further aspect, the invention relates to a method of identifying a candidate inhibitor of EFG polypeptide, said method comprising:
i) contacting a EFG polypeptide according to claim 5 or 6 with a test compound ;
ii) determining whether said compound selectively binds to said polypeptide, said binding indicating that said compound is a candidate inhibitor.

The invention also relates to a method of identifying a candidate inhibitor of EFG polypeptide, said method comprising:
a) contacting said polypeptide with a test compound;
b) determining whether said compound selectively inhibits the activity of said polypeptide, said inhibition indicating that said compound is a candidate inhibitor.

According to a further aspect, the invention relates to a method for locating at least one gene essential for the growth of a haploid fungus, said method comprising the following successive steps:
- generation of diploid strain from haploid fungal strain ;
- mutagenesis of said diploid strain;
- haploidisation of the diploid transformant strain, in selection conditions such that the absence of haploid progeny is indicative of mutagenesis occuring in said essential gene;
said mutagenesis being *an in vivo* transposon mutagenesis.

Preferably, the fungus belongs to the *Aspergillus* genus or the *Penicillium* genus. Preferably, the fungus is *Aspergillus fumigatus.*

Preferably, the transposon is the *impala160* transposon or its derivatives, the selection medium is a benomyl-containing medium, the transposon *impala160* is carried by a plasmid pNIpyr, the diploid strain is chosen between CEA225, CEA 226, CEA 227, and CEA 280:
- strain DH5α (pNIpyr): this strain (CNCM I-2815) is derivated from strain DH5α of *E. coli* K-12 transformed by a derivative of pBR322 carrying the *impala160* transposon of *Fusarium oxysporum,* in which has been inserted the *pyrG* gene of *Aspergillus nidulans;* transformed in a niaD- pyrG- of *Aspergillus fumigatus,* this plasmid confers prototrophy to uridin and uracil, and allows to select transposition of *impala160::pyr* by growth in a medium that contains nitrate as the sole nitrogen source.
- CEA 225 (CNCM I-2816), CEA 226 (CNCM I-2817), CEA 227 (CNCM I-2818), and CEA 280 are diploid strains of *Aspergillus fumigatus* derivated from the strain CBS 144-89 by gene transformation, spontaneous mutagenesis, cross and transformation by plasmid pNIpyr. Genotype: pyrG1/pyrG1 w1/F2/+ +/r7F1 cnx1/+ niaD1/niaD2 X/X::pNipyr.

The invention also provides a method for locating at least one gene essential for the growth of a fungus of the *Penicillium* genus which exhibits a parasexual cycle and the diploids of which are stable.

Further, the heterozygous diploid strains are useful tools for direct screening of active molecules against *A. fumigatus.* Accordingly, the invention provides a method of screening of compounds that are active against *A. fumigatus* comprising:
- preparing an *A. fumigatus* strain that is heterozygous for an EFG gene (heterozygous EFGn/efgn) ;
- preparing an *A. fumigatus* strain that is homozygous for the EFG gene (homozygous EFGn/EFGn) ;
- comparing the effect of a candidate compound on the heterozygous EFGn/efgn and on the homozygous EFGn/EFGn,
the higher inhibiting effect on the heterozygous EFGn/efgn than on the homozygous EFGn/EFGn indicating that the compound is an inhibitor.

This method involves typically the comparison for one EFG gene (for instance for EFG1 gene (n=1), comparison between EFG1/efg1 strain and EFG1/EFG1 strain).
Thus, a population of heterozygous diploid *A. fumigatus* G/g::impala can be screened in order to identify one or more strains that are more sensitive to a fungicidal compound which action mechanism is unknown : the characterization of the *gene G* will allow to identify the action mechanism of said fungicidal compound.

According to a further aspect, the invention relates to an isolated nucleic acid sequence described above, obtainable by a method of locating described above.

According to a further aspect, the invention relates to a composition capable of inhibiting haploid fungal growth, said composition comprising at least onecompound capable of inhibiting the expression of at least one EFG gene the nucleic acid sequence of which is described above.
The composition is typically either pharmaceutical or fungicidal.

### BRIEF DESCRIPTION OF THE SEQUENCE LISTING

SEQ ID N°1,4,7,10,13,16,19,22,25,28,31,34,37,40,43,46,49,52,55,58,92,96,100,104,108,112, 116,120,124,128,132,136,140,144,148,152,156,160,164,168 are cDNA sequences encoding *Aspergillus fumigatus* EFG protein. In the whole application, the expression "SEQ ID N°1,4,7,10,13,16,19,22,25,28,31,34,37,40,43,46,49,52,55,58,92,96,100,104,108,112, 116,120,124,128,132,136,140,144,148,152,156,160,164,168" means the group consisting of SEQ ID N°1, SEQ ID N°4, SEQ ID N°7, SEQ ID N°10, SEQ ID N°13, SEQ ID N°16, SEQ ID N°19, SEQ ID N°22, SEQ ID N°25, SEQ ID N°28, SEQ ID N°31, SEQ ID N°34, SEQ ID N°37, SEQ ID N°40, SEQ ID N°43, SEQ ID N°46, SEQ ID N°49, SEQ ID N°52, of SEQ ID N°55, SEQ ID N°58, SEQ ID N°92, SEQ ID N°96, SEQ ID N°100, SEQ ID N°104, SEQ ID N°108, SEQ ID N°112, SEQ ID N°116, SEQ ID N°120, SEQ ID N°124, SEQ ID N°128, SEQ ID N°132, SEQ ID N°136, SEQ ID N°140, SEQ ID N°144, SEQ ID N°148, SEQ ID N°152, SEQ ID N°156, SEQ ID N°160, of SEQ ID N°164, SEQ ID N°168.

SEQ ID N°3,6,9,12,15,18,21,24,27,30,33,36,39,42,45,48,51,54,57,60,94,98,102,106,110,114, 118,122,126,130,134,138,142,146,150,154,158,162,166,170 are the amino acid sequences of *Aspergillus fumigatus* EFG polypeptides. In the whole application, the expression "SEQ ID N°3,6,9,12,15,18,21,24,27,30,33,36,39,42,45,48,51,54,57,60,94,98,102,106,110,114, 118,122,126,130,134,138,142,146,150,154,158,162,166,170" means the group consisting of SEQ ID N°3, SEQ ID N°6, SEQ ID N°9, SEQ ID N°12, SEQ ID N°15, SEQ ID N°18, SEQ ID N°21, SEQ ID N°24, SEQ ID N°27, SEQ ID N°30, SEQ ID N°33, SEQ ID N°36, SEQ ID N°39, SEQ ID N°42, SEQ ID N°45, SEQ ID N°48, SEQ ID N°51, SEQ ID N°54, of SEQ ID N°57, SEQ ID N°60, SEQ ID N°94, SEQ ID N°98, SEQ ID N°102, SEQ ID N°106, SEQ ID N°110, SEQ ID N°114, SEQ ID N°118, SEQ ID N°122, SEQ ID N°126, SEQ ID N°130, SEQ ID N°134, SEQ ID N°138, SEQ ID N°142, SEQ ID N°146, SEQ ID N°150, SEQ ID N°154, SEQ ID N°158, SEQ ID N°162, of SEQ ID N°166, SEQ ID N°170.

An analogous construction is to be applied when having the expression "SEQ ID N°2,5,8,14,17,20,23,26,29,32,35,38,41,44,47,50,53,56,59,93,97,101,105,109,113,11 7,121,125,129,133,137,141,145,149,153,157,161,165,169".
**Table 1.** *A. fumigatus* essential genes^{a a}For each identified integration of *impala160::pyrG,* the corresponding TIGR contig number is indicated (www.tigr.org) with its length (kb) and the position of the TA where transposon integration occur (bp).

**Table 1**

| | | *A. fumigatus* genome sequence version of November 2001 | | | | | | *A. fumigatus* genome sequence version of February 2003 | | | | | | | |
|---|---|---|---|---|---|---|---|---|---|---|---|---|---|---|---|
| Clone Id | Strain Id | Nucleic acid SEQ ID | ORF SEQ_ID | Amino acid SEQ_ID | Contig TIGR n° | Contig length (kb) | impala160::p yrG location | gene SEQ_ID | Nucleic acid SEQ ID | ORF SEQ ID | Amino acid SEQ ID | Contig TIGR | Contig length n° (kb) | Coordinates of gene SEQ ID on contig | impala160::p yrG location |
| 10-80 | CEA231 | 1 | 2 | 3 | 131 | 27.2 | 15399 | 103 | 104 | 105 | 106 | 4940 | 205712 | 54154-50350 | 53934 |
| 10-291 | CEA233 | 4 | 5 | 6 | 164 | 45.3 | 40601 | 111 | 112 | 113 | 114 | 4865 | 598154 | 3495-6359 | 4705 |
| 7-1-19 | CEA254 | 7 | 8 | 9 | 43 | 93.1 | 72592 | 119 | 120 | 121 | 122 | 4911 | 85632 | 43163-41221 | 42151 |
| 10-3-7 | CEA255 | 10 | 11 | 12 | 408 | 71.1 | 53190 | 123 | 124 | 125 | 126 | 4899 | 1041326 | 441274-438167 | 440657 |
| 2-6-4 | CEA256 | 13 | 14 | 15 | 110 | 207.8 | 105657 | 127 | 128 | 129 | 130 | 4938 | 1796676 | 582107-579544 | 581561 |
| 2-1-1 | CEA257 | 16 | 17 | 18 | 493 | 42.2 | 14886 | 131 | 132 | 133 | 134 | 4951 | 825238 | 8362-11737 | 11010 |
| 2-10-16 | CEA258 | 19 | 20 | 21 | 190 | 22.1 | 7131 | 135 | 136 | 137 | 138 | 4912 | 206341 | 46084-42446 | 43492 |
| 5-4-21 | CEA259 | 22 | 23 | 24 | 1327 | 4.7 | 2928 | 139 | 140 | 141 | 142 | 4963 | 622595 | 373462-376145 | 375531 |
| 2-10-21 | EA260 | 25 | 26 | 27 | 493 | 42.8 | 36145 | 143 | 144 | 145 | 146 | 4849 | 22910 | 12560-15101 | 13405 |
| 7-5-9 | CEA261 | 28 | 29 | 30 | 93 | 53.7 | 11506 | 147 | 148 | 149 | 150 | 4857 | 593293 | 164191-165827 | 164994 |
| 10-2-18 | CEA262 | 31 | 32 | 33 | 1366 | 3.6 | 1870 | 151 | 152 | 153 | 154 | 4903 | 416417 | 3571-1535 | 3157 |
| 9-11 | CEA230 | 34 | 35 | 36 | 1754 | 6.3 | 603 | 99 | 100 | 101 | 102 | 4899 | 1041326 | 9642-7242 | 7285 |
| 4-3-3 | CEA263 | 37 | 38 | 39 | 838 | 9.8 | 8261 | 155 | 156 | 157 | 158 | 4944 | 310661 | 159432-161250 | 159931 |
| 11-6-11 | CEA264 | 40 | 41 | 42 | 846 | 16.7 | 3278 | 159 | 160 | 161 | 162 | 4899 | 1041326 | 65039-62439 | 64261 |
| 8-47 | CEA228 | 43 | 44 | 45 | 960 | 5.1 | 223 | 91 | 92 | 93 | 94 | 4842 | 368858 | 234347-231296 | 234245 |
| 10-304 | CEA234 | 46 | 47 | 48 | 408 | 71.1 | 55903 | 115 | 116 | 117 | 118 | 4899 | 1041326 | 443110- 444619 | 443430 |
| 10-175 | CEA232 | 49 | 50 | 51 | 221 | 76.1 | 67069 | 107 | 108 | 109 | 110 | 4938 | 1796676 | 211008- 213420 | 211352 |
| 11-4-9 | CEA265 | 52 | 53 | 54 | 573 | 36.5 | 13244 | 163 | 164 | 165 | 166 | 4826 | 811005 | 355652-358190 | 356111 |
| | | | | | | | | | | | | | | | |
| 2-10-18 | CEA266 | 55 | 56 | 57 | 585 | 39.5 | 24940 | 167 | 168 | 169 | 170 | 4898 | 422562 | 329309-331987 | 330474 |
| 8-62 | CEA229 | 58 | 59 | 60 | 221 | 76.1 | 60969 | 95 | 96 | 97 | 98 | 4938 | 1796676 | 215653-219466 | 217453 |
| 6-8-13 | CEA280 | | | | 6 | 212.7 | 208370 | 171 | 172 | 173 | 174 | 4925 | 1085193 | 997952-996381 | 997591 |
| | | | | | | | | | | | | | | | |
| *5-3-11* | *CEA281. 1* | | | | *792* | *13.7* | *1353* | *175* | *176* | *177* | *178* | *4839* | *130518* | 10030-12622 | *12332* |
| *5-3-11* | *CEA281. 2* | | | | *792* | *13.7* | *1353* | *179* | *180* | *181* | *182* | *4839* | *130518* | 12269-14135 | *12332* |
| *10-4-20* | *CEA282. 1* | | | | *443* | *89.1* | *67662* | *183* | *184* | *185* | *186* | *4929* | *586561* | 328110- 325663 | *328147* |
| *10-4-20* | *CEA282. 2* | | | | *443* | *89.1* | *67662* | *187* | *188* | *189* | *190* | *4929* | *586561* | 328075- 330267 | *328147* |
| *11-6-20* | *CEA283* | | | | *716* | *14.3* | *9480* | *191* | | | | *4910* | *185565* | 9638-11637 | *10637* |
| *4-3-4* | *CEA284. 1* | | | | *652* | *29.4* | *18411* | *192* | *193* | *194* | *195* | *4899* | *1041326* | 472441-476776 | *476988* |
| *4-3-4* | *CEA284. 2* | | | | *652* | *29.4* | *18411* | *196* | *197* | *198* | *199* | *4899* | *1041326* | 477626-479684 | *476988* |

**Table 1 bis**

| Clone Id | Protein Id | March 2002 Amino acid SEQ ID | March 2003 Amino acid SEQ ID | Size introns +exons (nt) | Size protein (aa) | Location of imp160::pyr G relative to A in start codon (nt) | S. cerevisiae closest homologue | Essential in S. cerevisiae | Function | Functional category |
|---|---|---|---|---|---|---|---|---|---|---|
| 10-80 | CEA231_prot | 3 | 106 | 2805 | 934 | -280 | DBP10/YDL031w | yes | ATP-dependent RNA helicase | RNA processing |
| 10-291 | CEA233_prot | 6 | 114 | 1865 | 574 | 733 | NAR1/YNL240c | yes | Nuclear architecture related protein | Nuclear architecture |
| 7-1-19 | CEA254 | 9 | 122 | 943 | 200 | 511 | GUK1/YDR454c | yes | Guanylate kinase | Nucleotide metabolism |
| 10-3-7 | CEA255 _prot | 12 | 126 | 2108 | 657 | 57 | SRP101/YDR292c | yes | Signal recognition particle receptor - alpha subunit | Protein transport |
| 2-6-4 | CEA256_prot | 15 | 130 | 1564 | 460 | 46 | WBP1/YEL002c | yes | Oligosaccharyl transferase beta subunit | Protein modification |
| 2-1-1 | CEA257 _prot | 18 | 134 | 2376 | 715 | 2148 | YGL245w | yes | Glutamate-tRNA synthetase | Protein synthesis |
| 2-10-16 | CEA258 _prot | 21 | 138 | 2639 | 809 | 2191 | CDC27/YBL084c | yes | Cell division control protein | Cell cycle control |
| 5-4-21 | CEA259 _prot | 24 | 142 | 1707 | 568 | 1569 | RSC9/YML127w | yes | Component of the chromatin remodeling complex | Chromatin structure |
| 2-10-21 | CEA260 _prot | 27 | 146 | 1542 | 493 | 345 | SPE2/YOL052c | yes | S-adenosylmethionine decarboxylase | Metabolism |
| 7-5-9 | CEA261 _prot | 30 | 150 | 637 | 139 | 303 | RPL17A/YKL180 w RPL17B/YJL177w | no | Ribosomal protein of the large subunit of the ribosome(L17) | Protein synthesis |
| 10-2-18 | CEA262 _prot | 33 | 154 | 1037 | 256 | -131 | RPL1A/YGL135w RPL1B/YPL220w | no | Ribosomal protein of the large subunit of the ribosome(L1) | Protein synthesis |
| 9-11 | CEA230 | 36 | 102 | 1401 | 399 | 1316 | MSW1/YDR268w | no | Mitochondrial tryptophanyl-tRNA synthetase | Protein synthesis |
| 4-3-3 | CEA263 _prot | 39 | 158 | 819 | 227 | -1 | GOS1/YHL031c | no | SNARE protein | Protein transport |
| 11-6-11 | CEA264 _prot | 42 | 162 | 1601 | 394 | 278 | RIM11/YMR139w | no | Serine/threonine-protein kinase | Cell cycle control |
| 8-47 | CEA228 _prot | 45 | 94 | 2052 | 683 | -398 | YFL034w | no | Probable membrane protein; Yfl034wp | Unknown |
| 10-304 | CEA234 _prot | 48 | 118 | 461 | 141 | -179 | RPL14A/YHL001 w RPL14B/YKL006w | no | Ribosomal protein of the large subunit of the ribosome (L14) | Protein synthesis |
| 10-175 | CEA232 _prot | 51 | 110 | 1413 | 428 | -105 | HEM15/YOR176w | yes | Ferrochelatase | Heme biosynthesis |
| 11-4-9 | CEA265 _prot | 54 | 166 | 1539 | 512 | -40 | COX10/YPL172c | no | Protoheme IX farnesyltransferase | Heme biosynthesis |
| 2-10-18 | CEA266 _prot | 57 | 170 | 1629 | 542 | 195 | TRF4/YOL115w | no | Topoisomerase | DNA replication |
| 8-62 | CEA229 _prot | 60 | 98 | 2814 | 937 | 1300 | no hit found | | unknown function | Unknown |
| 6-8-13 | CEA280 _prot | | 174 | 573 | 190 | 138 | no hit found | | weak homology to S. pombeGTPase activator protein and to myocilin | unknown |
| *5-3-11* | *CEA281. 1 _prot* | | *178* | 1974 | 609 | 2037 | *PAC2*/*YER007w* | *no* | *tubulin folding cofactor E* | *Cytoskeleton* |
| *5-3-11* | *CEA281.2 _prot* | | *182* | 963 | 291 | -290 | *no hit found* | | *unknown function* | *unknown* |
| *10-4-20* | *CEA282. 1 _prot* | | *186* | 1448 | 464 | -537 | *PBP2*/*YBR233w* | *no* | *hnRNP complex protein*/*PAB1-binding protein* | *RNAprocessin* |
| *10-4-20* | *CEA282. 2 _prot* | | *190* | 1143 | 344 | -427 | *SEC3*/*YER008c* | *yes* | *unknown function* | *Protein secretion* |
| *4-3-4* | *CEA284. 1_prot* | | *195* | 3336 | 1059 | 4049 | *ENA5*/*YDR038c* | *no* | *P-type ATPase* | *Ion transport* |
| *4-3-4* | *CEA284.2 _prot* | | *199* | 1059 | 352 | -1137 | *no hit found* | | *secondary metabolite biosynthesis protein* | *unknown* |

### BRIEF DESCRIPTION OF THE DRAWINGS

Figure **1****:** Strategy for the identification of essential genes in *A. fumigatus.* A stable diploid strain heterozygous for spore color markers (*w1, r7*) is randomly mutagenized with the transposable element *impala160::pyrG* (*imp::pyr*). During haploidization on benomyl containing media, random loss of chromosomes gives rise to two sub-populations of colored haploid conidia (*w1* or *r7*): one bearing the transposon-inactivated allele (population A) and one bearing wild type allele (population B). The ability to form haploid progenies on non selective haploidization medium and the inability on selective haploidization medium (without uridine and uracile) leads to the identification of mutant strains with an insertion in an essential gene.

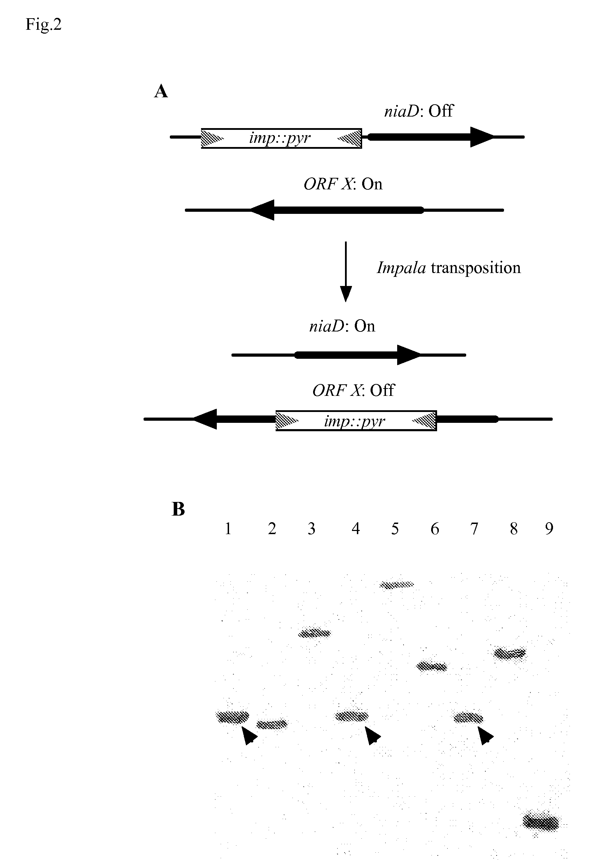

**Figure 2****:** *In vivo* random transposon mutagenesis in *A. fumigatus.* (A) Schematic representation of *impala160::pyrG* transposition in a *A. fumigatus* strain transformed by pNIpyr. Expression of the nitrate reductase gene (*niaD*) is prevented by the presence of the transposable element *impala160::pyG* (*imp::pyr*) into the promoter region. Positive selection of transposition events is obtained by selection of nitrate- utilizing revertants which appear as a result of the excision of *imp::pyr* and the restoration of a functional *niaD* promoter. Selection of *imp::pyr* reintegration events is ensured by the presence of *pyrG* in the transposable element when transposition events are induced in a *A. fumigatus pyrG* strain and in the absence of uridine and uracile. (B) Southern blot analysis of parental diploid transformants (lane 1: CEA225; lane 4: CEA226; lane 7: CEA227) and diploid revertants (lane 2: Rev 225-1; lane 3: Rev 225-2; lane 5: Rev 226-1; lane 6: Rev 226-2; lane 8: Rev 227-1; lane 9: Rev 227-2). Hybridization with a probe for *impala160::pyrG* revealed integration of the transposable element into the promoter of the *niaD* gene in the three parental transformants (arrowheads) and integration at apparent random sites in the genome of the diploid revertants.

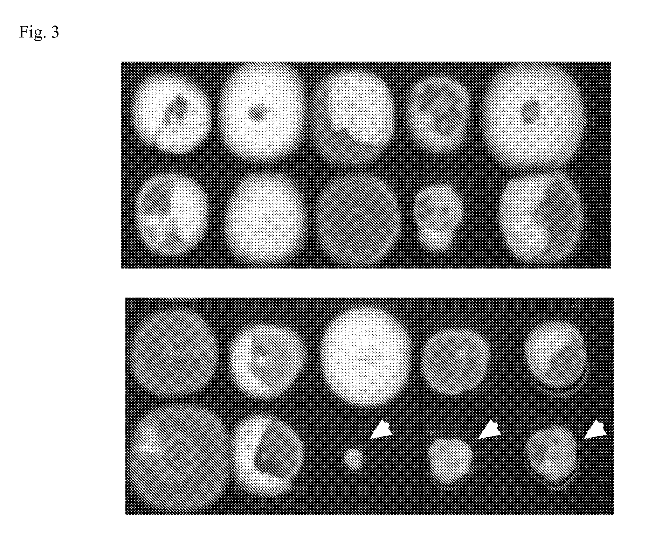

**Figure 3****:** Parasexual screening. Haploidization of 10 diploid revertants on non-selective (A) and selective (B) media.
   Random segregation of chromosomes is visualized by the production of differently colored haploid conidia (see Fig.1: allele *w1* and *r7*). On selective haploidization medium, in the case of plasmid integration in an essential gene, a residual growth phenotype is observed (arrowheads). For these revertants, haploid spores obtained on non selective haploidization medium were tested for the absence of the transposable element in order to confirm the essential phenotype.

### DETAILED DESCRIPTION OF THE INVENTION

The present invention is based on the discovery of novel molecules, referred to herein as EFG protein and nucleic acid molecules, encoding proteins Essential For Growth expressed in *Aspergillus fumigatus.*

An artificial *A. fumigatus* diploid strain with one copy of the *impala160* transposon from *Fusarium oxysporum* integrated into its genome was used to generate a library of diploid strains with random transposon integration. Among *ca.* 2,300 heterozygous diploid strains screened by parasexual genetics, 1.2% have a copy of the transposable element integrated into a gene essential for fungal growth. Homologues of genes essential for *Saccharomyces cerevisiae* growth have been identified, as well as genes that do not share homologues in other fungal species.
The term "EFG genes" refers to genes that are necessary for efficient fungal growth. An efficient growth refers to the normal growth of this fungus in absence of inhibitor of at least one of these genes. Inhibitors may be used to inhibit normal expression of at least one of the EFG genes identified herein.

Isolated EFG proteins of the present invention, have an amino acid sequences sufficiently homologous to the amino acid sequence of SEQ ID N°3,6,9,12,15,18,21,24,27,30,33,36,39,42,45,48,51,54,57,60,94,98,102,106,110,114,118, 122,126,130,134,138,142,146,150,154,158,162,166,170, or are encoded by a nucleotide sequence sufficiently homologous to one of SEQ ID N°1,4,7,10,13,16,19,22,25,28,31,34,37,40,43,46,49,52,55,58,92,96,100,104,108,112,116, 120,124,128,132,136,140,144,148,152,156,160,164,168. As used herein, the term "sufficiently homologous" refers to a first amino acid or nucleotide sequence which contains a sufficient or minimum number of identical or equivalent (e.g., an amino acid residue which has a similar side chain) amino acid residues or nucleotides to a second amino acid or nucleotide sequence such that the first and second amino acid or nucleotide sequences share common structural domains or motifs and/or a common functional activity. For example, amino acid or nucleotide sequences which share common structural domains have at least about 30-40% identity, preferably 40-50% identity, more preferably 50-60%, and even more preferably 60-70%, 70-80%, 80, 90%, 95%, 97%, 98%, 99% or 99.8% identity across the amino acid sequences of the domains are defined herein as sufficiently homologous. Furthermore, amino acid or nucleotide sequences which share at least 30%, preferably 40%, more preferably 60%, 70%, 80%, 90%, 95%, 97%, 98%, 99% or 99.8% identity and share a common functional activity are defined herein as sufficiently homologous.

Homologues are thus defined as those genes or gene products that show a significant level of identity or similarity at the nucleotide or amino acid level, respectively, as indicated above.

Orthologous genes are defined herein as those genes or gene products from two different species which, upon individual comparison to the gene set of the other species, appear reciprocally as the closest homologues.

As used interchangeably herein, a "EFG activity", "biological activity of EFG" or "functional activity of EFG", refers to an activity exerted by a EFG protein, polypeptide or nucleic acid molecule as determined *in vivo, or in vitro,* according to appropriate techniques.

The level of inhibition of EFG activity may depend on the number of the EFG genes that are inhibited and on the EFG genes inhibited. The inhibition of at least one EFG gene results in an inhibition of at least 5, 10, 20, 40, 60, 80, 90, 95% of the efficient growth. Preferably, the inhibition is of 100%, meaning the total suppression of growth and of the toxic effect of the fungus.

In one embodiment, a EFG activity is a direct activity, such as an association with a EFG-target molecule or most preferably EFG activity. As used herein, a "target molecule" is a molecule with which a EFG protein binds or interacts in nature, such that EFG-mediated function is achieved. Alternatively, a EFG activity is an indirect activity, such as an activity mediated by interaction of the EFG protein with a EFG target molecule such that the target molecule modulates a downstream cellular activity (e.g., interaction of an EFG molecule with a EFG target molecule can modulate the activity of that target molecule on an intracellular signaling pathway).

### I. EFG Nucleic Acids

The inventors have identified and completely characterized 21 EFG cDNA indicated in Table 1 and Table Ibis. For instance EFG2 refers to a 1735 nucleotide (nt) sequence in length (SEQ ID N°4) which comprises the nucleic acid of SEQ ID N°5 of 1592 nt in length, which encodes the protein of SEQ ID N°6, which is 530 amino acid residues in length.

One aspect of the invention pertains to purified or isolated nucleic acid molecules that encode EFG proteins or biologically active portions thereof, as well as nucleic acid fragments thereof. Fragments may be used for example as hybridization probes to identify EFG-encoding nucleic acids (e.g., EFG mRNA) and fragments for use as probes (e.g. for detection of EFG nucleic acid molecules) or primers (e.g. for sequencing, genotyping, amplification or mutation of EFG nucleic acid molecules). As used herein, the term "nucleic acids" and "nucleic acid molecule" is intended to include DNA molecules (e.g., cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA. Throughout the present specification, the expression "nucleotide sequence" may be employed to designate indifferently a polynucleotide or a nucleic acid. More precisely, the expression "nucleotide sequence" encompasses the nucleic material itself and is thus not restricted to the sequence information (i.e., the succession of letters chosen among the four base letters) that biochemically characterizes a specific DNA or RNA molecule. Also, used interchangeably herein are terms "nucleic acids", "oligonucleotides", and "polynucleotides".

An "isolated" nucleic acid molecule is one which is separated from other nucleic acid molecules which are present in the natural source of the nucleic acid. Preferably, an "isolated" nucleic acid is free of sequences which naturally flank the nucleic acid (i.e., sequences located at the 5' and 3' ends of the nucleic acid) in the genomic DNA of the organism from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

A nucleic acid molecule of the present invention, e.g., a nucleic acid molecule having a nucleotide sequence of SEQ ID N°1,4,7,10,13,16,19,22,25,28,31,34,37,40,43,46,49,52,55,58,92,96,100,104,108,112,116, 120,124,128,132,136,140,144,148,152,156,160,164,168, or a portion thereof, can be isolated using standard molecular biology techniques and the sequence information provided herein. Using all or portion of the nucleic acid sequence of SEQ ID N°1,4,7,10,13,16,19,22,25,28,31,34,37,40,43,46,49,52,55,58,92,96,100,104,108,112,116, 120,124,128,132,136,140,144,148,152,156,160,164,168, as a hybridization probe, EFG nucleic acid molecules can be isolated using standard hybridization and cloning techniques (e.g., as described in Sambrook, J., Fritsh, E. F., and Maniatis, T. Molecular Cloning. A Laboratory Manual. 2nd, ed., Cold Spring Harbor Laboratory, Cold Spring Harbor Laboratory Press, Cold Spring Harbor, N.Y., 1989).

Moreover, a nucleic acid molecule encompassing all or a portion of a sequence of SEQ ID N°1,4,7,10,13,16,19,22,25,28,31,34,37,40,43,46,49,52,55,58,92,96,100,104,108,112,116, 120,124,128,132,136,140,144,148,152,156,160,164,168, can be isolated by the polymerase chain reaction (PCR) using synthetic oligonucleotide primers designed based upon a sequence of SEQ ID N°1,4,7,10,13,16,19,22,25,28,31,34,37,40,43,46,49,52,55,58,92,96,100,104,108,112,116, 120,124,128,132,136,140,144,148,152,156,160,164,168.

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to EFG nucleotide sequences can be prepared by standard synthetic techniques, e.g., using an automated DNA synthesizer.

In a preferred embodiment, an isolated nucleic acid molecule of the invention comprises, consists essentially of, or consists of the nucleotide sequence of SEQ ID N°1,4,7,10,13,16,19,22,25,28,31,34,37,40,43,46,49,52,55,58,92,96,100,104,108,112,116, 120,124,128,132,136,140,144,148,152,156,160,164,168, or fragment thereof. The sequences of SEQ ID N°1,4,7,10,13,16,19,22,25,28,31,34,37,40,43,46,49,52,55,58,92,96,100,104,108,112,116, 120,124,128,132,136,140,144,148,152,156,160,164,168, correspond to *A. fumigatus* EFG cDNA.

Also encompassed by the EFG nucleic acids of the invention are nucleic acid molecules which are complementary to EFG nucleic acids described herein. Preferably, a complementary nucleic acid is sufficiently complementary to a nucleotide sequence of SEQ ID N°1,4,7,10,13,16,19,22,25,28,31,34,37,40,43,46,49,52,55,58,92,96,100,104,108,112,116, 120,124,128,132,136,140,144,148,152,156,160,164,168, such that it can hybridize to a nucleotide sequence of SEQ N°1,4,7,10,13,16,19,22,25,28,31,34,37,40,43,46,49,52,55,58,92,96,100,104,108,112,116, 120,124,128,132,136,140,144,148,152,156,160,164,168.

Another object of the invention is a purified, isolated, or recombinant nucleic acid encoding a EFG polypeptide comprising an amino acid sequence of SEQ ID N°3,6,9,12,15,18,21,24,27,30,33,36,39,42,45,48,51,54,57,60,94,98,102,106,110,114,118, 122,126,130,134,138,142,146,150,154,158,162,166,170, or fragments thereof. Preferred polynucleotides of the invention also include purified, isolated, or recombinant EFG cDNAs consisting of, consisting essentially of, or comprising a sequence of SEQ ID N°1,4,7,10,13,16,19,22,25,28,31,34,37,40,43,46,49,52,55,58,92,96,100,104,108,112,116, 120,124,128,132,136,140,144,148,152,156,160,164,168. Particularly preferred nucleic acids of the invention include isolated, purified, or recombinant polynucleotides comprising a contiguous span of at least 12, 15, 18, 20, 25, 30, 35, 40, 50, 60, 70, 80, 90, 100, 150, 200, 500, 1000 or 2000 nucleotides (upper lengths of the fragments to be adapted to the length of the nucleotide sequence) of a sequence of SEQ ID N°1,4,7,10,13,16,19,22,25,28,31,34,37,40,43,46,49,52,55,58,92,96,100,104,108,112,116, 120,124,128,132,136,140,144,148,152,156,160,164,168, or the complements thereof.

Moreover, the nucleic acid molecule of the invention can comprise only a portion of a nucleic acid sequence of SEQ ID N°1,4,7,10,13,16,19,22,25,28,31,34,37,40,43,46,49,52,55,58,92,96,100,104,108,112,116, 120,124,128,132,136,140,144,148,152,156,160,164,168, for example a fragment which can be used as a probe or primer or a fragment encoding a biologically active portion of a EFG protein. The nucleotide sequence determined from the cloning of the EFG genes allows for the generation of probes and primers designed for use in identifying and/or cloning other EFG family members, as well as EFG homologues from other species. The probe/primer typically comprises substantially purified oligonucleotide. The oligonucleotide typically comprises a region of nucleotide sequence that hybridizes under stringent conditions to at least about 12, preferably about 25, more preferably about 40, 50 or 75 consecutive nucleotides of a sequence of SEQ ID N°1,4,7,10,13,16,19,22,25,28,31,34,37,40,43,46,49,52,55,58,92,96,100,104,108,112,116, 120,124,128,132,136,140,144,148,152,156,160,164,168, or a sequence complementary thereto.

A nucleic acid fragment encoding a "biologically active portion of a EFG protein" can be prepared by isolating a portion of a nucleotide sequence of SEQ ID N°1,4,7,10,13,16,19,22,25,28,31,34,37,40,43,46,49,52,55,58,92,96,100,104,108,112,116, 120,124,128,132,136,140,144,148,152,156,160,164,168, which encodes a polypeptide having a EFG biological activity (the biological activities of the EFG proteins described herein), expressing the encoded portion of the EFG protein (e.g., by recombinant expression in vitro) and assessing the activity of the encoded portion of the EFG protein.

The invention further encompasses nucleic acid molecules that differ from a nucleotide sequence of SEQ ID N°1,4,7,10,13,16,19,22,25,28,31,34,37,40,43,46,49,52,55,58,92,96,100,104,108,112,116, 120,124,128,132,136,140,144,148,152,156,160,164,168, due to degeneracy of the genetic code and thus encode the same EFG proteins as those encoded by a nucleotide sequence of SEQ ID N°1,4,7,10,13,16,19,22,25,28,31,34,37,40,43,46,49,52,55,58,92,96,100,104,108,112,116, 120,124,128,132,136,140,144,148,152,156,160,164,168. In another embodiment, an isolated nucleic acid molecule of the invention comprises a nucleotide sequence encoding a protein having an amino acid sequence of SEQ ID N°3,6,9,12,15,18,21,24,27,30,33,36,39,42,45,48,51,54,57,60,94,98,102,106,110,114,118, 122,126,130,134,138,142,146,150,154,158,162,166,170.

In addition to the EFG nucleotide sequences of SEQ ID N°1,4,7,10,13,16,19,22,25,28,31,34,37,40,43,46,49,52,55,58,92,96,100,104,108,112,116, 120,124,128,132,136,140,144,148,152,156,160,164,168, it will be appreciated by those skilled in the art that DNA sequence polymorphisms that lead to changes in the amino acid sequences of the EFG proteins may exist within a population (e.g., the fungal population). Such genetic polymorphism in the EFG genes may exist among individuals within a population due to natural allelic variation. As used herein, the terms "gene" and "recombinant gene" refer to nucleic acid molecules comprising an open reading frame encoding an EFG protein, preferably a fungal EFG protein. Such natural allelic variations can typically result in 1-5% variance in the nucleotide sequence of a EFG gene. Any and all such nucleotide variations and resulting amino acid polymorphisms in EFG genes that are the result of natural allelic variation and, most preferably, that do not alter the functional activity of a EFG protein are intended to be within the scope of the invention.

Nucleic acid molecules corresponding to natural allelic variants and homologues of the EFG cDNAs of the invention can be isolated based on their homology to the EFG nucleic acids disclosed herein using the cDNAs disclosed herein, or a portion thereof, as a hybridization probe according to standard hybridization techniques under stringent hybridization conditions.

As used herein, the term "hybridizes under stringent conditions" is intended to describe conditions for hybridization and washing under which nucleotide sequences at least 60% homologous to each other typically remain hybridized to each other. Preferably, the conditions are such that sequences at least about 70%, more preferably at least about 80%, even more preferably at least about 85%, 90%, 95% or 98% homologous to each other typically remain hybridized to each other. Stringent conditions are known to those skilled in the art and can be found in Current Protocols in Molecular Biology, John Wiley & Sons, N.Y. (1989), 6.3.1-6.3.6. A preferred, non-limiting example of stringent hybridization conditions are hybridization in 6 * sodium chloride/sodium citrate (SSC) at about 45°C, followed by one or more washes in 0.2 *SSC, 0.1% SDS at 50-65°C. Preferably, an isolated nucleic acid molecule of the invention that hybridizes under stringent conditions to the sequence of SEQ ID NO: corresponds to a naturally-occurring nucleic acid molecule. As used herein, a "naturally-occurring" nucleic acid molecule refers to a RNA or DNA molecule having a nucleotide sequence that occurs in nature (e.g., encodes a natural protein).

In addition to naturally-occurring allelic variants of the EFG sequences that may exist in the population, the skilled artisan will further appreciate that changes can be introduced by mutation into a nucleotide sequence of SEQ ID N°1,4,7,10,13,16,19,22,25,28,31,34,37,40,43,46,49,52,55,58,92,96,100,104,108,112,116, 120,124,128,132,136,140,144,148,152,156,160,164,168, thereby leading to changes in the amino acid sequence of the encoded EFG proteins, without altering the functional ability of the EFG proteins. For example, nucleotide substitutions leading to amino acid substitutions at "non-essential" amino acid residues can be made in a sequence of N°3,6,9,12,15,18,21,24,27,30,33,36,39,42,45,48,51,54,57,60,94,98,102,106,110,114,118, 122,126,130,134,138,142,146,150,154,158,162,166,170. A "non-essential" amino acid residue is a residue that can be altered from the wild-type sequence of EFG (e.g., the sequence of SEQ ID NO:1) without altering the biological activity, whereas an "essential" amino acid residue is required for biological activity. For example, amino acid residues that are conserved among the EFG proteins of the present invention, are predicted to be less unamenable to alteration. Furthermore, additional conserved amino acid residues may be amino acids that are conserved between the EFG proteins of the present invention and other members of the *Aspergillus* family and/or of other fungi.

Thus, the invention further encompasses nucleic acid molecules that are homologous to the nucleic acids of *A. fumigatus* described above and that are isolated from target phytopathogenic fungi, namely *Botrytis cinerea, Mycosphaerella graminicola, Stagnospora nodorum, Blumeria graminis, Colleotrichum lindemuthianum, Puccinia graminis, Leptosphaeria maculans, Fusarium oxysporum, Fusarium graminearum, Venturia inaequalis,* most preferably fungi of the genus *Magnaporthe,* even most preferably *Magnaporthe grisea.*

Accordingly, another aspect of the invention pertains to nucleic acid molecules encoding EFG proteins and biologically active fragments thereof that contain changes in amino acid residues that are not essential for activity. Such EFG proteins differ in amino acid sequence of SEQ ID N°3,6,9,12,15,18,21,24,27,30,33,36,39,42,45,48,51,54,57,60,94,98,102,106,110,114,118, 122,126,130,134,138,142,146,150,154,158,162,166,170, yet retain biological activity. In one embodiment, the isolated nucleic acid molecule comprises a nucleotide sequence encoding a protein, wherein the protein comprises an amino acid sequence at least about 60% homologous to an amino acid sequence of SEQ ID N°3,6,9,12,15,18,21,24,27,30,33,36,39,42,45,48,51,54,57,60,94,98,102,106,110,114,118, 122,126,130,134,138,142,146,150,154,158,162,166,170. Preferably, the protein encoded by the nucleic acid molecule is at least about 65-70% homologous to a sequence of SEQ ID N°3,6,9,12,15,18,21,24,27,30,33,36,39,42,45,48,51,54,57,60,94,98,102,106,110,114,118, 122,126,130,134,138,142,146,150,154,158,162,166,170, more preferably sharing at least about 75-80% identity with SEQ ID N°3,6,9,12,15,18,21,24,27,30,33,36,39,42,45,48,51,54,57,60,94,98,102,106,110,114,118, 122,126,130,134,138,142,146,150,154,158,162,166,170, even more preferably sharing at least about 85%, 90%, 92%, 95%, 97%, 98%, 99% or 99.8% identity with a sequence of SEQ ID N°3,6,9,12,15,18,21,24,27,30,33,36,39,42,45,48,51,54,57,60,94,98,102,106,110,114,118, 122,126,130,134,138,142,146,150,154,158,162,166,170.

An isolated nucleic acid molecule encoding a EFG protein homologous to a protein of SEQ ID N°3,6,9,12,15,18,21,24,27,30,33,36,39,42,45,48,51,54,57,60,94,98,102,106,110,114,118, 122,126,130,134,138,142,146,150,154,158,162,166,170, can be created by introducing one or more nucleotide substitutions, additions or deletions into a nucleotide sequence of SEQ ID N°1,4,7,10,13,16,19,22,25,28,31,34,37,40,43,46,49,52,55,58,92,96,100,104,108,112,116, 120,124,128,132,136,140,144,148,152,156,160,164,168, such that one or more amino acid substitutions, additions or deletions are introduced into the encoded protein. Mutations can be introduced into a sequence of N°1,4,7,10,13,16,19,22,25,28,31,34,37,40,43,46,49,52,55,58,92,96,100,104,108,112,116, 120,124,128,132,136,140,144,148,152,156,160,164,168, by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in a EFG protein is preferably replaced with another amino acid residue from the same side chain family. Alternatively, in another embodiment, mutations can be introduced randomly along all or part of a EFG coding sequence, such as by saturation mutagenesis. Following mutagenesis the encoded protein can be expressed recombinantly and the activity of the protein can be determined.
The biological EFG activity of the protein fragments and mutants described above can be assayed according to the tests known from the one skilled in the art.

Primers and probes of the invention can be prepared by any suitable method, including, for example, cloning and restriction of appropriate sequences and direct chemical synthesis by a method such as the phosphodiester method of Narang SA, Hsiung HM, Brousseau R, Methods Enzymol 1979;68:90-98, the phosphodiester method of Brown EL, Belagaje R, Ryan MJ, Khorana HG, Methods Enzymol 1979;68:109-151, the diethylphosphoramidite method of Beaucage et al., Tetrahedron Lett 1981, 22: 1859-1862 and the solid support method described in EP 0 707 592.

Any of the polynucleotides of the present invention can be labeled, if desired, by incorporating any label known in the art to be detectable by spectroscopic, photochemical, biochemical, immunochemical, or chemical means. For example, useful labels include radioactive substances (including, ³²P, ³⁵S, ³H, ¹²⁵I), fluorescent dyes (including, 5-bromodesoxyuridin, fluorescein, acetylaminofluorene, digoxigenin) or biotin. Preferably, polynucleotides are labeled at their 3' and 5' ends. A label can also be used to capture the primer, so as to facilitate the immobilization of either the primer or a primer extension product, such as amplified DNA, on a solid support. A capture label is attached to the primers or probes and can be a specific binding member which forms a binding pair with the solid's phase reagent's specific binding member (e.g. biotin and streptavidin). Therefore depending upon the type of label carried by a polynucleotide or a probe, it may be employed to capture or to detect the target DNA. Further, it will be understood that the polynucleotides, primers or probes provided herein, may, themselves, serve as the capture label.

The probes of the present invention are useful for a number of purposes. They can be notably used in Southern hybridization to genomic DNA. The probes can also be used to detect PCR amplification products. They may also be used to detect mismatches in the EFG gene or mRNA using other techniques.

Any of the polynucleotides, primers and probes of the present invention can be conveniently immobilized on a solid support. Solid supports are known to those skilled in the art. A solid support, as used herein, refers to any material which is insoluble, or can be made insoluble by a subsequent reaction. The solid support can be chosen for its intrinsic ability to attract and immobilize the capture reagent. Alternatively, the solid phase can retain an additional receptor which has the ability to attract and immobilize the capture reagent. The additional receptor can include a charged substance that is oppositely charged with respect to the capture reagent itself or to a charged substance conjugated to the capture reagent. As yet another alternative, the receptor molecule can be any specific binding member which is immobilized upon (attached to) the solid support and which has the ability to immobilize the capture reagent through a specific binding reaction. The receptor molecule enables the indirect binding of the capture reagent to a solid support material before the performance of the assay or during the performance of the assay. The solid phase thus can be a plastic, derivatized plastic, magnetic or non-magnetic metal, glass or silicon surface of a test tube, microtiter well, sheet, bead, microparticle, chip, sheep (or other suitable animal's) red blood cells, duracytes and other configurations known to those of ordinary skill in the art. The polynucleotides of the invention can be attached to or immobilized on a solid support individually or in groups of at least 2, 5, 8, 10, 12, 15, 20, or 25 distinct polynucleotides of the invention to a single solid support. In addition, polynucleotides other than those of the invention may be attached to the same solid support as one or more polynucleotides of the invention.

Consequently, the invention also comprises a method for detecting the presence of a nucleic acid comprising a nucleotide sequence of SEQ ID N°1,4,7,10,13,16,19,22,25,28,31,34,37,40,43,46,49,52,55,58,92,96,100,104,108,112,116, 120,124,128,132,136,140,144,148,152,156,160,164,168, a fragment or a variant thereof and a complementary sequence thereto in a sample, said method comprising the following steps of:
a) bringing into contact a nucleic acid probe or a plurality of nucleic acid probes which can hybridize with a nucleotide sequence included in a nucleic acid sequence of SEQ ID N°1,4,7,10,13,16,19,22,25,28,31,34,37,40,43,46,49,52,55,58,92,96,100,104,108,112,116, 120,124,128,132,136,140,144,148,152,156,160,164,168, a fragment or a variant thereof and a complementary sequence thereto and the sample to be assayed; and
b) detecting the hybrid complex formed between the probe and a nucleic acid in the sample.

Any polynucleotide provided herein may be attached in overlapping areas or at random locations on a solid support. Alternatively the polynucleotides of the invention may be attached in an ordered array wherein each polynucleotide is attached to a distinct region of the solid support which does not overlap with the attachment site of any other polynucleotide. Preferably, such an ordered array of polynucleotides is designed to be "addressable" where the distinct locations are recorded and can be accessed as part of an assay procedure. Addressable polynucleotide arrays typically comprise a plurality of different oligonucleotide probes that are coupled to a surface of a substrate in different known locations. The knowledge of the precise location of each polynucleotides location makes these "addressable" arrays particularly useful in hybridization assays. Any addressable array technology known in the art can be employed with the polynucleotides of the invention. One particular embodiment of these polynucleotide arrays is known as the Genechips, and has been generally described in US Patent 5,143,854; PCT publications WO 90/15070 and 92/10092.

### II. EFG Polypeptides and Anti-EFG Antibodies

One aspect of the invention pertains to isolated EFG proteins, and biologically active portions thereof, as well as polypeptide fragments suitable for use as immunogens to raise fungus, preferably *Aspergillus,* most preferably *A. fumigatus,* anti-EFG antibodies. In one embodiment, native EFG proteins can be isolated from cells or tissue sources by an appropriate purification scheme using standard protein purification techniques. In another embodiment, EFG proteins are produced by recombinant DNA techniques. Alternative to recombinant expression, a EFG protein or polypeptide can be synthesized chemically using standard peptide synthesis techniques.

An "isolated" or "purified" protein or biologically active portion thereof is substantially free of cellular material or other contaminating proteins from the cell or tissue source from which the EFG protein is derived, or substantially free from chemical precursors or other chemicals when chemically synthesized. The language "substantially free of cellular material" includes preparations of EFG protein in which the protein is separated from cellular components of the cells from which it is isolated or recombinantly produced. In one embodiment, the language "substantially free of cellular material" includes preparations of EFG protein having less than about 30% (by dry weight) of non-EFG protein (also referred to herein as a "contaminating protein"), more preferably less than about 20% of non-EFG protein, still more preferably less than about 10% of non-EFG protein, and most preferably less than about 5% non-EFG protein. When the EFG protein or biologically active portion thereof is recombinantly produced, it is also preferably substantially free of culture medium, i.e., culture medium represents less than about 20%, more preferably less than about 10%, and most preferably less than about 5% of the volume of the protein preparation.

The term "polypeptide" refers to a polymer of amino acids without regard to the length of the polymer; thus, peptides, oligopeptides, and proteins are included within the definition of polypeptide. This term also does not specify or exclude post-expression modifications of polypeptides, for example, polypeptides which include the covalent attachment of glycosyl groups, acetyl groups, phosphate groups, lipid groups and the like are expressly encompassed by the term polypeptide. Also included within the definition are polypeptides which contain one or more analogs of an amino acid (including, for example, non-naturally occurring amino acids, amino acids which only occur naturally in an unrelated biological system, modified amino acids from mammalian systems etc.), polypeptides with substituted linkages, as well as other modifications known in the art, both naturally occurring and non-naturally occurring.

The term "recombinant polypeptide" is used herein to refer to polypeptides that have been artificially designed and which comprise at least two polypeptide sequences that are not found as contiguous polypeptide sequences in their initial natural environment, or to refer to polypeptides which have been expressed from a recombinant polynucleotide.

Biologically active portions of a EFG protein include peptides comprising amino acid sequences sufficiently homologous to or derived from an amino acid sequence of the EFG protein having a sequence of SEQ ID N°3,6,9,12,15,18,21,24,27,30,33,36,39,42,45,48,51,54,57,60,94,98,102,106,110,114,118, 122,126,130,134,138,142,146,150,154,158,162,166,170, which include less amino acids than the full length EFG proteins, and exhibit at least one activity of a EFG protein. Typically, biologically active portions comprise a domain or motif with at least one activity of the EFG protein. A biologically active portion of a EFG protein can be a polypeptide which is, for example at least 15, 25, 50, 100, 150, 200, 300, 400, 500, or more amino acids in length. The upper length just mentioned is of course to be adapted according to the size of the protein.

In a preferred embodiment, the EFG protein comprises, consists essentially of, or consists of the amino acid sequence of SEQ ID N°3,6,9,12,15,18,21,24,27,30,33,36,39,42,45,48,51,54,57,60,94,98,102,106,110,114,118, 122,126,130,134,138,142,146,150,154,158,162,166,170. The invention also concerns the polypeptide encoded by a nucleotide sequence selected from the group consisting of SEQ ID N°1,4,7,10,13,16,19,22,25,28,31,34,37,40,43,46,49,52,55,58,92,96,100,104,108,112,116, 120,124,128,132,136,140,144,148,152,156,160,164,168, a complementary sequence thereof or a fragment thereto. The present invention embodies isolated, purified, and recombinant polypeptides comprising a contiguous span of at least 6 amino acids, preferably at least 8 to 10 amino acids, more preferably at least 12, 15, 20, 25, 30, 40, 50, 100, 200, 300, 400, 500, 600 or 650 amino acids in length (upper length defined as mentioned above).

In other embodiments, the EFG protein is substantially homologous to SEQ ID N°3,6,9,12,15,18,21,24,27,30,33,36,39,42,45,48,51,54,57,60,94,98,102,106,110,114,118, 122,126,130,134,138,142,146,150,154,158,162,166,170, and retains the functional activity (at least 50, 60, 80, 90, 99%) of a protein of SEQ ID N°3,6,9,12,15,18,21,24,27,30,33,36,39,42,45,48,51,54,57,60,94,98,102,106,110,114,118, 122,126,130,134,138,142,146,150,154,158,162,166,170 yet differs in amino acid sequence due to natural allelic variation or mutagenesis, as described in detail in subsection I above. Accordingly, in another embodiment, the EFG protein is a protein which comprises an amino acid sequence at least about 60% homologous to an amino acid sequence of SEQ ID N°3,6,9,12,15,18,21,24,27,30,33,36,39,42,45,48,51,54,57,60,94,98,102,106,110,114,118, 122,126,130,134,138,142,146,150,154,158,162,166,170, and retains the functional activity of an EFG proteins of SEQ ID N°3,6,9,12,15,18,21,24,27,30,33,36,39,42,45,48,51,54,57,60,94,98,102,106,110,114,118, 122,126,130,134,138,142,146,150,154,158,162,166,170, respectively.
Preferably, the protein is at least about 30%, 40%, 50%, 60%, 70%, 80%, 85%, 90%, 92%, 95%, 97%, 98%, 99% or 99.8% homologous to a sequence of SEQ ID N°3,6,9,12,15,18,21,24,27,30,33,36,39,42,45,48,51,54,57,60,94,98,102,106,110,114,118, 122,126,130,134,138,142,146,150,154,158,162,166,170.

To determine the percent homology of two amino acid sequences or of two nucleic acids, the sequences are aligned for optimal comparison purposes (e.g., gaps can be introduced in the sequence of a first amino acid or nucleic acid sequence for optimal alignment with a second amino or nucleic acid sequence and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, 90% or 95% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position (i.e., as used herein amino acid or nucleic acid "identity" is equivalent to amino acid or nucleic acid "homology").

The comparison of sequences and determination of percent homology between two sequences can be accomplished using a mathematical algorithm, preferably the alignment method of Needleman and Wush,J.Mol.Biol.,1970,n°48,p443, using the GAP GCC package (Devereux et al.,Nucl.Acid.Res.,1984,voll2,p387). An other non-limiting example of a mathematical algorithim utilized for the comparison of sequences is the algorithm of Karlin and Altschul (1990) Proc. Natl. Acad. Sci. USA 87:2264-68, modified as in Karlin and Altschul (1993) Proc. Natl. Acad. Sci. USA 90:5873-77. Such an algorithm is incorporated into the NBLAST and XBLAST programs (version 2.0) of Altschul, et al. (1990) J. Mol. Biol. 215:403-10. BLAST nucleotide searches can be performed with the NBLAST program, score=100, wordlength=12 to obtain nucleotide sequences homologous to EFG nucleic acid molecules of the invention. BLAST protein searches can be performed with the XBLAST program, score=50, wordlength=3 to obtain amino acid sequences homologous to EFG protein molecules of the invention. To obtain gapped alignments for comparison purposes, Gapped BLAST can be utilized as described in Altschul et al., (1997) Nucleic Acids Research 25(17):3389-3402. When utilizing BLAST and Gapped BLAST programs, the default parameters of the respective programs (e.g., XBLAST and NBLAST) can be used. See http://www.ncbi.nlm.nih.gov. Another preferred, non-limiting example of a mathematical algorithim utilized for the comparison of sequences is the algorithm of Myers and Miller, CABIOS (1989). Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used.

The invention also provides EFG chimeric or fusion proteins. As used herein, a EFG "chimeric protein" or "fusion protein" comprises a EFG polypeptide operatively linked, preferalby fused in frame, to a non-EFG polypeptide. In a preferred embodiment, a EFG fusion protein comprises at least one biologically active portion of a EFG protein. In another preferred embodiment, a EFG fusion protein comprises at least two biologically active portions of a EFG protein. For example, in one embodiment, the fusion protein is a GST-EFG fusion protein in which the EFG sequences are fused to the C-terminus of the GST sequences. Such fusion proteins can facilitate the purification of recombinant EFG. In another embodiment, the fusion protein is a EFG protein containing a heterologous signal sequence at its N-terminus, such as for example to allow for a desired cellular localization in a certain host cell.

The EFG fusion proteins of the invention can be incorporated into pharmaceutical compositions and administered to a subject in vivo. Moreover, the EFG-fusion proteins of the invention can be used as immunogens to produce anti-EFG antibodies in a subject, to purify EFG ligands and in screening assays to identify molecules which inhibit the interaction of EFG with a EFG target molecule.

The present invention also pertains to variants of the EFG proteins which function as either EFG mimetics or as EFG inhibitors. Variants of the EFG proteins can be generated by mutagenesis, e.g., discrete point mutation or truncation of a EFG protein. An agonist of the EFG proteins can retain substantially the same, or a subset, of the biological activities of the naturally occurring form of a EFG protein. An antagonist of a EFG protein can inhibit one or more of the activities of the naturally occurring form of the EFG protein by, for example, competitively inhibiting the EFG activity of a EFG protein. Thus, specific biological effects can be elicited by treatment with a variant of limited function.

In a prefered embodiment, variants of a EFG protein which function as EFG antagonists can be identified by screening combinatorial libraries of mutants, e.g., truncation mutants, of a EFG protein for EFG protein antagonist activity. In one embodiment, a variegated library of EFG variants is generated by combinatorial mutagenesis at the nucleic acid level and is encoded by a variegated gene library. A variegated library of EFG variants can be produced by, for example, enzymatically ligating a mixture of synthetic oligonucleotides into gene sequences such that a degenerate set of potential EFG sequences is expressible as individual polypeptides, or alternatively, as a set of larger fusion proteins (e.g., for phage display) containing the set of EFG sequences therein. There are a variety of methods which can be used to produce libraries of potential EFG variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential EFG sequences.

In addition, libraries of fragments of a EFG protein coding sequence can be used to generate a variegated population of EFG fragments for screening and subsequent selection of variants of a EFG protein. In one embodiment, a library of coding sequence fragments can be generated by treating a double stranded PCR fragment of a EFG coding sequence with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S 1 nuclease, and ligating the resulting fragment library into an expression vector. By this method, an expression library can be derived which encodes N-terminal, C-terminal and internal fragments of various sizes of the EFG protein.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of EFG proteins. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected.

In one embodiment, cell based assays can be exploited to analyze a variegated EFG library.

Modified EFG proteins can be used for such purposes as enhancing therapeutic or prophylactic efficacy, or stability (e.g., ex vivo shelf life and resistance to proteolytic degradation in vivo). Such modified peptides, when designed to retain at least one activity of the naturally occurring form of the protein, are considered functional equivalents of the EFG protein described in more detail herein. Such modified peptide can be produced, for instance, by amino acid substitution, deletion, or addition.

Whether a change in the amino acid sequence of a peptide results in a functional EFG homolog (e.g. functional in the sense that it acts to mimic or antagonize the wild-type form) can be readily determined by assessing the ability of the variant peptide to produce a response in cells in a fashion similar to the wild-type EFG protein or competitively inhibit such a response. Peptides in which more than one replacement has taken place can readily be tested in the same manner.

A wide range of techniques are known in the art for screening gene products of combinatorial libraries made by point mutations, as well as for screening cDNA libraries for gene products having a certain property. Such techniques will be generally adaptable for rapid screening of the gene libraries generated by the combinatorial mutagenesis of EFG proteins. The most widely used techniques for screening large gene libraries typically comprises cloning the gene library into replicable expression vectors, transforming appropriate cells with the resulting library of vectors, and expressing the combinatorial genes under conditions in which detection of a desired activity facilitates relatively easy isolation of the vector encoding the gene whose product was detected.

The invention also provides for identification and reduction to functional minimal size of the EFG domains of the subject EFG proteins to generate mimetics, e.g. peptide or non-peptide agents, which are able to disrupt binding of a polypeptide of the present invention with a EFG target protein. Thus, such mutagenic techniques as described above are also useful to map the determinants of EFG proteins which participate in protein-protein interactions involved in, for example, binding to a EFG target protein. To illustrate, the critical residues of a EFG protein which are involved in molecular recognition of the EFG target can be determined and used to generate EFG target-13P-derived peptidomimetics that competitively inhibit binding of the EFG to the EFG target. By employing, for example, scanning mutagenesis to map tile amino acid residues of a particular EFG protein involved in binding a EFG target, peptidomimetic compounds can be generated which mimic those residues in binding to a EFG target, and which, by inhibiting binding of the EFG protein to the EFG target protein, can interfere with the function of a EFG target in transcriptional regulation of one or more genes. For instance, non hydrolyzable peptide analogs of such residues can be generated using retro-inverse peptides (e.g., see U.S. Patents 5,116,947 and 5,219,089; and Pallai et al. (1983) Int J Pept Protein Res 21:84-92), benzodiazepine (e.g., see Freidinger et al. in Peptides: Chemistry and Biology, G.R. Marshall ed., ESCOM Publisher: Leiden, Netherlands, 1988), azepine (e.g., see Huffman et al. in Peptides.- Chemistry and Biology, G.R. Marshall ed., ESCOM Publisher: Leiden, Netherlands, 1988).

An isolated EFG protein, or a portion or fragment thereof, can be used as an immunogen to generate antibodies that bind EFG using standard techniques for polyclonal and monoclonal antibody preparation. Even if they are internal fungus protein, EFG proteins may induce an immunitary response in contact with the host organism. A full-length EFG protein can be used or, alternatively, the invention provides antigenic peptide fragments of EFG for use as immunogens. The antigenic peptide of EFG comprises at least 8 amino acid residues of an amino acid sequence of SEQ ID N°3,6,9,12,15,18,21,24,27,30,33,36,39,42,45,48,51,54,57,60,94,98,102,106,110,114,118, 122,126,130,134,138,142,146,150,154,158,162,166,170, and encompasses an epitope of EFG such that an antibody raised against the peptide forms a specific immune complex with EFG. Preferably, the antigenic peptide comprises at least 10 amino acid residues, more preferably at least 15 amino acid residues, even more preferably at least 20 amino acid residues, and most preferably at least 30 amino acid residues.

Preferred epitopes encompassed by the antigenic peptide are regions of EFG that are located on the surface of the protein, e.g., hydrophilic regions.

A EFG immunogen typically is used to prepare antibodies by immunizing a suitable subject, (e.g., rabbit, goat, mouse or other mammal) with the immunogen. An appropriate immunogenic preparation can contain, for example, recombinantly expressed EFG protein or a chemically synthesized EFG polypeptide. The preparation can further include an adjuvant, such as Freund's complete or incomplete adjuvant, or similar immunostimulatory agent. Immunization of a suitable subject with an immunogenic EFG preparation induces a polyclonal anti-EFG antibody response.

Accordingly, another aspect of the invention pertains to anti-EFG antibodies. The term "antibody" as used herein refers to immunoglobulin molecules and immunologically active portions of immunoglobulin molecules, i.e., molecules that contain an antigen binding site which specifically binds (immunoreacts with) an antigen, such as EFG polypeptides. Examples of immunologically active portions of immunoglobulin molecules include F(ab) and F(ab').sub.2 fragments which can be generated by treating the antibody with an enzyme such as pepsin. The invention provides polyclonal and monoclonal antibodies that bind EFG polypeptides. The term "monoclonal antibody" or "monoclonal antibody composition", as used herein, refers to a population of antibody molecules that contain only one species of an antigen binding site capable of immunoreacting with a particular epitope of EFG polypeptides. A monoclonal antibody composition thus typically displays a single binding affinity for a particular EFG protein with which it immunoreacts.

The invention concerns antibody compositions, either polyclonal or monoclonal, capable of selectively binding, or selectively bind to an epitope-containing a polypeptide comprising a contiguous span of at least 6 amino acids, preferably at least 8 to 10 amino acids, more preferably at least 12, 15, 20, 25, 30, 40, 50, or 100 amino acids of a sequence of SEQ ID N°3,6,9,12,15,18,21,24,27,30,33,36,39,42,45,48,51,54,57,60,94,98,102,106,110,114,118, 122,126,130,134,138,142,146,150,154,158,162,166,170 (upper length according to the total length of each EFG protein). The invention also concerns a purified or isolated antibody capable of specifically binding to a mutated EFG protein or to a fragment or variant thereof comprising an epitope of the mutated EFG protein

Polyclonal anti-EFG antibodies can be prepared as described above by immunizing a suitable subject with a EFG immunogen. The anti-EFG antibody titer in the immunized subject can be monitored over time by standard techniques, such as with an enzyme linked immunosorbent assay (ELISA) using immobilized EFG proteins. If desired, the antibody molecules directed against EFG proteins can be isolated from the mammal (e.g., from the blood) and further purified by well known techniques, such as protein A chromatography to obtain the IgG fraction. At an appropriate time after immunization, e.g., when the anti-EFG antibody titers are highest, antibody-producing cells can be obtained from the subject and used to prepare monoclonal antibodies by standard techniques.

Any of the many well known protocols used for fusing lymphocytes and immortalized cell lines can be applied for the purpose of generating an anti-EFG monoclonal antibody (see, e.g., G. Galfre et al. (1977) Nature 266:55052; Gefter et al. Somatic Cell Genet., cited supra; Lerner, Yale J Biol. Med, cited supra; Kenneth, Monoclonal Antibodies, cited supra). Moreover, the ordinarily skilled worker will appreciate that there are many variations of such methods which also would be useful. Typically, the immortal cell line (e.g., a myeloma cell line) is derived from the same mammalian species as the lymphocytes. For example, murine hybridomas can be made by fusing lymphocytes from a mouse immunized with an immunogenic preparation of the present invention with an immortalized mouse cell line. Preferred immortal cell lines are mouse myeloma cell lines that are sensitive to culture medium containing hypoxanthine, aminopterin and thymidine ("HAT medium"). Any of a number of myeloma cell lines can be used as a fusion partner according to standard techniques, e.g., the P3-NS1/1-Ag4-1, P3-x63-Ag8.653 or Sp2/O-Ag14 myeloma lines. These myeloma lines are available from ATCC.

Alternative to preparing monoclonal antibody-secreting hybridomas, a monoclonal anti-EFG antibody can be identified and isolated by screening a recombinant combinatorial immunoglobulin library (e.g., an antibody phage display library) with EFG to thereby isolate immunoglobulin library members that bind EFG genes. Kits for generating and screening phage display libraries are commercially available (e.g., the Pharmacia Recombinant Phage Antibody System, Catalog No. 27-9400-01; and the Stratagene SurfZAP.TM. Phage Display Kit, Catalog No. 240612).

Additionally, recombinant anti-EFG antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art.

An anti-EFG antibody (e.g., monoclonal antibody) can be used to isolate EFG by standard techniques, such as affinity chromatography or immunoprecipitation. An anti-EFG antibody can facilitate the purification of natural EFG from cells and of recombinantly produced EFG expressed in host cells. Moreover, an anti-EFG antibody can be used to detect EFG protein (e.g., in a cellular lysate or cell supernatant) in order to evaluate the abundance and pattern of expression of the EFG protein. Anti-EFG antibodies can be used diagnostically to monitor protein levels in tissue as part of a clinical testing procedure, e.g., to, for example, determine the efficacy of a given treatment regimen.

### III. Recombinant Expression Vectors and Host Cells.

Another aspect of the invention pertains to vectors, preferably expression vectors, containing a nucleic acid encoding a EFG protein (or a portion thereof). Vectors may have particular use in the preparation of a recombinant protein of the invention.

As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions.

The recombinant expression vectors of the invention comprise a EFG nucleic acid of the invention in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (e.g., in an in vitro transcription/translation system or in a host cell when the vector is introduced into the host cell). The term "regulatory sequence" is intended to include promoters, enhancers and other expression control elements (e.g., polyadenylation signals). Such regulatory sequences are described, for example, in Goeddel; Gene Expression Technology: Methods in Enzymology 185, Academic Press, San Diego, Calif. (1990), the disclosure of which is incorporated herein by reference in its entirety. Regulatory sequences include those which direct constitutive expression of a nucleotide sequence in many types of host cell and those which direct expression of the nucleotide sequence only in certain host cells (e.g., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (e.g., EFG proteins, mutant forms of EFG proteins, fusion proteins, or fragments of any of the preceding proteins, etc.).

The recombinant expression vectors of the invention can be designed for expression of EFG proteins in prokaryotic or eukaryotic cells. For example, EFG proteins can be expressed in bacterial cells such as E. coli, insect cells (using baculovirus expression vectors) yeast cells, or mammalian cells. Suitable host cells are discussed further in Goeddel, Gene Expression Technology: Methods in Enzymology 185, Academic Press, San Diego, Calif. (1990), the disclosure of which is incorporated herein by reference in its entirety. Alternatively, the recombinant expression vector can be transcribed and translated in vitro, for example using T7 promoter regulatory sequences and T7 polymerase.

Expression of proteins in prokaryotes is most often carried out in E. coli with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes:1) to increase expression of recombinant protein; 2) to increase the solubility of the recombinant protein; and 3) to aid in the purification of the recombinant protein by acting as a ligand in affinity purification. Typical fusion expression vectors include pGEX (Pharmacia Biotech Inc; Smith, D. B. and Johnson, K. S. (1988) Gene 67:31-40), pMAL (New England Biolabs, Beverly, Mass.) and pRIT5 (Pharmacia, Piscataway, N.J.), the disclosures of which are incorporated herein by reference in their entireties, which fuse glutathione S-transferase (GST), maltose E binding protein, or protein A, respectively, to the target recombinant protein.

Purified fusion proteins can be utilized in EFG activity assays, (e.g., direct assays or competitive assays, or to generate antibodies specific for EFG proteins, for example.

The invention further provides a recombinant expression vector comprising a DNA molecule of the invention cloned into the expression vector in an antisense orientation. That is, the DNA molecule is operatively linked to a regulatory sequence in a manner which allows for expression (by transcription of the DNA molecule) of an RNA molecule which is antisense to EFG mRNA. Regulatory sequences operatively linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes see Weintraub, H. et al., Antisense RNA as a molecular tool for genetic analysis, Reviews--Trends in Genetics, Vol. 1(1) 1986, the disclosure of which is incorporated herein by reference in its entirety.
Of course, in the present invention, antisense vectors are particularly useful for inhibiting EFG genes expression, most preferably *A.fumigatus* EFG genes.
Antisense constructs may be designed to bind to the promoter and other control regions, exons, introns or even exon-intron boundaries of a gene. Antisense RNA constructs, or DNA encoding such antisense RNAs, may be employed to inhibit gene transcription or translation or both within a host cell, either in vitro or in vivo, such as within a host animal, including a human subject.
Although shorter oligomers are easier to make and increase in vivo accessibility, numerous other factors are involved in determining the specificity of hybridization. Both binding affinity and sequence specificity of an oligonucleotide to its complementary target increases with increasing length. It is contemplated that oligonucleotides of 8, 9, 10, 11, 12, 13, 14, 15, 16, 17, 18, 19, 20 or more base pairs will be used. One can readily determine whether a given antisense nucleic acid is effective at targeting of the corresponding host cell gene simply by testing the constructs in vitro to determine whether the endogenous gene's function is affected or whether the expression of related genes having complementary sequences is affected.

Another aspect of the invention pertains to host cells into which a recombinant expression vector of the invention has been introduced. The terms "host cell" and "recombinant host cell" are used interchangeably herein. It is understood that such term refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, a EFG protein can be expressed in bacterial cells such as E. coli, insect cells, yeast or mammalian cells (such as Chinese hamster ovary cells (CHO) or COS cells or human cells).

Vector DNA can be introduced into prokaryotic or eukaryotic cells via conventional transformation or transfection techniques, that can be found in Sambrook, et al. (Molecular Cloning: A Laboratory Manual. 2nd, ed., Cold Spring Harbor Laboratory, Cold Spring Harbor Laboratory Press, Cold Spring Harbor, N.Y., 1989, the disclosure of which is incorporated herein by reference in its entirety), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cells along with the gene of interest.

A host cell of the invention, such as a prokaryotic or eukaryotic host cell in culture, can be used to produce (i.e., express) a EFG protein. Accordingly, the invention further provides methods for producing a EFG protein using the host cells of the invention. In one embodiment, the method comprises culturing the host cell of invention (into which a recombinant expression vector encoding a EFG protein has been introduced) in a suitable medium such that a EFG protein is produced. In another embodiment, the method further comprises isolating a EFG protein from the medium or the host cell.

In another embodiment, the invention encompasses providing a cell capable of expressing a EFG protein, culturing said cell in a suitable medium such that a EFG protein is produced, and isolating or purifying the EFG protein from the medium or cell.

In certain indications, it may be desirable to activate transcription at specific times after administration of the gene therapy vector. This may be done with such promoters as those that are hormone or cytokine regulatable.

### IV. Drug screening assays.

The invention provides a method (also referred to herein as a "screening assay") for identifying inhibitors, i.e., candidate or test compounds or agents (e.g., preferably small molecules, but also peptides, peptidomimetics or other drugs) which bind to EFG proteins, have an inhibitory effect on, for example, EFG expression or preferably EFG activity, or have an inhibitory effect on, for example, the activity of an EFG target molecule. Assays may be cell based or non-cell based assays. Drug screening assays may be binding assays or more preferentially functional assays.

In preferred embodiments, an assay is a cell-based assay in which a cell which expresses a EFG protein or biologically active portion thereof is contacted with a test compound and the ability of the test compound to inhibit EFG activity determined. Determining the ability of the test compound to inhibit EFG activity can be accomplished by monitoring the bioactivity of the EFG protein or biologically active portion thereof.

The invention further encompasses compounds capable of inhibiting EFG activity. Inhibiting EFG activity refers to the inhibition of EFG gene expression such that fungus growth is inhibited. Preferably, a EFG inhibitor is a selective EFG inhibitor.

In a preferred embodiment, an inhibitor is capable of inhibiting EFG activity of at least one EFG protein of SEQ ID N°3,6,9,12,15,18,21,24,27,30,33,36,39,42,45,48,51,54,57,60,94,98,102,106,110,114,118, 122,126,130,134,138,142,146,150,154,158,162,166,170, or a fragment or a variant thereof as previously described. Compounds will be assayed for the activities indicated in Table 1 and Table 1bis.
For instance, the following standard enzymatic tests are appropriated : ATP dependant RNA helicase, guanylate kinase, RNA synthetase, SAM decarboxylase, protein kinase, ferro-chelatase.
Assays are made by using compounds already known to have an effect on the activity tested. For instance compounds known to inhibit protein kinase activity will be tested.

In one embodiment, the invention provides assays for screening candidate or test compounds which are target molecules of a EFG protein or polypeptide or biologically active portion thereof. In another embodiment, the invention provides assays for screening candidate or test compounds which bind to or modulate the activity of a EFG protein or polypeptide or biologically active portion thereof. The test compounds of the present invention can be obtained using any of the numerous approaches in combinatorial library methods known in the art, including: biological libraries; spatially addressable parallel solid phase or solution phase libraries; synthetic library methods requiring deconvolution; the 'one-bead one-compound' library method; and synthetic library methods using affinity chromatography selection. The biological library approach is used with peptide libraries, while the other four approaches are applicable to peptide, non-peptide oligomer or small molecule libraries of compounds (Lam, K. S. (1997) Anticancer Drug Des. 12:145).

Examples of methods for the synthesis of molecular libraries can be found in the art, for example in: DeWitt et al. (1993) Proc. Natl. Acad. Sci. U.S.A. 90:6909; Erb et al. (1994) Proc. Natl. Acad. Sci. USA 91:11422; Zuckermann et al. (1994). J. Med. Chem. 37:2678; Cho et al. (1993) Science 261:1303; Carrell et al. (1994) Angew. Chem. Int. Ed. Engl. 33:2059; Carell et al. (1994) Angew. Chem. Int. Ed. Engl. 33:2061; and in Gallop et al. (1994) J. Med. Chem. 37:1233.

Libraries of compounds may be presented in solution (e.g., Houghten (1992) Biotechniques 13:412-421), or on beads (Lam (1991) Nature 354:82-84), chips (Fodor (1993) Nature 364:555-556), bacteria (Ladner U.S. Pat. No. 5,223,409), spores (Ladner U.S. Pat. No. '409), plasmids (Cull et al. (1992) Proc Natl Acad Sci USA 89:1865-1869) or on phage (Scott and Smith (1990) Science 249:386-390); (Devin (1990) Science 249:404-406); (Cwirla et al. (1990) Proc. Natl. Acad. Sci. 87:6378-6382); (Felici (1991) J. Mol. Biol. 222:301-310); (Ladner supra.).

Determining the ability of the test compound to inhibit EFG activity can also be accomplished, for example, by coupling the EFG protein or biologically active portion thereof with a radioisotope or enzymatic label such that binding of the EFG protein or biologically active portion thereof to its cognate target molecule can be determined by detecting the labeled EFG protein or biologically active portion thereof in a complex. For example, compounds (e.g., EFG protein or biologically active portion thereof) can be labeled with.sup.125 I, .sup.35 S, .sup.14 C, or .sup.3 H, either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

It is also within the scope of this invention to determine the ability of a compound (e.g., EFG protein or biologically active portion thereof) to interact with its cognate target molecule without the labeling of any of the interactants. For example, a microphysiometer can be used to detect the interaction of a compound with its cognate target molecule without the labeling of either the compound or the receptor. McConnell, H. M. et al. (1992) Science 257:1906-1912. A microphysiometer such as a cytosensor is an analytical instrument that measures the rate at which a cell acidifies its environment using a light-addressable potentiometric sensor (LAPS). Changes in this acidification rate can be used as an indicator of the interaction between compound and receptor.

In a preferred embodiment, the assay comprises contacting a cell which expresses a EFG protein or biologically active portion thereof, with a target molecule to form an assay mixture, contacting the assay mixture with a test compound, and determining the ability of the test compound to inhibit the activity of the EFG protein or biologically active portion thereof, wherein determining the ability of the test compound to inhibit the activity of the EFG protein or biologically active portion thereof, comprises determining the ability of the test compound to inhibit a biological activity of the EFG expressing cell (e.g., determining the ability of the test compound to inhibit transduction or protein: protein interactions).

In another preferred embodiment, the assay comprises contacting a cell which is responsive to a EFG protein or biologically active portion thereof, with a EFG protein or biologically-active portion thereof, to form an assay mixture, contacting the assay mixture with a test compound, and determining the ability of the test compound to modulate the activity of the EFG protein or biologically active portion thereof, wherein determining the ability of the test compound to modulate the activity of the EFG protein or biologically active portion thereof comprises determining the ability of the test compound to modulate a biological activity of the EFG gene-responsive cell (e.g., determining the ability of the test compound to modulate signal transduction or protein:protein interactions).

In another embodiment, an assay is a cell-based assay comprising contacting a cell expressing a EFG target molecule with a test compound and determining the ability of the test compound to modulate (e.g. stimulate or inhibit) the activity of the EFG target molecule. Determining the ability of the test compound to modulate the activity of a EFG target molecule can be accomplished, for example, by determining the ability of the EFG protein to bind to or interact with the EFG target molecule.

Determining the ability of the EFG protein to bind to or interact with a EFG target molecule can be accomplished by one of the methods described above for determining direct binding. In a preferred embodiment, determining the ability of the EFG protein to bind to or interact with a EFG target molecule can be accomplished by determining the activity of the target molecule. For example, the activity of the target molecule can be determined by detecting induction of a cellular second messenger of the target, detecting catalytic/enzymatic activity of the target an appropriate substrate, detecting the induction of a reporter gene (comprising a target-responsive regulatory element operatively linked to a nucleic acid encoding a detectable marker, e.g., luciferase), or detecting a target-regulated cellular response, for example, signal transduction or protein:protein interactions.

In yet another embodiment, an assay of the present invention is a cell-free assay in which a EFG protein or biologically active portion thereof is contacted with a test compound and the ability of the test compound to bind to the EFG protein or biologically active portion thereof is determined. Binding of the test compound to the EFG protein can be determined either directly or indirectly as described above. In a preferred embodiment, the assay includes contacting the EFG protein or biologically active portion thereof with a known compound which binds EFG (e.g., a EFG target molecule) to form an assay mixture, contacting the assay mixture with a test compound, and determining the ability of the test compound to interact with a EFG protein, wherein determining the ability of the test compound to interact with a EFG protein comprises determining the ability of the test compound to preferentially bind to EFG or biologically active portion thereof as compared to the known compound.

In another embodiment, the assay is a cell-free assay in which a EFG protein or biologically active portion thereof is contacted with a test compound and the ability of the test compound to modulate (preferably inhibit) the activity of the EFG protein or biologically active portion thereof is determined. Determining the ability of the test compound to modulate the activity of a EFG protein can be accomplished, for example, by determining the ability of the EFG protein to bind to a EFG target molecule by one of the methods described above for determining direct binding. Determining the ability of the EFG protein to bind to a EFG target molecule can also be accomplished using a technology such as real-time Biomolecular Interaction Analysis (BIA). Sjolander, S. and Urbaniczky, C. (1991) Anal. Chem. 63:2338-2345 and Szabo et al. (1995) Curr. Opin. Struct. Biol. 5:699-705. As used herein, "BIA" is a technology for studying biospecific interactions in real time, without labeling any of the interactants (e.g., BIAcore). Changes in the optical phenomenon of surface plasmon resonance (SPR) can be used as an indication of real-time reactions between biological molecules.

In an alternative embodiment, determining the ability of the test compound to modulate the activity of a EFG protein can be accomplished by determining the ability of the EFG protein to further modulate the activity of a downstream effector (e.g., a growth factor mediated signal transduction pathway component) of a EFG target molecule. For example, the activity of the effector molecule on an appropriate target can be determined or the binding of the effector to an appropriate target can be determined as previously described.

In yet another embodiment, the cell-free assay involves contacting a EFG protein or biologically active portion thereof with a known compound which binds the EFG protein to form an assay mixture, contacting the assay mixture with a test compound, and determining the ability of the test compound to interact with the EFG protein, wherein determining the ability of the test compound to interact with the EFG protein comprises determining the ability of the EFG protein to preferentially bind to or modulate the activity of a EFG target molecule.

The cell-free assays of the present invention are amenable to use of both soluble and/or membrane-bound forms of isolated proteins (e.g. EFG proteins or biologically active portions thereof or molecules to which EFG targets bind). In the case of cell-free assays in which a membrane-bound form an isolated protein is used it may be desirable to utilize a solubilizing agent such that the membrane-bound form of the isolated protein is maintained in solution.

In more than one embodiment of the above assay methods of the present invention, it may be desirable to immobilize either EFG or its target molecule to facilitate separation of complexed from uncomplexed forms of one or both of the proteins, as well as to accommodate automation of the assay. Binding of a test compound to a EFG protein, or interaction of a EFG protein with a target molecule in the presence and absence of a candidate compound, can be accomplished in any vessel suitable for containing the reactants. Examples of such vessels include microtitre plates, test tubes, and micro-centrifuge tubes. In one embodiment, a fusion protein can be provided which adds a domain that allows one or both of the proteins to be bound to a matrix. For example, glutathione-S-transferase/EFG fusion proteins or glutathione-S-transferase/target fusion proteins can be adsorbed onto glutathione sepharose beads (Sigma Chemical, St. Louis, Mo.) or glutathione derivatized microtitre plates, which are then combined with the test compound or the test compound and either the non-adsorbed target protein or EFG protein, and the mixture incubated under conditions conducive to complex formation (e.g., at physiological conditions for salt and pH). Following incubation, the beads or microtitre plate wells are washed to remove any unbound components, the matrix immobilized in the case of beads, complex determined either directly or indirectly, for example, as described above. Alternatively, the complexes can be dissociated from the matrix, and the level of EFG binding or activity determined using standard techniques.

Other techniques for immobilizing proteins on matrices can also be used in the screening assays of the invention. For example, either a EFG protein or a EFG target molecule can be immobilized utilizing conjugation of biotin and streptavidin. Biotinylated EFG protein or target molecules can be prepared from biotin-NHS (N-hydroxy-succinimide) using techniques well known in the art (e.g., biotinylation kit, Pierce Chemicals, Rockford, Ill.), and immobilized in the wells of streptavidin-coated 96 well plates (Pierce Chemical). Alternatively, antibodies reactive with EFG protein or target molecules but which do not interfere with binding of the EFG protein to its target molecule can be derivatized to the wells of the plate, and unbound target or EFG protein trapped in the wells by antibody conjugation. Methods for detecting such complexes, in addition to those described above for the GST-immobilized complexes, include immunodetection of complexes using antibodies reactive with the EFG protein or target molecule, as well as enzyme-linked assays which rely on detecting an enzymatic activity associated with the EFG protein or target molecule.

In another embodiment, modulators of EFG expression are identified in a method wherein a cell is contacted with a candidate compound and the expression of EFG mRNA or protein in the cell is determined. The level of expression of EFG mRNA or protein in the presence of the candidate compound is compared to the level of expression of EFG mRNA or protein in the absence of the candidate compound. The candidate compound can then be identified as a modulator of EFG expression based on this comparison. For example, when expression of EFG mRNA or protein is greater (statistically significantly greater) in the presence of the candidate compound than in its absence, the candidate compound is identified as a stimulator of EFG mRNA or protein expression. Alternatively, when expression of EFG mRNA or protein is less (statistically significantly less) in the presence of the candidate compound than in its absence, the candidate compound is identified as an inhibitor of EFG mRNA or protein expression. The level of EFG mRNA or protein expression in the cells can be determined by methods described herein for detecting EFG mRNA or protein.

In yet another aspect of the invention, the EFG proteins can be used as "bait proteins" in a two-hybrid assay or three-hybrid assay (see, e.g., U.S. Pat. No. 5,283,317; Zervos et al. (1993) Cell 72:223-232).

This invention further pertains to novel agents identified by the above-described screening assays and to processes for producing such agents by use of these assays. Accordingly, in one embodiment, the present invention includes a compound or agent obtainable by a method comprising the steps of any one of the aformentioned screening assays (e.g., cell-based assays or cell-free assays). For example, in one embodiment, the invention includes a compound or agent obtainable by a method comprising contacting a cell which expresses a EFG target molecule with a test compound and the determining the ability of the test compound to bind to, or modulate the activity of, the EFG target molecule. In another embodiment, the invention includes a compound or agent obtainable by a method comprising contacting a cell which expresses a EFG target molecule with a EFG protein or biologically-active portion thereof, to form an assay mixture, contacting the assay mixture with a test compound, and determining the ability of the test compound to interact with, or modulate the activity of, the EFG target molecule. In another embodiment, the invention includes a compound or agent obtainable by a method comprising contacting a EFG protein or biologically active portion thereof with a test compound and determining the ability of the test compound to bind to, or modulate (e.g., stimulate or inhibit) the activity of, the EFG protein or biologically active portion thereof. In yet another embodiment, the present invention included a compound or agent obtainable by a method comprising contacting a EFG protein or biologically active portion thereof with a known compound which binds the EFG protein to form an assay mixture, contacting the assay mixture with a test compound, and determining the ability of the test compound to interact with, or modulate the activity of the EFG protein.

Accordingly, it is within the scope of this invention to further use an agent identified as described herein in an appropriate animal model. For example, an agent identified as described herein (e.g., a EFG modulating agent, an antisense EFG nucleic acid molecule, a EFG-specific antibody, or a EFG-binding partner) can be used in an animal model to determine the efficacy, toxicity, or side effects of treatment with such an agent. Alternatively, an agent identified as described herein can be used in an animal model to determine the mechanism of action of such an agent. Furthermore, this invention pertains to uses of novel agents identified by the above-described screening assays for treatments as described herein.

The present invention also pertains to uses of novel agents identified by the above-described screening assays for diagnoses, prognoses, and treatments as described herein. Accordingly, it is within the scope of the present invention to use such agents in the design, formulation, synthesis, manufacture, and/or production of a drug or pharmaceutical composition for use in diagnosis, prognosis, or treatment, as described herein. For example, in one embodiment, the present invention includes a method of synthesizing or producing a drug or pharmaceutical composition by reference to the structure and/or properties of a compound obtainable by one of the above-described screening assays. For example, a drug or pharmaceutical composition can be synthesized based on the structure and/or properties of a compound obtained by a method in which a cell which expresses a EFG target molecule is contacted with a test compound and the ability of the test compound to bind to, or modulate the activity of, the EFG target molecule is determined. In another exemplary embodiment, the present invention includes a method of synthesizing or producing a drug or pharmaceutical composition based on the structure and/or properties of a compound obtainable by a method in which a EFG protein or biologically active portion thereof is contacted with a test compound and the ability of the test compound to bind to, or inhibit the activity of, the EFG protein or biologically active portion thereof is determined.

### V. Methods of treatment.

EFG inhibitors identified according to the methods in the section titled "Drug Screening Assays" can be further tested for their ability to ameliorate or prevent the pathologies associated to *Aspergillus* fungus, and more particularly to *A.fumigatus,* namely invasive pulmonary aspergillosis.

An "individual" treated by the methods of this invention is a vertebrate, particularly a mammal (including model animals of human disease, farm animals, sport animals, and pets), and typically a human.

"Treatment" refers to clinical intervention in an attempt to alter the natural course of the individual being treated, and may be performed either for prophylaxis or during the course of clinical pathology. Desirable effects include preventing occurrence or recurrence of disease, alleviation of symptoms, diminishment of any direct or indirect pathological consequences of the disease, such as hyperresponsiveness, inflammation, or necrosis, lowering the rate of disease progression, amelioration or palliation of the disease state, and remission or improved prognosis. The "pathology" associated with a disease condition is anything that compromises the well-being, normal physiology, or quality of life of the affected individual.

Treatment is performed by administering an effective amount of a EFG inhibitor. An "effective amount" is an amount sufficient to effect a beneficial or desired clinical result, and can be administered in one or more doses.

The criteria for assessing response to therapeutic modalities employing the compositions of this invention are dictated by the specific condition, measured according to standard medical procedures appropriate for the condition.

### VI. Pharmaceutical Compositions.

Compounds capable of inhibiting EFG activity, preferably small molecules but also including peptides, EFG antisens nucleic acid molecules, EFG proteins inhibitors, and anti-EFG antibodies (also referred to herein as "active compounds") of the invention can be incorporated into pharmaceutical compositions suitable for administration. Such compositions typically comprise a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Supplementary active compounds can also be incorporated into the compositions.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL.TM. (BASF, Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Where the active compound is a protein, peptide or anti-EFG antibody, sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. For administration by inhalation, the compounds are delivered in the form of an aerosol spray from pressured container or dispenser which contains a suitable propellant, e.g., a gas such as carbon dioxide, or a nebulizer. Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art. Most preferably, active compound is delivered to a subject by intravenous injection.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art, for example, as described in U.S. Pat. No. 4,522,811, or are commercially available.

It is especially advantageous to formulate oral or preferably parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

The pharmaceutical compositions can be included in a container, pack, or dispenser together with instructions for administration.

### VII. Diagnostic and Prognostic Uses

The nucleic acid molecules, proteins, protein homologues, and antibodies described herein can be used in one or more of the following methods: diagnostic assays, prognostic assays, monitoring clinical trials, and pharmacogenetics; and in drug screening and methods of treatment (e.g., therapeutic and prophylactic) as further described herein.

The invention provides diagnostic and prognositc assays for detecting EFG members, as further described. Also provided are diagnostic and prognostic assays for detecting interactions between EFG members and EFG target molecules.

The isolated nucleic acid molecules of the invention can be used, for example, to detect EFG mRNA (e.g., in a biological sample) or a genetic alteration in a EFG gene, and to modulate a EFG activity, as described further below. The EFG proteins can be used to screen for drugs or compounds which modulate, preferably inhibit EFG activity.

Accordingly one embodiment of the present invention involves a method of use (e.g., a diagnostic assay, prognostic assay, or a prophylactic/therapeutic method of treatment) wherein a molecule of the present invention (e.g., a EFG protein, EFG nucleic acid, or most preferably a EFG inhibitor or activator) is used.

### VIII. Fungicidal Compositions.

The invention also deals with fungicidal incorporating at least one compound capable to inhibit fungal growth, by inhibiting EFG genes, in particular in diseases of plants due to phytopathogenic fungi, namely *Botrytis cinerea, Mycosphaerella graminicola, Stagnospora nodorum, Blumeria graminis, Colleotrichum lindemuthianum, Puccinia graminis, Leptosphaeria maculans, Fusarium oxysporum, Fusarium graminearum, Venturia inaequalis,* most preferably fungi of the genus *Magnaporthe,* even most preferably *Magnaporthe grisea.*

The invention thus also provides a method of combating fungi at a locus infested or liable to be infested therewith, which comprises applying to the locus the compound of the invention.

The invention also provides an agricultural composition comprising the compound of the invention in admixture with an agriculturally acceptable diluent or carrier.

The composition can comprise one or more additional active ingredients, for example compounds known to possess plant-growth regulant, herbicidal, fungicidal (such as metalaxyl, oxadixyl, ofurace, benalaxyl and furalaxyl; cymoxanil; mancozeb; chlorothalonil; folpet; captan; famoxadone; fenamidone; spiroxamine; fluazinam; dimethomorph; strobilurins, such as kresoxim-methyl, azoxystrobin and trifloxystrobin, pyrimethanil, cyprodinil; mepanipyrim; and iprodione), insecticidal or acaricidal properties.

The composition of the invention may include for example a dispersing agent, emulsifying agent or wetting agent. Usually they are in the form of an aqueous concentrate.

The concentration of the active ingredient in the composition of the present invention, as applied to plants is preferably within the range of 0.0001 to 1.0 percent by weight, especially 0.0001 to 0.01 percent by weight. In a primary composition, the amount of active ingredient can vary widely and can be, for example, from 5 to 95 percent by weight of the composition.

In the method of the invention, the compound is generally applied to seeds, plants or their habitat. Thus, the compound can be applied directly to the soil before, at or after drilling so that the presence of active compound in the soil can control the growth of fungi which may attack seeds. When the soil is treated directly the active compound can be applied in any manner which allows it to be intimately mixed with the soil such as by spraying, by broadcasting a solid form of granules, or by applying the active ingredient at the same time as drilling by inserting it in the same drill as the seeds. A suitable application rate is within the range of from 5 to 1000 g per hectare, more preferably from 10 to 500 g per hectare.

Alternatively, the active compound can be applied directly to the plant by, for example, spraying or dusting either at the time when the fungus has begun to appear on the plant or before the appearance of fungus as a protective measure. In both such cases the preferred mode of application is by foliar spraying. It is generally important to obtain good control of fungi in the early stages of plant growth as this is the time when the plant can be most severely damaged. The spray or dust can conveniently contain a pre- or post-emergence herbicide if this is thought necessary. Sometimes, it is practicable to treat the roots of a plant before or during planting, for example, by dipping the roots in a suitable liquid or solid composition. When the active compound is applied directly to the plant a suitable rate of application is from 0.025 to 5 kg per hectare, preferably from 0.05 to 1 kg per hectare.

Having generally described this invention, a further understanding can be obtained by reference to certain specific examples which are provided herein for purposes of illustration only, and are not intended to be limiting unless otherwise specified.

### Example 1: A. fumigatus strain construction.

Media and growth conditions were as follows. A. *fumigatus* strains were propagated at 37°C on complete medium or minimal medium with 0.5 mM of various nitrogen sources (sodium glutamate, ammonium tartrate, sodium nitrate, sodium nitrite and hypoxanthine) (Cove 1966). Uridine and uracil were added at a concentration of 5 mM when appropriate. Liquid cultures used for DNA-mediated transformation and genomic DNA preparation were grown in YG (0.5% Yeast Extract, 2% glucose). DNA-mediated transformation was achieved either on protoplasts as described previously (d'Enfert 1996; Osmani *et al.* 1987) or by electroporation of intact conidia as described (Weidner *et al.* 1998).

*A. fumigatus* stable diploids appropriate for transposon mutagenesis were obtained using the following procedure. In summary, insertional mutagenesis (Weidner *et al.*1998) of strain CEA17 has led to the isolation of spore color mutants CEA82 and CEA85. White strain CEA88 and reddish strain CEA94 are chlorate resistant derivatives of CEA82 and CEA85 with uncharacterized mutations in a gene involved in the biosynthesis of the molybdene cofactor (*cnx*) and in the nitrate reductase gene (*niaD*), respectively. Strains CEA125 (*w1, cnx1, pyrG1*) and CEA129 *(r7, niaD2, pyrG1*) were obtained from strains CEA88 and CEA94 by growth on media containing 5-fluoro-orotic acid (1 mg/ml) which selects for *pyrG* mutants. Simultaneous growth of CEA125 and CEA129 on minimal medium with nitrate as sole nitrogen source yielded heterokaryons that produced grey-green spores similar to that of *A. fumigatus* haploid wild-type strains. This led to the isolation of the stable diploid strain CEA131 (*w1*/+,+/*r7, cnx1*/+, +/*niaD2, pyrG1*/*pyrG1*). A chlorate resistant derivative of CEA131 was identified that was unable to use nitrate as the sole nitrogen source and was defective at both *niaD* alleles. This strain is referred to as CEA153 (*w1*/+,+/*r7, cnx1*/+, *niaD4*/*niaD2, pyrG1*/*pyrG1*). On minimal medium containing nitrate, spontaneous reversion of the haploid strain CEA113 and the diploid strain CEA153 for nitrate utilization was not observed.

### Example 2: in vivo transposon mutagenesis in A. fumigatus.

Plasmid pNIL160 has been described³⁰. A 2.2 kb *Bam*HI fragment from ppyrG containing the *A. nidulans pyrG* gene was cloned at the *Nhe*I restriction site in *impala160,* yielding pNIpyr. *Nde*I-digested pNIpyr was introduced into genomic DNA of strains CEA113 and CEA153 by electroporation of intact conidia as described¹⁹ yielding the haploid strain CEA165 and the diploid strains CEA225, 226 and 227, respectively. *impala160::pyrG* transposition occurs on minimal medium containing nitrate supplemented with 0.02% Triton X-100 at 37°C for 3 days. Genomic DNA preparation and Southern analysis techniques were essentially performed according to Sambrook *et al.* (1989) and Ausubel *et al.* (1992).

Plasmid pNIpyr, a derivative of pNIL160³⁰, carries the *A. nidulans niaD* gene encoding nitrate reductase with a copy of *impala160* inserted 10 bp upstream of the translation initiation codon of *niaD* and modified by the insertion of the *A. nidulans pyrG* gene between the 3'-end of the transposase-encoding gene and the 3' inverted terminal repeat. pNIpyr was introduced in *A. fumigatus* strain CEA153, a *stable pyrG, niaD* diploid strain, heterozygous for spore color markers. Because of the insertion of *impala160::pyrG* into the *niaD* promoter, the *niaD* allele carried by pNIpyr is not functional. However, when diploid transformants were grown on selective minimal medium with nitrate as sole nitrogen source, pyrG⁺, NiaD⁺ revertants were observed at a frequency of 10⁻⁵-10⁻⁶. Southern analysis showed that these transformants have only one copy of *impala160::pyrG* integrated into their genome and that all the revertants resulted of *impala* transposition events from the *niaD* promoter to an apparent random site located elsewhere in the *A. fumigatus* genome (Fig. 2). Sequence analysis of the *niaD* promoter region in all revertants revealed a footprint of usually 5 bp associated with *impala* excision. Characterization of integration targets by sequencing and comparison to public genomic sequences (Tables 1 and 1bis, and data not shown) revealed that the transposition of *impala160::pyrG* in *A. fumigatus* 1) occurs at a genomic TA dinucleotide which is duplicated during the integration process; 2) is apparently random without sequence preference (except the TA dinucleotide); and 3) is not associated with genomic rearrangements. All of these characteristics are typical for transposition of the *Tc1-mariner* family members and was previously observed for *impala160* transposition events in *F. oxysporum²⁸, A. nidulans²⁹* and *M. grisea³⁰.* Therefore, *impala* appears as the most suitable tool to generate random tagged mutation in *A. fumigatus* since insertional mutagenesis through DNA-mediated transformation results in various types of rearrangements of the transforming and genomic DNA.

### Example 3: parasexual genetic screening.

Haploidization of A. *fumigatus* diploid strains was conducted on selective haploidization medium [complete medium containing 1.2 µg/ml benomyl (ALDRICH, 10 mg/ml in DMSO)] or on non-selective haploidization medium (selective haploidization medium plus uridine and uracil) for 5 days at 37°C. Haploid progenies are easily identified by the production of white and reddish-colored sectors after haploidization of grey-green diploid strains.

Three diploid transformants (namely *A. fumigatus* CEA225, CEA226 and CEA227) were used to generate a collection of random diploid heterozygous revertants. Haploidization of heterozygous strains was induced by the destabilizing reagent benomyl and results from mitotic chromosomal non-disjunction^{18,31}. Since each revertant has a single mutated chromosomal locus tagged by *impala160::pyrG,* parasexual genetics on selective and non-selective haploidization media permits to distinguish insertions that occur in non-essential versus essential chromosomal targets (Fig. 1). Strains CEA225, CEA226, CEA227 and 97% of 2,386 revertants showed no difference on selective and non-selective haploidization media after two independent tests, indicating that integration of pNIpyr into the genome of the parental transformants and integration of *impala160:.pyrG* in the diploid revertants had not occurred into an essential chromosomal region. On selective haploidization medium, 73 revertants (3%) did not yield haploid conidia as evidenced by the absence of colored sectors (Fig. 3). Diploid strains of *A. fumigatus* are hypersensitive to benomyl and only haploid strains can grow at the benomyl concentration used³¹. However, the transient formation of aneuploids leads to the formation of a cal on selective haploidization medium that potentially over-grow haploid strains with morphological defects. To identify mutants which could have been selected in our screening because of the slow growth of haploid progenies rather than the lethality of the insertion, *ca.* 10⁶ haploid progenies obtained on non-selective haploidization medium were tested for the presence of *impala160::pyrG* by growth on selective medium. Twenty nine diploid revertants (29/2,386 = 1.2%) never yielded haploid pyrG⁺ progenies and were defined as carrying an integration of *impala160::pyrG* into a chromosomal locus essential for *A. fumigatus* growth.

### Example 4A: sequence determination.

Genomic sequences bordering *impala160::pyrG* are determined by an adaptation of a two-step PCR strategy developed by Chun *et al.*³⁶ and using transposon specific primers. More precisely concerning the two step PCR, first, ca. 100 ng of genomic DNA were amplified in 50 µl using oligonucleotides ppyrl and PCRall or ppyr3 and PCRall (4 pmol/µl final) and the following amplification protocol: a denaturation step at 94°C for 3 min. followed by 5 cycles of the following steps: denaturation at 94°C for 30 sec, annealing at 35°C for 30 sec, extension at 72°C for 1 min, and 30 cycles of the following steps: denaturation at 94°C for 30 sec, annealing at 45°C for 30 sec, extension at 72°C for 1 min. A last elongation step was done at 72°C for 3 min. Final concentrations for MgCl₂ and dNTPs were 3 mM and 0.2 mM, respectively. One microliter of the PCR reaction was subjected to a second amplification using similar reaction conditions and oligonucleotides ppyr2 and PCRa12N (if ppyrl and PCRall had been used in the first reaction) or ppyr4 and PCRa12N (if ppyr3 and PCRall had been used in the first reaction). The following amplification protocol was used: 30 cycles of the following steps: denaturation at 94°C for 30 sec, annealing at 60°C for 30 sec, extension at 72°C for 1 min. A last elongation step was done at 72°C for 3 min. In some instances, oligonucleotides PCRal3, PCRa14, and PCRa15 were used in place of PCRall. PCR products were separated by electrophoresis on a 2% TBE-agarose gel and major PCR products were purified with the Qiaquick Gel Purification Kit (Qiagen, France) according to the supplier's instructions. Purified PCR products are sequenced using ppyr2 or ppyr4 as primers (ESGS, Evry, France). Nucleotide sequences obtained in this manner and trimmed for ppyrG sequences are compared using blastx or blastn (Altschul *et* al. 1990) to protein databases and to the preliminary sequence data of the A. fumigatus genome project which were obtained from The Institute for Genomic Research (TIGR) website (http://www.tigr.org).

More precisely concerning the transposon specific primers, two primers were used [primers Imp1: ATGAAGGCGTAAGTTCCTTGC (SEQ ID No.61) and Imp2: GTGTGGAGGAAGAAAGAGC (SEQ ID No.62)]. Sequencing reactions were performed by ESGS (Evry, France) with the primer Imp2 directly on the major PCR product purified from agarose gel using the Qiaquick gel extraction kit (QIAGEN). After elimination of transposon sequences, genomic tags were compared to the *A. fumigatus* TIGR genomic data (www.tigr.org). Results present in this study were obtained from the sequence release of November 14, 2001. At this time, the shotgun sequencing of A. *fumigatus* progressed to 6X sequence coverage (28.7 Mb in 1,578 assemblies of over 1,000 bp in size). From TIGR data, specific primers were designed and used in standard PCR reactions and confirmed the absence of genomic rearrangements after transposon integration and the presence of a wild type chromosomal locus in each of the diploid revertants tested.

**Table 2. Primer sequences**

| | |
|---|---|
| ppyr1 (5'end) | GGAAGACGGGCAGTTAGTCC (SEQ ID No.63) |
| ppyr3 (3'end) | CCCAGGCTTTACACTTTATGC (SEQ ID No.64) |
| PCRal 1 | GGCCACGCGTCGACTAGTAG(N)₁₀GATAT (SEQ ID No.65) |
| PCRal 3 | GGCCACGCGTCGACTAGTAC(N)₁₀ACGTC (SEQ ID No.66) |
| PCRal 4 | GGCCACGCGTCGACTAGTAC(N)₁₀TGGAC (SEQ ID No.67) |
| PCRal 5 | GGCCACGCGTCGACTAGTAC(N)₁₀ACGTG (SEQ ID No.68) |
| ppyr2N (5'end) | CGAAGTTGACGTTCAGTATGC (SEQ ID No.69) |
| ppyr4N (3'end) | TGACCATGATTACGCCAAGC (SEQ ID No.70) |
| PCRal 2N | GGCCACGCGTCGACTAGTAC (SEQ ID No.71) |

Ppyr primers are specific of the ppyrG plasmid used for insertional mutagenesis. The pentamer at the 5' end of the random primer (PCRall, 3, 4 and 5) is expected to bind once in a kb. It is chosen according to the GC% of A. fumigatus (ca.50%) and does not occur in the region of ppyrG located between ppyr primers and the linearized plasmid end.

### Example 4B: characterization of A. fumigatus essential genes.

Genomic sequences bordering *impala160::pyrG* were obtained for 21 of the 29 diploid strains mentioned above. Except for one strain (4-1-3), corresponding genomic regions were identified (Table 1 and Table 1bis) in the public preliminary sequence data of the *A. fumigatus* genome available at The Institute for Genomic Research (http://www.tigr.org). Similarity searches of the NCBI non-redundant sequence database performed using the BLASTx algorithm³² identified three main categories of insertional mutants (Tables 1 and 1bis). The first category includes 15 strains that have an insertion of *impala160::pyrG* into genes with homologues in other fungal species. The second category is composed of three strains with *impala160::pyrG* integration occurring into intergenic regions. The last category includes two strains with *impala160::pyrG* integration in genes without homologues in public databases and classified as A. *fumigatus* specific essential genes.

In nine of the fifteen strains of the first category, integration of *impala160::pyrG* occurs into homologues of genes demonstrated as essential for *S*. *cerevisiae* growth (Tables 1 and Ibis). These genes are involved in a broad range of essential biological processes such as protein synthesis (*YGL245W*), protein maturation *(WBPI)* and protein transport (*SRP101*), nuclear architecture (*NAR1*), RNA processing (*DBD10*), nucleotide metabolism (*GUK1*), chromatine structure (*RSC9*) and cell cycle control (*CDC27*). Four additional *impala160::pyrG* integrations occur into genes encoding homologues of non-essential S. *cerevisiae* proteins for which essentiality in *A. fumigatus* is not unexpected: ribosomal proteins *RPL1* and *RPL17* are duplicated in the yeast genome and therefore are not independently essential although the double mutation is lethal; a null mutation of *MSW1* encoding the yeast tryptophanyl-tRNA synthetase localized in mitochondria leads to a slow growth phenotype; and *S*; *cerevisiae gos1* null mutant fail to germinate at 37°C, the temperature of the lethality screen.

Two genes encoding homologues of *S*. *cerevisiae* proteins that are not essential for yeast growth, namely Rim11p and Yfl034w (Tables 1 and Ibis), have been identified as essential for *A. fumigatus* growth. These proteins are conserved among lower eukaryotes but their role has not been precisely evaluated yet. Analysis of the Rim11p and its *Schizosaccharomyces pombe* counterpart *(SKPI)* suggest that these protein might be involved in spore formation and for mitosis, although they are not essential for viability in these two species. That the corresponding homologues are essential in *A. fumigatus* might be explained by their implication in additional essential pathways and for discrepencies in the contribution of similar biological pathways to the biology of *A. fumigatus* and other lower eukaryotes. The second class of mutants includes three revertants in which transposon integration occurs in the vicinity (<200 bp) of the deduced translation initiation codon of three genes which are likely to be essential for *A. fumigatus* growth based to their homology to genes essential for S. *cerevisiae* growth: *RPL14* (revertant 10-304) encodes an essential ribosomal protein and *COX10* (revertant 10-175) and *HEM15* (revertant 11-4-9) are required for heme biosynthesis (Table1). It is likely that *impala* integration prevents proper expression of these three genes but the inventors cannot exclude an additional effect on genes divergently transcribed from these intergenic regions.

### Example 5: impala transposition characteristics.

The correlation between the parasexual genetics phenotype and the nature of the mutated genes supports the idea that genes genuinely essential for *A. fumigatus* growth can be identified among diploid heterozygous mutants. However, the proportion of *impala160::pyrG* integration in essential genes observed in this study (1.2%) is lower than expected. In *S*. *cerevisiae,* 17% of the 6,200 genes are essentia1¹³. Considering a similar frequency of essential genes in *A. fumigatus,* a genome size of 30 Mb with approximately 8000 genes, the inventors have estimated at 8 to 10% the frequency of heterozygous diploids that should have an essential phenotype after parasexual genetics. In an attempt to understand the observed lower frequency, the inventors have characterized insertions of *impala160::pyrG* in different contexts. In fourteen diploid revertants not classified as essential but that showed an altered growth pattern on selective haploidization medium only two revertants had an insertion of *impala160::pyrG* in ORFs greater than two kb and these were not similar to known proteins. Interestingly, five *impala160::pyrG* integration (36%) were located 5' (<200 bp) of the deduced translation initiation codon of genes which have homologues in databases, including a tRNA seryl transferase essential in S. *cerevisiae.* In 18 random diploid strains with a non-essential phenotype only one transposon insertion occured in a protein-coding region, corresponding to the homologue of the non-essential *A*. *nidulans amdA* gene, while five insertions (28%) were found 5' (<300 bp) of start codons of homologues of non-essential *S. cerevisiae* genes (data not shown). Similar results have been obtained upon analysis of *impala160::pyrG* integrations in a haploid *A. fumigatus* strain (data not shown). These results suggest that *impala160::pyrG* has a tendency to insert preferentially into noncoding regions thus resulting in phenotypically silent mutations as already observed for other transposons³⁴. However, the inventors positive screening by parasexual analysis enrich for insertions that lie predominantly in ORFs.

In summary, through the analysis of 2,364 *A. fumigatus* diploid insertional mutants the inventors have been able to identify 21 previously uncharacterized essential genes (SEQ IDN°1,4,7,10,13,16,19,22,25,28,31,34,37,40,43,46,49,52,55,58,92,96,100,104,108,112,116, 120,124,128,132,136,140,144,148,152,156,160,164,168) several of which could not have been predicted as essential for *A. fumigatus* growth based on the knowledge gained from studies in other fungal species. Despite an apparent not truly random distribution of the *impala* element, transposon mutagenesis is a promising tool for insertional mutagenesis in filamentous fungi. A large scale genomic approach is now underway for the systematic identification of essential *A. fumigatus* genes by automatisation of the strategy. This represent an important step for the definition of novel antifungal treatments. Interestingly, the inventors have observed that some heterozygous diploid strains with an integration of *impala* in genes necessary for efficient growth show reduced growth compared to a parental diploid strain (data not shown). In *S. cerevisiae* or *C*. *albicans,* several heterozygous diploids with a mutation in a gene essential for growth also show reduced growth or increased sensitivity to drugs targeting the corresponding gene product^{15,35}. This phenomenon, referred to as haploinsufficiency, can form the basis for the identification of antifungal components that target the product of the mutated gene^{15,35}. In order to achieve the results described the inventors used the following experimental protocols.

### Example 6: cDNA analysis.

PCR were carried on DNA prepared from a *A. fumigatus* cDNA library in order to:
1) check for expression of a subset of genes identified by transposon;
2) confirm the location of postulated exon/intron splice sites; and
3) identify transcription start sites and polyadenylation sites.

The *A. fumigatus* cDNA library was obtained from M. Monod (CHUV, Lausanne, Switzerland). It was constructed by InVitrogen using cDNA prepared from *A fumigatus* strain Y1090 and Lambda gT11 as the cloning vector. Following amplification, DNA of the library was prepared using the Qiagen Lambda Midi Kit.

PCR were performed on an aliquote of the prepared DNA using standard reaction conditions. PCR used universal primers (Gt11f1, Gt11f3, gt11Rev) corresponding to regions of Lambda flanking the cDNA cloning sites and primers specific to each of the candidate genes.

PCR primers used herein are listed in Table 3 below:

**Table 3. Primer sequences**

| **Oligonucleotide** | **5'-3' sequence** | |
|---|---|---|
| 10.175.2 | GTTGGATCTTTGGGTTCTG | (SEQ ID No.72) |
| 10.304.4 | CGCGAATCTGATGACATAGC | (SEQ ID No.73) |
| 10.4.20.2 | CTCTTCGCTTCATCGTACCC | (SEQ ID No.74) |
| 11.4.9.4 | ATTAGTCCATGCGAGCATCC | (SEQ ID No.75) |
| 11.6.20.2 | GCCTGAGCCTAGTCCATCAC | (SEQ ID No.76) |
| 2.1.1.1 | CTCGCAGGTCGATTTCACTC | (SEQ ID No.77) |
| 2.1.1.2 | GGAGGAAACCTTGTCACCAC | (SEQ ID No.78) |
| 2.1.1.5 | TACCGAGAAGGAGGTCATGG | (SEQ ID No.79) |
| 2.1.1.6 | TCCAGTCAAGGTTGGTGATG | (SEQ ID No.80) |
| 2.1.1.9 | CGAGACCATCCTACCTCAG | (SEQ ID No.81) |
| 4.3.4.2 | ACACTCACCGCCTTAACCAC | (SEQ ID No.82) |
| 5.3.11.2 | AGTGCCCTTCATTCAGTTCC | (SEQ ID No.83) |
| 6.8.13.2 | GCGACTTTGAGGGAACTATCC | (SEQ ID No.84) |
| 7.5.9.3 | CACCACCCACCTTATGAAGC | (SEQ ID No.85) |
| 7.5.9.4 | ACCAGGAGAATCAGCGACAC | (SEQ ID No.86) |
| 8.62.2 | GGGACGAAGAATACGAGCTG | (SEQ ID No.87) |
| Gt11f1 | CTGAATATCGACGGTTTCC | (SEQ ID No.88) |
| Gt11f3 | GCACATTGGCTGAATATCG | (SEQ ID No.89) |
| Gt11Rev | TTGACACCAGACCAACTGGTAATG | (SEQ ID No.90) |

The oligonucleotides that were used in the different PCR reactions are listed in Table 4 below. PCR products were gel purified using standard procedures and subjected to DNA sequencing using sequencing oligonucleotides as indicated in Table 4 below. Sequencing was performed at GenomeExpress (Grenoble, France) and Sequentia (Clermont-Ferrand, France).

**Table 4. Oligonucleotides for PCR and sequencing**

| **Gene id** | **March 2002 ORF SEQ ID** | **March 2003 ORF SEQ ID** | **PCR** | **5' oligo.** | **3' oligo.** | **sequencing oligo.** |
|---|---|---|---|---|---|---|
| CEA229_genomic | 59 | 97 | PCR1 | Gtllf3 | 8.62.2 | 8.62.2 |
| | | | | | | |
| CEA232_genomic | 50 | 109 | PCR2 | Gt11f1 | 10.175.2 | 10.175 |
| CEA232_genomic | 50 | 109 | PCR3 | Gt11f1 | 10.175.2 | 10.175.2 |
| | | | | | | |
| CEA234_genomic | 47 | 117 | PCR4 | Gtllf3 | 10.304.4 | 10.304.4 |
| | | | | | | |
| CEA257_genomic | 17 | 133 | PCR5 | 2.1.1.1 | 2.1.1.2 | 2.1.1.2 |
| CEA257_genomic | 17 | 133 | PCR6 | 2.1.1.5 | 2.1.1.6 | 2.1.1.5 |
| CEA257_genomic | 17 | 133 | PCR7 | 2.1.1.9 | Gt11Rev | 2.1.1.9 |
| CEA257_genomic | 17 | 133 | PCR8 | Gtllf3 | 2.1.1.2 | 2.1.1.2 |
| CEA261_genomic | 29 | 149 | PCR9 | 7.5.9.3 | 7.5.9.4 | 7.5.9.3 |
| CEA261_genomic | 29 | 149 | PCR10 | 7.5.9.3 | Gt11Rev | 7.5.9.3 |
| CEA265_genomic | 53 | 165 | PCR11 | GT11f1 | 11.4.9.4 | 11.4.9.4 |
| CEA280_genomic | | 173 | PCR12 | Gt11f3 | 6.8.13.2 | 6.8.13.2 |
| | | | | | | |
| *CEA281.1_enomic*/*CEA281.2_genomic* | | *177 and 181* | *PCR13* | *Gt11f3* | *5.3.11.2* | *5.3.11.2* |
| | | | | | | |
| *CEA282_genomic* | | *185 and 189* | *PCR14* | *GT11f1* | *0.4.20.2* | *10.4.20.2* |
| *CEA282_genomic* | | *186 and 189* | *PCR15* | *GT11f3* | *10.4.20.2* | *10.4.20.2* |
| | | | | | | |
| *CEA283_genomic* | | | *PCR16* | *GT11f1* | *11.6.20.2* | *11.6.20.2* |
| | | | | | | |
| *CEA284.1_genomic*/*CEA284.2_genomic* | | *194 and 198* | *PCR17* | *Gt11f1* | *4.3.4.2* | *4.3.4.2* |

Using this approach, we could confirm that sequences of SEQ ID N° 95, 107, 115, 131, 147, 171 are expressed in *A. fumigatus* grown in standard culture medium. Furthermore, the following results could be obtained:
1. PCR1 located the polyadenylation site 189 bp 3' of the proposed stop codon in SEQ ID N°95;
2. PCR3 located the transcription start of SEQ ID N°107, 103 bp 5' of the proposed start codon and confirmed the location of the first intron;
3. PCR4 located the transcription start site of SEQ ID N°115, 113 bp upstream of the proposed start codon, identified an intron in the 5'-untranslated region from position -84 to position -12 relative to the proposed start codon and confirmed the proposed location of the first and second introns;
4. PCR5 and PCR8 confirmed the proposed location of the first intron in SEQ ID N°131;
5. PCR6 confirmed the proposed location of the second and third introns in SEQ ID N°131;
6. PCR 7 located the polyadenylation site 106 bp 3' of the proposed stop codon in SEQ ID N°131;
7. PCR9 and PCR10 confirmed the proposed location of the third intron in SEQ ID N° 147 and located two alternative polyadenylation sites 143 bp and 167 bp 3' of the proposed stop codon in SEQ ID N°147.

### Example 7: comparison of A. fumigatus EFG proteins with proteins of other fungal species.

EFG proteins of SEQ ID 106 to SEQ ID 174 were systematically compared to the genome of A. nidulans using the TBLASTN algorithm, to the genome and gene set of Magnaporthe grisea using the TBLASTN algorithm and to the protein set of Neurospora crassa and Saccharomyces cerevisiae using the BLASTP algorithm.

*A*. nidulans sequence data were obtained from the Aspergillus Sequencing project at the Whitehead Institute for Genome Research (http://wwwgenome.wi.mit.edu/annotation/fungi/aspergillus/index.html). The first release referred to as the Monsanto release was used.

M. grisea sequence data were obtained from the Magnaporthe Sequencing Project [Ralph Dean, Fungal Genomics Laboratory at North Carolina State University (www.fungalgenomics.ncsu.edu), and Whitehead Institute/MIT Center for Genome Research (www-genome.wi.mit.edu) ; http://wwwgenome.wi.mit.edu/annotation/fungi/magnaporthe/index.html]. Release 2.1 was used.

N. crassa sequence data were obtained from the Neurospora Sequencing Project [Whitehead Institute/MIT Center for Genome Research (www-genome.wi.mit.edu); http://www-genome.wi.mit.edu/annotation/fungi/neurospora/index.html] Release 3 was used. S. cerevisiae sequence data were obtained from the Saccharomyces Genome Database (http://genome-www.stanford.edu/Saccharomyces/).

A. nidulans, M. grisea, N. crassa and S. cerevisiae closest homologues of the A. fumigatus EFG proteins were subsequently compared to the genome of A. fumigatus using the TBLASTN algorithm and the data available from the A. fumigatus genome project (http://tigrblast.tigr.org/ufing/) in order to evaluate whether these proteins are orthologues of the A. fumigatus EFG proteins (BDBH = yes) or are only homologues of the A. fumigatus EFG proteins with an A. fumigatus orthologue that differs from the A. fumigatus EFG protein (BDBH= no).

Results presented in Table 5 below show that:
- all EFG proteins have a orthologue in the genome of A. nidulans
- two EFG proteins are specific to Aspergilli (SEQ ID 98 and SEQ ID 170)
- one EFG protein is specific to filamentous ascomycetes (SEQ ID 174)
- the remaining 18 EFG proteins have orthologues in all investigated species.

Consequently targets might be identified that are specific to Aspergilli, to filamentous ascomycetes or that have a broad spectrum. This analysis is reinforced by the comparison that was made to human proteins, whose results are shown in Table 6 below. As expected, SEQ ID 98 and SEQ ID 174 do not have a homologue encoded by the human genome thus defining targets for antifungal drugs that would show limited side effects.

**Table 5**

| | | | | *S. cerevisiae* closest homologue | Protein length (aa) | Probability (e value) | Similarity | BDBH | Essential | A. nidulans homologue | BDBH | M. grisea homologue | BDBH | N. crassa homologue | BDBH |
|---|---|---|---|---|---|---|---|---|---|---|---|---|---|---|---|
| Clone Id | Protein Id | March 2002 Amino acid SEQ ID | March 2003 Amino acid SEQ ID | (p<0.01) | | | | | in *S.c.* | (%Pos length of alignment region in Af protein) | | (e-value %Pos length of match) | | (e-value %Pos length of match) | |
| 10-80 | CEA 231_ prot | 3 | 106 | DBP10/YD L031w | 995 | e-166 | 55% on 949 aa | yes | yes | AN161C8821 (92% 347 #1-345); ANI61C3840 (69% 166 #772-934 + 83% 55 #723-777); ANI61S3472 (83% 129 #471-599+92% 14 #600-613); ANI61S468 (57% 132 #418-547) | yes | MG04179.1 (0.0 65% 943) | yes | NCU077 12.1 (0.0 69% 958) | yes |
| 10-291 | CEA 233_ prot | 6 | 114 | NAR1/YN L240c | 491 | 4E-59 | 48% on 465 aa | yes | yes | ANI61C10656 (74% 313 #1-273+77% 270 #307-574) | yes | CEA233_homol_ Mgrisea (not in Mg gene Db; contig 2.226 1621..71) | yes | NCU032 04.1 (e-159 62% 613) | yes |
| 7-1-19 | CEA 254_ | 9 | 122 | GUK1/YD R454c | 187 | 2E-62 | 81% on 182 aa | yes | yes | ANI61C9151 (75% 132 #2-110+91% 88 #111-198) | yes | MG06764.1 (6e-58 77% 183) | yes | NCU063 00.1 (le- | yes |
| 10-3-7 | prot CEA 255_ prot | 12 | 126 | SRP101/Y DR292c | 621 | e-102 | 62% on 431 aa | yes | yes | ANI61C10591 (77% 346 #168-513+86% 160 #502-658); AN161C6709 (80% 149 #1-129) | yes | MG02663.1 (0.0 67% 654) | yes | 61 78% 181) NCU006 25.1(e-174 67% 620) | yes |
| 2-6-4 | CEA 256_ prot | 15 | 130 | WBP1/YE L002c | 430 | 2E-37 | 46% on 432 aa | yes | yes | ANI61C5302(69% 520 #1-460) | yes | MG02821.1 (e-125 66% 465) | yes | NCU006 69.1 (e-128 64% 469) | yes |
| 2-1-1 | CEA 257_ prot | 18 | 134 | YGL245w | 724 | 0.0 | 67% on 622 aa | yes | yes | ANI61CI0340 (83% 624 #92-715); ANI61C6256 (82% 62 #1-62) | yes | MG05956.1 (0.0 69% 633) | yes | NCU088 94.1 (0.0 72% 631) | yes |
| 2-10- 16 | CEA 258_ prot | 21 | 138 | CDC27/YB L084c | 758 | 4E-71 | 63% on 304 aa | yes | yes | ANI61C8961 (84% 402 #390-786 + 55% 229 #171-399 + 63% 53 #119-171); ANI61C6854 (87% 114 #1-114) | yes | MG06292.1 (e-174 57% 821) | yes | NCU002 13.1 (e-173 57% 820) | yes |
| 5-4- 21 | CEA 259_ prot | 24 | 142 | RSC9/YM L127w | 581 | 5E-29 | 45% on 414 aa | yes | yes | ANI61C10567 (92% 382 #1-382); ANI61C1244(81% 144 #425-568) | yes | MG02493.1 (6e-61 51% 470) | yes | NCU038 92.1 (7e-66 52% | yes |
| 2-10- 21 | CEA 260_ prot | 27 | 146 | SPE2/YOL 052c | 396 | 1E-54 | 51% on 462 aa | yes | yes | ANI61 C9610 (82% 247 #39-273+80% 236 #264-491 + 94% 36 #6-41) | yes | MG10635.1 (e-143 67% 479) | yes | 478) NCU010 83.1 (e-163 71% | yes |
| 7-5-9 | CEA 261_ prot | 30 | 150 | RPL17A/Y KL180w RPL17B/Y JL177w | 136 | 5E-40 | 92% on 115 aa | yes | no | ANI61C1126 (96% 70 #50-119+77% 69 #8-72) | yes | MG041 14.1 (3e-57 96% 118) | yes | 502) NCU070 14.1 (3e- 58 86% 139) | yes |
| 10-2- 18 | CEA 262_ prot | 33 | 154 | RPL1A/Y GL135w RPL1B/YP L220w | 255 | 9E-86 | 86% on 238 aa | yes | no | ANI61C3974 (96% 170 #43-212 + 94% 50 #207-256) | yes | MG06919.1 (e-116 93% 238) | yes | NCU014 52.1 (e-114 91% 241) | yes |
| 9-11 | CEA 230_ prot | 36 | 102 | MSW1/YD R268w | 379 | 2E-26 | 43% on 154 aa | yes | no | ANI61C6741 (79% 150 #249-398+84% 93 #53-145 + 56% 140 #137-249) | yes | MG00474.1 (6e-85 64% 351) | yes | NCU001 13.1 (e-101 67% 374) | yes |
| 4-3-3 | CEA 263_prot | 39 | 158 | GOS1/YH L031c | 223 | 8E-31 | 60% on 224 aa | yes | no | ANI61C8624 (93% 172 #56-227+91% 38 #23-60) | yes | MG04454.1 (5e-71 76% 226) | yes | NCU027 06.1 (7e-74 78% | yes |
| 11-6-11 | CEA 264_ prot | 42 | 162 | RIM11/Y MR139w | 370 | e-104 | 77% on 323 aa | no | no | ANI61C8742 (74% 246 #16-217 + 87% 33 #215-247 + 93% 15 #1-15); ANI61C11836 (79% 108 #263-350+88% 44 #351-394) | yes | MG03972.1 (0.0 93% 394) | yes | 224) NCU041 85.1 (0.0 92% 394) | yes |
| 8-47 | CEA 228_ prot | 45 | 94 | YFL034w | 1074 | 5E-42 | 60% on 248 aa | no | no | ANI61C7512 (75% 306 #374-679); AN161C7189 (49% 225 #1-217) | yes | MG08873.1 (1e-69 73% 240) | yes | NCU016 72.1 (2e-89 50% | yes |
| 10-304 | CEA 234_ prot | 48 | 118 | RPL14A/Y HL001w RPL14B/Y KL006w | 138 | 1E-24 | 63% on 130 aa | yes | no | ANI61 C6709 (90% 90 #43-132 + 79% 55 #1-55) | yes | MG02659.1 (3e-40 70% 132) | yes | 631) NCU006 34.1 (5e 35 38% 132) | yes |
| 10-175 | CEA 232_ prot | 51 | 110 | HEM15/Y OR176w | 393 | e-112 | 71% on 352 aa | yes | yes | ANI61C8249 (94% 200 #79-278+56% 132 #1 -132) | yes | MG01513.1 (e-172 78% 420) | yes | NCU082 91.1 (e-175 79% | yes |
| 11-4- 9 | CEA 265_ prot | 54 | 166 | COX10/YP L172c | 462 | 1E-45 | 43% on 341 aa | yes | no | ANI61C1412 (50% 301 #20-320) | yes | MG05944.1 (2e-79 52% 351) | yes | 422) NCU061 41.1 (6e-81 51% 351) | yes |
| 2-10-18 | CEA 266_ prot | 57 | 170 | no hit found | | | | | | ANI61C1220 (57% 305 #233-537) | yes | MG00073.1 (9 e-06 39% 254) | no | NCU055 88.1 (2e- 07 37% 289) no hit found | no |
| 8-62 | CEA 229_ prot | 60 | 98 | no hit found | | | | | | ANI61 C3462 (72% 472 #237-707 + 68% 59 #710-768); ANI61C1809 (63% 179 #762-926); AN161C9531 (80% 97 #7-103) | yes | no hit found | ND | | ND |
| 6-8- 13 | CEA 280_ prot | | 174 | no hit found | | | | | | ANI61C5802 (68% 182 #15-190) | yes | MG04487.1 (6e-11 45% 175) | yes | NCU099 96.1 (1e- 1146% 175) | yes |
| *5-3- 11* | *CEA 281. 1_pr ot* | | *178* | *PAC2*/*YER 007w* | *518* | *8.4 e-14* | *50% fragmented* | *yes* | *no* | *ANI61C7709 (76% 206 #280-482 + 74% 146 #60-205 + 96% 53 #6-58)* | *yes* | *MG00378.1 (2e-95 51 % 641)* | *yes* | *NCU091 39.1 (2e- 98 50% 634)* | *yes* |
| *5-3-11* | *CEA 281. 2_pr ot* | | *182* | *no hit found* | | | | | | *ANI61C868 (72% 169 #47-211)* | *yes* | *MG07998.1 (2e-48 52% 300)* | *yes* | *NCU040 32.1 (5e- 42 48% 355)* | *yes* |
| *10-4-20* | *CEA 282. 1_pr ot* | | *186* | *PBP2*/*YBR 233w* | *413* | *2.1 e-26* | *50% fragmented* | *yes* | *no* | *ANI61C4164 (79%205 #1-200 + 82% 158 171-328); ANI61C6581 (55% 142 #323-464)* | *yes* | *MG00514.1 (e-125 60% 485)* | *yes* | *NCU092 37.1 (e- 131 60% 492)* | *yes* |
| *10-4- 20* | *CEA 282. 2_pr ot* | | *190* | *SEC3*/*YER 008c* | *1337* | *2.2 e-05* | *44% on 277 aa* | *no* | *yes* | *ANI61C11139 (67% 281 #64-344); ANI61C4164 (71% 60 #1-60)* | *yes* | *MG00515.1 (2e-41 50% 315)* | *yes* | *NCU092 38.1 (4e- 36 49% 334)* | *yes* |
| *4-3-4* | *CEA 284. 1_pr ot* | | *195* | *ENA5*/*YDR 038c* | *1091* | *2.5 e-260* | *65% fragmeted* | *yes* | *no* | *ANI61C1006 (88% 336 #429-760 + 86% 260 #167-426 + 78% 190 #1-169); ANI61C1748 (84% 212 #851-1059 + 95% 73 #781-853)* | *yes* | *MG10730.1 (0.0 72% 1072)* | *yes* | *NCU050 46.1 (0.0 74% 1038)* | *yes* |
| *4-3-4* | *CEA 284. 2_pr ot* | | *199* | *no hit found* | | | | | | *ANI61C8195 (39% 126 #232-350); ANI61C3069 (63% 56 #35-90)* | *yes* | *MG10932.1 (2e-08 40% 117)* | *no* | *NCU007 23.1 (3e- 07 37% 106)* | *no* |

**Table 6**

| Clone Id | Protein Id | March 2002 Amino acid SEQ ID | March 2003 Amino acid SEQ ID | Nearest human homologue at the integratuion of site *imp160::pyrG* | Probability | Protein Length (aa) | Identitity (%) | Similitary (%) | Human protein Ref |
|---|---|---|---|---|---|---|---|---|---|
| 10-80 | CEA231_ prot | 3 | 106 | ATP-dependent RNA helicase | e-123 | 882 | 261/594 (43%) | 353/594 (58%) | NP_07697 7.2 |
| 10- 291 | CEA233_ prot | 6 | 114 | protein related to Narf | 4E-59 | 476 | 168/474 (35%) | 216/474 (45%) | NP_07193 8.1 |
| 7-1- 19 | CEA254_ prot | 9 | 122 | guanylate kinase 1 | 5E-52 | 197 | 98/179 (54%) | 131/179 (72%) | NP_00084 9.1 |
| 10-3- 7 | CEA255_ prot | 12 | 126 | signal recognition particle receptor | 1E-88 | 638 | 232/668 (34%) | 334/668 (49%) | NP_00313 0.1 |
| 2-6-4 | CEA256_ prot | 15 | 130 | dolichyl-diphosphooligosaccharide-protein glycosyltransferase | 4E-45 | 456 | 124/423 (29%) | 213/423 (50%) | NP_00520 7.2 |
| 2-1-1 | CEA257_ prot | 18 | 134 | glutamyl-prolyl tRNA synthetase | e-135 | 1440 | %) 260/614 (42%) | 372/614 (60%) | NP_00443 7.1 |
| 2-10-16 | CEA258_ prot | 21 | 138 | cell division cycle protein 27 | 5E-81 | 824 | 168/465 (36%) | 257/465 (55%) | NP_00124 7.2 |
| 5-4-21 | CEA259_ prot | 24 | 142 | inc finger protein of the cerebellum 2 | 0.001 | 532 | 28/102 (27%) | 37/102 (35%) | NP_00906 0.2 |
| 2-10-21 | CEA260_ prot | 27 | 146 | S-adenosylmethionine decarboxylase 1 | 5E-50 | 334 | 140/447 (31 %) | 215/447 (47%) | NP_00162 5.1 |
| 7-5-9 | CEA26 _ prot | 30 | 150 | ribosomal protein S 17; 40S ribosomal protein | 1E-36 | 135 | 85/119 (71%) | 98/119 (81%) | NP _00101 2.1 |
| 10-2- 18 | CEA262_ prot | 33 | 154 | ribosomal protein S3a; 40S ribosomal protein | 1E-76 | 264 | 151/246 (61%) | 190/246 (76%) | NP _00099 7.1 |
| 9-11 | CEA230_ prot | 36 | 102 | tryptophanyl tRNA synthetase 2 (mitochondrial) | 7E-27 | 360 | 64/183 (34%) | 104/183 (55%) | NP_05665 1.1 |
| 4-3-3 | CEA263_ prot | 39 | 158 | golgi SNAP receptor complex member 1; Golgi SNARE | 6E-21 | 250 | 67/247(27%) | 125/247 (50%) | NP_00486 2.1 |
| 11-6- 11 | CEA264_ prot | 42 | 162 | glycogen synthase kinase 3 beta | e-137 | 433 | 241/352 (68%) | 281/352 (79%) | NP_00208 4.2 |
| 8-47 | CEA228_ prot | 45 | 94 | hypothetical protein | 6E-28 | 421 | 69/188 (36%) | 100/188 (52%) | CAB39107 .1 |
| 8-62 | CEA229_ prot | 60 | 98 | no hit found | | | | | |
| 6-8- 13 | CEA280_ prot | | 174 | no hit found | | | | | |
| *5-3- 11* | *CEA281. 1-prot* | | *178* | *beta-tubulin cofactor E* | *5E-29* | *527* | *75*/*199 (37%) 55*/*211 (26%)* | *108*/*199 (54%) 91*/*211 (43%)* | *NP_00318 4.1* |
| *5-3- 11* | *CEA281. 2_prot* | | *182* | *WW domain-contaihing binding protein 4; formin binding protein 21* | *0. 020* | *376* | *22*/*64 (34%)* | *36*/*64 (56%)* | *NP_00911 8.1* |
| *10-4- 20* | *CEA282. 1_prot _prot* | | *186* | *poly(rC) binding protein 1; heterogenous nuclear ribonucleoprotein X* | *1E-14* | *356* | *59*/*194 (30%)* | *96*/*194 (49%)* | *NP_00618 7.1* |
| *10-4- 20* | *CEA282. 2_prot* | | *190* | *no hit found* | | | | | |
| 4*-3-4* | *CEA284. 2 prot* | | *199* | *no hit found* | | | | | |

### REFERENCES

1. Latgé, J. P. Aspergillus fumigatus and aspergillosis. Clin Microbiol Rev12, 310-350 (1999).
2. Latgé, J. The pathobiology of Aspergillus fumigatus. Trends Microbiol 9, 382-389 (2001).
3. Lin, S., Schranz, J. & Teutsch, S. Aspergillosis case-fatality rate: systematic review of the literature. Clin Infect Dis 32, 358-366. (2001).
4. McNeil, M. M. *et al.* Trends in mortality due to invasive mycotic diseases in the United States, 1980-1997. Clin Infect Dis33, 641-647. (2001).
5. Georgopapadakou, N. H. Antifungals: mechanism of action and resistance, established and novel drugs. Curr Opin Microbiol1,547-557 (1998).
6. Tkacz, J. S. & DiDomenico, B. Antifungals: what's in the pipeline. Curr Opin Microbiol4, 540-545. (2001).
7. Walsh, T. J. et al. New targets and delivery systems for antifungal therapy. Med Mycol38,335-347. (2000).
8. Groll, A. H., De Lucca, A. J. & Walsh, T. J. Emerging targets for the development of novel antifungal therapeutics. Trends Microbiol 6, 117-124 (1998).
9. Perfect, J. R. Fungal virulence genes as targets for antifungal chemotherapy. Antimicrob Agents Chemother40, 1577-1583 (1996).
10. Brown, J. S. et al. Signature-tagged and directed mutagenesis identify PABA synthetase as essential for Aspergillus fumigatus pathogenicity. Mol Microbiol 36, 1371-1380. (2000).
11. Reich, K. A. The search for essential genes. Res Microbiol151, 319-324 (2000).
12. Odds, F. C., Gow, N. A. & Brown, A. J. Fungal virulence studies come of age. Genome Biol 2 (2001).
13. Winzeler, E. A. et al. Functional characterization of the S. cerevisiae genome by gene deletion and parallel analysis. Science285, 901-906 (1999).
14. Vidan, S. & Snyder, M. Large-scale mutagenesis: yeast genetics in the genome era. Curr Opin Biotechnol 12,28-34. (2001).
15. De Backer, M. D. et al. An antisense-based functional genomics approach for identification of genes critical for growth of Candida albicans. Nat Biotechnol19, 235-241. (2001).
16. Brookman, J. L. & Denning, D. W. Molecular genetics in Aspergillus fumigatus. Curr Opin Microbiol3, 468-474. (2000).
17. Timberlake, W. E. in More Gene Manipulations in Fungi(eds. Bennett, J. W. & Lasure, L. L.) 51-85 (Academic Press Inc., Oxford, 1991).
18. Clutterbuck, A. J. Sexual and parasexual genetics of Aspergillus species. Biotechnology 23, 3-18 (1992).
19. Brown, J. S., Aufauvre-Brown, A. & Holden, D. W. Insertional mutagenesis of Aspergillus fumigatus. Mol Gen Genet 259, 327-335 (1998).
20. d'Enfert, C., Weidner, G., Mol, P. C. & Brakhage, A. A. Transformation systems of Aspergillus fumigatus. New tools to investigate fungal virulence. Contrib Microbiol2, 149-166 (1999).
21. Judson, N. & Mekalanos, J. J. Transposon-based approaches to identify essential bacterial genes. Trends Microbiol 8, 521-526. (2000).
22. Judson, N. & Mekalanos, J. J. TnAraOut, a transposon-based approach to identify and characterize essential bacterial genes. Nat Biotechnol18, 740-745. (2000).
23. Kumar, A. & Snyder, M. Emerging technologies in yeast genomics. Nat Rev Genet2, 302-312. (2001).
24. Ross-Macdonald, P. et al. Large-scale analysis of the yeast genome by transposon tagging and gene disruption. Nature402, 413-418 (1999).
25. Hamer, L. et al. Gene discovery and gene function assignment in filamentous fungi. Proc Natl Acad Sci U S A98, 5110-5115. (2001).
26. Brown, J. S. & Holden, D. W. Insertional mutagenesis of pathogenic fungi. Curr Opin Microbial 1,390-394 (1998).
27. Langin, T., Capy, P. & Daboussi, M. J. The transposable element impala, a fungal member of the Tc1-mariner superfamily. Mol Gen Genet 246, 19-28 (1995).
28. Hua-Van, A., Pamphile, J. A., Langin, T. & Daboussi, M. J. Transposition of autonomous and engineered impala transposons in Fusarium oxysporum and a related species. Mol Gen Genet 264, 724-731. (2001).
29. Li Destri Nicosia, M. G. et al. Heterologous transposition in Aspergillus nidulans. Mol Microbiol39, 1330-1344. (2001).
30. Villalba, F., Lebrun, M. H., Hua-Van, A., Daboussi, M. J. & Grosjean-Cournoyer, M. C. Transposon impala, a novel tool for gene tagging in the rice blast fungus Magnaporthe grisea. Mol Plant Microbe Interact 14, 308-315. (2001).
31. Hastie, A. C. Benlate-induced Instability of Aspergillus diploids. Nature226, 771(1970).
32. Altschul, S. F. et al. Gapped BLAST and PSI-BLAST: a new generation of protein database search programs. Nucleic Acids Res25, 3389-3402. (1997).
33. Akerley, B. J. et al. A genome-scale analysis for identification of genes required for growth or survival of Haemophilusinfluenzae. Proc Natl Acad Sci U S A 99, 966-971. (2002).
34. Craig, N. L. Target site selection in transposition. Annu Rev Biochem66, 437-474(1997).
35. Giaever, G. et al. Genomic profiling of drug sensitivities via induced haploinsufficiency. Nat Genet21, 278-283 (1999).
36. Chun, K. T., Edenberg, H. J., Kelley, M. R. & Goebl, M. G. Rapid amplification of uncharacterized transposon-tagged DNA sequences from genomic DNA. Yeast 13, 233-240 (1997).
37. Bouchara, J.P. et al. The search for virulence determinants in Aspergillus fumigatus; Trends Microbiol. 3(8), 327-330 (1995).
38. Langfelder, K. et al. Identification of a polyketide Synthase Genes (pKsP) of Aspergillus fumigatus involved in conidial pigment biosynthesis and virulence. Med Microbiol Immunol (Berl.) 187(2), 79-89 (1998).
39. Tsai, H.F. et al. A developmentally regulated gene cluster involved in conidial pigment biosynthesis in Aspergillus fumigatus. J. Bacteriol 181 (20), 6469-6477(1999).

## Claims

1. A nucleic acid encoding an Essential For Growth (EFG) polypeptide selected from the group consisting of:
(i) a nucleic acid molecule encoding a polypeptide comprising the amino acid sequence depicted in one of SEQ ID N°9 and 122;
(ii) a nucleic acid molecule comprising the nucleic acid sequence as depicted in one of SEQ ID N° 7 and 120;
(iii) a nucleic sequence having at least 80, 85, 90, 95, 98, 99% identity with a sequence of SEQ ID N° 7 or 120;
(iv) a nucleic acid molecule which hybridizes under stringent conditions to:
(a) a nucleic acid as defined in (i), (ii) and (iii), or
(b) a complementary strand of (a);
(v) a nucleic acid the sequence of which is degenerate as a result of the genetic code to the sequence of a nucleic acid as defined in (i), (ii), (iii) and (iv).

2. An isolated nucleic acid comprising a nucleotide sequence encoding:
i) an Essential For Growth (EFG) polypeptide comprising an amino acid sequence having at least 80% identity to a sequence of SEQ ID N° 9 or 122; or
ii) a biologically active fragment of said polypeptide.

3. An isolated nucleic acid, said nucleic acid comprising a nucleotide sequence encoding:
i) an Essential For Growth (EFG) polypeptide comprising an amino acid sequence which is orthologous to a sequence of SEQ ID N° 9 or 122; or
ii) a biologically active fragment of said polypeptide.

4. A nucleic acid sequence of any of claims 1 to 3 encoding a polypeptide of A. *fumigatus* exhibiting a biological function associated to fungal growth, said nucleic acid comprising a sequence of SEQ ID N° 8 or 121.

5. A nucleic acid sequence of claim 4, wherein said biological function associated a fungal growth is nucleotide metabolism.

6. The nucleic acid of claim 1, wherein said nucleic acid is operably linked to a promoter.

7. An expression cassette comprising the nucleic acid of claim 6.

8. A host cell comprising the expression cassette of claim 7.

9. A biologically active polypeptide encoded by a nucleic acid according to any of claims 1 to 5.

10. A polypeptide according to claim 9 or a biologically active fragment thereof, said polypeptide comprising an amino acid sequence of at least 80% amino acid sequence identity to a sequence of SEQ ID N° 9 or 122.

11. A method of identifying a candidate inhibitor of an Essential For Growth (EFG) polypeptide, said method comprising:
a) contacting an EFG polypeptide according to claim 9 or 10 with a test compound;
b) determining whether said compound selectively binds to said polypeptide, said binding indicated that said compound is a candidate inhibitor.

12. A method for detecting the presence of a nucleic acid comprising a nucleotide sequence of SEQ ID N° 7 or 120, a fragment or a variant thereof and a complementary sequence thereto in a sample, said method comprising the following steps of:
a) bringing into contact a nuclic acid probe or a plurality of nucleic acid probes which can hybridize with a nucleotide sequence included in a nucleic acid sequence of SEQ ID N° 7 or 120, a fragment or a variant thereof and a complementary sequence thereto and the sample to be assayed; and
b) detecting the hybrid complex formed between the probe and a nucleic acid in the sample.

13. A composition capable of inhibiting haploid fungal growth, wherein said composition comprises at least one compound capable of inhibiting the expression of at least one Essential For Growth (EFG) gene as defined in any of claims 1 to 5.

14. The composition of claim 13, which is a pharmaceutical composition.

15. The composition of claim 13 or 14, which is a fungicidal composition.
